# EUROPEAN PATENT APPLICATION

(11) **EP 1 832 588 A1**
(43) Date of publication of application: **12.09.2007**
(21) Application number: 05824476.5
(22) Date of filing: 28.12.2005
(51) Int. Cl.: C07D 487/04, A61K 31/5025, A61P 9/00, A61P 19/00, A61P 19/02, A61P 25/00, A61P 29/00, A61P 31/00, A61P 35/00, A61P 37/00, A61P 43/00

(54) **CONDENSED IMIDAZOLE COMPOUND AND USE THEREOF**

(30) Priority: 28.12.2004 JP 2004381947
(71) Applicant: Takeda Pharmaceutical Company Limited, Osaka-shi, Osaka 541-0045 (JP)
(72) Inventor: UCHIKAWA, Osamu Takeda Pharmaceutical Co. Ltd., Osaka-shi, Osaka 5328686 (JP); MIWATASHI, Seiji Takeda Pharmaceutical Co. Ltd., Osaka-shi, Osaka 5328686 (JP)
(74) Representative: Weber, Thomas
(86) International application number: PCT/JP2005/024279
(87) International publication number: WO 2006/070943

(57) **Abstract**

The present invention relates to a compound represented by the formula [I] 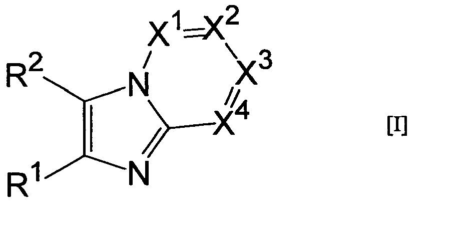 wherein X¹, X² and X³ are each an optionally substituted CH or a nitrogen atom, and any one of X¹, X² and X³ is a nitrogen atom, X⁴ is an optionally substituted CH, R¹ is an optionally substituted phenyl group or an optionally substituted heterocyclic group, and R² is an optionally substituted pyridin-4-yl group, an optionally substituted pyridine-N-oxide-4-yl group or an optionally substituted pyrimidin-4-yl group, or a salt thereof. The compound has superior p38 MAP kinase inhibitory activity and MMP-13 production inhibitory activity, and is useful as an agent for the prophylaxis or treatment and the like of an inflammatory disease, an autoimmune disease, a debilitating disease, an osteoarticular degenerative disease, a neurodegenerative disease, a vascular disease, a neoplastic disease or an infectious disease.

## Description

### Technical Field

The present invention relates to a novel condensed bicyclic imidazole compound having superior pharmaceutical actions such as a p38 MAP kinase inhibitory activity, a TNF-α production inhibitory activity, an MMP-13 production inhibitory activity and the like, particularly, a superior p38 MAP kinase inhibitory activity and an MMP-13 production inhibitory activity, a production method thereof, a pharmaceutical agent containing the compound and the like.

### Background Art

Cytokines such as TNF-α (tumor necrosis factor-α), IL-1 (interleukin-1) and the like are biological substances, which are produced by a variety of cells such as monocyte or macrophage in response to infection and other cellular stress (Koj, A., Biochim. Biophys. Acta, 1317, 84-94 (1996)). Although these cytokines play important roles in the immune response when they are present at an appropriate amount, it is thought that their overproduction is associated with a variety of inflammatory diseases (Dinarello, C.A., Curr. Opin. Immunol., 3, 941-948 (1991)).

p38 MAP kinase which was cloned as a homologue of MAP (Mitogen Activated Protein) kinase (hereinafter abbreviated as MAPK) is involved in the control of production of these cytokines and signal transduction system coupled with receptors, and there is a possibility that the inhibition of p38 MAP kinase provides a drug for treating inflammatory diseases (Stein, B., Anderson, D., Annual Report in Medicinal Chemistry, edited by Bristol, J.A., Academic Press, vol.31, pages 289-298, 1996).

In addition, MMP (matrix metalloprotease)-13 is a selective hydrolase of type II collagen, which is a major constituent component of cartilage tissue, and the production thereof is regulated by inflammatory cytokines, IL-1β and TNF-α.

Moreover, it is known that production of MMP-13 is promoted in the cartilage of patients suffering from osteoarthritis or rheumatoid arthritis. Thus, MMP-13 is considered to be particularly deeply involved in these pathologies. (Tardif, G., Roboul, P., Pelletier, J.-P., Martel-Pelletier, J., Mod. Rheumatol., 14, 197-204 (2004))

Heretofore, as a compound having a p38 MAP kinase-inhibitory activity, imidazole derivatives are described in JP-A-7-50317 (WO 93/14081), and oxazole derivatives are described in JP-A-9-505055 (WO 95/13067). In addition, imidazo[1,2-a]pyrimidine compounds are described in JP-A-2003-513977 (WO 2001/34605). As a compound having a TNF-α production inhibitory activity, bicyclic imidazole derivatives are described in JP-A-4-506215 (WO 90/15534) and JP-A-5-503919 (WO 91/00092).

As a compound having IL-1-induced MMP-13 production inhibitory activity, ERK inhibitors such as PD98059 and U0126, p38 MAP kinase inhibitors such as SB203580 and SB202190, JNK inhibitors such as SB203580, SP600125 and curcumin, curcumin which is an inhibitor of AP-1 and NF-κB, Bay-11-7085 and the like have been reported. (Liacini, A., Sylvester, J., Li, W.Q., Zafarullah, M., Matrix Biol., 21, 251-262 (2002) and Liacini, A., Sylvester, J., Li, W.Q., Huang, W., Dehnade, F., Ahmad, M., Zafarullaha, M., Exp. Cell Res., 288, 208-217 (2003))

In addition, as condensed bicyclic imidazole compounds, the following compounds and the like are known.
1) JP-A-2002-234875 describes an imidazole derivative represented by 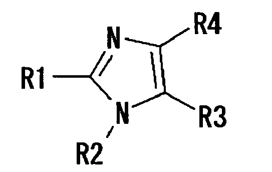 wherein R1 and R2 are each independently a hydrogen atom, an alkyl group or an alkenyl group, or in combination optionally form a ring together with a carbon atom and a nitrogen atom bonded thereto, R3 and R4 are each independently a hydrogen atom, an alkyl group, a cycloalkyl group, an alkenyl group, an alkynyl group, an aryl group or an aralkyl group, provided that when R3 is a phenyl group, R1 is not an isopropyl group and when R3 is a methyl group, R4 is not a hydrogen atom, or a salt thereof.
2) US Patent No. 6451520 describes an imidazole compound represented by 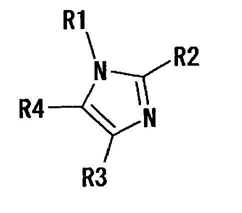 wherein R1 is an alkyl group, an aryl group, an aralkyl group or a heteroaryl group, R2 is a hydrogen atom or R1, or R1 and R2 in combination represent a hetero ring, R3 and R4 are each independently an aryl group or a heteroaryl group, or in combination represent a phenanthrene group wherein the 9th and 10th positions are condensed, and when R3 and R4 form a phenanthrene ring, R1 may be a hydrogen atom.

However, no compound has heretofore been found which is satisfactory in the action effect, safety, (oral) absorbability, (metabolism) stability and the like as a p38 MAP kinase inhibitor, TNF-α production inhibitor or MMP-13 production inhibitor. Accordingly, there is a demand for the development of a superior p38 MAP kinase inhibitor, TNF-α production inhibitor or MMP-13 production inhibitor as a pharmaceutical agent effective for the prophylaxis or treatment of cytokine-related diseases and the like.

### Disclosure of the Invention

The present inventors have conducted various studies and first synthesized a compound represented by the formula [I] 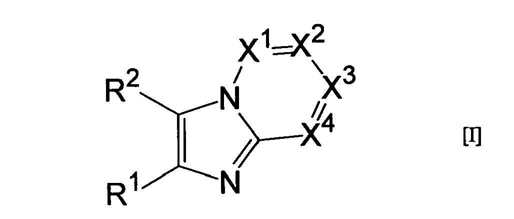 wherein
x¹, X² and X³ are each an optionally substituted CH or a nitrogen atom, and any one of X¹, X² and X³ is a nitrogen atom,
X⁴ is an optionally substituted CH,
R¹ is an optionally substituted phenyl group or an optionally substituted heterocyclic group, and
R² is an optionally substituted pyridin-4-yl group, an optionally substituted pyridine-N-oxide-4-yl group or an optionally substituted pyrimidin-4-yl group,
or a salt thereof [hereinafter sometimes to be abbreviated as compound (I)], and found that the obtained compound (I) unexpectedly has a superior pharmaceutical action based on its specific chemical structure, such as p38 MAP kinase inhibitory activity, TNF-α production inhibitory activity, MMP-13 production inhibitory activity and the like, particularly, superior p38 MAP kinase inhibitory activity and MMP-13 production inhibitory activity.

Moreover, the present inventors have found that the pharmaceutical agent of the present invention, which comprises compound (I) or a compound represented by the formula [I'] 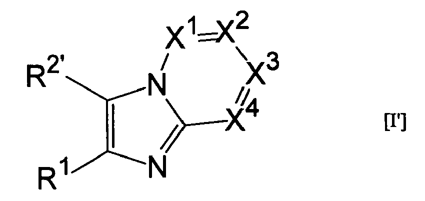 wherein
R^{2'} is an optionally substituted phenyl group or an optionally substituted heterocyclic group, and the other symbols are each as defined above [hereinafter sometimes to be abbreviated as compound (I')], not only has a superior pharmaceutical action such as p38 MAP kinase inhibitory activity, TNF-α production inhibitory activity, MMP-13 production inhibitory activity and the like, particularly, superior p38 MAP kinase inhibitory activity and MMP-13 production inhibitory activity, but also superior properties as a pharmaceutical product such as stability, lower toxicity and the like, and is sufficiently satisfactory as a pharmaceutical agent.

Based on these findings, the present inventors have completed the present invention.

Accordingly, the present invention relates to
[1] a compound represented by formula [I] 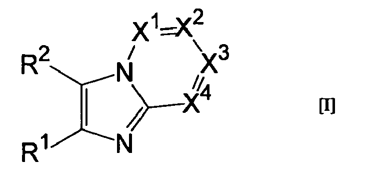 wherein
   X¹, X² and X³ are each an optionally substituted CH or a nitrogen atom, and any one of X¹, X² and X³ is a nitrogen atom,
   X⁴ is an optionally substituted CH,
   R¹ is an optionally substituted phenyl group or an optionally substituted heterocyclic group, and
   R² is an optionally substituted pyridin-4-yl group, an optionally substituted pyridine-N-oxide-4-yl group or an optionally substituted pyrimidin-4-yl group,
   or a salt thereof;
[2] the compound of the above-mentioned [1], wherein
   X¹, X² and X³ are each an optionally substituted CH or a nitrogen atom, and any one of X¹, X² and X³ is a nitrogen atom,
   X⁴ is CH,
   R¹ is an optionally substituted phenyl group or an optionally substituted heterocyclic group, and
   R² is an optionally substituted pyridin-4-yl group or an optionally substituted pyrimidin-4-yl group;
[3] the compound of the above-mentioned [1], wherein R¹ is an optionally substituted phenyl group;
[4] the compound of the above-mentioned [1], wherein
   X¹ is a nitrogen atom;
   X² is CH optionally substituted by (a) halogen atom, (b) C₁₋₆ alkylthio group, (c) C₁₋₆ alkylsulfonyl group, (d) C₁₋₆ alkoxy group, or (e) a group represented by the formula 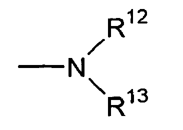
   (I) a pyridin-4-yl group substituted by substituent(s) selected from halogen atom, C₁₋₆ alkoxy group and C₁₋₆ alkyl group,
   (II) a pyridin-4-yl group substituted by substituent(s) represented by the formula 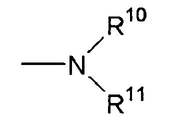 wherein
      R¹⁰ and R¹¹ are each
      (a) a hydrogen atom,
      (b) a C₁₋₆ alkyl group optionally substituted by group(s) selected from
         (i) C₁₋₆ alkoxy group,
         (ii) amino group optionally having 1 or 2 C₁₋₆ alkyl groups,
         (iii) hydroxy group,
         (iv) C₃₋₁₀ cycloalkyl group,
         (v) 5- to 10-membered heterocyclic group, and
         (vi) 5- to 7-membered saturated cyclic amino group,
      (c) a C₇₋₁₆ aralkyl group,
      (d) a C₃₋₁₀ cycloalkyl group optionally condensed with C₆₋₁₄ aryl ring,
      (e) a C₆₋₁₄ aryl group optionally substituted by substituent(s) selected from halogen atom, C₁₋₆ alkyl group and C₁₋₆ alkoxy group, or
      (f) the formula - (C=O) -R⁵, - (C=O) -NR⁶R^{6'} or -SO₂-R⁷
         wherein
         R⁵ is
         (i) a C₁₋₆ alkyl group optionally substituted by substituent(s) selected from C₁₋₆ alkyl-carbonyloxy group, hydroxy group, halogen atom, C₁₋₆ alkylthio group and C₁₋₆ alkylsulfonyl group;
         (ii) a C₃₋₁₀ cycloalkyl group;
         (iii) a C₆₋₁₄ aryl group optionally substituted by substituent(s) selected from C₁₋₆ alkoxy group optionally substituted by halogen atom, C₁₋₆ alkyl group optionally substituted by halogen atom, halogen atom, C₁₋₆ alkylthio group, C₁₋₆ alkylsulfonyl group and di-C₁₋₆ alkylamino group;
         (iv) a C₇₋₁₆ aralkyl group optionally substituted by C₁₋₆ alkoxy group;
         (v) a C₁₋₆ alkoxy group optionally substituted by halogen atom; or
         (vi) a 5- to 10-membered heterocyclic group optionally substituted by substituent(s) selected from halogen atom and C₁₋₆ alkyl group optionally substituted by halogen atom,
         R⁶ is
         (i) a hydrogen atom;
         (ii) a C₆₋₁₄ aryl group optionally substituted by substituent(s) selected from halogen atom, C₁₋₆ alkyl group, C₁₋₆ alkoxy group, C₁₋₆ alkyl-carbonyl group, C₁₋₆ alkylthio group and cyano group;
         (iii) a C₁₋₆ alkyl group optionally substituted by substituent(s) selected from hydroxy group, C₁₋₆ alkylsulfonyl group, oxo group, cyano group and halogen atom;
         (iv) a C₃₋₁₀ cycloalkyl group optionally substituted by hydroxy group; or
         (v) a 5- to 10-membered heterocyclic group optionally substituted by C₁₋₆ alkyl group;
         R^{6'} is a hydrogen atom or a C₁₋₆ alkyl group; or
         R⁶ and R^{6'} form, together with the nitrogen atom they are bonded to, a 5- to 7-membered saturated cyclic amino group optionally condensed with C₆₋₁₄ aryl ring optionally substituted by hydroxy group; and
         R⁷ is a C₆₋₁₄ aryl group; or
         R¹⁰ and R¹¹ form, together with the nitrogen atom they are bonded to, a 5- to 7-membered saturated cyclic amino group optionally substituted by oxo group,
   (III) a pyridine-N-oxide-4-yl group substituted by C₆₋₁₄ aryl-carbonylamino group optionally substituted by C₁₋₆ alkylsulfonyl group, or
   (IV) a pyrimidin-4-yl group substituted by substituent(s) selected from (1) amino group, (2) C₆₋₁₄ aryl-carbonylamino group wherein the aryl moiety is optionally substituted by C₁₋₆ alkyl group or C₁₋₆ alkoxy group, and (3) 5- to 10-membered heterocyclic group-carbonylamino group;
[5] 4-methyl-N-{4-[2-(3-methylphenyl)imidazo[1,2-b]pyridazin-3-yl]pyridin-2-yl}benzamide,
   N-{4-[2-(3-methylphenyl)imidazo[1,2-b]pyridazin-3-yl]pyridin-2-yl}cyclopropanecarboxamide,
   4-fluoro-N-{4-[2-(3-methylphenyl)imidazo[1,2-b]pyridazin-3-yl]pyridin-2-yl}benzamide,
   N-{4-[2-(4-fluoro-3-methylphenyl)imidazo[1,2-b]pyridazin-3-yl]pyridin-2-yl}-4-methoxybenzamide,
   N-ethyl-N'-{4-[2-(4-fluoro-3-methylphenyl)imidazo[1,2-b]pyridazin-3-yl]pyridin-2-yl}urea,
   4-fluoro-N-{4-[2-(4-fluoro-3-methylphenyl)imidazo[1,2-b]pyridazin-3-yl]pyridin-2-yl}benzamide,
   N-{4-[2-(4-fluoro-3-methylphenyl)imidazo[1,2-b]pyridazin-3-yl]pyridin-2-yl}cyclopropanecarboxamide,
   N-{4-[2-(4-fluoro-3-methylphenyl)imidazo[1,2-b]pyridazin-3-yl]pyridin-2-yl}-2-(methylthio)acetamide,
   N-{4-[2-(4-fluoro-3-methylphenyl)imidazo[1,2-b]pyridazin-3-yl]pyridin-2-yl}isonicotinamide,
   N-{4-[2-(4-fluoro-3-methylphenyl)imidazo[1,2-b]pyridazin-3-yl]pyridin-2-yl}-6-methylnicotinamide,
   N-{4-[2-(4-fluoro-3-methylphenyl)imidazo[1,2-b]pyridazin-3-yl]pyridin-2-yl}-1,3-benzothiazole-6-carboxamide,
   N-{4-[2-(4-fluoro-3-methylphenyl)imidazo[1,2-b]pyridazin-3-yl]pyridin-2-yl}-6-quinolinecarboxamide,
   N-{4-[2-(4-fluoro-3-methylphenyl)imidazo[1,2-b]pyridazin-3-yl]pyridin-2-yl}-N'-(2-hydroxyethyl)urea,
   N-(2-cyanoethyl)-N'-{4-[2-(4-fluoro-3-methylphenyl)imidazo[1,2-b]pyridazin-3-yl]pyridin-2-yl}urea,
   N-{4-[2-(4-fluoro-3-methylphenyl)imidazo[1,2-b]pyridazin-3-yl]pyridin-2-yl}-2-methylisonicotinamide,
   N-{4-[2-(4-fluoro-3-methylphenyl)imidazo[1,2-b]pyridazin-3-yl]pyridin-2-yl}nicotinamide
   or a salt thereof ;
[6] a prodrug of the compound of the above-mentioned [1];
[7] a pharmaceutical agent comprising a compound represented by formula [I'] 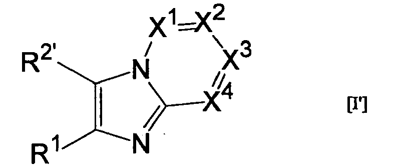 wherein
   R²' is an optionally substituted phenyl group or an optionally substituted heterocyclic group, and the other symbols are each as defined the above-mentioned [1],
   or a salt thereof or a prodrug thereof;
[8] the pharmaceutical agent of the above-mentioned [7], which is a p38 MAPK inhibitor and/or MMP-13 production inhibitor;
[9] the pharmaceutical agent of the above-mentioned [7], which is an agent for the prophylaxis or treatment of an inflammatory disease, an autoimmune disease, a debilitating disease, an osteoarticular degenerative disease, a neurodegenerative disease, a vascular disease, a neoplastic disease or an infectious disease;
[10] a method for the prophylaxis or treatment of an inflammatory disease, an autoimmune disease, a debilitating disease, an osteoarticular degenerative disease, a neurodegenerative disease, a vascular disease, a neoplastic disease or an infectious disease, which comprises administering an effective amount of a compound represented by formula [I'] 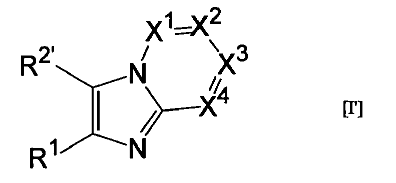 wherein
   R^{2'} is an optionally substituted phenyl group or an optionally substituted heterocyclic group, and the other symbols are each as defined the above-mentioned [1],
   or a salt thereof or a prodrug thereof to a mammal;
[11] use of a compound represented by formula [I'] 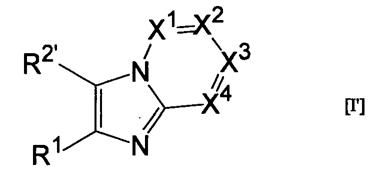 wherein
   R²' is an optionally substituted phenyl group or an optionally substituted heterocyclic group, and the other symbols are each as defined the above-mentioned [1],
   or a salt thereof or a prodrug thereof, for the production of an agent for the prophylaxis or treatment of an inflammatory disease, an autoimmune disease, a debilitating disease, an osteoarticular degenerative disease, a neurodegenerative disease, a vascular disease, a neoplastic disease or an infectious disease;
[12] a production method of the compound of the above-mentioned [1] or a salt thereof, which comprises condensing a compound represented by the formula [III] 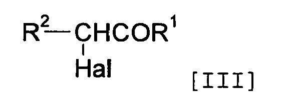
wherein
Hal is a halogen atom,
R¹ is an optionally substituted phenyl group or an optionally substituted heterocyclic group,
R² is an optionally substituted pyridin-4-yl group, an optionally substituted pyridine-N-oxide-4-yl group, or an optionally substituted pyrimidin-4-yl group,
or a salt thereof with a compound represented by formula [IV] 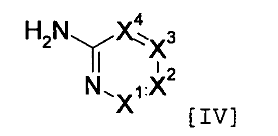 wherein
X¹, X² and X³ are each an optionally substituted CH or a nitrogen atom, and any one of X¹, X² and X³ is a nitrogen atom, and
X⁴ is an optionally substituted CH,
or a salt thereof, and, where desired, performing deprotection, acylation reaction, alkylation reaction, hydrogenation reaction, oxidation reaction, reduction reaction, carbon chain extension reaction and/or substituent exchange reaction.

### Best Mode for Embodying the Invention

### (Explanation of R¹)

In the aforementioned formula [I] and [I'], R¹ is an optionally substituted phenyl group or an optionally substituted heterocyclic group.

As the "heterocyclic group" of the "heterocyclic group optionally having substituent(s)", for example, a monovalent group obtained by removing optional one hydrogen atom from a 5- to 14-membered (monocyclic, bicyclic or tricyclic) heterocycle containing, besides carbon atom(s), 1 or 2 kinds of 1 to 4 hetero atoms selected from nitrogen atom, sulfur atom and oxygen atom, preferably (i) a 5- to 14-membered (preferably 5- to 10-membered, particularly preferably 5- or 6-membered) aromatic heterocycle, (ii) a 5- to 10-membered (preferably 5- to 7-membered) non-aromatic heterocycle or (iii) a 7- to 10-membered bridged heterocycle, and the like can be mentioned.

As the aforementioned "(i) 5- to 14-membered (preferably 5- to 10-membered) aromatic heterocycle", for example, an aromatic heterocycle such as thiophene, benzo[b]thiophene, benzo[b]furan, benzimidazole, benzoxazole, benzothiazole, benzisothiazole, naphtho[2,3-b]thiophene, furan, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isoxazole, furazan, phenoxazine and the like, or a ring formed by condensation of these rings (preferably monocycle) with one or plural (preferably 1 or 2) aromatic rings (e.g., benzene ring, etc.) and the like can be mentioned.

As the aforementioned "(ii) 5- to 10-membered (preferably 5- to 7-membered) non-aromatic heterocycle", for example, pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole, tetrahydrofuran, 3,4-dihydroquinoline, 1,2,3,4-tetrahydroquinoline, 3,4-dihydroisoquinoline, 1,2,3,4-tetrahydroisoquinoline and the like can be mentioned. Of these, a 5- to 7-membered saturated cyclic amino containing one nitrogen atom and optionally containing, besides carbon atoms, one or two kinds of 1 to 4 hetero atoms selected from a nitrogen atom, a sulfur atom and an oxygen atom is preferable, and as specific examples, pyrrolidin-1-yl, piperidino, piperazin-1-yl, morpholino, thiomorpholino, hexahydroazepin-1-yl and the like can be mentioned.

As the aforementioned "(iii) 7- to 10-membered bridged heterocycle", for example, quinuclidine, 7-azabicyclo[2.2.1]heptane and the like can be mentioned.

The preferable "heterocyclic group" is a 5- to 14-membered (preferably 5- to 10-membered, particularly preferably 5- or 6-membered) (monocyclic or bicyclic, preferably monocyclic) heterocyclic group preferably containing, besides carbon atom(s), 1 or 2 kinds of 1 to 4 hetero atoms selected from nitrogen atom, sulfur atom and oxygen atom. Specific examples include aromatic heterocyclic groups such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl and the like, non-aromatic heterocyclic groups such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, tetrahydrofuran-1-yl, 1,2,3,4-tetrahydrofuran-1-yl, 1,2,3,4-tetrahydroquinolin-1-yl and the like, and the like.

Of these, for example, a 5- or 6-membered heterocyclic group containing, besides carbon atom(s), 1 or 2 kinds of 1 to 3 hetero atoms selected from nitrogen atom, sulfur atom and oxygen atom, and the like are more preferable. Specifically, 2-thienyl, 3-thienyl, 2-pyridyl, 3-pyridyl, 4-pyridyl, 2-furyl, 3-furyl, pyrazinyl, 2-pyrimidinyl, 4-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, tetrahydrofuran-1-yl and the like can be mentioned.

As the "substituent" of the aforementioned "optionally substituted phenyl group or an optionally substituted heterocyclic group", for example,
optionally substituted hydrocarbon group;
optionally substituted amino group [for example, a group represented by formula 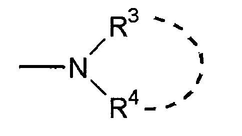 wherein R³ and R⁴ are each a hydrogen atom, an optionally substituted hydrocarbon group, an optionally substituted heterocyclic group or an optionally substituted acyl group, or R³ and R⁴ optionally form a ring together with the nitrogen atom they are bonded to;
oxo;
halogen atom (for example, fluorine, chlorine, bromine, iodine and the like);
C₁₋₃ alkylenedioxy (for example, methylenedioxy, ethylenedioxy and the like);
nitro;
cyano;
optionally substituted hydroxy group [for example, a group represented by R^{h}O-, R^{h}-CO-O-, R^{h}O-CO-O-, R^{h}-NH-CO-O- or R^{h}R^{h,}N-CO-O- (wherein R^{h} and R^{h}' are each a hydrogen atom, an optionally substituted hydrocarbon group or an optionally substituted heterocyclic group), specifically, hydroxy group; optionally halogenated C₁₋₈ alkoxy; C₁₋₆ alkoxy-carbonyl-C₁₋₆ alkoxy (e.g., ethoxycarbonylmethyloxy, etc.); C₆₋₁₄ aryloxy (e.g., phenyloxy, 1-naphthyloxy, 2-naphthyloxy, etc.) wherein the C₆₋₁₄ aryl moiety is optionally substituted by substituent(s) selected from halogen atom, C₁₋₆ alkoxy and C₁₋₆ alkyl; C₇₋₁₆ aralkyloxy (e.g., benzyloxy, phenethyloxy, etc.) wherein the C₇₋₁₆ aralkyl moiety is optionally substituted by group(s) selected from halogen atom, C₁₋₆ alkyl and C₁₋₆ alkoxy; C₁₋₆ alkyl-carbonyloxy (e.g., acetoxy, propionyloxy, etc.); C₆₋₁₄ aryl-carbonyloxy (e.g., benzoyloxy, naphthylcarbonyloxy, etc.) wherein the C₆₋₁₄ aryl moiety is optionally substituted by substituent(s) selected from halogen atom, C₁₋₆ alkoxy and C₁₋₆ alkyl; C₁₋₆ alkoxy-carbonyloxy (e. g. , methoxycarbonyloxy, ethoxycarbonyloxy, propoxycarbonyloxy, butoxycarbonyloxy, etc.); mono-C₁₋₆ alkyl-carbamoyloxy (e.g., methylcarbamoyloxy, ethylcarbamoyloxy, etc.); di-C₁₋₆ alkyl-carbamoyloxy (e.g., dimethylcarbamoyloxy, diethylcarbamoyloxy, etc.); C₆₋₁₄ aryl-carbamoyloxy (e.g., phenylcarbamoyloxy, naphthylcarbamoyloxy, etc.) wherein the C₆₋₁₄ aryl moiety is optionally substituted by substituent(s) selected from halogen atom, C₁₋₆ alkoxy and C₁₋₆ alkyl; nicotinoyloxy and the like]; optionally substituted mercapto group [for example, a group represented by R^{m}S- (wherein R^{m} is a hydrogen atom or an optionally substituted hydrocarbon group), specifically, mercapto; optionally halogenated C₁₋₆ alkylthio; C₆₋₁₄ arylthio (e.g., phenylthio, 1-naphthylthio, 2-naphthylthio, etc.) wherein the C₆₋₁₄ aryl moiety is optionally substituted by substituent(s) selected from halogen atom, C₁₋₆ alkoxy and C₁₋₆ alkyl; C₇₋₁₆ aralkylthio (e.g., benzylthio, phenethylthio, etc.) wherein the C₇₋₁₆ aralkyl moiety is optionally substituted by group(s) selected from halogen atom, C₁₋₆ alkyl and C₁₋₆ alkoxy, and the like];
carboxy;
optionally substituted acyl group;
optionally substituted alkoxycarbonyl group [for example, C₁₋₆ alkoxy-carbonyl (e.g., methoxycarbonyl, ethoxycarbonyl, propoxycarbonyl, tert-butoxycarbonyl, etc.) optionally substituted by halogen atom and the like];
optionally substituted aryloxycarbonyl group [for example, C₆₋₁₄ aryloxy-carbonyl (e.g., phenoxycarbonyl, etc.) wherein the C₆₋₁₄ aryl moiety is optionally substituted by substituent(s) selected from halogen atom, C₁₋₆ alkoxy and C₁₋₆ alkyl, and the like]; optionally substituted aralkyloxycarbonyl group [for example, C₇-16 aralkyloxy-carbonyl (e.g., benzyloxycarbonyl, phenethyloxycarbonyl, etc.) wherein the C₇₋₁₆ aralkyl moiety is optionally substituted by group(s) selected from halogen atom, C₁₋₆ alkyl and C₁₋₆ alkoxy, and the like];
thiocarbamoyl;
substituted sulfonyl group [for example, C₁₋₆ alkylsulfonyl (e.g., methylsulfonyl, ethylsulfonyl, etc.); C₆₋₁₄ arylsulfonyl (e.g., phenylsulfonyl, 1-naphthylsulfonyl, 2-naphthylsulfonyl, etc.)
wherein the C₆₋₁₄ aryl moiety is optionally substituted by substituent(s) selected from halogen atom, C₁₋₆ alkoxy and C₁₋₆ alkyl, and the like];
substituted sulfinyl group [for example, C₁₋₆ alkylsulfinyl (e.g., methylsulfinyl, ethylsulfinyl, etc.); C₆₋₁₄ arylsulfinyl (e.g., phenylsulfinyl, 1-naphthylsulfinyl, 2-naphthylsulfinyl, etc.)
wherein the C₆₋₁₄ aryl moiety is optionally substituted by substituent(s) selected from halogen atom, C₁₋₆ alkoxy and C₁₋₆ alkyl, and the like];
optionally substituted heterocyclic group, specifically, heterocyclic group [e.g., 5- to 7-membered saturated cyclic amino (e.g., pyrrolidin-1-yl, piperidino, piperazin-1-yl, morpholino, thiomorpholino, hexahydroazepin-1-yl, etc.), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.) and the like] optionally substituted by 1 to 3 substituents selected from below-mentioned the Substituent B group (preferably, C₁₋₆ alkyl (e.g., methyl, ethyl, propyl, isopropyl, butyl, isobutyl, sec-butyl, tert-butyl, pentyl, hexyl, etc.), C₆₋₁₄ aryl (e.g., phenyl, 1-naphthyl, 2-naphthyl, 2-biphenylyl, 3-biphenylyl, 4-biphenylyl, 2-anthryl, etc.), C₁₋₆ alkyl-carbonyl (e.g., acetyl, propionyl, etc.), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), oxo, etc.);
sulfo;
sulfamoyl;
sulfinamoyl;
sulfenamoyl and the like (hereinafter to be abbreviated as the Substituent A group) can be mentioned.

The "optionally substituted phenyl group or optionally substituted heterocyclic group" for R¹ may have, for example, 1 to 5, preferably 1 to 3, substituents selected from the above-mentioned substituent group A at substitutable positions, and when the number of substituents is 2 or more, the respective substituents may be the same or different.

As the "hydrocarbon group" of the "optionally substituted hydrocarbon group" of the Substituent A group and the "hydrocarbon group" of the "optionally substituted hydrocarbon group" for R³ or R⁴, for example, chain or cyclic hydrocarbon group (e.g., alkyl, alkenyl, alkynyl, cycloalkyl, aryl, aralkyl, etc.) and the like can be mentioned. Of these, chain or cyclic hydrocarbon group having 1 to 16 carbon atoms and the like are preferable.

As the "alkyl", for example, C₁₋₈ alkyl (e.g., methyl, ethyl, propyl, isopropyl, butyl, isobutyl, sec-butyl, tert-butyl, pentyl, hexyl, heptyl, octyl, etc.) and the like can be mentioned, preferably C₁₋₆ alkyl (e.g., methyl, ethyl, propyl, isopropyl, butyl, isobutyl, sec-butyl, tert-butyl, pentyl, hexyl, etc.) and the like can be mentioned, and more preferably C₁₋₃ alkyl (e.g., methyl, ethyl, propyl, isopropyl) and the like can be mentioned.

As the "alkenyl", for example, C₂₋₆ alkenyl (e.g., vinyl, allyl, isopropenyl, 1-butenyl, 2-butenyl, 3-butenyl, 2-methyl-2-propenyl, 1-methyl-2-propenyl, 2-methyl-1-propenyl, etc.) and the like can be mentioned.

As the "alkynyl", for example, C₂₋₆ alkynyl (e.g., ethynyl, propargyl, 1-butynyl, 2-butynyl, 3-butynyl, 1-hexynyl, etc.) and the like can be mentioned.

As the "cycloalkyl", for example, C₃₋₁₀ cycloalkyl (e.g., cyclopropyl, cyclobutyl, cyclopentyl, cyclohexyl, etc.) and the like can be mentioned.

As the "aryl", for example, C₆₋₁₄ aryl (e.g., phenyl, 1-naphthyl, 2-naphthyl, 2-biphenylyl, 3-biphenylyl, 4-biphenylyl, 2-anthryl, etc.) and the like can be mentioned.

As "aralkyl", for example, C₇₋₁₆ aralkyl (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, etc.) and the like can be mentioned.

As the "substituent" of the "optionally substituted hydrocarbon group" of the Substituent A group and the "substituent" of the "optionally substituted hydrocarbon group" for R³ and R⁴, for example,
oxo;
halogen atom (e.g., fluorine, chlorine, bromine, iodine, etc.); C₁₋₃ alkylenedioxy (e.g., methylenedioxy, ethylenedioxy, etc.); nitro;
cyano;
C₁₋₈ alkyl optionally substituted by substituent(s) selected from halogen atom, hydroxy group, C₁₋₆ alkoxy, mono-C₁₋₆ alkylamino, di-C₁₋₆ alkylamino, C₃₋₁₀ cycloalkyl, and heterocycle optionally substituted by substituent(s) selected from halogen atom, C₁₋₆ alkyl and C₁₋₆ alkoxy group, and the like;
optionally halogenated C₂₋₆ alkenyl;
carboxy C₂₋₆ alkenyl (e.g., 2-carboxyethenyl, 2-carboxy-2-methylethenyl, etc.);
optionally halogenated C₂₋₆ alkynyl;
optionally halogenated C₃₋₁₀ cycloalkyl;
C₆₋₁₄ aryl (e.g., phenyl, 1-naphthyl, 2-naphthyl, 2-biphenylyl, 3-biphenylyl, 4-biphenylyl, 2-anthryl, etc.) optionally substituted by substituent(s) selected from halogen atom, C₁₋₆ alkyl and C₁₋₆ alkoxy group;
optionally halogenated C₁₋₈ alkoxy;
C₁₋₆ alkoxy-carbonyl-C₁₋₆ alkoxy (e.g., ethoxycarbonylmethyloxy, etc.);
hydroxy group;
C₆₋₁₄ aryloxy (e.g., phenyloxy, 1-naphthyloxy, 2-naphthyloxy, etc.) wherein the C₆₋₁₄ aryl moiety is optionally substituted by substituent(s) selected from halogen atom, C₁₋₆ alkoxy and C₁₋₆ alkyl;
C₇₋₁₆ aralkyloxy (e.g., benzyloxy, phenethyloxy, etc.) wherein the C₇₋₁₆ aralkyl moiety is optionally substituted by group(s) selected from halogen atom, C₁₋₆ alkyl and C₁₋₆ alkoxy;
mercapto;
optionally halogenated C₁₋₆ alkylthio;
C₆₋₁₄ arylthio (e.g., phenylthio, 1-naphthylthio, 2-naphthylthio, etc.) wherein the C₆₋₁₄ aryl moiety is optionally substituted by substituent(s) selected from halogen atom, C₁₋₆ alkoxy and C₁₋₆ alkyl;
C₇₋₁₆ aralkylthio (e.g., benzylthio, phenethylthio, etc.) wherein the C₇₋₁₆ aralkyl moiety is optionally substituted by group(s) selected from halogen atom, C₁₋₆ alkyl and C₁₋₆ alkoxy;
amino;
mono-C₁₋₆ alkylamino optionally substituted by substituent(s) selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkyl and C₁₋₆ alkoxy group (e.g., methylamino, ethylamino, propylamino, t-butylamino, 1-ethylpropylamino, isopentylamino, 2-hydroxyethylamino, 2-methoxyethylamino, 2-piperidinoethylamino, 2-(N,N-diethylamino)ethylamino, cyclopropylmethylamino, thienylmethylamino, furylmethylamino, tetrahydrofuranylmethylamino, etc.);
mono-C₆₋₁₄ arylamino wherein the C₆₋₁₄ aryl moiety is optionally substituted by substituent(s) selected from halogen atom, C₁₋₆ alkoxy and C₁₋₆ alkyl (e.g., phenylamino, 1-naphthylamino, 2-naphthylamino, 2-methylphenylamino, 2,3-dimethylphenylamino, 4-methylphenylamino, 3-methylphenylamino, 4-fluoro-2-methylphenylamino, 2-methoxyphenylamino, etc.);
C₇₋₁₆ aralkylamino (e.g., benzylamino, 1-phenylethylamino, 2-phenylethylamino, etc.) wherein the C₇₋₁₆ aralkyl moiety is optionally substituted by group(s) selected from halogen atom, C₁₋₆ alkyl and C₁₋₆ alkoxy;
di-C₁₋₆ alkylamino (e.g., dimethylamino, diethylamino, ethylmethylamino, etc.) optionally substituted by group(s) selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkoxy and C₁₋₆ alkyl;
mono-C₃₋₁₀ cycloalkylamino optionally condensed with C₆₋₁₄ aryl group (e.g., cyclopropylamino, cyclobutylamino, cyclopentylamino, cyclohexylamino, 1,2,3,4-tetrahydronaphthalen-1-ylamino, etc.); di-C₃₋₁₀ cycloalkylamino (e.g., dicyclopropylamino, dicyclopentylamino, dicyclohexylamino, pentylhexylamino, etc.); di-C₆₋₁₄ arylamino (e.g., diphenylamino, etc.) wherein the C₆₋₁₄ aryl moiety is optionally substituted by substituent(s) selected from halogen atom, C₁₋₆ alkoxy and C₁₋₆ alkyl;
formyl;
carboxy;
C₁₋₆ alkyl-carbonyl optionally substituted by substituent(s) selected from hydroxy group and C₁₋₆ alkylcarbonyloxy group (e.g., acetyl, propionyl, 2-acetoxypropionyl, etc.);
C₃₋₁₀ cycloalkyl-carbonyl (e.g., cyclopropylcarbonyl, cyclopentylcarbonyl, cyclohexylcarbonyl, etc.);
C₁₋₆ alkoxy-carbonyl (e.g., methoxycarbonyl, ethoxycarbonyl, propoxycarbonyl, tert-butoxycarbonyl, etc.) optionally substituted by halogen atom;
C₆₋₁₄ aryl-carbonyl wherein the C₆₋₁₄ aryl moiety is optionally substituted by group(s) selected from halogen atom, C₁₋₆ alkyl and C₁₋₆ alkoxy (e.g., benzoyl, 4-methoxybenzoyl, 4-methylbenzoyl, 4-fluorobenzoyl, 1-naphthoyl, 2-naphthoyl, etc.);
C₇₋₁₆ aralkyl-carbonyl (e.g., phenylacetyl, 3-phenylpropionyl, etc.) wherein the C₇₋₁₆ aralkyl moiety is optionally substituted by group(s) selected from halogen atom, C₁₋₆ alkyl and C₁₋₆ alkoxy;
C₆₋₁₄ aryloxy-carbonyl (e.g., phenoxycarbonyl, etc.) wherein the C₆₋₁₄ aryl moiety is optionally substituted by substituent(s) selected from halogen atom, C₁₋₆ alkoxy and C₁₋₆ alkyl;
C₇₋₁₆ aralkyloxy-carbonyl (e.g., benzyloxycarbonyl, phenethyloxycarbonyl, etc.) wherein the C₇₋₁₆ aralkyl moiety is optionally substituted by group(s) selected from halogen atom, C₁₋₆ alkyl and C₁₋₆ alkoxy;
heterocyclylcarbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.) optionally substituted by substituent(s) selected from halogen atom, C₁₋₆ alkoxy and C₁₋₆ alkyl;
carbamoyl;
thiocarbamoyl;
mono-C₁₋₆ alkyl-carbamoyl (e.g., methylcarbamoyl, ethylcarbamoyl, etc.);
di-C₁₋₆ alkyl-carbamoyl (e.g., dimethylcarbamoyl, diethylcarbamoyl, ethylmethylcarbamoyl, etc.);
C₆₋₁₄ aryl-carbamoyl (e.g., phenylcarbamoyl, 1-naphthylcarbamoyl, 2-naphthylcarbamoyl, etc.) wherein the C₆₋₁₄ aryl moiety is optionally substituted by substituent(s) selected from halogen atom, C₁₋₆ alkoxy and C₁₋₆ alkyl;
heterocyclylcarbamoyl (e.g., 2-pyridylcarbamoyl, 3-pyridylcarbamoyl, 4-pyridylcarbamoyl, 2-thienylcarbamoyl, 3-thienylcarbamoyl, etc.) optionally substituted by substituent(s) selected from halogen atom, C₁₋₆ alkoxy and C₁₋₆ alkyl;
C₁₋₆ alkylsulfonyl (e.g., methylsulfonyl, ethylsulfonyl, etc.);
C₆₋₁₄ arylsulfonyl (e.g., phenylsulfonyl, 1-naphthylsulfonyl, 2-naphthylsulfonyl, etc.);
C₁₋₆ alkylsulfinyl (e.g., methylsulfinyl, ethylsulfinyl, etc.);
C₆₋₁₄ arylsulfinyl (e.g., phenylsulfinyl, 1-naphthylsulfinyl, 2-naphthylsulfinyl, etc.) wherein the C₆₋₁₄ aryl moiety is optionally substituted by substituent(s) selected from halogen atom, C₁₋₆ alkoxy and C₁₋₆ alkyl;
formylamino;
mono- or bis(C₁₋₆ alkyl-carbonyl)amino wherein the C₁₋₆ alkyl moiety is optionally substituted by substituent(s) selected from C₁₋₆ alkyl-carbonyloxy and hydroxy group (e.g., acetylamino, 2-acetoxypropionylamino, ethylcarbonylamino, 2-hydroxyethylcarbonylamino, propylcarbonylamino, isopropylcarbonylamino, t-butylcarbonylamino, neopentylcarbonylamino, dipropionylamino, etc.);
mono- or bis(C₆₋₁₄ aryl-carbonyl) amino wherein the C₆₋₁₄ aryl moiety is optionally substituted by substituent(s) selected from C₁₋₆ alkyl, C₁₋₆ alkoxy and halogen atom (e.g., benzoylamino, naphthoylamino, 4-methoxybenzoylamino, 4-methylbenzoylamino, 4-t-butylbenzoylamino, 4-chlorobenzoylamino, 3-fluorobenzoylamino, 2-fluorobenzoylamino, 4-fluorobenzoylamino, 3,4-dichlorobenzoylamino, bis(4-fluorobenzoyl)amino, etc.);
C₇₋₁₆ aralkyl-carbonylamino optionally substituted by C₁₋₆ alkoxy (e.g., benzylcarbonylamino, 4-methoxybenzylcarbonylamino, etc.); C₁₋₆ alkoxy-carbonylamino optionally substituted by halogen atom (e.g., 2,2,2-trichloroethyloxycarbonylamino);
C₁₋₆ alkyl-carbonylamino optionally substituted by substituent(s) selected from C₁₋₆ alkyl-carbonyloxy group (e.g., acetyloxy, etc.) and hydroxy group (e.g., acetylamino, ethylcarbonylamino, 2-hydroxyethylcarbonylamino, propylcarbonylamino, isopropylcarbonylamino, t-butylcarbonylamino, neopentylcarbonylamino, 2-acetoxypropionylamino, etc.);
mono- or di-C₃₋₁₀ cycloalkyl-carbonylamino (e.g., cyclopropylcarbonylamino, bis(cyclopropylcarbonyl)amino, etc.); heterocyclylcarbonylamino (e.g., thiophenecarbonylamino, nicotinoylamino, isonicotinoylamino, 2-pyridylcarbonylamino, 1-piperidinocarbonylamino, 3,4-dihydroisoquinolylcarbonylamino, etc.) optionally substituted by substituent(s) selected from halogen atom, C₁₋₆ alkyl and C₁₋₆ alkoxy;
C₁₋₆ alkoxy-carbonylamino (e.g., methoxycarbonylamino, ethoxycarbonylamino, propoxycarbonylamino, butoxycarbonylamino, etc.);
C₁₋₆ alkylsulfonylamino (e.g., methylsulfonylamino, ethylsulfonylamino, etc.);
mono- or bis(C₆₋₁₄ arylsulfonyl) amino (e.g., phenylsulfonylamino, 2-naphthylsulfonylamino, 1-naphthylsulfonylamino, bis(phenylsulfonyl)amino, etc.);
C₆₋₁₄ aryl-carbamoylamino wherein the C₆₋₁₄ aryl moiety is optionally substituted by substituent(s) selected from C₁₋₆ alkyl, C₁₋₆ alkoxy and halogen atom (e.g., phenylcarbamoylamino, 4-methylphenylcarbamoylamino, 2-methylphenylcarbamoylamino, 3-methylphenylcarbamoylamino, 4-methoxyphenylcarbamoylamino, 3,4-dichlorophenylcarbamoylamino, etc.);
C₁₋₆ alkyl-carbamoylamino (e.g., ethylcarbamoylamino, propylcarbamoylamino, isopropylcarbamoylamino);
C₃₋₁₀ cycloalkyl-carbamoylamino (e.g., cyclohexylcarbamoylamino); di-C₁₋₆ alkyl-carbamoylamino (e.g., diethylcarbamoylamino);
C₁₋₆ alkyl-carbonyloxy (e.g., acetoxy, propionyloxy, etc.);
C₆₋₁₄ aryl-carbonyloxy (e.g., benzoyloxy, naphthylcarbonyloxy, etc.) wherein the C₆₋₁₄ aryl moiety is optionally substituted by substituent(s) selected from halogen atom, C₁₋₆ alkoxy and C₁₋₆ alkyl;
C₁₋₆ alkoxy-carbonyloxy (e.g., methoxycarbonyloxy, ethoxycarbonyloxy, propoxycarbonyloxy, butoxycarbonyloxy, etc.); mono-C₁₋₆ alkyl-carbamoyloxy (e.g., methylcarbamoyloxy, ethylcarbamoyloxy, etc.);
di-C₁₋₆ alkyl-carbamoyloxy (e.g., dimethylcarbamoyloxy, diethylcarbamoyloxy, etc.);
C₆₋₁₄ aryl-carbamoyloxy (e.g., phenylcarbamoyloxy, naphthylcarbamoyloxy, etc.);
nicotinoyloxy;
heterocyclic group optionally substituted by substituent(s) selected from halogen atom, C₁₋₆ alkyl and C₁₋₆ alkoxy [e.g., 5-to 7-membered saturated cyclic amino optionally condensed with C₆₋₁₄ aryl (e.g., pyrrolidin-1-yl, piperidino, piperazin-1-yl, morpholino, thiomorpholino, 1-piperazinyl optionally having a substituent at the 4-position, 3,4-dihydroquinolin-1-yl, 3,4-dihydroisoquinolin-2-yl, 1,2,3,4-tetrahydroquinolin-1-yl, hexahydroazepin-1-yl, etc.), 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), etc.];
sulfo;
sulfamoyl;
sulfinamoyl;
sulfenamoyl and the like (hereinafter to be abbreviated as the Substituent B group)
can be mentioned.

As the "optionally substituted hydrocarbon group" of the Substituent A group, more specifically, (1) C₁₋₈ alkyl optionally substituted by substituent(s) selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkyl and C₁₋₆ alkoxy; (2) optionally halogenated C₂₋₆ alkenyl; (3) carboxy C₂₋₆ alkenyl (e.g., 2-carboxyethenyl, 2-carboxy-2-methylethenyl, etc.); (4) optionally halogenated C₂₋₆ alkynyl; (5) optionally halogenated C₃₋₁₀ cycloalkyl; (6) C₆₋₁₄ aryl optionally substituted by substituent(s) selected from halogen atom, C₁₋₆ alkyl and C₁₋₆ alkoxy group (e.g., phenyl, 1-naphthyl, 2-naphthyl, 2-biphenylyl, 3-biphenylyl, 4-biphenylyl, 2-anthryl, etc.); and the like can be mentioned.

As the "heterocyclic group" of the "optionally substituted heterocyclic group" for R³ or R⁴, those similar to the "heterocyclic group" of the "optionally substituted heterocyclic group" for R¹ can be used.

As the "substituent" of the "optionally substituted heterocyclic group" for R³ or R⁴, a substituent selected from the Substituent B group, particularly, a substituent selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkyl and C₁₋₆ alkoxy, and the like can be mentioned.

As the "optionally substituted acyl group" of the Substituent A group and "optionally substituted acyl group" for R³ or R⁴, for example, a group represented by the formula: -

(C=O)-R5,-(C=O)-NR⁶R^{6'} or -SO₂-R⁷

wherein R⁵ is a hydrogen atom, an optionally substituted hydrocarbon group or an optionally substituted heterocyclic group, R⁶ is a hydrogen atom, an optionally substituted hydrocarbon group or an optionally substituted heterocyclic group, R^{6'} is a hydrogen atom, an optionally substituted hydrocarbon group or an optionally substituted heterocyclic group, or R⁶ and R^{6'} optionally form a ring together with the nitrogen atom they are bonded to, and R⁷ is an optionally substituted hydrocarbon group or an optionally substituted heterocyclic group,
and the like can be mentioned.

As the "optionally substituted hydrocarbon group" for R^{h}, R^{h,}, R⁵, R⁶, R^{6'} or R⁷, those similar to the "optionally substituted hydrocarbon group" of the Substituent A group can be used.

As the "heterocyclic group" of the "optionally substituted heterocyclic group" for R^{h}, R^{h'}, R⁵, R⁶, R^{6'} or R⁷, those similar to the "heterocyclic group" of the "optionally substituted heterocyclic group" for R¹ can be used.

As the "substituent" of the "optionally substituted heterocyclic group" for R^{h}, R^{h}' R⁵, R⁶, R⁶' or R⁷, a substituent selected from the Substituent B group, particularly, a substituent selected from halogen atom, C₁₋₆ alkyl and C₁₋₆ alkoxy, and the like can be mentioned.

As specific examples of the "optionally substituted acyl group" of the Substituent A group and the "optionally substituted acyl group" for R³ or R⁴, for example, formyl;
C₁₋₆ alkyl-carbonyl optionally substituted by substituent(s) selected from the aforementioned the Substituent B group (e.g., acetyl, propionyl, 2-acetoxypropionyl, etc.);
C₃₋₁₀ cycloalkyl-carbonyl (e.g., cyclopropylcarbonyl, cyclopentylcarbonyl, cyclohexylcarbonyl, etc.) optionally substituted by substituent(s) selected from the aforementioned the Substituent B group;
C₁₋₆ alkoxy-carbonyl (e.g., methoxycarbonyl, ethoxycarbonyl, propoxycarbonyl, tert-butoxycarbonyl, etc.) optionally substituted by substituent(s) selected from the aforementioned the Substituent B group;
C₆₋₁₄ aryl-carbonyl optionally substituted by substituent(s) selected from the aforementioned the Substituent B group (e.g., benzoyl, 4-methoxybenzoyl, 4-methylbenzoyl, 4-fluorobenzoyl, 1-naphthoyl, 2-naphthoyl, etc.);
C₇₋₁₆ aralkyl-carbonyl (e.g., phenylacetyl, 3-phenylpropionyl, etc.) optionally substituted by substituent(s) selected from the aforementioned the Substituent B group;
C₆₋₁₄ aryloxy-carbonyl (e.g., phenoxycarbonyl, etc.) optionally substituted by substituent(s) selected from the aforementioned the Substituent B group;
C₇₋₁₆ aralkyloxy-carbonyl (e.g., benzyloxycarbonyl, phenethyloxycarbonyl, etc.) optionally substituted by substituent(s) selected from the aforementioned the Substituent B group;
heterocyclylcarbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.) optionally substituted by substituent(s) selected from the aforementioned the Substituent B group;
carbamoyl;
thiocarbamoyl;
mono-C₁₋₆ alkyl-carbamoyl (e.g., methylcarbamoyl, ethylcarbamoyl, etc.) optionally substituted by substituent(s) selected from the aforementioned the Substituent B group;
di-C₁₋₆ alkyl-carbamoyl (e.g., dimethylcarbamoyl, diethylcarbamoyl, ethylmethylcarbamoyl, etc.) optionally substituted by substituent(s) selected from the aforementioned the Substituent B group;
C₆₋₁₄ aryl-carbamoyl (e.g., phenylcarbamoyl, 1-naphthylcarbamoyl, 2-naphthylcarbamoyl, etc.) optionally substituted by substituent(s) selected from the aforementioned the Substituent B group;
heterocyclylcarbamoyl (e.g., 2-pyridylcarbamoyl, 3-pyridylcarbamoyl, 4-pyridylcarbamoyl, 2-thienylcarbamoyl, 3-thienylcarbamoyl, etc.) optionally substituted by substituent(s) selected from the aforementioned the Substituent B group;
C₁₋₆ alkylsulfonyl (e.g., methylsulfonyl, ethylsulfonyl, etc.) optionally substituted by substituent(s) selected from the aforementioned the Substituent B group;
C₆₋₁₄ arylsulfonyl (e.g., phenylsulfonyl, 1-naphthylsulfonyl, 2-naphthylsulfonyl, etc.) optionally substituted by substituent(s) selected from the aforementioned the Substituent B group;
C₁₋₆ alkylsulfinyl (e.g., methylsulfinyl, ethylsulfinyl, etc.); and the like can be mentioned.

As the ring formed by R³ and R⁴ together with the nitrogen atom they are bonded to, the ring formed by R⁶ and R^{6'} together with the nitrogen atom they are bonded to, and ring formed by R¹⁰ and R¹¹ together with the nitrogen atom they are bonded to, for example, a "optionally substituted 5- to 7-membered saturated cyclic amino group" can be mentioned. The "optionally substituted 5- to 7-membered saturated cyclic amino group" may be a fused ring condensed with C₆₋₁₄ aryl group (e.g., benzene ring, etc.) (e.g., optionally substituted 5- to 7-membered saturated cyclic amino group condensed with C₆₋₁₄ aryl group, etc.) and the like.

As the ring formed by R³ and R⁴ together with the nitrogen atom they are bonded to, the ring formed by R⁶ and R^{6'} together with the nitrogen atom they are bonded to, and ring formed by R¹⁰ and R¹¹ together with the nitrogen atom they are bonded to, specifically, for example, pyrrolidin-1-yl, piperidino, piperazin-1-yl, morpholino, thiomorpholino, 1-piperazinyl optionally having a substituent at the 4-position, 3,4-dihydroquinolin-1-yl, 3,4-dihydroisoquinolin-2-yl, 1,2,3,4-tetrahydroquinolin-1-yl, hexahydroazepin-1-yl and the like can be mentioned, and particularly preferably, piperidino, 1,2,3,4-tetrahydroquinolin-1-yl and the like can be mentioned.

As the "substituent" which the ring optionally has, a substituent selected from the Substituent B group, particularly, a substituent selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkyl and C₁₋₆ alkoxy, and the like can be mentioned.

As the aforementioned "C₁₋₈ alkyl optionally substituted by substituent(s) selected from halogen atom, hydroxy group, C₁₋₆ alkoxy, mono-C₁₋₆ alkylamino, di-C₁₋₆ alkylamino, C₃₋₁₀ cycloalkyl, and heterocyclic group optionally substituted by substituent(s) selected from halogen atom, C₁₋₆ alkyl and C₁₋₆ alkoxy", for example, C₁₋₈ alkyl (e.g., methyl, ethyl, propyl, isopropyl, butyl, isobutyl, sec-butyl, tert-butyl, pentyl, hexyl, heptyl, octyl, etc.) optionally substituted by 1 to 5, preferably 1 to 3, substituent(s) selected from halogen atom (e.g., fluorine, chlorine, bromine, iodine, etc.), hydroxy group, C₁₋₆ alkoxy (e.g., methoxy, ethoxy, propoxy, isopropoxy, butoxy, isobutoxy, sec-butoxy, pentyloxy, hexyloxy, etc.), mono-C₁₋₆ alkylamino (e.g., methylamino, ethylamino, propylamino, isopropylamino, butylamino, isobutylamino, sec-butylamino, pentylamino, hexylamino, etc.), di-C₁₋₆ alkylamino (e.g., dimethylamino, diethylamino, dipropylamino, ethylmethylamino, etc.), C₃₋₁₀ cycloalkyl (e.g., cyclopropyl, cyclobutyl, cyclopentyl, cyclohexyl, etc.), and heterocyclic group (e.g., pyrrolidin-1-yl, piperidino, piperazin-1-yl, morpholino, thiomorpholino, hexahydroazepin-1-yl, 1-furyl, 2-furyl, 1-thienyl, 2-thienyl, tetrahydro-1-furanyl, 1,2,3,4-tetrahydro-1-quinolinyl, etc.) optionally substituted by substituent(s) selected from halogen atom (e.g., fluorine, chlorine, bromine, iodine, etc.), C₁₋₆ alkyl (e.g., methyl, ethyl, etc.) and C₁₋₆ alkoxy (e.g., methoxy, ethoxy, propoxy, isopropoxy, butoxy, isobutoxy, sec-butoxy, pentyloxy, hexyloxy, etc.), and the like can be mentioned. As specific examples, methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 2-ethylpropyl, 5,5,5-trifluoropentyl, hexyl, 6,6,6-trifluorohexyl, 1-furylmethyl, tetrahydrofuran-1-ylmethyl, 1-thienylmethyl and the like can be mentioned.

As the aforementioned "optionally halogenated C₂₋₆ alkenyl", for example, C₂₋₆ alkenyl (for example, vinyl, propenyl, isopropenyl, 2-buten-1-yl, 4-penten-1-yl, 5-hexen-1-yl) optionally having 1 to 5, preferably 1 to 3 halogen atoms (for example, fluorine, chlorine, bromine, iodine and the like) and the like can be mentioned. As specific examples, vinyl, 1-propenyl, allyl, 3,3,3-trifluoro-1-propenyl and the like can be mentioned.

As the aforementioned "optionally halogenated C₂₋₆ alkynyl", C₂₋₆ alkynyl (for example, 2-butyn-1-yl, 4-pentyn-1-yl, 5-hexyn-1-yl and the like) optionally having 1 to 5, preferably 1 to 3 halogen atoms (for example, fluorine, chlorine, bromine, iodine and the like) and the like can be mentioned. As specific examples, 2-butyn-1-yl, 4-pentyn-1-yl, 5-hexyn-1-yl, 4,4,4-trifluoro-2-butyn-1-yl and the like can be mentioned.

As the aforementioned "optionally halogenated C₃₋₁₀ cycloalkyl", for example, C₃₋₁₀ cycloalkyl (for example, cyclopropyl, cyclobutyl, cyclopentyl, cyclohexyl and the like) optionally having 1 to 5, preferably 1 to 3 halogen atoms (for example, fluorine, chlorine, bromine, iodine and the like) and the like can be mentioned. As specific examples, cyclopropyl, cyclobutyl, cyclopentyl, cyclohexyl, 4,4-dichlorocyclohexyl, 2,2,3,3-tetrafluorocyclopentyl, 4-chlorocyclohexyl and the like can be mentioned.

As the aforementioned "optionally halogenated C₁₋₈ alkoxy", for example, C₁₋₈ alkoxy (for example, methoxy, ethoxy, propoxy, isopropoxy, butoxy, isobutoxy, sec-butoxy, pentyloxy, hexyloxy and the like) optionally having 1 to 5, preferably 1 to 3 halogen atoms (for example, fluorine, chlorine, bromine, iodine and the like) and the like can be mentioned. As specific examples, methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy and the like can be mentioned.

As the aforementioned "optionally halogenated C₁₋₆ alkylthio", for example, C₁₋₆ alkylthio (for example, methylthio, ethylthio, propylthio, isopropylthio, butylthio, sec-butylthio, tert-butylthio and the like) optionally having 1 to 5, preferably 1 to 3 halogen atoms (for example, fluorine, chlorine, bromine, iodine and the like) and the like can be mentioned. As specific examples, methylthio, difluoromethylthio, trifluoromethylthio, ethylthio, propylthio, isopropylthio, butylthio, 4,4,4-trifluorobutylthio, pentylthio, hexylthio and the like can be mentioned.

As the "heterocycle" of the aforementioned "heterocycle optionally substituted by substituent(s) selected from halogen atom, C₁₋₆ alkyl and C₁₋₆ alkoxy", for example, those similar to the heterocycle of the "optionally substituted heterocyclic group" for R¹ and the like can be mentioned.

As specific examples of the "heterocycle optionally substituted by substituent(s) selected from halogen atom, C₁₋₆ alkyl and C₁₋₆ alkoxy", for example, piperidino, morpholino, 3,4-dihydroquinolin-1-yl, 3,4-dihydroisoquinolin-2-yl, 1,2,3,4-tetrahydroquinolin-1-yl, 6,7-dimethoxy-1,2,3,4-tetrahydroquinolin-1-yl and the like can be mentioned.

Of these, as the "substituent" of the "optionally substituted phenyl group or optionally substituted heterocyclic group" for R¹, halogen atom and an optionally substituted hydrocarbon group are preferable, and an optionally substituted hydrocarbon group is more preferable.

As R¹, preferably an optionally substituted phenyl group and the like can be mentioned, and particularly preferably phenyl group substituted by C₁₋₆ alkyl group (e.g., methyl, etc.), halogen atom (e.g., chlorine, fluorine, etc.) and the like, and the like can be mentioned.

### (Explanation of R²)

In the formula [I], R² is an optionally substituted pyridin-4-yl group, an optionally substituted pyridine-N-oxide-4-yl group or an optionally substituted pyrimidin-4-yl group.

In the aforementioned formula [I], as the "substituent" of the "optionally substituted pyridin-4-yl group, optionally substituted pyridine-N-oxide-4-yl group or optionally substituted pyrimidin-4-yl group" for R², for example, the "substituent" selected from the Substituent A group exemplified for the "substituent" of the "optionally substituted phenyl group or optionally substituted heterocyclic group" for R¹, and the like can be mentioned.

As such "substituent" for R², for example, halogen atom, the aforementioned optionally substituted hydrocarbon group, a group represented by R^{h}O- (R^{h} is as defined above) and an optionally substituted amino group (e.g., a group represented by the aforementioned formula 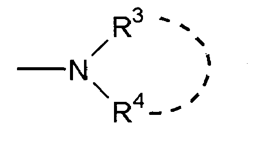 wherein R³ and R⁹ is as defined above) are preferable.

As the substituent which the "optionally substituted pyridin-4-yl group, optionally substituted pyridine-N-oxide-4-yl group or optionally substituted pyrimidin-4-yl group" for R² optionally has, particularly preferably a substituent selected from the following (I)-(VI):
(I) halogen atom (e.g., fluorine, etc.);
(II) C₁₋₆ alkoxy group (e.g., ethoxy, etc.);
(III) C₁₋₆ alkyl group (e.g., methyl, etc.);
(IV) amino group;
(V) 5- to 7-membered saturated cyclic amino group optionally substituted by oxo (e.g., tetrahydropyrimidinyl-2(1H)-one);
(VI) amino group mono- or di-substituted by substituent(s) selected from
   (1) C₃₋₁₀ cycloalkyl group optionally condensed with C₆₋₁₄ aryl ring (e.g., cyclopropyl, cyclobutyl, cyclopentyl, cyclohexyl, 1,2,3,4-tetrahydronaphthyl, etc.);
   (2) C₁₋₆ alkyl group (e.g., methyl, ethyl, propyl, butyl, isopentyl, 1-ethylpropyl, etc.);
   (3) hydroxy C₁₋₆ alkyl group (e.g., hydroxyethyl, etc.);
   (4) C₃₋₁₀ cycloalkyl C₁₋₆ alkyl group (e.g., cyclopropylmethyl, etc.);
   (5) C₁₋₆ alkoxy C₁₋₆ alkyl group (e.g., methoxyethyl, etc.);
   (6) 5- to 7-membered saturated cyclic amino C₁₋₆ alkyl group (e.g., piperidylethyl, pyrrolidilmethyl, etc.);
   (7) 5- or 6-membered heterocyclyl C₁₋₆ alkyl group (e.g., thienylmethyl, furylmethyl, 2-tetrahydrofuranylmethyl, etc.);
   (8) mono or di-C₁₋₆ alkylamino C₁₋₆ alkyl group (e.g., 2-(N, N-diethylamino)ethyl, etc.), preferably di-C₁₋₆ alkylamino C₁₋₆ alkyl group;
   (9) C₇₋₁₆ aralkyl group (e.g., benzyl, 1-phenylethyl, etc.);
   (10) C₆₋₁₄ aryl group optionally substituted by substituent(s) selected from C₁₋₆ alkoxy group (e.g., methoxy), C₁₋₆ alkyl group (e.g., methyl, etc.) and halogen atom (e.g., fluorine, etc.) (e.g., phenyl, 2-methylphenyl, 2,3-dimethylphenyl, 4-methylphenyl, 3-methylphenyl, 4-fluoro-2-methylphenyl, 2-methoxyphenyl, etc.);
   (11) C₁₋₆ alkyl-carbonyl group optionally substituted by substituent(s) selected from C₁₋₆ alkyl-carbonyloxy group (e.g., acetyloxy, etc.), hydroxy group, halogen atom (e.g., fluorine atom, etc.), C₁₋₆ alkylthio group (e.g., methylthio, etc.) and C₁-6 alkylsulfonyl group (e.g., methylsulfonyl, etc.) (e.g., acetyl, ethylcarbonyl, 2-hydroxyethylcarbonyl, propylcarbonyl, isopropylcarbonyl, t-butylcarbonyl, neopentylcarbonyl, 2-acetoxypropionyl, 3,3,3-trifluoro-1-propionyl, 2-methylthioethylcarbonyl, methylthiomethylcarbonyl, etc.);
   (12) C₃₋₁₀ cycloalkyl-carbonyl group (e.g., cyclopropylcarbonyl, etc.);
   (13) C₆₋₁₄ aryl-carbonyl group optionally substituted by substituent(s) selected from C₁₋₆ alkoxy group (e.g., methoxy, etc.) optionally substituted by halogen atom (e.g., fluorine atom, etc.), C₁₋₆ alkyl group (e.g., methyl, t-butyl) optionally substituted by halogen atom (e.g., fluorine atom, etc.), halogen atom (e.g., chlorine, fluorine, etc.), C₁₋₆ alkylthio group (e.g., methylthio, etc.), C₁₋₆ alkylsulfonyl group (e.g., methylsulfonyl, etc.) and di-C₁₋₆ alkylamino group (e.g., dimethylamino, etc.) (e.g., benzoyl, 4-methoxybenzoyl, 4-methylbenzoyl, 4-t-butylbenzoyl, 4-chlorobenzoyl, 3-fluorobenzoyl, 2-fluorobenzoyl, 4-fluorobenzoyl, 3,4-dichlorobenzoyl, 4-fluoro-2-methoxybenzoyl, 4-(methylthio)benzoyl, 4-(dimethylamino)benzoyl, 4-fluoro-3-methoxybenzoyl, 3-fluoro-4-methoxybenzoyl, 4-(trifluoromethyl)benzoyl, 4-(trifluoromethoxy)benzoyl, 4-chloro-2-methylbenzoyl, 2-methylbenzoyl, 3-methylbenzoyl, 4-methoxy-2-methylbenzoyl, 1-naphthylcarbonyl, 2-naphthylcarbonyl, etc.);
   (14) C₇₋₁₆ aralkyl-carbonyl group optionally substituted by C₁₋₆ alkoxy group (e.g., benzylcarbonyl, 4-methoxybenzylcarbonyl, etc.);
   (15) C₁₋₆ alkoxy-carbonyl group optionally substituted by halogen atom (e.g., chlorine, etc.) (e.g., 2,2,2-trichloroethyloxycarbonyl, etc.);
   (16) C₆₋₁₄ aryl-sulfonyl group (e.g., phenylsulfonyl, etc.);
   (17) mono- or di-C₆₋₁₄ aryl-carbamoyl group wherein the C₆₋₁₄ aryl moiety is optionally substituted by substituent(s) selected from C₁₋₆ alkyl group (e.g., methyl, etc.), C₁₋₆ alkoxy group (e.g., methoxy, etc.), halogen atom (e.g., chlorine, etc.), C₁₋₆ alkyl-carbonyl group (e.g., acetyl, etc.), C₁₋₆ alkylthio group (e.g., methylthio, etc.) and cyano group (e.g., phenylcarbamoyl, 4-methylphenylcarbamoyl, 2-methylphenylcarbamoyl, 3-methylphenylcarbamoyl, 4-methoxyphenylcarbamoyl, 3,4-dichlorophenylcarbamoyl, 4-cyanophenylcarbamoyl, 4-methylthiophenylcarbamoyl, etc.);
   (18) mono-C₁₋₆ alkyl-carbamoyl group or di-C₁₋₆ alkyl-carbamoyl group (e.g., diethylcarbamoyl, etc.) optionally substituted by substituent(s) selected from hydroxy group, C₁₋₆ alkylsulfonyl group (e.g., methylsulfonyl, etc.), oxo group, cyano group and halogen atom (e.g., fluorine atom, chlorine atom, etc.) (e.g., methylcarbamoyl, ethylcarbamoyl, 2-cyanoethylcarbamoyl, propylcarbamoyl, isopropylcarbamoyl, 4-hydroxybutylcarbamoyl, 2-hydroxypropylcarbamoyl, 1-ethylpropylcarbamoyl, tert-butylcarbamoyl, sec-butylcarbamoyl, 3-chloropropylcarbamoyl, 2,2,2-trifluoroethylcarbamoyl, methylsulfonylmethylcarbamoyl, methylsulfonylethylcarbamoyl, etc.);
   (19) mono- or di-C₃₋₁₀ cycloalkyl-carbamoyl group optionally substituted by hydroxy group (e.g., cyclopropylcarbamoyl, 4-hydroxycyclohexylcarbamoyl, cyclohexylcarbamoyl, etc.);
   (20) 5- to 10-membered heterocyclic group optionally substituted by C₁₋₆ alkyl group;
   (21) 5- to 7-membered saturated cyclic amino-carbonyl group optionally condensed with C₆₋₁₄ aryl ring (e.g., benzene ring), which is optionally substituted by hydroxy group (e.g., 1-piperidinocarbonyl, 3,4-dihydroisoquinolylcarbonyl, morpholinocarbonyl, 4-hydroxypiperidine-1-carbonyl, etc.);
   (22) 5- to 10-membered heterocyclyl-carbonyl group optionally substituted by substituent(s) selected from halogen atom (e.g., chlorine atom) and C₁₋₆ alkyl (e.g., methyl) optionally substituted by halogen atom (e.g., fluorine atom, chlorine atom) (e.g., 4-pyridylcarbonyl, 4-chloro-3-pyridylcarbonyl, 2-chloro-3-pyridylcarbonyl, 6-chloro-3-pyridylcarbonyl, 2-pyridylcarbonyl, 2-chloro-4-pyridylcarbonyl, 6-trifluoromethyl-3-pyridylcarbonyl, 6-methyl-3-pyridylcarbonyl, 5-chloro-3-pyridylcarbonyl, 2-methyl-3-pyridylcarbonyl, 5-methyl-3-pyridylcarbonyl, 2-chloro-5-methyl-4-pyridylcarbonyl, 3-thiophenecarbonyl, 3-furylcarbonyl, 2-pyrazinecarbonyl, 1,3-benzothiazole-6-carbonyl, 6-quinolinecarbonyl, 8-quinolinecarbonyl, 6-methyl-4-pyridylcarbonyl, 3-pyridylcarbonyl, etc.);
   (23) carbamoyl; and
   (24) 5- to 10-membered heterocyclyl-carbamoyl group optionally substituted by C₁₋₆ alkyl (e.g., methyl, etc.) (e.g., 3-thienylcarbamoyl, 3-pyridylcarbamoyl, (3-methyl-1H-pyrazol-5-yl)carbamoyl, (tetrahydro-2H-pyran-4-yl)carbamoyl, etc.).

As the di-substituted amino group, for example, amino group di-substituted by substituents selected from
(1) C₁₋₆ alkyl-carbonyl group (e.g., ethylcarbonyl, etc.);
(2) C₃₋₁₀ cycloalkyl-carbonyl group (e.g., cyclopropylcarbonyl, etc.);
(3) C₆₋₁₄ aryl-carbonyl group optionally substituted by halogen atom (e.g., 4-fluorobenzoyl, etc.); and
(4) C₆₋₁₄ aryl-sulfonyl group (e.g., phenylsulfonyl, etc.) and the like are preferable.

The "optionally substituted pyridin-4-yl group, optionally substituted pyridine-N-oxide-4-yl group or optionally substituted pyrimidin-4-yl group" for R² optionally has 1 to 5, preferably 1 to 3, substituents, for example, substituents selected from the aforementioned the Substituent A group, preferably substituents cited in the above-mentioned (I)-(VI), at substitutable positions, and when the number of substituents is 2 or more, the respective substituents may be the same or different.

### (Explanation of X¹, X² and X³)

In the formula [I] and [I'], X¹, X² and X³ are each an optionally substituted CH or a nitrogen atom, and any one of X¹, X²and X³ is a nitrogen atom. X⁴ is an optionally substituted CH.

As the "substituent" when any of X¹-X³ is "optionally substituted CH" and the "substituent" of the "optionally substituted CH" for X⁴, for example, those similar to the "substituents" selected from the Substituent A group exemplified for the substituent of the "optionally substituted phenyl group or optionally substituted heterocyclic group" for R¹ can be mentioned. The "substituent" of the "optionally substituted CH" is preferably halogen atom, a group represented by R^{m}S- (R^{m} is as defined above), a group represented by R^{h}O- (R^{h} is as defined above), substituted sulfinyl group, substituted sulfonyl group, a group represented by the formula 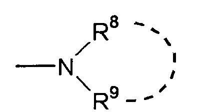 wherein R⁸ and R⁹ are each a hydrogen atom, an optionally substituted hydrocarbon group, or R⁸ and R⁹ form a ring together with the nitrogen atom they are bonded to, and the like.

As the "optionally substituted hydrocarbon group" for the aforementioned formula R⁸ and/or R⁹, for example, those similar to "optionally substituted hydrocarbon group" of the Substituent A group exemplified for the substituent of the "optionally substituted phenyl group or optionally substituted heterocyclic group" for R¹ can be mentioned.

Alternatively, R⁸ and R⁹ form a ring together with the nitrogen atom they are bonded to. As the ring formed by R⁸ and R⁹ together with the nitrogen atom they are bonded to, for example, those similar to the "ring formed by R³ and R⁴ together with the nitrogen atom they are bonded to" above-mentioned in the "explanation of R²" can be mentioned.

As particularly preferable "substituent" of the "optionally substituted CH" for X¹, X², X³ or X⁴, for example, a substituent selected from
(a) halogen atom (e.g., chlorine atom, etc.),
(b) C₁₋₆ alkyl group (e.g., methyl, tert-butyl, etc.),
(c) C₁₋₆ alkylthio group (e.g., methylthio group, etc.),
(d) C₁₋₆ alkylsulfinyl group (e.g., methylsulfinyl group, etc.),
(e) C₁₋₆ alkylsulfonyl group (e.g., methylsulfonyl group, etc.),
(f) C₁₋₆ alkoxy group (e.g., methoxy group, etc.), and
(g) formula 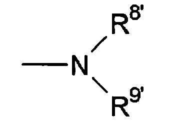 wherein R^{8'} and R^{9'} are each a substituent selected from
   (i) hydrogen atom,
   (ii) C₁₋₆ alkyl group (e.g., methyl, etc.)
   (iii) C₇₋₁₆ aralkyl group (e.g., benzyl, etc.), and
   (iv) C₃₋₁₀ cycloalkyl group (e.g., cyclohexyl, etc.) and the like.

### (Explanation of R^{2'})

In the formula [I'], R^{2'} is an optionally substituted phenyl group or an optionally substituted heterocyclic group.

As the "optionally substituted phenyl group or optionally substituted heterocyclic group" for R^{2'}, for example, those similar to the "optionally substituted phenyl group or optionally substituted heterocyclic group" for R¹ can be mentioned.

As compound (I), for example, compounds shown in the following (A)-(F), and the like are preferably used.

### (A) Compound (I) wherein

R¹ is an optionally substituted phenyl group;
R² is an optionally substituted pyridin-4-yl group;
X¹ is a nitrogen atom; and
X² and X³ are each an optionally substituted CH.

### (B) Compound (I) wherein

R¹ is a phenyl group optionally substituted by substituent(s) selected from halogen atom (e.g., chlorine atom, fluorine atom) and C₁₋₆ alkyl group (e.g., methyl group);
R² is
(I) a pyridin-4-yl group substituted by substituent(s) selected from halogen atom (e.g., fluorine atom, etc.), C₁₋₆ alkoxy group (e.g., ethoxy, etc.) and C₁₋₆ alkyl group (e.g., methyl),
(II) a pyridin-4-yl group substituted by substituent(s) represented by the formula 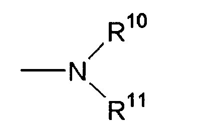 wherein
   R¹⁰ and R¹¹ are each
   (a) a hydrogen atom,
   (b) a C₁₋₆ alkyl group(e.g., methyl, ethyl, propyl, butyl, isopentyl, 1-ethylpropyl) optionally substituted by group(s) selected from
      (i) C₁₋₆ alkoxy group (e.g., methoxy, etc.),
      (ii) amino group optionally having 1 or 2 C₁₋₆ alkyl groups (e.g., ethyl, etc.),
      (iii) hydroxy group,
      (iv) C₃₋₁₀ cycloalkyl group (e.g., cyclopropyl, cyclobutyl, cyclopentyl, cyclohexyl, etc.),
      (v) 5- to 10-membered (preferably 5 or 6-membered) heterocyclic group (e.g., furyl, thienyl, tetrahydrofuranyl, etc.), and
      (vi) 5- to 7-membered saturated cyclic amino group (e.g., piperidyl, etc.),
   (c) a C₇₋₁₆ aralkyl group (e.g., benzyl, 1-phenylethyl, etc.),
   (d) a C₃₋₁₀ cycloalkyl group (e.g., cyclopropyl, cyclobutyl, cyclopentyl, cyclohexyl, etc.) optionally condensed with C₆₋₁₄ aryl ring (e.g., benzene ring),
   (e) a C₆₋₁₄ aryl group (e.g., phenyl, etc.) optionally substituted by substituent(s) selected from halogen atom (e.g., fluorine), C₁₋₆ alkyl group (e.g., methyl) and C₁₋₆ alkoxy group (e.g., methoxy), or
   (f) the formula -(C=O)-R⁵, -(C=O)-NR⁶R^{6'} or -SO₂-R⁷
   wherein
   R⁵ is
   (i) a C₁₋₆ alkyl group (e.g., methyl, ethyl, propyl, isopropyl, t-butyl, neopentyl, etc.) optionally substituted by substituent(s) selected from C₁₋₆ alkyl-carbonyloxy group (e.g., acetyloxy, etc.), hydroxy group, halogen atom (e.g., fluorine, etc.), C₁₋₆ alkylthio group (e.g., methylthio, etc.) and C₁₋₆ alkylsulfonyl group (e.g., methylsulfonyl, etc.);
   (ii) a C₃₋₁₀ cycloalkyl group (e.g., cyclopropyl, etc.) ;
   (iii) a C₆₋₁₄ aryl group (e.g., phenyl, naphthyl, etc.) optionally substituted by substituent(s) selected from C₁₋₆ alkoxy group (e.g., methoxy, etc.) optionally substituted by halogen atom (e.g., fluorine, etc.), C₁₋₆ alkyl group (e.g., methyl, t-butyl, etc.) optionally substituted by halogen atom (e.g., fluorine, etc.), halogen atom (e.g., chlorine, fluorine, etc.), C₁₋₆ alkylthio group (e.g., methylthio, etc.), C₁₋₆ alkylsulfonyl group (e.g., methylsulfonyl, etc.) and di-C₁₋₆ alkylamino group (e.g., dimethylamino, etc.);
   (iv) a C₇₋₁₆ aralkyl group (e.g., benzyl, etc.) optionally substituted by C₁₋₆ alkoxy group (e.g., methoxy, etc.);
   (v) a C₁₋₆ alkoxy group (e.g., ethoxy, etc.) optionally substituted by halogen atom (e.g., chlorine, etc.); or
   (vi) a 5- to 10-membered heterocyclic group (e.g., thienyl, furyl, pyridyl, pyrazinyl, benzothiazolyl, quinolyl, etc.) optionally substituted by substituent(s) selected from halogen atom (e.g., chlorine, etc.) and C₁₋₆ alkyl group (e.g., methyl, etc.) optionally substituted by halogen atom (e.g., fluorine, chlorine, etc.),
   R⁶ is
   (i) a hydrogen atom;
   (ii) a C₆₋₁₄ aryl group (e.g., phenyl, etc.) optionally substituted by substituent(s) selected from halogen atom (e.g., chlorine, etc.), C₁₋₆ alkyl group (e.g., methyl, etc.), C₁₋₆ alkoxy group (e.g., methoxy, etc.), C₁₋₆ alkyl-carbonyl group (e.g., acetyl, etc.), C₁₋₆ alkylthio group (e.g., methylthio, etc.) and cyano group;
   (iii) a C₁₋₆ alkyl group (e.g., methyl, ethyl, propyl, isopropyl, butyl, tert-butyl, 1-ethylpropyl, etc.) optionally substituted by substituent(s) selected from hydroxy group, C₁₋₆ alkylsulfonyl group (e.g., methylsulfonyl, etc.), oxo group, cyano group and halogen atom (e.g., fluorine, chlorine, etc.);
   (iv) a C₃₋₁₀ cycloalkyl group (e.g., cyclopropyl, cyclohexyl, etc.) optionally substituted by hydroxy group; or
   (v) a 5- to 10-membered heterocyclic group (e.g., thienyl, pyridyl, tetrahydropyranyl, etc.) optionally substituted by C₁₋₆ alkyl group (e.g., methyl, etc.); R^{6'} is a hydrogen atom or a C₁₋₆ alkyl group (e.g., ethyl, etc.); or
      R⁶ and R⁶' form, together with the nitrogen atom they are bonded to, a 5- to 7-membered saturated cyclic amino group (e.g., piperidino, morpholino, etc.) optionally condensed with C₆₋₁₄ aryl ring (e.g., benzene ring, etc.) optionally substituted by hydroxy group; and
      R⁷ is a C₆₋₁₄ aryl group (e.g., phenyl, etc.); or
      R¹⁰ and R¹¹ form, together with the nitrogen atom they are bonded to, a 5- to 7-membered saturated cyclic amino group (e.g., tetrahydropyrimidinyl, etc.) optionally substituted by oxo group,
(III) a pyridine-N-oxide-4-yl group substituted by C₆₋₁₄ aryl-carbonylamino group (e.g., phenylcarbonylamino, etc.) optionally substituted by C₁₋₆ alkylsulfonyl group (e.g., methylsulfonyl, etc.), or
(IV) a pyrimidin-4-yl group substituted by substituent(s) selected from (1) amino group, (2) C₆₋₁₄ aryl-carbonylamino group (e.g., phenylcarbonylamino, etc.) wherein the aryl moiety is optionally substituted by C₁₋₆ alkyl group (e.g., methyl, etc.) or C₁₋₆ alkoxy group (e.g., methoxy, etc.), and (3) 5- to 10-membered heterocyclic group-carbonylamino group (e.g., pyridylcarbonylamino, etc.);
   X¹ is a nitrogen atom;
   X² is CH optionally substituted by the substituent(s) selected from
   (a) halogen atom (e.g., chlorine atom, etc.),
   (b) C₁₋₆ alkylthio group (e.g., methylthio, etc.),
   (c) C₁₋₆ alkylsulfonyl group (e.g., methylsulfonyl, etc.),
   (d) C₁₋₆ alkoxy group (e.g., methoxy, etc.), or
   (e) the formula 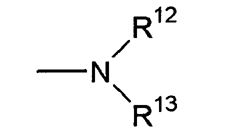 wherein R¹² and R¹³ are each
      (i) a hydrogen atom,
      (ii) a C₁₋₆ alkyl group (e.g., methyl, etc.),
      (iii) a C₇₋₁₆ aralkyl group (e.g., benzyl, etc.), or
      (iv) a C₃₋₁₀ cycloalkyl group (e.g., cyclohexyl, etc.);
   X³ is CH optionally substituted by C₁₋₆ alkyl group (e.g., tert-butyl, etc.); and
   X⁴ is CH optionally substituted by C₁₋₆ alkyl group (e.g., methyl, etc.), C₁₋₆ alkoxy group (e.g., methoxy, etc.), C₁₋₆ alkylthio group (e.g., methylthio, etc.) or C₁₋₆ alkylsulfinyl group (e.g., methylsulfinyl, etc.).

### (C) Compound (I) wherein

R¹ is a phenyl group optionally substituted by substituent(s) selected from halogen atom (e.g., chlorine atom, fluorine atom) and C₁₋₆ alkyl group (e.g., methyl group);
R² is a pyridin-4-yl group substituted by substituent(s) represented by the formula 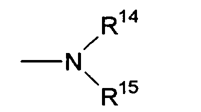 wherein R¹⁴ and R¹⁵ are each a group selected from
(a) a hydrogen atom,
(b) a C₁₋₆ alkyl group optionally substituted by substituent(s) selected from
   (i) C₃₋₁₀ cycloalkyl group (e.g., cyclopropyl, cyclobutyl, cyclopentyl, cyclohexyl, etc.), and
   (ii) 5- to 10-membered (preferably 5- or 6-membered) heterocyclic group (e.g., furyl, thienyl, tetrahydrofuranyl, etc.) (e.g., methyl, ethyl, propyl, butyl, isopentyl, 1-ethylpropyl, cyclopropylmethyl, methoxyethyl, thienylmethyl, furylmethyl, tetrahydrofuranylmethyl, etc.),
(c) a C₇₋₁₆ aralkyl group (e.g., benzyl, 1-phenylethyl, etc.),
(d) a C₃₋₁₀ cycloalkyl group (e.g., cyclopropyl, cyclobutyl, cyclopentyl, cyclohexyl, etc.) optionally condensed with C₆₋₁₄ aryl ring (e.g., benzene ring),
(e) a C₆₋₁₄ aryl group (e.g., phenyl, etc.) optionally substituted by group(s) selected from halogen atom (e.g., fluorine atom, etc.), C₁₋₆ alkyl group (e.g., methyl, etc.) and C₁₋₆ alkoxy group (e.g., methoxy, etc.), and
(f) an acyl group represented by the formula -(C=O)-R⁵ or - (C=O)-NR⁶R^{6'}
   wherein
   R⁵ is
   (i) C₆₋₁₄ aryl group (e.g., phenyl, etc.) optionally substituted by group(s) selected from halogen atom (e.g., chlorine, fluorine, etc.), C₁₋₆ alkyl group (e.g., methyl, t-butyl, etc.) and C₁₋₆ alkoxy group (e.g., methoxy, etc.), or
   (ii) a 5- to 10-membered heterocyclic group optionally substituted by substituent(s) selected from halogen atom (e.g., chlorine, fluorine, etc.) and C₁₋₆ alkyl group (e.g., methyl group, etc.) optionally substituted by halogen atom (e.g., chlorine, fluorine, etc.),
   R⁶ is
   (i) a C₆₋₁₄ aryl group (e.g., phenyl, etc.) optionally substituted by substituent(s) selected from halogen atom (e.g., chlorine, etc.), C₁₋₆ alkyl group (e.g., methyl, etc.) and C₁₋₆ alkoxy group (e.g., methoxy, etc.), or
   (ii) a C₁₋₆ alkyl group (e.g., methyl group, ethyl group, propyl group, butyl group, isopropyl group, tert-butyl group, etc.) optionally substituted by substituent(s) selected from hydroxy group, C₁₋₆ alkylsulfonyl group (e.g., methylsulfonyl group), oxo group, cyano group and halogen atom (e.g., fluorine, chlorine, etc.),
      R^{6'} is a hydrogen atom or a C₁₋₆ alkyl group (e.g., ethyl, etc.), or
      R⁶ and R^{6'} form, together with the nitrogen atom they are bonded to, a 5- to 7-membered saturated cyclic amino group (e.g., piperidino, morpholino, etc.) optionally condensed with C₆₋₁₄ aryl ring (e.g., benzene ring, etc.);
      X¹ is a nitrogen atom;
      X² is CH optionally substituted by substituent(s) represented by the formula 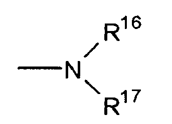
   wherein R¹⁶ and R¹⁷ are each a hydrogen atom or a C₁₋₆ alkyl group; X³ is CH; and
   X⁴ is CH.

### (D) Compound (I) produced in Examples 1-64.

### (E) 4-[6-chloro-2-(3-methylphenyl)imidazo[1,2-b]pyridazin-3-yl]-N-cyclohexylpyridin-2-amine (Example compound 2)

4-[6-chloro-2-(3-methylphenyl)imidazo[1,2-b]pyridazin-3-yl]-N-(4-fluoro-2-methylphenyl)pyridin-2-amine (Example compound 3-1)
4-[6-chloro-2-(3-methylphenyl)imidazo[1,2-b]pyridazin-3-yl]-N-(2-methylphenyl)pyridin-2-amine (Example compound 3-2)
N-(cyclopropylmethyl)-4-[2-(3-methylphenyl)imidazo[1,2-b]pyridazin-3-yl]pyridin-2-amine (Example compound 5)
N-cyclohexyl-4-[2-(3-methylphenyl)imidazo[1,2-b]pyridazin-3-yl]pyridin-2-amine (Example compound 6-1)
N-cyclopentyl-4-[2-(3-methylphenyl)imidazo[1,2-b]pyridazin-3-yl]pyridin-2-amine (Example compound 6-2)
N-(2-methylphenyl)-4-[2-(3-methylphenyl)imidazo[1,2-b]pyridazin-3-yl]pyridin-2-amine (Example compound 6-10) 4-[2-(3-methylphenyl)imidazo[1,2-b]pyridazin-3-yl]-N-(tetrahydrofuran-2-ylmethyl)pyridin-2-amine (Example compound 6-16)
N-(2,2-dimethylpropyl)-4-[2-(3-methylphenyl)imidazo[1,2-b]pyridazin-3-yl]pyridin-2-amine (Example compound 6-18)
N-(1-ethylpropyl)-4-[2-(3-methylphenyl)imidazo[1,2-b]pyridazin-3-yl]pyridin-2-amine (Example compound 6-20)
3-[2-(cyclohexylamino)pyridin-4-yl]-N,N-dimethyl-2-(3-methylphenyl)imidazo[1,2-b]pyridazin-6-amine (Example compound 7)
3-[2-(cyclohexylamino)pyridin-4-yl]-N-methyl-2-(3-methylphenyl)imidazo[1,2-b]pyridazin-6-amine (Example compound 8-1)
4-methyl-N-{4-[2-(3-methylphenyl)imidazo[1,2-b]pyridazin-3-yl]pyridin-2-yl}benzamide (Example compound 20-1)
4-methoxy-N-{4-[2-(3-methylphenyl)imidazo[1,2-b]pyridazin-3-yl]pyridin-2-yl}benzamide (Example compound 20-2)
N-{4-[2-(3-methylphenyl)imidazo[1,2-b]pyridazin-3-yl]pyridin-2-yl}cyclopropanecarboxamide (Example compound 20-10)
4-fluoro-N-{4-[2-(3-methylphenyl)imidazo[1,2-b]pyridazin-3-yl]pyridin-2-yl}benzamide (Example compound 20-11)
N-{4-[2-(4-fluoro-3-methylphenyl)imidazo[1,2-b]pyridazin-3-yl]pyridin-2-yl}-4-methylbenzamide trifluoroacetate (Example compound 21-15)
N-{4-[2-(4-fluoro-3-methylphenyl)imidazo[1,2-b]pyridazin-3-yl]pyridin-2-yl}-4-methoxybenzamide (Example compound 21-16)
N-{4-[2-(4-fluoro-3-methylphenyl)imidazo[1,2-b]pyridazin-3-yl]pyridin-2-yl}cyclopropanecarboxamide trifluoroacetate (Example compound 21-19)
N-ethyl-N'-{4-[2-(4-fluoro-3-methylphenyl)imidazo[1,2-b]pyridazin-3-yl]pyridin-2-yl}urea (Example compound 28-3)
N-{4-[2-(4-fluoro-3-methylphenyl)imidazo[1,2-b]pyridazin-3-yl]pyridin-2-yl}-N'-isopropylurea (Example compound 28-5)
N-{4-[2-(4-fluoro-3-methylphenyl)imidazo[1,2-b]pyridazin-3-yl]pyridin-2-yl}-4-methoxybenzamide (Example compound 39-3) 4-fluoro-N-{4-[2-(4-fluoro-3-methylphenyl)imidazo[1,2-b]pyridazin-3-yl]pyridin-2-yl}benzamide (Example compound 41-1)
N-{4-[2-(4-fluoro-3-methylphenyl)imidazo[1,2-b]pyridazin-3-yl]pyridin-2-yl}cyclopropanecarboxamide (Example compound 42-3)
N-{4-[2-(4-fluoro-3-methylphenyl)imidazo[1,2-b]pyridazin-3-yl]pyridin-2-yl}isonicotinamide (Example compound 42-23)
6-chloro-N-{4-[2-(4-fluoro-3-methylphenyl)imidazo[1,2-b]pyridazin-3-yl]pyridin-2-yl}nicotinamide (Example compound 42-24)
N-{4-[2-(4-fluoro-3-methylphenyl)imidazo[1,2-b]pyridazin-3-yl]pyridin-2-yl}-6-methylnicotinamide (Example compound 42-27)
N-{4-[2-(4-fluoro-3-methylphenyl)imidazo[1,2-b]pyridazin-3-yl]pyridin-2-yl}-1,3-benzothiazole-6-carboxamide (Example compound 42-35)
N-{4-[2-(4-fluoro-3-methylphenyl)imidazo[1,2-b]pyridazin-3-yl]pyridin-2-yl}-6-quinolinecarboxamide (Example compound 42-36)
N-{4-[2-(4-fluoro-3-methylphenyl)imidazo[1,2-b]pyridazin-3-yl]pyridin-2-yl}-N'-(2-hydroxyethyl)urea (Example compound 51-11)
N-(2-cyanoethyl)-N'-{4-[2-(4-fluoro-3-methylphenyl)imidazo[1,2-b]pyridazin-3-yl]pyridin-2-yl}urea (Example compound 51-14)
N-{4-[2-(4-fluoro-3-methylphenyl)imidazo[1,2-b]pyridazin-3-yl]pyridin-2-yl}-2-methylisonicotinamide (Example compound 58)

### (F)

4-methyl-N-{4-[2-(3-methylphenyl)imidazo[1,2-b]pyridazin-3-yl]pyridin-2-yl}benzamide (Example compound 20-1)
N-{4-[2-(3-methylphenyl)imidazo[1,2-b]pyridazin-3-yl]pyridin-2-yl}cyclopropanecarboxamide (Example compound 20-10)
4-fluoro-N-{4-[2-(3-methylphenyl)imidazo[1,2-b]pyridazin-3-yl]pyridin-2-yl}benzamide (Example compound 20-11)
N-{4-[2-(4-fluoro-3-methylphenyl)imidazo[1,2-b]pyridazin-3-yl]pyridin-2-yl}-4-methoxybenzamide (Example compound 21-16)
N-ethyl-N'-{4-[2-(4-fluoro-3-methylphenyl)imidazo[1,2-b]pyridazin-3-yl]pyridin-2-yl}urea (Example compound 28-3) 4-fluoro-N-{4-[2-(4-fluoro-3-methylphenyl)imidazo[1,2-b]pyridazin-3-yl]pyridin-2-yl}benzamide (Example compound 41-1)
N-{4-[2-(4-fluoro-3-methylphenyl)imidazo[1,2-b]pyridazin-3-yl]pyridin-2-yl}cyclopropanecarboxamide (Example compound 42-3)
N-{4-[2-(4-fluoro-3-methylphenyl)imidazo[1,2-b]pyridazin-3-yl]pyridin-2-yl}-2-(methylthio)acetamide (Example compound 42-6)
N-{4-[2-(4-fluoro-3-methylphenyl)imidazo[1,2-b]pyridazin-3-yl]pyridin-2-yl}isonicotinamide (Example compound 42-23)
N-{4-[2-(4-fluoro-3-methylphenyl)imidazo[1,2-b]pyridazin-3-yl]pyridin-2-yl}-6-methylnicotinamide (Example compound 42-27)
N-{4-[2-(4-fluoro-3-methylphenyl)imidazo[1,2-b]pyridazin-3-yl]pyridin-2-yl}-1,3-benzothiazole-6-carboxamide (Example compound 42-35)
N-{4-[2-(4-fluoro-3-methylphenyl)imidazo[1,2-b]pyridazin-3-yl]pyridin-2-yl}-6-quinolinecarboxamide (Example compound 42-36)
N-{4-[2-(4-fluoro-3-methylphenyl)imidazo[1,2-b]pyridazin-3-yl]pyridin-2-yl}-N'-(2-hydroxyethyl)urea (Example compound 51-11)
N-(2-cyanoethyl)-N'-{4-[2-(4-fluoro-3-methylphenyl)imidazo[1,2-b]pyridazin-3-yl]pyridin-2-yl}urea (Example compound 51-14)
N-{4-[2-(4-fluoro-3-methylphenyl)imidazo[1,2-b]pyridazin-3-yl]pyridin-2-yl}-2-methylisonicotinamide (Example compound 58)
N-{4-[2-(4-fluoro-3-methylphenyl)imidazo[1,2-b]pyridazin-3-yl]pyridin-2-yl}nicotinamide (Example compound 59)

As preferable examples of the compound (I), compound (I)
wherein
R¹ is an optionally substituted phenyl group;
R² is an optionally substituted pyridin-4-yl group;
X¹ is a nitrogen atom; and
x² and X³ are each an optionally substituted CH
can be also mentioned.

As the salt of compound (I) or (I'), for example, a metal salt, an ammonium salt, a salt with an organic base, a salt with an inorganic acid, a salt with an organic acid, a salt with a basic amino acid or an acidic amino acid, etc. As a suitable example of the metal salt, for example, alkali metal salts such as sodium salt, potassium salt and the like; alkaline earth metal salts such as calcium salt, magnesium salt, barium salt and the like; aluminum salt and the like can be mentioned. As a suitable example of the salt with an organic base, for example, salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc., and the like can be mentioned. As a suitable example of the salt with an inorganic acid, for example, salts with hydrochloric acid, hydrobromic acid, nitric acid, sulfuric acid, phosphoric acid, etc., and the like can be mentioned. As a suitable example of the salt with an organic acid, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc., and the like can be mentioned. As a suitable example of the salt with a basic amino acid, for example, salts with arginine, lysine, ornithine, etc., and the like can be mentioned. As a suitable example of the salt with an acidic amino acid, for example, salts with aspartic acid, glutamic acid, etc., and the like can be mentioned.

Of these, pharmaceutically acceptable salts are preferable. For example, when a compound has an acidic functional group therein, inorganic salts such as alkali metal salts (e.g., sodium salt, potassium salt and the like), alkaline earth metal salts (e.g., calcium salt, magnesium salt, barium salt and the like), and the like, ammonium salts and the like can be mentioned, and when a compound has a basic functional group therein, salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like can be mentioned.

Hereinafter a typical production method of compound (I) is explained as an example of the production method for compounds (I) and (I'), but the production method is not limited to the method. Compounds (Ia), (Ib), (Ic), (Id), (Ie), (If), (Ig), (Ih), (Ii), (Ij), (Ik) and (Im) are encompassed in the compound (I). Furthermore, compound (I') can be produced with referring to the production method of compound (I).

Compound (I) is obtained by the methods shown in the following Reaction Schemes 1, 2, 3, 4, 5 and 6 or a method analogous thereto and the like.

The compounds in Reaction Schemes 1, 2, 3, 4, 5 and 6 may form a salt, and as the salt, for example, those similar to salts of Compound (I) and the like can be mentioned.

For Compounds (IV), (V), (VI), (VII), (VIII), (IX) and (X), commercially available compounds can be used, or they can be produced according to a method known per se or a method analogous thereto. 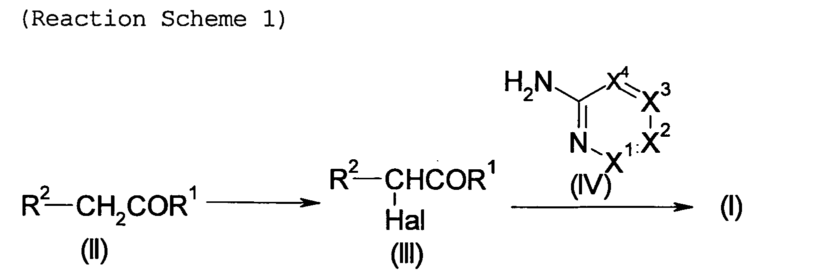 In the Reaction Scheme 1, Hal is a halogen atom, and R¹, R², X¹, X², X³ and X⁴ are each as defined for the formula [I].

Compound (II) and (III) can be produced, for example, according to the methods described in WO00/64894, WO01/10865 and WO01/74811, or a method known per se, or a method analogous thereto.

Compound (III) can be obtained by treating compound (II) with halogen. Where desired, this reaction is carried out in the presence of a base or a basic salt.

The amount of halogen to be used is about 1.0 mol to about 5.0 mol, preferably about 1.0 mol to about 2.0 mol relative to 1 mol of compound (II).

As the "halogen", bromine, chlorine, iodine and the like can be mentioned.

The amount of the base to be used is about 1.0 mol to about 10.0 mol, preferably about 1.0 mol to about 3.0 mol, per 1 mol of compound (II).

As the "base", for example, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

The amount of the basic salt to be used is about 1.0 mol to about 10.0 mol, preferably about 1.0 mol to about 3.0 mol, per 1 mol of compound (II).

As the "basic salt", for example, sodium carbonate, potassium carbonate, cesium carbonate, sodium hydrogen carbonate, sodium acetate, potassium acetate and the like can be mentioned.

It is advantageous to carry out this reaction without a solvent or in the presence of an inert solvent for the reaction. The solvent is not particularly limited as long as the reaction proceeds and, for example, ethers, aromatic hydrocarbons, aliphatic hydrocarbons, amides, halogenated hydrocarbons, nitriles, sulfoxides, organic acids, aromatic amines or a mixture of two or more of them and the like are used.

The reaction temperature is generally about -20°C to about 150°C, preferably about 0°C to about 100°C.

The reaction time is generally about 5 minutes to about 24 hours, preferably about 10 minutes to about 14 hours.

Although the product can be used as a reaction solution itself or as a crude product in the next reaction, it can be isolated from the reaction mixture by a conventional method, and can be easily purified by a separation method such as recrystallization, distillation, chromatography and the like.

Compound (I) is obtained by condensation of compound (III) with compound (IV). Where desired, this reaction is carried out in the presence of a base.

Where compound (IV) is commercially available, a commercially available product can be used. Alternatively, compound (IV) can be obtained by a method known per se or a method analogous thereto and the like.

The amount of compound (IV) to be used is about 0.5 mol to about 5.0 mol, preferably about 0.8 mol to about 1.5 mol, per 1 mol of compound (III).

The amount of the base to be used is about 1 mol to about 30 mol, preferably about 1 mol to about 5 mol, per 1 mol of compound (III).

As the "base", for example, basic salts such as sodium hydroxide, potassium hydroxide, lithium hydroxide, sodium carbonate, potassium carbonate, cesium carbonate, sodium hydrogen carbonate, disodium hydrogenphosphate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds and, for example, halogenated hydrocarbons, aliphatic hydrocarbons, aromatic hydrocarbons, ethers, amides, alcohols, nitriles or mixtures of two or more of them and the like are used.

The reaction temperature is about -5°C to about 200°C, preferably about 5°C to about 150°C.

The reaction time is generally about 0.5 hour to about 120 hours, preferably about 2 hours to about 72 hours.

Although the product can be used as a reaction solution itself or as a crude product in the next reaction, it can be isolated from the reaction mixture by a conventional method, and can be easily purified by a separation method such as recrystallization, distillation, chromatography and the like.

Compounds (Ib) and (In), in which R³¹ is an acyl group, are obtained by the method shown in Reaction Scheme 2 or a method analogous thereto. 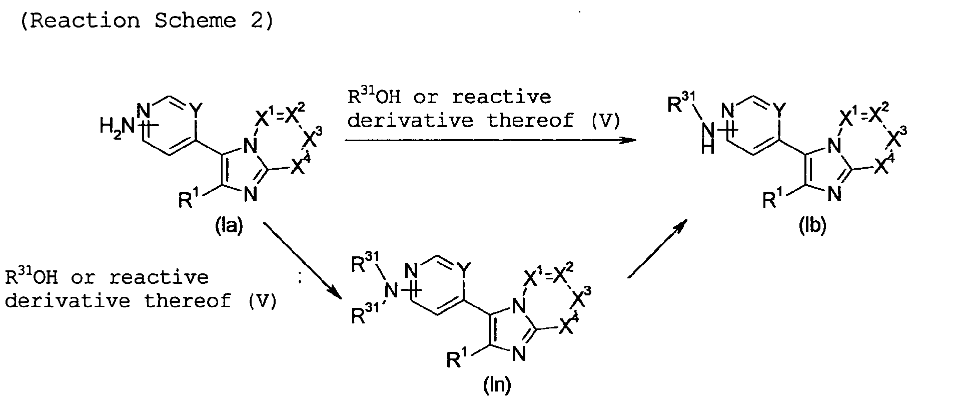 In the Reaction Scheme 2, R³¹ is an optionally substituted acyl group, Y is an optionally substituted CH or a nitrogen atom, and R¹, X¹, X², X³ and X⁴ are each as defined for the formula [I].

Compound (Ib) is obtained by reacting the corresponding amine (Ia) and compound (V), where desired, in the presence of a base or an acid.

As the acid R³¹OH (wherein R³¹ is as defined above) or its reactive derivative compound (V), for example, acid halide, acid anhydride, ester and the like can be mentioned.

As the "optionally substituted acyl group" represented by R³¹, for example, those similar to the "optionally substituted acyl group" represented by R³ or R⁴ can be mentioned.

As the "optionally substituted CH or nitrogen atom" represented by Y, for example, the "optionally substituted CH or nitrogen atom" represented by X¹, X² and/or X³ can be mentioned.

The amount of compound (V) to be used is about 1 mol to about 5 mol, preferably about 1 mol to about 2 mol, per 1 mol of the corresponding amine (Ia).

The amount of the base or the acid to be used is about 0.8 mol to about 5 mol, preferably about 1 mol to about 2 mol, per 1 mol of the corresponding amine (Ia).

As the "base", for example, triethylamine, pyridine, 4-dimethylaminopyridine and the like can be mentioned.

As the "acid", for example, methanesulfonic acid, p-toluenesulfonic acid, camphorsulfonic acid and the like can be mentioned.

It is advantageous to carry out this reaction without a solvent or in the presence of an inert solvent for the reaction. The solvent is not particularly limited as long as the reaction proceeds and, for example, ethers, aromatic hydrocarbons, aliphatic hydrocarbons, amides, halogenated hydrocarbons, nitriles, sulfoxides, aromatic amines or mixtures of two or more of them and the like are used.

The reaction temperature is about -20°C to about 150°C, preferably about 0°C to about 100°C.

The reaction time is generally about 5 minutes to about 24 hours, preferably about 10 minutes to about 5 hours.

Although the product can be used as a reaction solution itself or as a crude product in the next reaction, it can be isolated from the reaction mixture by a conventional method, and can be easily purified by a separating method such as recrystallization, distillation, chromatography and the like.

Alternatively, compound (Ib) is obtained by reacting the below-mentioned compound (In), where desired, in the presence of a base or an acid.

The amount of the base or the acid to be used is about 1 mol to about 100 mol, preferably about 2 mol to about 50 mol, per 1 mol of the corresponding compound (In).

As the "base", for example, primary and secondary amines such as ammonia, methylamine, ethylamine, dimethylamine, diethylamine, piperidine, morpholine and the like, and the like can be mentioned.

As the "acid", for example, inorganic acids such as hydrochloric acid, hydrobromic acid, sulfate, nitric acid and the like, and the like can be mentioned.

It is advantageous to carry out this reaction without a solvent or in the presence of an inert solvent for the reaction. The solvent is not particularly limited as long as the reaction proceeds and, for example, ethers, aromatic hydrocarbons, aliphatic hydrocarbons, amides, halogenated hydrocarbons, nitriles, sulfoxides, alcohols, water or mixtures of two or more of them and the like are used.

The reaction temperature is about -20°C to about 150°C, preferably about 0°C to about 100°C.

The reaction time is generally about 5 minutes to about 48 hours, preferably about 10 minutes to about 14 hours.

Although the product can be used as a reaction solution itself or as a crude product in the next reaction, it can be isolated from the reaction mixture by a conventional method, and can be easily purified by a separating method such as recrystallization, distillation, chromatography and the like.

Compound (In) is obtained by reacting the corresponding amine (Ia) and compound (V), where desired, in the presence of a base or an acid.

As the acid R³¹OH (wherein R³¹ is as defined above.) or its reactive derivative compound (V), for example, acid halide, acid anhydride, ester and the like can be mentioned.

As the "optionally substituted acyl group"represented by R³¹, for example, those similar to the "optionally substituted acyl group" represented by R³ or R⁴ can be mentioned.

As the "optionally substituted CH or nitrogen atom" represented by Y, for example, the "optionally substituted CH or nitrogen atom" represented by X¹, X² and/or X³ can be mentioned.

The amount of compound (V) to be used is about 1 mol to about 10 mol, preferably about 2 mol to about 5 mol, per 1 mol of the corresponding amine (Ia).

The amount of the base or the acid to be used is about 0.8 mol to about 10 mol, preferably about 2 mol to about 5 mol, per 1 mol of the corresponding amine (Ia).

As the "base", for example, triethylamine, pyridine, 4-dimethylaminopyridine and the like can be mentioned.

As the "acid", for example, methanesulfonic acid, p-toluenesulfonic acid, camphorsulfonic acid and the like can be mentioned.

It is advantageous to carry out this reaction without a solvent or in the presence of an inert solvent for the reaction. The solvent is not particularly limited as long as the reaction proceeds and, for example, ethers, aromatic hydrocarbons, aliphatic hydrocarbons, amides, halogenated hydrocarbons, nitriles, sulfoxides, aromatic amines or mixtures of two or more of them and the like are used.

The reaction temperature is about -20°C to about 150°C, preferably about 0°C to about 100°C.

The reaction time is generally about 5 minutes to about 48 hours, preferably about 10 minutes to about 24 hours.

Although the resultant product can be used as a reaction solution itself or as a crude product in the next reaction, it can be isolated from the reaction mixture according to a conventional method, and can be easily purified by a separation method such as recrystallization, distillation, chromatography and the like.

Compound (Id) is obtained by the method shown in Reaction Scheme 3 or a method analogous thereto. 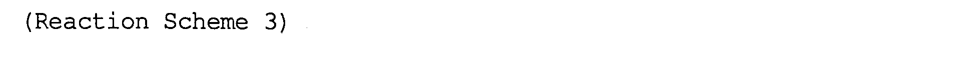 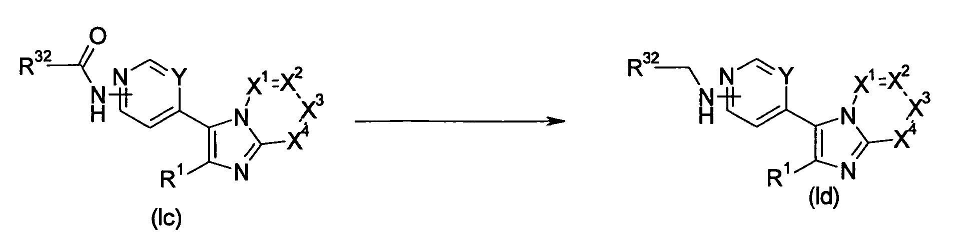 In Reaction Scheme 3, R³² is a hydrogen atom, an optionally substituted hydrocarbon group or an optionally substituted heterocyclic group, Y is as defined for Reaction Scheme 2, and R¹, X¹, X², X³ and X⁴ are each as defined for the formula [I].

Compound (Id) is obtained by reducing the amide group of compound (Ic) with a reducing agent.

As the "optionally substituted hydrocarbon group" represented by R³², for example, those similar to the "optionally substituted hydrocarbon group" in the Substituent A group can be mentioned.

As the "optionally substituted heterocyclic group" represented by R³², for example, those similar to the "optionally substituted heterocyclic group" for R¹ can be mentioned.

The amount of the reducing agent to be used is about 1.0 mol to about 5.0 mol, preferably about 1.0 mol to about 3.0 mol, per 1 mol of compound (Ic).

As the "reducing agent", for example, metal hydrides such as aluminum hydride, diisobutyl aluminum hydride and the like, metal hydrogen complex compounds such as aluminum lithium hydride, sodium borohydride and the like, borane complexes such as borane tetrahydrofuran complex, borane dimethylsulfide complex and the like, alkylboranes such as thexylborane, disiamylborane and the like, and the like can be mentioned.

In this reaction, where desired, an acid can be added with the reducing agent.

The amount of the acid to be used is about 0.8 mol to about 5.0 mol, preferably about 1.0 mol to about 3.0 mol, per 1 mol of compound (Ic).

As the "acid", for example, Lewis acids such as aluminum chloride and the like can be mentioned.

It is advantageous to carry out this reaction without a solvent or in the presence of an inert solvent for the reaction. The solvent is not particularly limited as long as the reaction proceeds and, for example, ethers, aromatic hydrocarbons, aliphatic hydrocarbons, halogenated hydrocarbons or mixtures of two or more of them and the like are used.

The reaction temperature is about -78°C to about 150°C, preferably about 0°C to about 100°C. The reaction time is generally about 5 minutes to about 24 hours, preferably about 10 minutes to about 5 hours.

Although the product can be used as a reaction solution itself or as a crude product in the next reaction, it can be isolated from the reaction mixture by a conventional method, and can be easily purified by a separating method such as recrystallization, distillation, chromatography and the like.

Compound (If) is also obtained according to the method shown in the following Reaction Scheme 4. 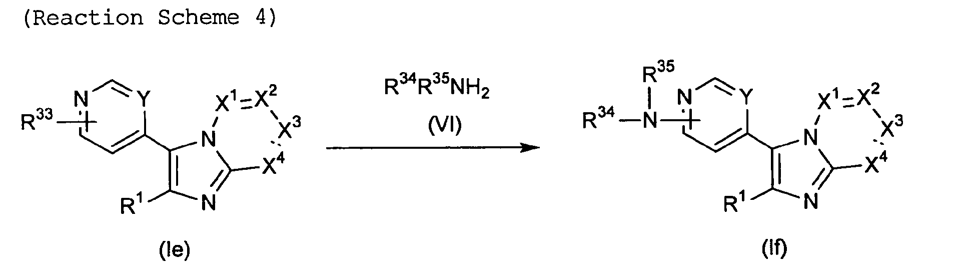 In Reaction Scheme 4, R³³ is a leaving group, R³⁴ and R³⁵ are each a hydrogen atom, an optionally substituted hydrocarbon group, an optionally substituted heterocyclic group or an optionally substituted acyl group, Y is as defined for Reaction Scheme 2, and R¹, X¹, X², X³ and X⁴ are each as defined for the formula [I].

Compound (If) is obtained by condensation of compound (Ie) with an amine R³⁴R³⁵ NH (VI) (wherein R³⁴ and R³⁵ are each as defined above), where desired, in the presence of a base.

In compound (Ie), as the "leaving group" represented by R³³, for example, a halogen atom, an optionally halogenated alkylsulfonyl group, an optionally substituted arylsulfonyl group and the like can be mentioned. As the "halogen atom", for example, fluorine, chlorine, bromine, iodine and the like can be mentioned. As the "optionally halogenated alkylsulfonyl group", for example, methanesulfonyl group, trifluoromethanesulfonyl group and the like can be mentioned. As the "optionally substituted arylsulfonyl group", for example, phenylsulfonyl group, 4-methylphenylsulfonyl group and the like can be mentioned.

In compound (VI) and (If), as the "optionally substituted hydrocarbon group", "optionally substituted heterocyclic group" and "optionally substituted acyl group" each as represented by R³⁴ and R³⁵, for example, those represented as the "optionally substituted hydrocarbon group", "optionally substituted heterocyclic group" and "optionally substituted acyl group" for R³ and R⁴, and the like can be mentioned, and preferably, the substituents each as exemplified above for R¹⁰ and R¹¹, and the like can be mentioned.

The amount of the amine (VI) to be used is about 1.0 mol to about 100.0 mol, preferably about 1 mol to about 50 mol, per 1 mol of compound (Ie).

The amount of the base to be used is about 0.1 mol to about 50 mol, preferably about 1 mol to about 20 mol, per 1 mol of compound (Ie).

As the "base", for example, basic salts such as sodium carbonate, potassium carbonate and the like, metal alkoxides such as sodium methoxide, sodium ethoxide, potassium tert-butoxide and the like, organic bases such as aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like, and the like can be used.

It is advantageous to carry out this reaction without a solvent or in the presence of an inert solvent for the reaction. The solvent is not particularly limited as long as the reaction proceeds and, for example, alcohols, ethers, aromatic hydrocarbons, aliphatic hydrocarbons, halogenated hydrocarbons, sulfoxides, water or mixtures of two or more of them and the like are used.

The reaction time is generally about 10 minutes to about 50 hours, preferably about 30 minutes to about 18 hours.

The reaction temperature is about 0°C to about 300°C, preferably about 20°C to about 250°C.

Although the product can be used as a reaction solution itself or as a crude product in the next reaction, it can be isolated from the reaction mixture by a conventional method, and can be easily purified by a separating method such as recrystallization, distillation, chromatography and the like.

Compound (Ig) and (Ii) are obtained by the methods shown in the following Reaction Scheme 5. 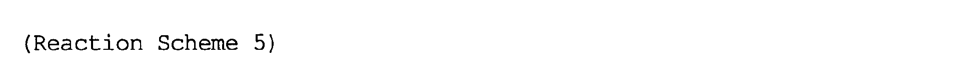 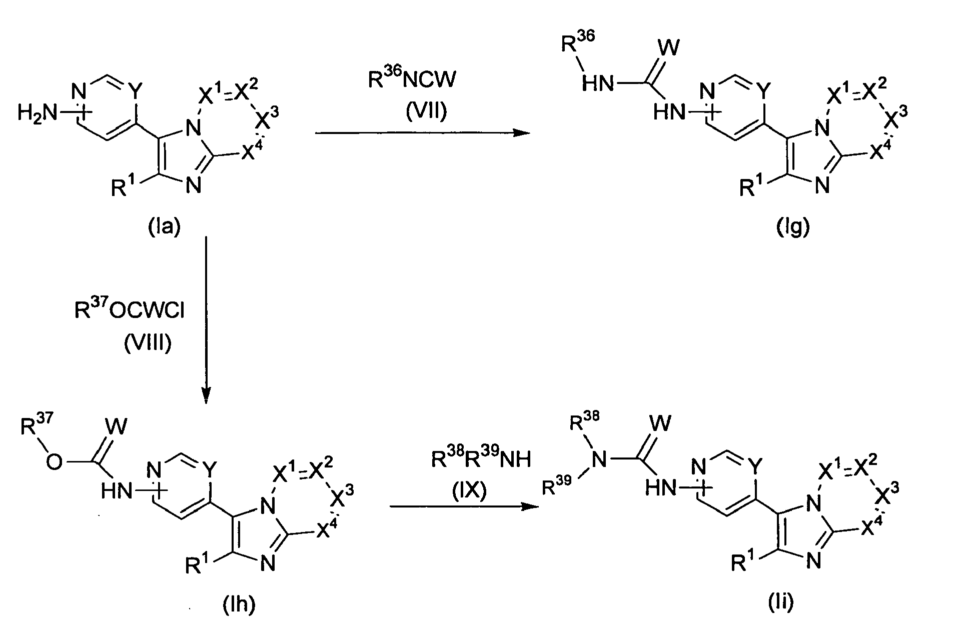 In Reaction Scheme 5, R³⁶ is an optionally substituted
hydrocarbon group or a heterocyclic group optionally having substituents, R³⁷ is a C₁₋₃ alkyl group substituted by halogen atoms, R³⁸ and R³⁹ are each a hydrogen atom or an optionally substituted hydrocarbon, or R³⁸ and R³⁹ may form a ring with the nitrogen atom to which they are bonded, W is an oxygen atom or a sulfur atom, Y is as defined for Reaction Scheme 2, and R¹, X¹, X², X³ and X⁴ are as defined for the formula [I].

Compound (Ig) is obtained by condensation of compound (Ia) with compound (VII), where desired, in the presence of a base.

As the "optionally substituted hydrocarbon group" represented by R³⁶, for example, those exemplified as the "optionally substituted hydrocarbon group" in the Substituent A group, and the like can be mentioned. As the "optionally substituted heterocyclic group" represented by R³⁶, for example, the "optionally substituted heterocyclic group" exemplified as the "optionally substituted heterocyclic group" for R¹ and the like can be mentioned. As preferable R³⁶, for example, the substituents exemplified for R⁶, and the like can be mentioned.

As the "optionally substituted hydrocarbon" represented by R³⁸ and R³⁹, for example, the "optionally substituted hydrocarbon group" exemplified in the Substituent A group and the like can be mentioned, and preferably, the substituents as exemplified above for each of R⁶ and R^{6'}, and the like can be mentioned.

As the "ring" formed by R³⁸ and R³⁹ with the nitrogen atom to which they are bonded, for example, a 5- to 7-membered saturated cyclic amino group optionally condensed with a C₆₋₁₄ aryl group can be mentioned, and preferably, those as exemplified above as a ring formed by R⁶ and R⁶' with the nitrogen atom to which they are bonded (e.g., piperidinyl, 3,4-dihydroquinolyl, etc.) can be mentioned.

The amount of compound (VII) to be used is about 0.8 mol to about 50 mol, preferably about 2 mol to about 20 mol, per 1 mol of compound (Ia).

The amount of the base to be used is about 0.1 mol to about 3 mol, preferably about 0.3 mol to about 1.2 mol, per 1 mol of compound (Ia).

As the "base", for example, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds and, for example, aliphatic hydrocarbons, aromatic hydrocarbons, ethers, amides, sulfoxides or mixtures of two or more of them and the like are used.

The reaction temperature is generally about 0°C to about 200°C, preferably about 20°C to about 100°C.

The reaction time is generally about 8 hours to about 120 hours, preferably about 12 hours to about 72 hours.

Although the product can be used as a reaction solution itself or as a crude product in the next reaction, it can be isolated from the reaction mixture by a conventional method, and can be easily purified by a separating method such as recrystallization, distillation, chromatography and the like.

Compound (Ii) is obtained by reacting compound (Ih), which is obtained by treating compound (Ia) with compound (VIII), with an amine R³⁸R³⁹ NH₂ (IX) (wherein R³⁸ and R³⁹ are each as defined above).

In Reaction Scheme 5, as the "C₁₋₃ alkyl group substituted by halogen atoms" represented by R³⁷, for example, 2,2,2-trichloroethyl group and the like can be mentioned.

Compound (Ih) is obtained by condensation of compound (Ia) with compound (VIII), where desired, in the presence of a base.

The amount of compound (VIII) to be used is about 0.8 mol to about 2 mol, preferably about 0.8 mol to about 1.2 mol, per 1 mol of compound (Ia).

The amount of the base to be used is about 1 mol to about 10 mol, preferably about 1 mol to about 3 mol, per 1 mol of compound (Ia).

As the "base", for example, alkali metals such as sodium hydroxide, potassium hydroxide, lithium hydroxide and the like, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds and, for example, ethers, esters, aromatic hydrocarbons, aliphatic hydrocarbons, amides, halogenated hydrocarbons, nitriles, sulfoxides, organic acids, aromatic amines or mixtures of two or more of them and the like are used.

The reaction temperature is about -20°C to about 150°C, preferably about 0°C to about 100°C. The reaction time is generally about 5 minutes to about 24 hours, preferably about 1 hour to about 14 hours.

Although the product can be used as a reaction solution itself or as a crude product in the next reaction, it can be isolated from the reaction mixture by a conventional method, and can be easily purified by a separation method such as recrystallization, distillation, chromatography and the like.

Compound (Ii) can be obtained by reacting compound (Ih) with an amine R³⁸R³⁹NH (IX) (wherein R³⁸ and R³⁹ are each as defined above). Where desired, this reaction is carried out in the presence of a base.

The amount of compound (IX) to be used is about 0.5 mol to about 3.0 mol, preferably about 0.8 mol to about 2 mol, per 1 mol of compound (Ih).

The amount of the base to be used is about 1 mol to about 3.0 mol, preferably about 1 mol to about 2 mol, per 1 mol of compound (Ih).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, N,N-diisopropylethylamine, 4-dimethylaminopyridine, N,N-dimethylaniline, N-methylpiperidine, N-methylpyrrolidine, N-methylmorpholine and the like, and the like can be mentioned.

It is advantageous to carry out this reaction without a solvent or in the presence of an inert solvent for the reaction. The solvent is not particularly limited as long as the reaction proceeds and, for example, halogenated hydrocarbons, aliphatic hydrocarbons, aromatic hydrocarbons, ethers, amides, sulfoxides, nitriles or mixtures of two or more of them and the like are used.

The reaction temperature is about -5°C to about 200°C, preferably about 5°C to about 150°C. The reaction time is generally about 5 minutes to about 72 hours, preferably about 1 hour to about 30 hours.

Although the product can be used as a reaction solution itself or as a crude product in the next reaction, it can be isolated from the reaction mixture by a conventional method, and can be easily purified by a separation method such as recrystallization, distillation, chromatography and the like.

Compound (Ik) and (Im) are obtained by the methods shown in the following Reaction Scheme 6. 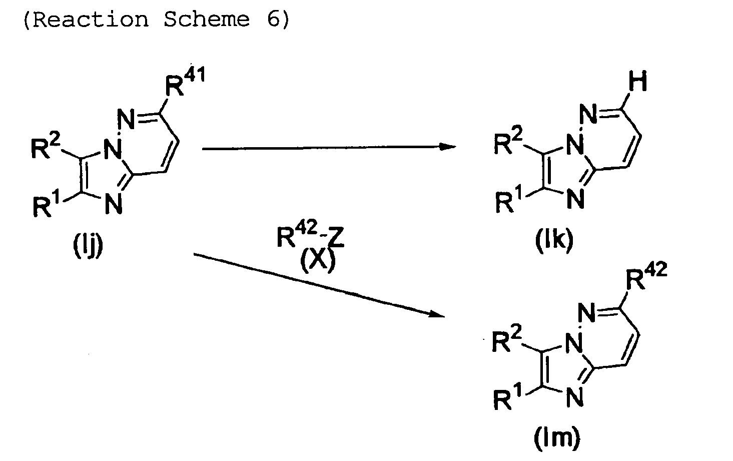 In Reaction Scheme 6, R⁴¹ is a leaving group, R⁴² is an optionally substituted amino group, a group represented by R^{m}S- (R^{m} is as defined above) or a group represented by R^{h}O- (R^{h} is as defined above), Z is hydrogen or a metal such as sodium, potassium and the like, and R¹ and R² are as defined for the formula [I].

In compound (Ij), the "leaving group" represented by R⁴¹ is a leaving group such as halogen atom and the like. As the halogen atom, for example, fluorine, chlorine, bromine, iodine atom and the like can be mentioned.

As the "optionally substituted amino group", "group represented by R^{m}S-" and "group represented by R^{h}O-" represented by R⁴², for example, the "optionally substituted amino group", "group represented by R^{m}S-" and "group represented by R^{h}O-" exemplified in the Substituent A group can be mentioned respectively.

Compound (Ik) is obtained by reducing compound (Ij).

As the reducing agent used for the reduction, for example, metal hydrides such as aluminum hydride, diisobutylaluminum hydride and the like, metal hydrogen complex compounds such as lithium aluminum hydride, sodium borohydride and the like, borane complexes such as borane tetrahydrofuran complex, borane dimethylsulfide complex and the like, alkylboranes such as thexylborane, disiamylborane and the like, diborane, or metals such as zinc, aluminum, tin, iron and the like, alkali metal such as sodium, lithium or the like/liquid ammonia (Birch reduction) and the like can be mentioned. As the hydrogenolysis catalyst, for example, catalysts such as palladium carbon, platinum oxide, Raney nickel, Raney cobalt and the like, and the like are used. The amount of the reducing agent to be used is, for example, about 1.0 mol to about 10 mol, preferably about 1.0 mol to about 3.0 mol, per 1 mol of compound (Ij), in the case where a metal hydride is used; about 1.0 mol to about 10 mol, preferably about 1.0 mol to about 3.0 mol, per 1 mol of compound (Ij), in the case where a metal hydrogen complex compound is used; about 1.0 mol to about 5.0 mol, per 1 mol of compound (Ij), in the case where a borane complex, an alkylborane or a diborane is used; about 1.0 equivalent amount to 20 equivalent amount, preferably about 1 equivalent amount to 5 equivalent amount in the case where a metal is used; about 1 equivalent amount to 20 equivalent amount, preferably about 1 equivalent amount to 5 equivalent amount, in the case where an alkali metal is used; and in the case where hydrogen addition is used, the amount of the catalyst such as palladium carbon, platinum oxide, Raney-nickel, Raney cobalt or the like is about 5 wt% to 1000 wt%, preferably about 10 wt% to about 300 wt% relative to compound (Ij).

It is advantageous to carry out this reaction without a solvent or in the presence of an inert solvent for the reaction. The solvent is not particularly limited as long as the reaction proceeds and, for example, alcohols, ethers, aliphatic hydrocarbons, aromatic hydrocarbons, amides, organic acids or a mixed solvent thereof and the like are used.

Reaction time varies depending on the kind and amount of the reducing agent to be used or the activity and amount of the catalyst, and is generally about 0.5 hr to about 30 hr, preferably about 1 hr to about 14 hr.

The reaction temperature is about 0°C to about 120°C, preferably about 20°C to about 80°C.

Where a hydrogenolysis catalyst is used, the pressure of hydrogen is generally about 1 atm to about 100 atm.

In this reaction, where desired, an acid or a base may be added with the reducing agent.

The amount of the acid to be used is about 0.8 mol to about 5.0 mol, preferably about 1.0 mol to about 3.0 mol, per 1 mol of compound (Ij).

As the "acid", for example, Lewis acids such as aluminum chloride and the like, organic acids such as acetic acid and the like, inorganic acids such as hydrochloric acid and the like can be mentioned.

The amount of the base to be used is about 1.0 mol to about 5.0 mol, preferably about 1.0 mol to about 3.0 mol, per 1 mol of compound (Ij).

As the "base", for example, basic salts such as sodium carbonate, potassium carbonate, cesium carbonate, sodium hydrogen carbonate, sodium acetate and the like, aromatic amines such as pyridine, lutidine and the like, tertiary amines such as triethylamine, tripropylamine, tributylamine, cyclohexyldimethylamine, N,N-diisopropylethylamine, 4-dimethylaminopyridine, N,N-dimethylaniline, N-methylpiperidine, N-methylpyrrolidine, N-methylmorpholine and the like, and the like can be mentioned.

Although the product can be used as a reaction solution itself or as a crude product in the next reaction, it can be isolated from the reaction mixture by a conventional method, and can be easily purified by a separating method such as recrystallization, distillation, chromatography and the like.

Compound (Im) can be obtained by condensing compound (Ij) with compound (X) (wherein R⁴² and Z are as defined above) optionally in the presence of a base.

The amount of compound (X) to be used is about 1 mol to about 100 mol, preferably about 2 mol to about 50 mol, per 1 mol of compound (Ij).

The amount of the base to be used is about 0.1 mol to about 50 mol, preferably about 1 mol to about 20 mol, per 1 mol of compound (Ij).

As the "base", for example, alkali metal hydrides such as sodium hydride, potassium hydride and the like, basic salts such as sodium carbonate, potassium carbonate and the like, metal alkoxides such as sodium methoxide, sodium ethoxide, potassium tert-butoxide and the like, organic bases such as aromatic amines such as pyridine, lutidine and the like, tertiary amines such as triethylamine, tripropylamine, tributylamine, cyclohexyldimethylamine, 4-dimethylaminopyridine, N,N-dimethylaniline, N-methylpiperidine, N-methylpyrrolidine, N-methylmorpholine and the like, and the like, and the like are used.

It is advantageous to carry out this reaction without a solvent or in the presence of an inert solvent for the reaction. The solvent is not particularly limited as long as the reaction proceeds and, for example, alcohols, ethers, aromatic hydrocarbons, aliphatic hydrocarbons, halogenated hydrocarbons, sulfoxides, water or mixtures of two or more of them and the like are used.

The reaction time is generally about 10 minutes to about 50 hours, preferably about 30 hours to about 24 hours.

The reaction temperature is generally about 0°C to about 300°C, preferably about 20°C to about 250°C.

Although the product can be used as a reaction solution itself or as a crude product in the next reaction, it can be isolated from the reaction mixture by a conventional method, and can be easily purified by a separating method such as recrystallization, distillation, chromatography and the like.

In the respective reactions mentioned above, when starting compounds have an amino group, a carboxy group, a hydroxy group as a substituent, a protecting group which is generally used in the peptide chemistry and the like may be introduced into these groups and, after reaction, an object compound can be obtained by removing protecting groups if needed.

As protecting groups for an amino group, for example, formyl or C₁₋₆ alkyl-carbonyl (e.g., acetyl, propionyl, etc.), phenylcarbonyl, C₁₋₆ alkoxy-carbonyl (e.g., methoxycarbonyl, ethoxycarbonyl, etc.), C₆₋₁₄ aryloxycarbonyl (e.g., phenyloxycarbonyl, etc.), C₇₋₁₆ aralkyloxy-carbonyl (e.g., benzyloxycarbonyl, etc.), trityl, phthaloyl and the like, each of which may have substituent(s), are used. As these substituents, halogen atoms (e.g., fluorine, chlorine, bromine, iodine and the like), C₁₋₆ alkyl-carbonyl (e.g., acetyl, propionyl, valeryl, etc.), nitro and the like are used, wherein the number of the substituents is 1 to 3.

As protecting group(s) for a carboxyl group, for example, C₁₋₆ alkyl (e.g., methyl, ethyl, propyl, isopropyl, butyl, tert-butyl and the like), phenyl, trityl, silyl and the like, each of which may have substituents, are used. As these substituents, halogen atom (e.g., fluorine, chlorine, bromine, iodine and the like), formyl, C₁₋₆ alkyl-carbonyl (e.g., acetyl, propionyl, butylcarbonyl and the like), nitro, C₁₋₆ alkyl (e.g., methyl, ethyl, tert-butyl and the like), C₆₋₁₄ aryl (e.g., phenyl, naphthyl and the like) and the like are used, wherein the number of substituents is 1 to 3.

As a protecting group for a hydroxy group, for example, C₁₋₆ alkyl (e.g., methyl, ethyl, propyl, isopropyl, butyl, tert-butyl, etc.), phenyl, C₇₋₁₆ aralkyl (e.g., benzyl, etc.), formyl, C₁₋₆ alkyl-carbonyl (e.g., acetyl, propionyl, etc.), C₆₋₁₄ aryloxycarbonyl (e.g., phenyloxycarbonyl, etc.), C₇₋₁₆ aralkyloxy-carbonyl (e.g., benzyloxycarbonyl, etc.), tetrahydropyranyl, tetrahydrofuranyl, silyl and the like, each of which may have substituents, are used. As these substituents, halogen atom (e.g., fluorine, chlorine, bromine, iodine, etc.), C₁₋₆ alkyl (e.g., methyl, ethyl, tert-butyl, etc.), C₇₋₁₆ aralkyl (e.g., benzyl, etc.), C₆₋₁₄ aryl (e.g., phenyl, naphthyl, etc.), nitro and the like are used, wherein the number of substituents is 1 to 4.

In addition, as a method of removing a protecting group, a method known *per* se or a method according to such method is used and, for example, method by treating with acid, base, ultraviolet rays, hydrazine, phenylhydrazine, sodium N-methyldithiocarbamate, tetrabutylammonium fluoride, palladium acetate and the like or a method of reduction is used.

In any case, when desired further, compound (I) or (I') can be synthesized by optionally applying further known deprotection, acylation, alkylation, hydrogenation, oxidation, reduction, carbon chain extension and substituent exchange reactions alone or a combination of two or more of them. As these reactions, those described in, for example, Shinjikkenkagakukoza 14, vol. 15, 1977 (Maruzen Press) and the like are adopted.

As the aforementioned "alcohols", for example, methanol, ethanol, propanol, isopropanol, tert-butanol and the like can be mentioned.

As the aforementioned "ethers", for example, diethyl ether, diisopropyl ether, diphenyl ether, tetrahydrofuran, dioxane, 1,2-dimethoxyethane and the like can be mentioned.

As the aforementioned "halogenated hydrocarbons", for example, dichloromethane, chloroform, 1,2-dichloroethane, carbon tetrachloride and the like can be mentioned.

As the aforementioned "aliphatic hydrocarbons", for example, hexane, pentane, cyclohexane and the like can be mentioned.

As the aforementioned "aromatic hydrocarbons", for example, benzene, toluene, xylene, chlorobenzene and the like can be mentioned.

As the aforementioned "aromatic amines", for example, pyridine, lutidine, quinoline and the like can be mentioned.

As the aforementioned "amides", for example, N,N-dimethylformamide, N,N-dimethylacetamide, hexamethylphosphoric triamide and the like can be mentioned.

As the aforementioned "ketones", for example, acetone, methylethyl ketone and the like can be mentioned.

As the aforementioned "sulfoxides", for example, dimethyl sulfoxide and the like can be mentioned.

As the aforementioned "nitriles", for example, acetonitrile, propionitrile and the like can be mentioned.

As the aforementioned "organic acids", for example, acetic acid, propionic acid, trifluoroacetic acid and the like can be mentioned.

When an object product is obtained in a free form by the above reaction, it may be converted into a salt according to conventional methods or, when an object product is obtained as a salt, it can be converted into a free form or another salt according to conventional methods. Compound (I) or (I') of the present invention thus obtained can be isolated and purified from the reaction solution by the known methods, for example, phase transfer, concentration, solvent extraction, fractional distillation, crystallization, recrystallization, chromatography and the like.

When compound (I) or (I') is present as a configurational isomer, diastereomer, conformer or the like, each can be optionally isolated, where desired, by the above separation and purification methods. In addition, when compound (I) or (I') is in the form of its racemate, they can be separated into S- and R-forms by any conventional optical resolution.

When compound (I) or (I') includes stereoisomers, each isomer and mixtures of respective isomers are encompassed in the scope of the present invention.

In addition, compound (I) or (I') may be a hydrate or non-hydrate.

Compound (I) or (I') may be labeled with an isotope (e.g., ³H, ¹⁴C, ³⁵S and the like) or the like.

A prodrug for compound (I) or (I') refers to a compound which is converted to compound (I) or (I') as a result of a reaction with an enzyme, gastric acid, etc. under physiological conditions in vivo, thus a compound that undergoes enzymatic oxidation, reduction, hydrolysis, etc. to convert into compound (I) or (I') and a compound that undergoes hydrolysis and the like by gastric acid, etc. to convert into compound (I) or (I'). As a prodrug for compound (I) or (I'), a compound obtained by subjecting an amino group in compound (I) or (I') to acylation, alkylation or phosphorylation (e.g., a compound obtained by subjecting an amino group in compound (I) or (I') to eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation, tert-butylation, etc.); a compound obtained by subjecting a hydroxy group in compound (I) or (I') to acylation, alkylation, phosphorylation and boration (e.g., a compound obtained by subjecting a hydroxy group in compound (I) or (I') to acetylation, palmitoylation, propanoylation, pivaloylation, succinylation, fumarylation, alanylation, dimethylaminomethylcarbonylation, etc.); a compound obtained by subjecting a carboxyl group in compound (I) or (I') to esterification or amidation (e.g., a compound obtained by subjecting a carboxyl group in compound (I) to ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification and methylamidation, etc.) and the like can be mentioned. Any of these compounds can be produced from compound (I) or (I') by a method known per se.

A prodrug for compound (I) may also be one which is converted to compound (I), (Ia), (Ib), (Ic) or (Id), or (I') under physiological conditions as described in "IYAKUHIN no KAIHATSU (Development of Pharmaceuticals)", Vol. 7, Design of Molecules, p. 163-198, Published by HIROKAWA SHOTEN (1990).

Since the compounds of the present invention (I) and (I') show superior p38 MAP kinase inhibitory activity, TNF-α inhibitory activity (TNF-α production inhibitory activity, TNF-α action inhibitory activity) and MMP-13 production inhibitory activity (e.g., IL-1 induced MMP-13 production inhibitory activity), particularly, superior p38 MAP kinase inhibitory activity and MMP-13 production inhibitory activity, they are also useful as safe pharmaceutical products based on these actions.

For example, the pharmaceutical agent of the present invention containing compound (I) or (I') can be used as an agent for the prophylaxis or treatment of p38 MAP kinase related disease, TNF-α related disease, IL-1-related disease or MMP-13 related disease, more specifically, inflammatory disease (e.g., acute pancreatitis, chronic pancreatitis, asthma, adult respiratory distress syndrome, chronic obstructive pulmonary diseases (COPD), inflammatory bone disease, inflammatory pulmonary disease, inflammatory bowel disease, hepatitis, systemic inflammatory response syndrome (SIRS), postoperative or posttraumatic inflammation, pneumonia, hepatitis, nephritis, meningitis, cystitis, pharyngolaryngitis, stomach mucosa injury, meningitis, spondylitis, chronic pneumonia, bronchitis, lung infarction, silicosis, pulmonary sarcoidosis, etc.), autoimmune disease (e.g., chronic rheumatoid arthritis, ankylosing spondylitis, psoriasis, multiple sclerosis (MS), polymyositis, dermatomyositis (DM), polyarteritis nodosa (PN), mixed connective tissue disease (MCTD), Sjogren's syndrome nephritis, systemic lupus erythematosus, scleroderma, lupus erythematosus profundus, chronic thyroiditis, Graves' disease, autoimmune gastritis, Type I and Type II diabetes, autoimmune hemolytic anemia, autoimmune neutrophenia, thrombocytopenia, atopic dermatitis, chronic active hepatitis, myasthenia gravis, multiple sclerosis, inflammatory colitis, inflammatory bowel disease (IBD), Crohn's disease, graft-versus-host disease, Addison's disease, abnormal immune response, arthritis, dermatitis, radiodermatitis, etc.), osteoarticular degenerative disease (e.g., osteoporosis, osteoarthritis, etc.), debilitating disease (e.g., irritable bowel syndrome (IBS), acute or chronic diarrhea, etc.), neurodegenerative disease (e.g., Alzheimer's disease, Parkinson's syndrome, amyotropic lateral sclerosis, Huntington's disease, cerebral ischemia, traumatic neurodegenerative disease, multiple sclerosis, etc.), vascular disease (e.g., cerebral apoplexy (e.g., cerebral hemorrhage, cerebral infarction, brain edema), spinal trauma, myocardial ischemia, ischemic cardiac diseases, renal ischemia, renal failure, angina pectoris, myocardial infarction , congestive heart failure, arteriosclerosis, cardiac hypertrophy, dialysis hypotension, disseminated intravascular coagulation syndrome, etc.), neoplastic disease (e.g., malignant tumor, neovascular glaucoma, infantile hemangioma, multiple myeloma, acute myeloblastic leukemia, chronic sarcoma, multiple myeloma, chronic myeloid leukemia, metastasis melanoma, Kaposi's sarcoma , vascular proliferation, cachexia, etc.) or infectious disease (e.g., infections, toxemia, sepsis, septic shock, endotoxic shock , pulmonary tuberculosis, viral disease (e.g., cytomegaloviral pneumonia, adenoviral cold, acquired immunodeficiency syndrome (AIDS), conjunctivitis, AIDS encephalopathy, etc.), etc.) and the like in mammal (e.g., mouse, rat, hamster, rabbit, cat, dog, bovine, sheep, monkey, human, etc.). Preferably, it can be used as an agent for the prophylaxis or treatment of rheumatoid arthritis, osteoarthritis and the like.

As used herein, the "prophylaxis" of the above-mentioned diseases means, for example, administration of a pharmaceutical containing the compound of the present invention to patients before onset of a disease but having a high risk of the onset due to some factor associated with the disease or patients who developed the disease but without subjective symptoms, or administration of a pharmaceutical containing the compound of the present invention to patients having a risk of recurrence of disease after treatment of the disease.

The pharmaceutical agent of the present invention containing compound (I) or (I') shows low toxicity, and can be safely administered orally or parenterally (e.g., topical, rectal, intravenous administration, etc.) as a pharmaceutical agent of the compound (I) or (I') as it is or after admixing with a pharmacologically acceptable carrier to give, for example, tablet (including sugar-coated tablet and film-coated tablet), powder, granule, capsules (including soft capsules), liquid, injection, suppository, sustained-release preparation and the like, according to a methods known per se used for the general production method for pharmaceutical preparations. The content of the compound (I) or (I') in a pharmaceutical agent of the present invention is about 0.01 to about 100% by weight relative to the whole pharmaceutical agent. The dose varies depending on administration subjects, administration route, diseases, and the like. The preparation may be administered, for example, to a patient with rheumatoid arthritis (body weight about 60 kg), about 0.01 to about 30 mg active ingredient (compound (I) or (I'))/kg body weight per day, preferably about 0.1 to about 20 mg/kg body weight per day, more preferably about 1 to about 20 mg/kg body weight per day, which is given once or divided into several doses a day as an oral preparation.

As a pharmacologically acceptable carrier which may be used for preparing a pharmaceutical agent of the present invention, the conventional various organic or inorganic carriers as a pharmaceutical material, for example, excipient, lubricant, binder and disintegrating agent in solid preparations, or solvent, solubilizing agent, suspending agent, isotonizing agent, buffer and soothing agent in liquid preparations, and the like can be mentioned. Further, if needed, additives such as the conventional preservative, antioxidant, colorant, sweetening agent, adsorbing agent, wetting agent and the like can be appropriately used at an appropriate amount.

As an excipient, for example, lactose, saccharose, D-mannitol, starch, corn starch, crystalline cellulose, light silicic acid anhydride and the like can be mentioned.

As a lubricant, for example, magnesium stearate, calcium stearate, talc, colloidal silica and the like can be mentioned.

As a binder, for example, crystalline cellulose, saccharose, D-mannitol, dextrin, hydroxypropylcellulose, hydroxypropylmethylcellulose, polyvinylpyrrolidone, starch, sucrose, gelatin, methylcellulose, sodium carboxymethylcellulose and the like can be mentioned.

As a disintegrating agent, for example, starch, carboxymethyl cellulose, calcium carboxymethyl cellulose, sodium carboxymethyl starch, L-hydroxypropylcellulose and the like can be mentioned.

As a solvent, for example, water for injection, alcohol, propylene glycol, macrogol, sesame oil, corn oil, olive oil and the like can be mentioned.

As a solubilizing agent, for example, polyethylene glycol, propylene glycol, D-mannitol, benzyl benzoate, ethanol, tris-aminomethane, cholesterol, triethanolamine, sodium carbonate, sodium citrate and the like can be mentioned.

As a suspending agent, for example, surfactants such as stearyl triethenolamine, sodium lauryl sulfate, lauryl aminopropionate, lecithin, benzalkonium chloride, benzethonium chloride, glyceryl monostearate and the like; hydrophilic polymers such as polyvinyl alcohol, polyvinylpyrrolidone, sodium carboxymethyl cellulose, methylcellulose, hydroxymethylcellulose, hydroxyethylcellulose, hydoxypropylcellulose and the like, and the like can be mentioned.

As an isotonizing agent, for example, glucose, D-sorbitol, sodium chloride, glycerin, D-mannitol and the like can be mentioned.

As a buffer, for example, buffering solutions such as phosphate, acetate, carbonate, citrate and the like, and the like can be mentioned.

As a soothing agent, for example, benzyl alcohol and the like can be mentioned.

As a preservative, for example, p-hydroxybenzoates, chlorobutanol, benzyl alcohol, phenethyl alcohol, dehydroacetic acid, sorbic acid and the like can be mentioned.

As an antioxidant, for example, sulfites, ascorbic acid, α-tocopherol and the like can be mentioned.

The prophylactic or therapeutic agent of the present invention can also be used together with other pharmaceutical agent. For example, when compound (I) or (I') is used as a p38 MAP kinase inhibitor, TNF-α production inhibitor or MMP-13 production inhibitor, the following drug can be used in combination.
(1) non-steroidal antiinflammatory drugs (NSAIDs)
   (i) Classical NSAIDs
      alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesylate, camostat mesylate, ulinastatin, colchicine, probenecid, sulfinpyrazone, benzbromarone, allopurinol, sodium aurothiomalate, hyaluronate sodium, sodium salicylate, morphine hydrochloride, salicylic acid, atropine, scopolamine, morphine, pethidine, levorphanol, oxymorphone or a salt thereof and the like.
   (ii) cyclooxygenase inhibitor (COX-1 selective inhibitor, COX-2 selective inhibitor and the like)
      salicylic acid derivatives (e.g., celecoxib, rofecoxib, aspirin, etc.), MK-663, valdecoxib, SC-57666, tiracoxib, S-2474, diclofenac, indomethacin, loxoprofen and the like.
   (iii) drug concurrently having COX inhibitory activity and 5-lipoxygenase inhibitory activity
      ML-3000, p54 (COX inhibitor & 5-lipoxygenase inhibitor) and the like.
   (iv) nitric oxide-releasing NSAIDs
(2) disease-modifying anti-rheumatic drugs (DMARDs)
   (i) Gold preparation
      Auranofin and the like.
   (ii) penicillamine
      D-penicillamine
   (iii) sulfasalazine
   (iv) antimalarial drug
      chloroquine and the like.
   (v) pyrimidine synthesis inhibitor
      leflunomide and the like.
   (vi) Prograf
(3) anti-cytokine drug
   (I) protein drug
      (i) TNF inhibitor
         etanercept, infliximab, D2E7, CDP-571, PASSTNF-α, soluble TNF-α receptor, TNF-α binding protein, anti-TNF-α antibody and the like.
      (ii) interleukin-1 inhibitor
         anakinra (interleukin-1 receptor antagonist), soluble interleukin-1 receptor and the like.
      (iii) interleukin-6 inhibitor
         MRA (anti-interleukin-6 receptor antibody), anti-interleukin-6 antibody and the like.
      (iv) interleukin-10 drug
         interleukin-10 and the like.
      (v) interleukin-12 inhibitor
         anti-interleukin-12 antibody and the like.
      (vi) drug concurrently having interferon-α and -γ inhibitory activity and TNF-α inhibitory activity (polyclonal antibody) AGT-1
   (II) non-protein drug
      (i) MAP kinase inhibitor
         PD-98059 and the like.
      (ii) gene modulator
         SP-100030, inhibitor of molecule involved in signal transduction, such as NF-κ, NF-κB, IKK-1, IKK-2, AP-1 and the like, and the like
      (iii) cytokine production inhibitor
         T-614, SR-31747, sonatimod and the like.
      (iv) TNF-α converting enzyme inhibitor
      (v) interleukin-1β converting enzyme inhibitor
         HMR3480/VX-740 and the like.
      (vi) interleukin-6 antagonist
         SANT-7 and the like.
      (vii) interleukin-8 inhibitor
         IL-8 antagonist, CXCR1 & CXCR2 antagonist and the like.
      (viii) chemokine antagonist
         MCP-1 antagonist and the like.
      (ix) interleukin-2 receptor antagonist
         Denileukin, diftitox and the like.
      (x) therapeutic vaccines
         TNF-α vaccine and the like.
      (xi) gene therapy drug
         gene therapy drugs aiming at promoting the expression of gene having an anti-inflammatory action such as interleukin-4, interleukin-10, soluble interleukin-1 receptor, soluble TNF-α receptor and the like.
      (xii) antisense compound
         ISIS-104838 and the like.
(4) immunomodulator (immunosuppressant)
   (i) T cell differentiation modulator
      ethyl 6,7-dimethoxy-4-(3,4-dimethoxyphenyl)-2-(1,2,4-triazol-1-ylmethyl)quinoline-3-carboxylate (JP-A-7-118266)
   (ii) others
      methotrexate, cyclophosphamide, MX-68, atiprimod dihydrochloride, BMS-188667, CKD-461, rimexolone, cyclosporine, tacrolimus, gusperimus, azathiopurine, antilymphocyte serum, freeze-dried sulfonated normal immunoglobulin, erythropoietin, colony stimulating factor, interleukin, interferon and the like.
(5) steroid
   dexamethasone, hexestrol, methimazole, betamethasone, triamcinolone, triamcinolone acetonide, fluocinonide, fluocinolone acetonide, predonisolone, methylpredonisolone, cortisone acetate, hydrocortisone, fluorometholone, beclomethasone dipropionate, estriol and the like.
(6) c-Jun N terminal kinase (JNK) inhibitor
   Compounds described in WO00/35906, WO00/35909, WO00/35921, WO00/64872 or WO00/75118 and the like.
(7) angiotensin converting enzyme inhibitor
   enalapril, captopril, ramipril, lisinopril, cilazapril, perindopril and the like.
(8)angiotensin II receptor antagonist
   candesartan, cilexetil (TCV-116), valsartan, irbesartan, olmesartan, eprosartan and the like.
(9) diuretic drug
   hydrochlorothiazide, spironolactone, furosemide, indapamide, bendrofluazide, cyclopenthiazide and the like.
(10) cardiotonic drug
   digoxin, dobutamine and the like.
(11) β receptor antagonist
   carvedilol, metoprolol, atenolol and the like.
(12) Ca sensitizer
   MCC-135 and the like.
(13) Ca channel antagonist
   nifedipine, diltiazem, verapamil and the like.
(14) anti-platelet drug, anticoagulator
   heparin, aspirin, warfarin and the like.
(15) HMG-CoA reductase inhibitor
   atorvastatin, simvastatin and the like.
(16) contraceptive
   (i) sex hormone or derivatives thereof
      gestagen or a derivative thereof (progesterone, 17α-hydroxy progesterone, medroxyprogesterone, medroxyprogesterone acetate, norethisterone, norethisterone enanthate, norethindrone, norethindrone acetate, norethynodrel, levonorgestrel, norgestrel, ethynodiol diacetate, desogestrel, norgestimate, gestodene, progestin, etonogestrel, drospirenone, dienogest, trimegestone, nestorone, chlormadinone acetate, mifepristone, nomegestrol acetate, Org-30659, TX-525, EMM-310525) or a combination agent of a gestagen or a derivative thereof and an estrogen or a derivative thereof (estradiol, estradiol benzoate, estradiol cypionate, estradiol dipropionate, estradiol enanthate, estradiol hexahydrobenzoate, estradiol phenylpropionate, estradiol undecanoate, estradiol valerate, estrone, ethinylestradiol, mestranol) and the like.
   (ii) antiestrogen
      ormeloxifene, mifepristone, Org-33628 and the like.
   (iii) spermatocide
      ucarcide and the like.
(17) others
   (i) T cell inhibitors
      IR-501 (T cell receptor peptide) and the like.
   (ii) inosine monophosphate dehydrogenase (IMPDH) inhibitor mycophenolate mofetil, VX-497 and the like.
   (iii) adhesion molecule inhibitor
      ISIS-2302, selectin inhibitor, ELAM-1, VCAM-1, ICAM-1 and the like.
   (iv) thalidomide
   (v) cathepsin inhibitor
   (vi) matrix metalloprotease (MMPs) inhibitor
      BB-3644, CGS-27023A, Bay-12-9566, KB-R7785, L-758354, POL-641 and the like.
   (vii) glucose-6-phosphate dehydrogenase inhibitor
      CBF-BS2 and the like.
   (viii) Dihydroorotate dehydrogenase (DHODH) inhibitor
   (ix) phosphodiesterase IV (PDE IV) inhibitor
      CG-1088 and the like.
   (x) phospholipase A₂ inhibitor
   (xi) iNOS inhibitor
      NOX-200 and the like.
   (xii) microtubule stimulating drug
      paclitaxel and the like.
   (xiii) microtubule inhibitor
      reumacon and the like.
   (xiv) MHC class II antagonist
      ZD-2315 and the like.
   (xv) prostacyclin agonist
      iloprost and the like.
   (xvi) CD4 antagonist
      4162W94, keliximab and the like.
   (xvii) CD23 antagonist
   (xviii) LTB4 receptor antagonist
      CGS-25019C and the like.
   (xix) 5-lipoxygenase inhibitor
      zileuton and the like.
   (xx) cholinesterase inhibitor
      galanthamine and the like.
   (xxi) tyrosine kinase inhibitor
      YT-146 and the like.
   (xxii) cathepsin B inhibitor
   (xxiii) adenosine deaminase inhibitor
      pentostatin and the like.
   (xxiv) osteogenesis stimulator
      (2R,4S)-(-)-N-[4-(diethoxyphosphorylmethyl)phenyl]-1,2,4,5-tetrahydro-4-methyl-7,8-methylenedioxy-5-oxo-3-benzothiepin-2-carboxamide or a salt thereof (JP-A-8-231659) and the like.
   (xxv) dipeptidylpeptidase inhibitor
      TMC-2A and the like.
   (xxvi) TRK-530, TOK-8801
   (xxvii) collagen agonist
      AI-200 and the like.
   (xxviii) capsaicin cream
   (xxix) hyaluronic acid derivative
      synvisc (hylan G-F 20), orthovisc and the like.
   (xxx) glucosamine sulfate
   (xxxi) amiprilose
      Other concomitant drugs besides the above-mentioned include, for example, antibacterial agent, antifungal agent, antiprotozoal agent, antibiotic, antitussive and expectorant drug, sedative, anesthetic, antiulcer drug, antiarrhythmic agent, hypotensive diuretic drug, anticoagulant, tranquilizer, antipsychotic, antitumor drug, hypolipidemic drug, muscle relaxant, anticonvulsant, antidepressant, antiallergic drug, cardiac stimulants, therapeutic drug for arrhythmia, vasodilator, vasoconstrictor, hypotensive diuretic, therapeutic drug for diabetes, antinarcotic, vitamin, vitamin derivative, antiasthmatic, therapeutic agent for pollakisuria/anischuria, therapeutic agent for atopic dermatitis, therapeutic agent for allergic rhinitis, hypertensor, endotoxin-antagonist or - antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Specific examples thereof include the following.

(1) Antibacterial agent
   A. sulfa drug
      sulfamethizole, sulfisoxazole, sulfamonomethoxine, sulfamethizole, salazosulfapyridine, silver sulfadiazine and the like.
   B. quinoline antibacterial agent
      nalidixic acid, pipemidic acid trihydrate, enoxacin, norfloxacin, ofloxacin, tosufloxacin tosilate, ciprofloxacin hydrochloride, lomefloxacin hydrochloride, sparfloxacin, fleroxacin and the like.
   C. antiphthisic
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   D. antiacidfast bacterium drug
      diaphenylsulfone, rifampicin and the like.
   E. antiviral drug
      idoxuridine, acyclovir, vidarabine, gancyclovir and the like.
   F. anti-HIV agent
      zidovudine, didanosine, zalcitabine, indinavir sulfate ethanolate, ritonavir and the like.
   G. antispirochetele
   H. antibiotic
      tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt thereof, griseofulvin, lankacidin-group [Journal of Antibiotics (J. Antibiotics), 38, 877-885(1985)], azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone, fluconazole, itraconazole and the like] and the like.
(2) antifungal agent
   A. polyethylene antibiotic (e.g., amphotericin B, nystatin, trichomycin, etc.)
   B. griseofulvin, pyrrolnitrin and the like.
   C. cytosine metabolism antagonist (e.g., flucytosine)
   D. imidazole derivative (e.g., econazole, clotrimazole, miconazole nitrate, bifonazole and croconazole)
   E. triazole derivative (e.g. fluconazole and itraconazole)
   F. thiocarbamic acid derivative (e.g. trinaphthol), and the like
(3) antiprotozoal agent
   metronidazole, tinidazole, diethylcarbamazine citrate, quinine hydrochloride, quinine sulfate and the like.
(4) antitussive and expectorant drug
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, cloperastine, protokylol, isoproterenol, salbutamol, terbutaline, oxymetebanol, morphine hydrochloride, dextromethorfan hydrobromide, oxycodone hydrochloride, dimemorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(5) sedative
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(6) anesthetic
   (6-1) local anesthetic
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine and the like.
   (6-2) general anesthetic
      A. inhalation anesthetic (e.g., ether, halothane, nitrous oxide, isoflurane, enflurane, etc.),
      B. intravenous anesthetic (e.g., ketamine hydrochloride, droperidol, thiopental sodium, thiamylal sodium, pentobarbital, etc.) and the like.
(7) antiulcer drug
   histidine hydrochloride, lansoprazole, metoclopramide, pirenzepine, cimetidine, ranitidine, famotidine, urogastrone, oxethazaine, proglumide, omeprazole, sucralfate, sulpiride, cetraxate, gefarnate, aldioxa, teprenone, prostaglandin and the like.
(8) antiarrhythmic agent
   A. Na channel blocker (e.g., quinidine, procainamide, disopyramide, ajmaline, lidocaine, mexiletine, phenytoin),
   B. β-blocker (e.g., propranolol, alprenolol, bufetolol hydrochloride, oxprenolol, atenolol, acebutolol, metoprolol, bisoprolol, pindolol, carteolol, arotinolol hydrochloride),
   C. K channel blocker (e.g., amiodarone),
   D. Ca channel blocker (e.g., verapamil, diltiazem) and the like.
(9) hypotensive diuretic drug
   hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophylline, and the like.
(10) anticoagulant
   heparin sodium, sodium citrate, activated protein C, tissue factor pathway inhibitor, antithrombin III, dalteparin sodium, warfarin potassium, argatroban, gabexate, sodium citrate, ozagrel sodium, ethyl icosapentate, beraprost sodium, alprostadil, ticlopidine hydrochloride, pentoxifylline, dipyridamole, tisokinase, urokinase, streptokinase and the like.
(11) tranquilizer
   diazepam, lorazepam, oxazepam, chlordiazepoxide, medazepam, oxazolam, cloxazolam, clotiazepam, bromazepam, etizolam, fludiazepam, hydroxyzine and the like.
(12) antipsychotic
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(13) antitumor drug
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, busulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuprorelin acetate, buserelin acetate and the like.
(14) antihypolipidemic drug
   clofibrate, ethyl 2-chloro-3-[4-(2-methyl-2-phenylpropoxy)phenyl]propionate [Chemical and Pharmaceutical Bulletin (Chem. Pharm. Bull), 38, 2792-2796 (1990)], pravastatin, simvastatin, probucol, bezafibrate, clinofibrate, nicomol, cholestyramine, dextran sulfate sodium and the like.
(15) muscle relaxant
   pridinol, tubocurarine, pancuronium, tolperisone hydrochloride, chlorphenesin carbamate, baclofen, chlormezanone, mephenesin, chlorzoxazone, eperisone, tizanidine and the like.
(16) anticonvulsant
   phenytoin, ethosuximide, acetazolamide, chlordiazepoxide, trimethadione, carbamazepine, phenobarbital, primidone, sulthiame, sodium valproate, clonazepam, diazepam, nitrazepam and the like.
(17) antidepressant
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(18) antiallergic drug
   diphenhydramine, chlorpheniramine, tripelennamine, metodilamine, clemizole, diphenylpyraline, methoxyphenamine, sodium cromoglicate, tranilast, repirinast, amlexanox, ibudilast, ketotifen, terfenadine, mequitazine, azelastine hydrochloride, epinastine, ozagrel hydrochloride, pranlukast hydrate, seratrodast and the like.
(19) cardiac
   trans-π-oxocamphor, terephyllol, aminophylline, etilefrine, dopamine, dobutamine, denopamine, aminophylline, bencirin, amrinone, pimobendan, ubidecarenone, digitoxin, digoxin, methyldigoxin, lanatoside C, G-strophanthin and the like.
(20) vasodilator
   oxyfedrine, diltiazem, tolazoline, hexobendine, bamethan, clonidine, methyldopa, guanabenz and the like.
(21) vasoconstrictor
   dopamine, dobutamine denopamine and the like.
(22) hypotensive diuretic
   Hexamethonium bromide, pentolinium, mecamylamine, ecarazine, clonidine, diltiazem, nifedipine and the like.
(23) antidiabetic drug
   tolbutamide, chlorpropamide, acetohexamide, glibenclamide, tolazamide, acarbose, epalrestat, troglitazone, glucagon, glymidine, glipizide, phenformin, buformin, metformin and the like.
(24) antinarcotic
   levallorphan, nalorphine, naloxone or a salt thereof and the like.
(25) liposoluble vitamins
   A. vitamin A: vitamin A₁, vitamin A₂ and retinol palmitate
   B. vitamin D: vitamin D₁, D₂, D₃, D₄ and D₅
   C. vitamin E: α-tocopherol, β-tocopherol, γ-tocopherol, δ-tocopherol, dl-α-tocopherol nicotinate
   D. vitamin K: vitamin K₁, K₂, K₃ and K₄
   E. folic acid (vitamin M) and the like.
(26) vitamin derivative
   various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like, and the like.
(27) antiasthmatic
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlkast hydrate, seratrodast, dexamethasone, prednisolone, hydrocortisone, hydrocortisone sodium succinate, beclometasone dipropionate and the like.
(28) therapeutic agent for pollakisuria/anischuria
   flavoxate hydrochloride and the like.
(29) therapeutic agent for atopic dermatitis
   sodium cromoglicate and the like.
(30) therapeutic agent for allergic rhinitis
   sodium cromoglicate, chlorpheniramine maleate, alimemazine tartrate, clemastine fumarate, homochlorcyclizine hydrochloride, terfenadine, mequitazine and the like.
(31) hypertensive drug
   dopamine, dobutamine, denopamine, digitoxin, digoxin, methyldigoxin, lanatoside C, G-strophanthin and the like.
(32) Others
   hydroxycam, diacerein, megestrol acetate, nicergoline, prostaglandins and the like.

As regards the use of the combination, the administration time of the compound (I) or (I') and the concomitant drug is not restricted, and the compound (I) or a pharmaceutical composition thereof and the concomitant drug or a pharmaceutical composition thereof can be administered to an administration subject simultaneously, or may be administered at different times. The dosage of the concomitant drug may be determined according to the dose clinically used, and can be appropriately selected depending on an administration subject, administration route, disease, combination and the like.

The administration mode of the compound (I) or (I') is not particularly restricted, and it is sufficient that the compound (I) or (I') and the concomitant drug are combined in administration. Examples of such administration mode include the following methods:
(1) The compound (I) or (I'), or a pharmaceutical composition thereof and the concomitant drug are simultaneously produced to give a single preparation which is administered. (2) The compound (I) or (I'), or a pharmaceutical composition thereof of the present invention and the concomitant drug or a pharmaceutical composition thereof are separately produced to give two kinds of preparations which are administered simultaneously by the same administration route. (3) The compound (I) or (I'), or a pharmaceutical composition thereof and the concomitant drug or a pharmaceutical composition thereof are separately produced to give two kinds of preparations which are administered by the same administration route only at the different times. (4) The compound (I) or (I'), or a pharmaceutical composition thereof and the concomitant drug or a pharmaceutical composition thereof are separately produced to give two kinds of preparations which are administered simultaneously by the different administration routes. (5) The compound (I) or (I'), or a pharmaceutical composition thereof and the concomitant drug or a pharmaceutical composition thereof are separately produced to give two kinds of preparations which are administered by the different administration routes only at different times (e.g., the compound (I) or (I'), or a pharmaceutical composition thereof and the concomitant drug or a pharmaceutical composition thereof are administered in this order, or in the reverse order).

The compounding ratio of the compound (I) or (I') to the concomitant drug in the combination agent of the present invention can be appropriately selected depending on an administration subject, administration route, diseases and the like.

For example, the content of the compound (I) or (I') in the combination agent of the present invention differs depending on the form of a preparation, and usually from about 0.01 to 100% by weight, preferably from about 0.1 to 50% by weight, further preferably from about 0.5 to 20% by weight, based on the preparation.

The content of the concomitant drug in the combination agent of the present invention differs depending on the form of a preparation, and usually from about 0.01 to 100% by weight, preferably from about 0.1 to 50% by weight, further preferably from about 0.5 to 20% by weight, based on the preparation.

The content of additives such as a carrier and the like in the combination agent of the present invention differs depending on the form of a preparation, and usually from about 1 to 99.99% by weight, preferably from about 10 to 90% by weight, based on the preparation.

The same contents can be employed when compound (I) or (I') and a concomitant drug are separately prepared.

While the dose varies depending on the kind of compound (I) or (I') or a pharmacologically acceptable salt thereof, administration route, symptom, age of patient and the like, it is, for example, about 0.1 to about 30 mg/kg body weight, preferably about 1 to about 20 mg/kg mg/kg body weight, as compound (I) or (I') for 1 kg body weight, for one day by oral administration to a patient (body weight about 60 kg) with rheumatoid arthritis. The dose may be administered once a day, or in several portions a day.

While the dose of the pharmaceutical composition of the present invention when it is a sustained-release preparation varies depending on the kind and content of compound (I) or (I'), dosage form, duration of drug-release, subject animal of administration (e.g., mammal such as mouse, rat, hamster, guinea pig, rabbit, cat, dog, bovine, horse, swine, sheep, monkey, human and the like) and administration object, in the case of parenteral administration, for example, about 0.1 to about 100 mg of compound (I) or (I') only needs to be released in one week from the administered preparation.

The amount of the concomitant drug can be set at any value unless side effects are problematical. The daily dosage in terms of the concomitant drug differs depending on the severity, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, usually from about 0.001 to 2000 mg, preferably from about 0.01 to 500 mg, further preferably from about 0.1 to 100 mg, per 1 kg of a mammal and this is usually administered once to 4-times divided in a day.

For administration of a combination agent of the present invention, the compound (I) or (I') may be administered after administration of the concomitant drug or the concomitant drug may be administered after administration of the compound (I) or (I'), though they may be administered simultaneously. When administered at a time interval, the interval varies depending on the effective ingredient, drug form and administration method, and, for example, when the concomitant drug is administered first, a method in which the compound (I) or (I') is administered within time range of from 1 minute to 3 days, preferably from 10 minutes to 1 day, more preferably from 15 minutes to 1 hour, after administration of the concomitant drug, is exemplified. When the compound (I) or (I') is administered first, a method in which the concomitant drug is administered within time range of from 1 minute to 1 day, preferably from 10 minutes to 6 hours, more preferably from 15 minutes to 1 hour, after administration of the compound (I) or (I'), is exemplified.

### Examples

The present invention is further described in detail with reference to Reference Examples, Examples, Preparative Examples and Experimental Example, which are not intended to restrict the invention and may be modified without departing from the scope of the invention.

The "room temperature" in the following Reference Examples and Examples means a temperature of generally from about 10°C to about 35°C. Unless otherwise specified, "%" means weight percent. However, yield shows mol/mol%.

Other abbreviations used in the specification mean the following.
s: singlet
d: doublet
t: triplet
q: quartet
dd: double doublet
ddd: double double doublet
dt: double triplet
dq: double quartet
br: broad
J: coupling constant
Hz: Hertz
CDCl₃: deuterated chloroform
DMSO-d₆: deuterated dimethylsulfoxide
CD₃OD: deuterated methanol
¹H-NMR: protone nuclear magnetic resonance
o: ortho
m: meta
p: para
n: normal
sec: secondary
tert: tertiary

The SEQ ID NOs of the sequence listing in the specification show the following sequences.
[SEQ ID NO: 1]
   nucleotide sequence of primer P38-U
[SEQ ID NO: 2]
   nucleotide sequence of primer PAG-L
[SEQ ID NO: 3]
   nucleotide sequence of primer MKK-U
[SEQ ID NO: 4]
   nucleotide sequence of primer MKK-L
[SEQ ID NO: 5]
   nucleotide sequence of primer SER-U
[SEQ ID NO: 6]
   nucleotide sequence of primer SER-L

### (Reference Example 1)

### 2-Bromo-2-(2-fluoropyridin-4-yl)-1-(3-methylphenyl)ethanone

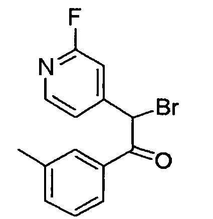

Bromine (13 mL, 0.25 mol) was added dropwise to a solution of 2-(2-fluoropyridin-4-yl)-1-(3-methylphenyl)ethanone (54 g, 0.24 mol) obtained by the method described in WO 00/64894 in acetic acid (250 mL), and the reaction mixture was stirred at 80°C for 90 min. The reaction mixture was allowed to be cooled to room temperature, and the solvent was removed by evaporation under reduced pressure. Saturated aqueous sodium hydrogen carbonate solution was added to the residue, and the mixture was extracted with ethyl acetate. The extract was washed with saturated brine and dried over anhydrous sodium sulfate, and the solvent was removed by evaporation under reduced pressure. The obtained crystals were recrystallized from ethyl acetate-hexane to give the title compound (yield 54 g, 74%).
Melting point 66-67°C (ethyl acetate-hexane)
¹H-NMR (CDCl₃) δ: 2.43 (3H, s), 6.20 (1H, s), 7.12 (1H, m), 7.31-7.43 (3H, m), 7.77-7.81 (2H, m), 8.23 (1H, d, J=5.2Hz).

### (Reference Example 2)

### 6-Chloro-4-methylpyridazin-3-amine

The compound was synthesized according to the method described in US 4104385.

### (Reference Example 3)

### 6-Chloro-5-tert-butylpyridazin-3-amine

The compound was synthesized according to the method described in WO 1996/8496.

### (Reference Example 4)

### 6-Chloro-4-methoxypyridazin-3-amine

The compound was synthesized according to the method described in WO 2004/108690.

### (Reference Example 5)

### tert-Butyl 4-[2-hydroxy-2-(3-methylphenyl)ethenyl]-2-pyrimidinyl carbamate

The compound was synthesized according to the method described in WO 2002/062792.

### (Reference Example 6)

### tert-Butyl 4-[2-hydroxy-2-(4-fluoro-3-methylphenyl)ethenyl]-2-pyrimidinylcarbamate

The compound was synthesized in the same manner as in Reference Example 5 using N-(4-fluoro-3-methylbenzoyl)propyleneimine instead of N-(3-methylbenzoyl)propyleneimine.
Melting point 193-194°C (ethyl acetate-isopropyl ether)
¹H-NMR (CDCl₃) δ: 1.58 (9H, s), 2.32 (3H, d, J=1.9Hz), 5.94 (1H, s), 6.53 (1H, d, J=5.5Hz), 7.03 (1H, t, J=8.9Hz), 7.67-7.74 (1H, m), 7.75-7.81 (1H, m), 8.16 (1H, d, J=5.5Hz), 8.35 (1H, brs).

### (Reference Example 7)

### 6-Chloro-4-(methylthio)pyridazin-3-amine

The compound of Reference Example 7 was synthesized in the same manner as in Reference Example 4 and using sodium methylthiolate instead of sodium methoxide.
Melting point 186-187°C (ethanol)
¹H-NMR (CDCl₃) δ: 2.55 (3H, s), 4.89 (2H, brs), 6.98 (1H, s).

### (Example 1)

### 6-Chloro-3-(2-fluoropyridin-4-yl)-2-(3-methylphenyl)imidazo[1,2-b]pyridazine

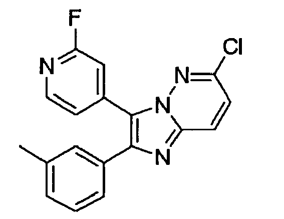

2-Bromo-2-(2-fluoropyridin-4-yl)-1-(3-methylphenyl)ethanone (50.0 g, 162 mmol) obtained in Reference Example 1, 3-amino-6-chloropyridazine (27.3 g, 211 mmol) and disodium hydrogenphosphate (34.5 g, 243 mmol) were heated under reflux for 6 hr in ethanol (500 mL). The reaction mixture was allowed to be cooled to room temperature, and the solvent was removed by evaporation under reduced pressure. To the residue was added saturated aqueous sodium hydrogen carbonate solution, and the mixture was extracted with chloroform. The extract was dried over anhydrous magnesium sulfate, and the solvent was removed by evaporation under reduced pressure. The obtained crude crystals were recrystallized from ethanol to give the title compound (yield 41.0 g, 75%).
Melting point 215-216°C (ethanol)
¹H-NMR (CDCl₃) δ: 2.38 (3H, s), 7.16-7.35 (5H, m), 7.42-7.45 (1H, m), 7.53 (1H,s), 8.00 (1H, d, J=9.4Hz), 8.28 (1H, d, J=5.3Hz).

### (Example 2)

### 4-[6-Chloro-2-(3-methylphenyl)imidazo[1,2-b]pyridazin-3-yl]-N-cyclohexylpyridin-2-amine

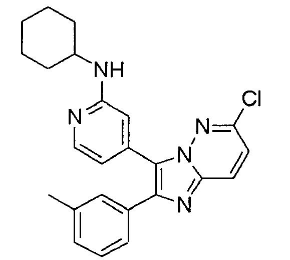

6-Chloro-3-(2-fluoropyridin-4-yl)-2-(3-methylphenyl)imidazo[1,2-b]pyridazine (1.50 g, 4.43 mmol) obtained in Example 1 and cyclohexylamine (15 mL) were stirred at 150°C for 90 min under microwave irradiation. The reaction mixture was allowed to be cooled to room temperature, and saturated aqueous sodium hydrogen carbonate solution was added to the mixture, and the mixture was extracted with ethyl acetate. The extract was washed with saturated brine and dried over anhydrous sodium sulfate, and the solvent was removed by evaporation under reduced pressure. The obtained residue was purified by silica gel column chromatography (ethyl acetate: hexane = 3:7 - 8:2) and recrystallized from ethyl acetate to give the title compound (yield 750 mg, 40%).
Melting point 191-192°C (ethyl acetate)
¹H-NMR (CDCl₃) δ: 1.09-1.43 (6H, m), 1.57-1.80 (2H, m), 1.96-2.05 (2H, m), 2.36(3H, s), 3.32-3.42 (1H, m), 4.57 (1H, d, J=7.7Hz), 6.63 (1H, s), 6.75 (1H, dd, J=5.2, 1.4Hz), 7.09-7.25 (3H, m), 7.40 (1H, d, J=7.7Hz), 7.57 (1H, s), 7.94 (1H, d, J=9.3Hz), 8.12 (1H, dd, J=5.2, 0.6Hz).
Elemental analysis: for C₂₄H₂₄ClN₅
Calculated: C, 68.97; H, 5.79; N, 16.76.
Found: C, 68.98; H, 5.85; N, 15.78.

### (Example 3)

The compounds of Examples 3-1 and 3-2 below were synthesized in the same manner as in Example 2 respectively using 4-fluoro-2-methylaniline or 2-methylaniline, instead of cyclohexylamine.

### Example compound 3-1: 4-[6-chloro-2-(3-methylphenyl)imidazo[1,2-b]pyridazin-3-yl]-N-(4-fluoro-2-methylphenyl)pyridin-2-amine

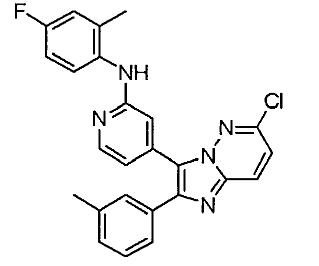

Melting point 191-192°C (ethyl acetate)
¹H-NMR (CDCl₃) δ: 2.27 (3H, s), 2.37 (3H, s), 6.27 (1H, s), 6.75-6.81 (2H, m), 6.89-6.93 (2H, m), 7.10 (1H, d, J=9.5Hz), 7.18-7.26 (3H, m), 7.35 (1H, d, J=7.5Hz), 7.52 (1H, s), 7.92 (1H, d, J=9.5Hz), 8.22 (1H, d, J=5.4Hz).
Elemental analysis: for C₂₅H₁₉ClFN₅
Calculated: C, 67.64; H, 4.31; N, 15.78.
Found: C, 67.66; H, 4.25; N, 15.73.

### Example compound 3-2: 4-[6-chloro-2-(3-methylphenyl)imidazo[1,2-b]pyridazin-3-yl]-N-(2-methylphenyl)pyridin-2-amine

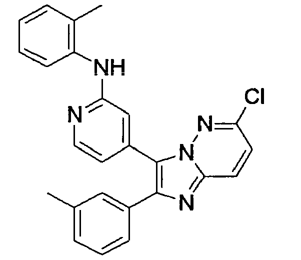

Melting point 191-192°C (ethyl acetate)
¹H-NMR (CDCl₃) δ: 2.29 (3H, s), 2.38 (3H, s), 6.44 (1H, s), 6.93 (1H, dd, J=5.2, 1.4Hz), 6.98-7.12 (4H, m), 7.19-7.32 (4H, m), 7.39 (1H, d, J=7.5Hz), 7.55 (1H, s), 7.94 (1H, d, J=9.3Hz), 8.25 (1H, dd, J=5.2, 0.7Hz).
Elemental analysis: for C₂₅H₂₀ClN₅
Calculated: C, 70.50; H, 4.73; N, 16.44.
Found: C, 70.59; H, 4.80; N, 16.43.

### (Example 4)

### 3-(2-fluoropyridin-4-yl)-2-(3-methylphenyl)imidazo[1,2-b]pyridazine

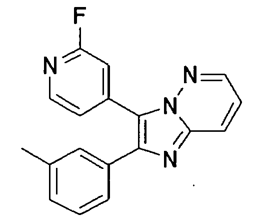

6-Chloro-3-(2-fluoropyridin-4-yl)-2-(3-methylphenyl)imidazo[1,2-b]pyridazine (500 mg, 1.48 mmol) obtained in Example 1 and 10% palladium carbon powder (50% water-containing product, 100 mg) were stirred in acetic acid solvent (50 mL) at 50°C for 7 hr under hydrogen atmosphere at the hydrogen pressure of 0.5 MPa. The reaction mixture was allowed to be cooled to room temperature and filtrated, and the solvent was removed by evaporation under reduced pressure. To the residue was added saturated aqueous sodium hydrogen carbonate solution, and the mixture was extracted with ethyl acetate. The extract was washed with saturated brine and dried over anhydrous sodium sulfate, and the solvent was removed by evaporation under reduced pressure. The obtained residue was purified by silica gel chromatography (ethyl acetate: hexane = 3:7 - 8:2), and the obtained crystals were recrystallized from ethyl acetate to give the title compound (yield 248 mg, 55%).
Melting point 144-145°C (ethyl acetate)
¹H-NMR (CDCl₃) δ: 2.38 (3H, s), 7.15-7.28 (3H, m), 7.33-7.37 (2H, m), 7.42-7.46(1H, m), 7.55 (1H, s), 8.03-8.07 (1H, m), 8.24 (1H, d, J=5.2Hz), 8.39 (1H, dd, J=4.5, 1.5Hz).
Elemental analysis: for C₁₈H₁₃FN₄
Calculated: C, 71.04; H, 4.31; N, 18.41.
Found: C, 71.10; H, 4.31; N, 18.46.

### (Example 5)

### N-(Cyclopropylmethyl)-4-[2-(3-methylphenyl)imidazo[1,2-b]pyridazin-3-yl]pyridin-2-amine

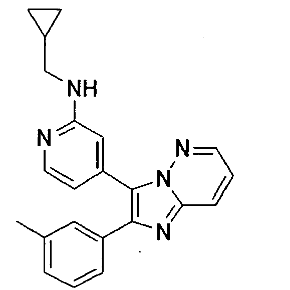

3-(2-Fluoropyridin-4-yl)-2-(3-methylphenyl)imidazo[1,2-b]pyridazine (150 mg, 0.493 mmol) obtained in Example 4 and 1-cyclopropylmethylamine (1.0 mL) were stirred under microwave irradiation at 150°C for 90 min. The reaction mixture was concentrated under reduced pressure, and the obtained residue was purified by silica gel chromatography (ethyl acetate: hexane = 3:7 - 8:2) and recrystallized from ethyl acetate to give the title compound (yield 146 mg, 83%).
Melting point 134-135°C (ethyl acetate)
¹H-NMR (CDCl₃) δ: 0.20-0.25 (2H, m), 0.49-0.55 (2H, m), 1.03-1.09 (1H, m), 2.36(3H, s), 3.10 (2H, m), 4.73 (1H, t, J=5.3Hz), 6.68-6.69 (1H, m), 6.79 (1H, dd, J=5.5, 1.4Hz), 7.06-7.23 (3H, m), 7.41 (1H, d, J=7.4Hz), 7.64 (1H, s), 8.01 (1H, dd, J=9.1, 1.7Hz), 8.16 (1H, m), 8.33 (1H, dd, J=4.4, 1.7Hz).
Elemental analysis: for C₂₂H₂₁N₅
Calculated: C, 74.34; H, 5.96; N, 19.70.
Found: C, 74.27; H, 5.97; N, 19.70.

### (Example 6)

The compounds of Examples 6-1 - 6-26 below were synthesized in the same manner as in Example 5 and respectively using cyclohexylamine, cyclopentylamine, cyclobutylamine, cyclopropylamine, 2-aminoethanol, 2-methoxyaniline, 2,3-dimethylaniline, 4-methylaniline, 3-methylaniline, 2-methylaniline, aniline, 1,2,3,4-tetrahydro-1-naphthylamine, 2-methoxyethylamine, 1-(2-aminoethyl)piperidine, 1-(2-thienyl)methylamine, tetrahydrofurfurylamine, furfurylamine, neopentylamine, N,N-diethylethylenediamine, 3-aminopentane, (1S)-1-phenylethylamine, (1R)-1-phenylethylamine, benzylamine, 1-butylamine, 1-propylamine or an aqueous solution of ethylamine, instead of 1-cyclopropylmethylamine.

### Example compound 6-1: N-cyclohexyl-4-[2-(3-methylphenyl)imidazo[1,2-b]pyridazin-3-yl]pyridin-2-amine

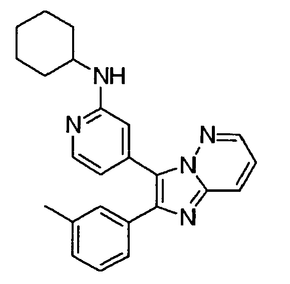

Melting point 169-170°C (ethyl acetate)
¹H-NMR (CDCl₃) δ: 1.06-1.39 (5H, m), 1.57-1.75 (3H, m), 1.92-2.00 (2H, m), 2.37(3H, s), 3.34-3.43 (1H, m), 4.51 (1H, d, J=8.0Hz), 6.58 (1H, s), 6.84 (1H, dd, J=5.2, 1.4Hz), 7.08-7.23 (3H, m), 7.43 (1H, d, J=7.7Hz), 7.62 (1H, s), 8.02 (1H, dd, J=9.2, 1.7Hz), 8.17 (1H, dd, J=5.2, 0.7Hz), 8.35 (1H, dd, J=4.4, 1.7Hz).
Elemental analysis: for C₂₄H₂₅N₅
Calculated: C, 75.17; H, 6.57; N, 18.26.
Found: C, 75.03; H, 6.57; N, 18.25.

### Example compound 6-2: N-cyclopentyl-4-[2-(3-methylphenyl)imidazo[1,2-b]pyridazin-3-yl]pyridin-2-amine

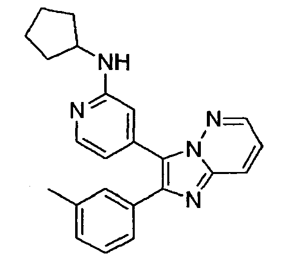

Melting point 130-131°C (ethyl acetate)
¹H-NMR (CDCl₃) δ: 1.41-1.96 (8H, m), 2.36 (3H, s), 3.84-3.90 (1H, m), 4.65 (1H, d, J=6.9Hz), 6.33 (1H, d, J=1.1Hz), 6.83 (1H, dd, J=5.2, 1.5Hz), 7.06-7.25 (3H, m), 7.43 (1H, d, J=7.8Hz), 7.63 (1H, s), 8.00 (1H, dd, J=9.1, 1.6Hz), 8.16 (1H, dd, J=5.2, 0.8Hz), 8.33 (1H, dd, J=4.4, 1.6Hz).
Elemental analysis: for C₂₃H₂₃N₅
Calculated: C, 74.77; H, 6.27; N, 18.96.
Found: C, 74.65; H, 6.26; N, 18.90.

### Example compound 6-3: N-cyclobutyl-4-[2-(3-methylphenyl)imidazo[1,2-b]pyridazin-3-yl]pyridin-2-amine

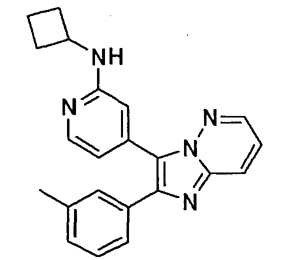

Melting point 152-153°C (ethyl acetate)
¹H-NMR (CDCl₃) δ: 1.67-1.87 (4H, m), 2.27-2.34 (2H, m), 2.37 (3H, s), 3.99-4.06 (1H, m), 4.83 (1H, d, J=6.6Hz), 6.57 (1H, s), 6.84 (1H, dd, J=5.2, 1.4Hz), 7.06-7.24 (3H, m), 7.41 (1H, d, J=8.1Hz), 7.62 (1H, s), 8.01 (1H, dd, J=9.2, 1.6Hz), 8.15 (1H, d, J=5.2Hz), 8.33 (1H, dd, J=4.4, 1.6Hz).
Elemental analysis: for C₂₂H₂₁N₅
Calculated: C, 74.34; H, 5.96; N, 19.70.
Found: C, 74.19; H, 6.01; N, 19.52.

### Example compound 6-4: N-cyclopropyl-4-[2-(3-methylphenyl)imidazo[1,2-b]pyridazin-3-yl]pyridin-2-amine

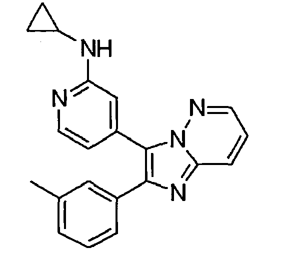

Melting point 202-203°C (ethyl acetate)
¹H-NMR (CDCl₃) δ : 0.43-0.63 (4H, m), 2.37-2.41 (4H, m), 5.13 (1H, s), 6.97-6.99 (2H, m), 7.08-7.25 (3H, m), 7.42 (1H, d, J=7.5Hz), 7.63 (1H, s), 8.02 (1H, dd, J=9.1, 1.7Hz), 8.18 (1H, dd, J=4.5, 1.5Hz), 8.35 (1H, dd, J=4.4, 1.7Hz).
Elemental analysis: for C₂₁H₁₉N₅
Calculated: C, 73.88; H, 5.61; N, 20.51.
Found: C, 73.60; H, 5.61; N, 20.28.

### Example compound 6-5: 2-({4-[2-(3-methylphenyl)imidazo[1,2-b]pyridazin-3-yl]pyridin-2-yl}amino)ethanol

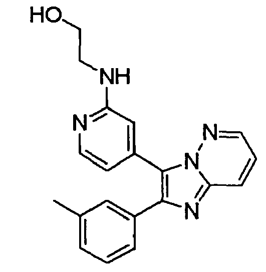

Melting point 176-177°C (ethyl acetate)
¹H-NMR (CDCl₃) δ: 2.38 (3H, s), 3.48-3.47 (2H, m), 3.81 (2H, t, J=4.7Hz), 4.77 (1H, br), 4.95 (1H, t, J=5.5Hz), 6.78-6.84 (2H, m), 7.11 (1H, dd, J=9.2, 4.3Hz), 7.15-7.19 (1H, m), 7.23 (1H, t, J=7.5Hz), 7.39 (1H, d, J=7.5Hz), 7.64 (1H, s), 8.03 (1H, dd, J=9.2, 1.7Hz), 8.11 (1H, d, J=6.0Hz), 8.34 (1H, dd, J=4.3, 1.7Hz).
Elemental analysis: for C₂₀H₁₉N₅O
Calculated: C, 69.55; H, 5.54; N, 20.28.
Found: C, 69.41; H, 5.58; N, 20.03.

### Example compound 6-6: N-(2-methoxyphenyl)-4-[2-(3-methylphenyl)imidazo[1,2-b]pyridazin-3-yl]pyridin-2-amine

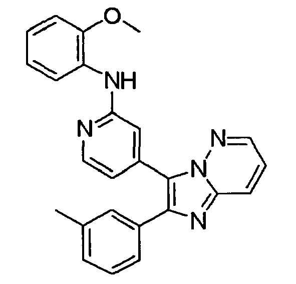

Melting point 157-158°C (ethyl acetate) ¹H-NMR (CDCl₃) δ: 2.39 (3H, s), 3.87 (3H, s), 6.79-6.95 (3H, m), 7.02-7.07 (2H, m), 7.12 (1H, dd, J=9.2, 4.3Hz), 7.17-7.23 (2H, m), 7.25-7.29 (1H, m), 7.44 (1H, d, J=7.5Hz), 7.63 (1H, s), 7.73 (1H, dd, J=7.9, 1.5Hz), 8.03 (1H, dd, J=9.2, 1.7Hz), 8.33 (1H, d, J=5.3Hz), 8.36 (1H, dd, J=4.3, 1.7Hz).
Elemental analysis: for C₂₅H₂₁N₅O
Calculated: C, 73.69; H, 5.19; N, 17.19.
Found: C, 73.51; H, 5.20; N, 17.17.

### Example compound 6-7: N-(2,3-dimethylphenyl)-4-[2-(3-methylphenyl)imidazo[1,2-b]pyridazin-3-yl]pyridin-2-amine

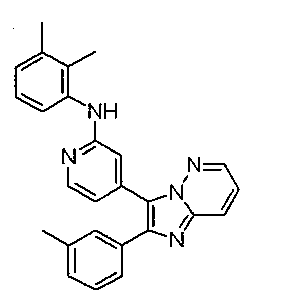

Melting point 187-188°C (ethyl acetate)
¹H-NMR (CDCl₃) δ: 2.12 (3H, s), 2.25 (3H, s), 2.37 (3H, s), 6.38 (1H, s), 6.75 (1H, s), 6.86-6.95 (2H, m), 6.97-7.05 (2H, m), 7.09 (1H, dd, J=9.1, 4.3Hz), 7.16-7.29 (2H, m), 7.38 (1H, d, J=7.4Hz), 7.55 (1H, s), 7.99 (1H, d, J=9.1Hz), 8.28 (1H, d, J=5 .1Hz), 8.32 (1H, d, J=4.3Hz).
Elemental analysis: for C₂₆H₂₃N₅
Calculated: C, 77.01; H, 5.72; N, 17.27.
Found: C, 76.71; H, 5.68; N, 17.05.

### Example compound 6-8: N-(4-methylphenyl)-4-[2-(3-methylphenyl)imidazo[1,2-b]pyridazin-3-yl]pyridin-2-amine

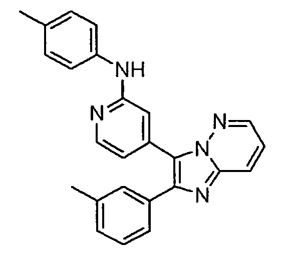

Melting point 213-214°C (ethyl acetate) .
¹H-NMR (CDCl₃) δ: 2.28 (3H, s), 2.39 (3H, s), 6.65 (1H, s), 6.98-7.07 (6H, m), 7.11 (1H, dd, J=9.0, 4.4Hz), 7.19-7.32 (2H, m), 7.42 (1H, d, J=7.4Hz), 7.60 (1H, s), 8.01 (1H, dd, J=9.0, 1.7Hz), 8.28 (1H, dd, J=4.9, 0.9Hz), 8.35 (1H, dd, J=4.4, 1.7Hz).
Elemental analysis: for C₂₅H₂₁N₅
Calculated: C, 76.70; H, 5.41; N, 17.89.
Found: C, 76.42; H, 5.42; N, 17.69.

### Example compound 6-9: N-(3-methylphenyl)-4-[2-(3-methylphenyl)imidazo[1,2-b]pyridazin-3-yl]pyridin-2-amine

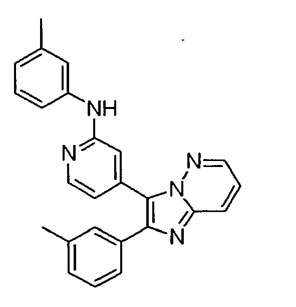

Melting point 195-196°C (ethyl acetate)
¹H-NMR (CDCl₃) δ: 2.27 (3H, s), 2.39 (3H, s), 6.61 (1H, s), 6.81 (1H, d, J=7.5Hz) 6.91-6.98 (1H, m), 7.01-7.23 (6H, m), 7.27 (1H, t, J=7.4Hz), 7.43 (1H, d, J=7.5Hz), 7.62 (1H, s), 8.02 (1H, dd, J=9.1, 1.6Hz), 8.30 (1H, d, J=5.3Hz), 8.36 (1H, dd, J=4.4, 1.6Hz).
Elemental analysis: for C₂₅H₂₁N₅
Calculated: C, 76.70; H, 5.41; N, 17.89.
Found: C, 76.58; H, 5.46; N, 17.68.

### Example compound 6-10: N-(2-methylphenyl)-4-[2-(3-methylphenyl)imidazo[1,2-b]pyridazin-3-yl]pyridin-2-amine

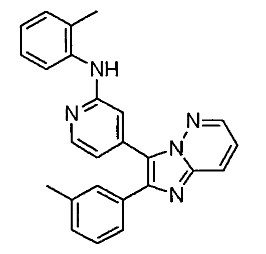

Melting point 208-209°C (ethyl acetate)
¹H-NMR (CDCl₃) δ: 2.25 (3H, s), 2.39 (3H, s), 6.36 (1H, s), 6.92-7.03 (3H, m), 7.06 (1H, dd, J=5.3, 1.6Hz), 7.08-7.13 (1H, m), 7.15-7.23 (3H, m), 7.27 (1H, t, J=7.5Hz), 7.40 (1H, d, J=7.5Hz), 7.58 (1H, s), 8.00 (1H, dd, J=9.1, 1.7Hz), 8.30 (1H, dd, J=5.3, 0.8Hz), 8.34 (1H, dd, J=4.5, 1.7Hz).
Elemental analysis: for C₂₅H₂₁N₅
Calculated: C, 76.70; H, 5.41; N, 17.89.
Found: C, 76.43; H, 5.44; N, 17.73.

### Example compound 6-11: 4-[2-(3-methylphenyl)imidazo[1,2-b]pyridazin-3-yl]-N-phenylpyridin-2-amine

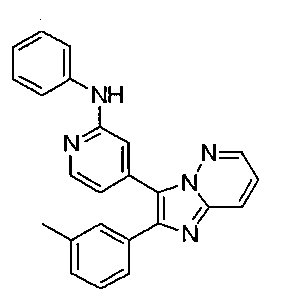

¹H-NMR (CDCl₃) δ: 2.40 (3H, s), 6.66 (1H, s), 6.95-7.02 (1H, m), 7.10-7.32 (9H, m), 7.42 (1H, d, J=7.5Hz), 7.61 (1H, s), 8.02 (1H, dd, J=9.2, 1.5Hz), 8.31 (1H, d, J=5.3Hz), 8.36 (1H, dd, J=4.3, 1.5Hz).

### Example compound 6-12: 4-[2-(3-methylphenyl)imidazo[1,2-b]pyridazin-3-yl]-N-(1,2,3,4-tetrahydronaphthalen-1-yl)pyridin-2-amine

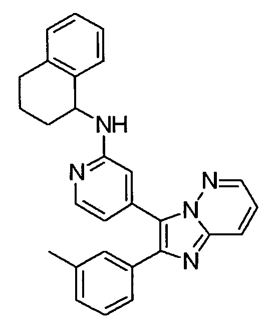

Melting point 195-196°C (ethyl acetate)
¹H-NMR (CDCl₃) δ: 1.78-2.05 (4H, m), 2.38 (3H, s), 2.71-2.88 (2H, m), 4.75 (1H, d, J=8.3Hz), 4.95-5.05 (1H, m), 6.71 (1H, s), 6.87 (1H, dd, J=5.3, 1.3Hz), 7.08-7.19 (5H, m), 7.25 (1H, t, J=7.5Hz), 7.36-7.42 (1H, m), 7.45 (1H, d, J=7.5Hz), 7.64 (1H, s), 8.02 (1H, dd, J=9.0, 1.7Hz), 8.22 (1H, d, J=5.3Hz), 8.35 (1H, dd, J=4.5, 1.7Hz).
Elemental analysis: for C₂₈H₂₅N₅
Calculated: C, 77.93; H, 5.84; N, 16.23.
Found: C, 77.90; H, 5.84; N, 16.09.

### Example compound 6-13: N-(2-methoxyethyl)-4-[2-(3-methylphenyl)imidazo[1,2-b]pyridazin-3-yl]pyridin-2-amine

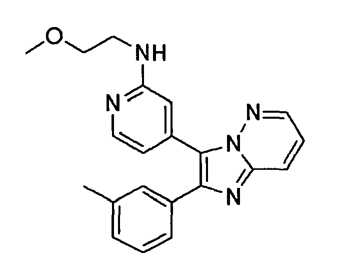

Melting point 99-100°C (ethyl acetate)
¹H-NMR (CDCl₃) δ: 2.37 (3H, s), 3.38 (3H, s), 3.48-3.61 (4H, m), 4.87 (1H, t, J=5.4Hz), 6.73 (1H, s), 6.80 (1H, dd, J=5.3, 1.4Hz), 7.10 (1H, dd, J=9.0, 4.4Hz), 7.17 (1H, d, J=7.4Hz), 7.21-7.25 (1H, m), 7.41 (1H, d, J=7.4Hz), 7.65 (1H, s), 8.02 (1H, dd, J=9.0, 1.7Hz), 8.18 (1H, d, J=5.3Hz), 8.34 (1H, dd, J=4.4, 1.7Hz).
Elemental analysis: for C₂₁H₂₁N₅O
Calculated: C, 70.17; H, 5.89; N, 19.48.
Found: C, 70.10; H, 5.89; N, 19.66.

### Example compound 6-14: 4-[2-(3-methylphenyl)imidazo[1,2-b]pyridazin-3-yl]-N-[2-(piperidin-1-yl)ethyl]pyridin-2-amine

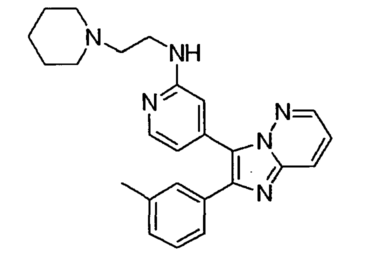

5 Melting point 143-144°C (ethyl acetate)
¹H-NMR (CDCl₃) δ: 1.42-1.46 (2H, m), 1.52-1.58 (4H, m), 2.37 (7H, s), 2.53 (2H, t, J=6.2Hz), 3.31 (2H, m), 5.22 (1H, t, J=5.0Hz), 6.70 (1H, s), 6.77 (1H, dd, J=5.2, 1.1Hz), 7.08 (1H, dd, J=9.6, 4.4Hz), 7.13-7.16 (1H, m), 7.19-7.23 (1H, m), 7.41 (1H, d, J=7.4Hz), 7.65 (1H, s), 7.99-8.02 (1H, m), 8.17 (1H, d, J=5.2Hz), 8.33 (1H, dd, J=4.4, 1.4Hz).
Elemental analysis: for C₂₅H₂₈N₆
Calculated: C, 72.79; H, 6.84; N, 20.37.
Found: C, 72.68; H, 6.74; N, 20.27.

### Example compound 6-15: 4-[2-(3-methylphenyl)imidazo[1,2-b]pyridazin-3-yl]-N-(2-thienylmethyl)pyridin-2-amine

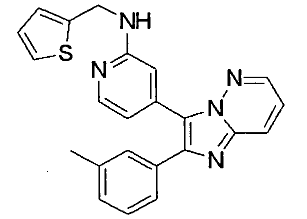

Melting point 185-186°C (ethyl acetate)
¹H-NMR (CDCl₃) δ: 2.37 (3H, s), 4.69 (2H, d, J=5.7Hz), 4.90 (1H, t, J=5.7Hz), 6.78 (1H, s), 6.88 (1H, dd, J=5.2, 1.5Hz), 6.92-6.98 (2H, m), 7.08-7.25 (4H, m), 7.41 (1H, d, J=7.4Hz), 7.62 (1H, s), 8.02 (1H, dd, J=1.7, 9.1Hz), 8.22 (1H, d, J=5.2Hz), 8.33 (1H, dd, J=4.5, 1.7Hz).
Elemental analysis: for C₂₃H₁₉N₅S
Calculated: C, 69.50; H, 4.82; N, 17.62.
Found: C, 69.27; H, 4.78; N, 17.56.

### Example compound 6-16: 4-[2-(3-methylphenyl)imidazo[1,2-b]pyridazin-3-yl]-N-(tetrahydrofuran-2-ylmethyl)pyridin-2-amine

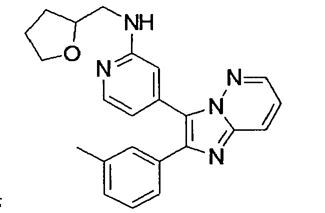

Melting point 137-138°C (ethyl acetate)
¹H-NMR (CDCl₃) δ: 1.61-1.72 (1H, m), 1.87-2.05 (3H, m), 2.37 (3H, s), 3.22-3.31 (1H, m), 3.54-3.62 (1H, m), 3.73-3.81 (1H, m), 3.85-3.92 (1H, m), 4.06-4.14 (1H, m), 4.87 (1H, t, J=5.2Hz), 6.74 (1H, d, J=0.8Hz), 6.79 (1H, dd, J=5.2, 1.4Hz), 7.08-7.25 (3H, m), 7.41 (1H, d, 7.4Hz), 7.64 (1H, s), 8.02 (1H, dd, J=9.1, 1.7Hz), 8.17 (1H, d, J=5.2Hz), 8.34 (1H, dd, J=4.5, 1.7Hz).
Elemental analysis: for C₂₃H₂₃N₅O
Calculated: C, 71.67; H, 6.01; N, 18.17.
Found: C, 71.66; H, 5.98; N, 18.29.

### Example compound 6-17: N-(2-furylmethyl)-4-[2-(3-methylphenyl)imidazo[1,2-b]pyridazin-3-yl]pyridin-2-amine

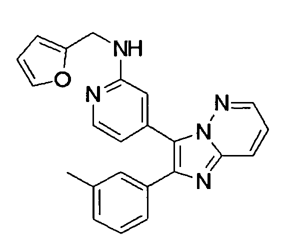

Melting point 150-151°C (ethyl acetate)
¹H-NMR (CDCl₃) δ: 2.37 (3H, s), 4.51 (2H, d, J=5.7Hz), 4.87 (1H, t, J=5.7Hz), 6.20 (1H, d, J=3.3Hz), 6.31 (1H, dd, J=3.3, 1.8Hz), 6.79 (1H, d, J=0.8Hz), 6.86 (1H, m), 7.09-7.25 (3H, m), 7.35 (1H, dd, J=1.9, 0.8Hz), 7.40 (1H, d, J=7.7Hz), 7.63 (1H, s), 8.02 (1H, dd, J=9.1, 1.7Hz), 8.21 (1H, d, J=5.5Hz), 8.34 (1H, dd, J=4.4, 1.7Hz).
Elemental analysis: for C₂₃H₁₉N₅O
Calculated: C, 72.42; H, 5.02; N, 18.36.
Found: C, 72.38; H, 5.05; N, 18.36.

### Example compound 6-18: N-(2,2-dimethylpropyl)-4-[2-(3-methylphenyl)imidazo[1,2-b]pyridazin-3-yl]pyridin-2-amine

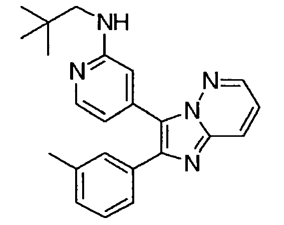

Melting point 157-158°C (ethyl acetate)
¹H-NMR (CDCl₃) δ: 0.95 (9H, s), 2.37 (3H, s), 3.02 (2H, d, J=6.0Hz), 4.63 (1H, m), 6.67 (1H, d, J=0.8Hz), 6.80 (1H, dd, J=5.2, 1.5Hz) 7.07-7.24 (3H, m), 7.42 (1H, d, J=7.7Hz), 7.63 (1H, s), 8.01 (1H, dd, J=9.1, 1.6Hz), 8.15 (1H, dd, J=5.2, 0.8Hz), 8.34 (1H, dd, J=4.4, 1.6Hz).
Elemental analysis: for C₂₃H₂₅N₅
Calculated: C, 74.36; H, 6.78; N, 18.85.
Found: C, 74.27; H, 6.74; N, 18.80.

### Example compound 6-19: N,N-diethyl-N'-{4-[2-(3-methylphenyl)imidazo[1,2-b]pyridazin-3-yl]pyridin-2-yl}ethane-1,2-diamine

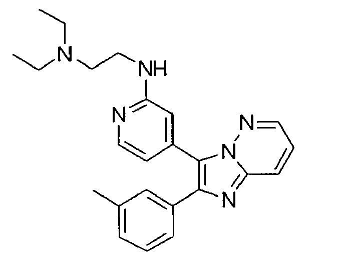

Melting point 97-98°C (ethyl acetate)
¹H-NMR (CDCl₃) δ: 1.02 (6H, t, J=7.2Hz), 2.37 (3H, s), 2.56 (4H, q, J=7.2Hz), 2.68 (2H, t, J=6.0Hz), 3.30-3.32 (2H, m), 5.22 (1H, m), 6.72 (1H, s), 6.78 (1H, m), 7.08-7.26 (3H, m), 7.43 (1H, d, J=8.0Hz), 7.66 (1H, s), 8.02 (1H, dd, J=9.2, 1.8Hz), 8.18 (1H, dd, J=5.1, 0.9Hz), 8.35 (1H, dd, J=4.5, 1.8Hz).
Elemental analysis: for C₂₄H₂₈N₆
Calculated: C, 71.97; H, 7.05; N, 20.98.
Found: C, 71.78; H, 7.01; N, 21.10.

### Example compound 6-20: N-(1-ethylpropyl)-4-[2-(3-methylphenyl)imidazo[1,2-b]pyridazin-3-yl]pyridin-2-amine

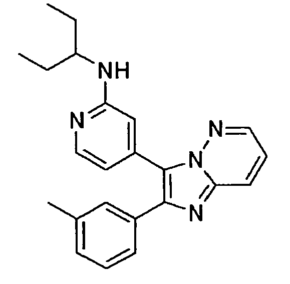

Melting point 155-156°C (ethyl acetate)
¹H-NMR (CDCl₃) δ: 0.87 (6H, t, J=7.4Hz), 1.35-1.48 (2H, m), 1.49-1.62 (2H, m), 2.36 (3H, s), 3.31-3.42 (1H, m), 4.40 (1H, d, J=8.9Hz), 6.57 (1H, s), 6.85 (1H, dd, J=5.3, 1.5Hz), 7.10 (1H, dd, J=9.0, 4.5Hz), 7.13-7.26 (2H, m), 7.45 (1H, d, J=7.5Hz), 7.62 (1H, s), 8.02 (1H, dd, J=9.0, 1.7Hz), 8.17 (1H, d, J=5.3Hz), 8.35 (1H, dd, J=4.5, 1.7Hz).
Elemental analysis: for C₂₃H₂₅N₅
Calculated: C, 74.36; H, 6.78; N, 18.85.
Found: C, 74.23; H, 6.69; N, 18.62.

### Example compound 6-21: 4-[2-(3-methylphenyl)imidazo[1,2-b]pyridazin-3-yl]-N-[(1S)-1-phenylethyl]pyridin-2-amine

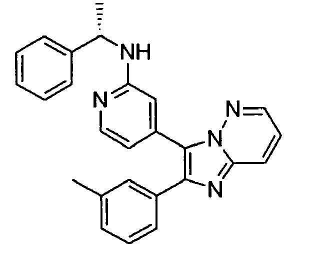

Melting point 154-155°C (ethyl acetate)
¹H-NMR (CDCl₃) δ: 1.52 (3H, d, J=6.8Hz), 2.37 (3H, s), 4.58-4.67 (1H, m), 5.01 (1H, d, J=6.0Hz), 6.55 (1H, s), 6.84 (1H, dd, J=5.3, 1.5Hz), 7.06 (1H, dd, J=9.1, 4.4Hz), 7.15-7.29 (7H, m), 7.35 (1H, d, J=7.0Hz), 7.56 (1H, s), 7.97 (1H, dd, J=9.1, 1.7Hz), 8.15 (1H, d, J=5.3Hz), 8.22 (1H, dd, J=4.4, 1.7Hz).
Elemental analysis: for C₂₆H₂₃N₅
Calculated: C, 77.01; H, 5.72; N, 17.27.
Found: C, 76.97; H, 5.68; N, 17.35.

### Example compound 6-22: 4-[2-(3-methylphenyl)imidazo[1,2-b]pyridazin-3-yl]-N-[(1R)-1-phenylethyl]pyridin-2-amine

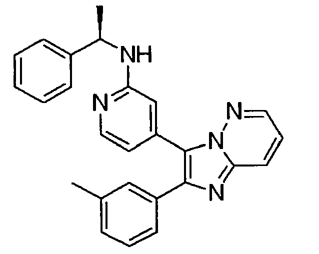

Melting point 154-155°C (ethyl acetate)
¹H-NMR (CDCl₃) δ: 1.52 (3H, d, J=6.8Hz), 2.37 (3H, s), 4.58-4.68 (1H, m), 5.02 (1H, d, J=6.0Hz), 6.55 (1H, s), 6.84 (1H, dd, J=5.3, 1.5Hz), 7.05 (1H, dd, J=9.1, 4.4Hz), 7.15-7.29 (7H, m), 7.35 (1H, d, J=7.0Hz) , 7.57 (1H, s), 7.97 (1H, dd, J=9.1, 1.7Hz), 8.16 (1H, d, J=5.3Hz), 8.22 (1H, dd, J=4.4, 1.7Hz).
Elemental analysis: for C₂₆H₂₃N₅
Calculated: C, 77.01; H, 5.72; N, 17.27.
Found: C, 77.08; H, 5.74; N, 17.31.

### Example compound 6-23: N-benzyl-4-[2-(3-methylphenyl)imidazo[1,2-b]pyridazin-3-yl]pyridin-2-amine

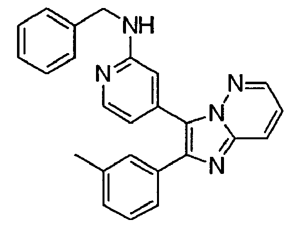

Melting point 193-194°C (ethyl acetate)
¹H-NMR (CDCl₃) δ: 2.37 (3H, s), 4.48 (2H, d, J=5.7Hz), 4.92 (1H, t, J=5.7Hz), 6.72 (1H, s), 6.86 (1H, dd, J=5.2, 1.2Hz), 7.09 (1H, dd, J=9.1, 4.4Hz), 7.16-7.33 (7H, m), 7.40 (1H, d, J=7.4Hz), 7.62 (1H, s), 8.01 (1H, dd, J=9.1, 1.7Hz), 8.20 (1H, d, J=5.2Hz), 8.30 (1H, dd, J=4.4, 1.7Hz).
Elemental analysis: for C₂₅H₂₁N₅
Calculated: C, 76.70; H, 5.41; N, 17.89.
Found: C, 76.44; H, 5.36; N, 17.70.

### Example compound 6-24: N-butyl-4-[2-(3-methylphenyl)imidazo[1,2-b]pyridazin-3-yl]pyridin-2-amine

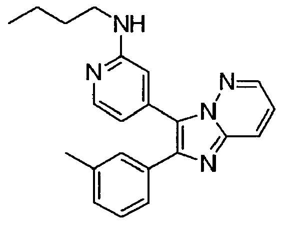

Melting point 128-129°C (ethyl acetate)
¹H-NMR (CDCl₃) δ: 0.93 (3H, t, J=7.3Hz), 1.32-1.46 (2H, m), 1.52-1.61 (2H, m), 2.37 (3H, s), 3.16-3.27 (2H, m), 4.58 (1H, t, J=5.4Hz), 6.66 (1H, s), 6.82 (1H, dd, J=5.3, 1.1Hz), 7.10 (1H, dd, J=9.1, 4.4Hz), 7.16 (1H, d, J=7.5Hz), 7.23 (1H, t, J=7.5Hz), 7.43 (1H, d, J=7.5Hz), 7.65 (1H, s), 8.02 (1H, dd, J=9.1, 1.6Hz), 8.18 (1H, d, J=5.3Hz), 8.34 (1H, dd, J=4.4, 1.6Hz).
Elemental analysis: for C₂₂H₂₃N₅
Calculated: C, 73.92; H, 6.49; N, 19.59.
Found: C, 74.01; H, 6.56; N, 19.59.

### Example compound 6-25: 4-[2-(3-methylphenyl)imidazo[1,2-b]pyridazin-3-yl]-N-propylpyridin-2-amine

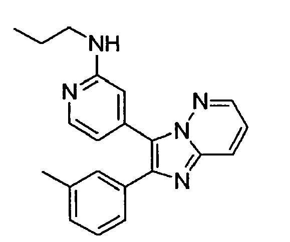

Melting point 128-129°C (ethyl acetate)
¹H-NMR (CDCl₃) δ: 0.96 (3H, t, J=7.5Hz), 1.54-1.66 (2H, m), 2.37 (3H, s), 3.16-3.22 (2H, m), 4.59 (1H, t, J=5.7Hz), 6.66 (1H, s), 6.82 (1H, dd, J=5.2, 1.4Hz), 7.10 (1H, dd, J=9.1, 4.4Hz), 7.16 (1H, m), 7.20-7.25 (1H, m), 7.43 (1H, d, J=7.4Hz), 7.65 (1H, s), 8.02 (1H, dd, J=9.1, 1.7Hz), 8.18 (1H, d, J=5.2Hz), 8.35 (1H, dd, J=4.4, 1.7Hz).
Elemental analysis: for C₂₁H₂₁N₅
Calculated: C, 73.44; H, 6.16; N, 20.39.
Found: C, 73.52; H, 6.13; N, 20.42.

### Example compound 6-26: N-ethyl-4-[2-(3-methylphenyl)imidazo[1,2-b]pyridazin-3-yl]pyridin-2-amine

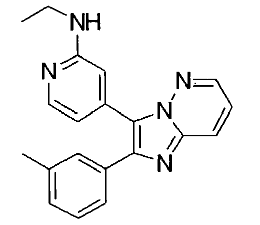

Melting point 151-153°C (ethyl acetate)
¹H-NMR (CDCl₃) δ: 1.23 (3H, t, J=7.1Hz), 2.37 (3H, s), 3.23-3.32 (2H, m), 4.53 (1H, t, J=5.1Hz), 6.67-6.68 (1H, m), 6.81 (1H, dd, J=5.1, 1.7Hz), 7.10 (1H, dd, J=9.3, 4.5Hz), 7.16 (1H, d, J=7.4Hz), 7.20-7.25 (1H, m), 7.42-7.44 (1H, m), 7.66 (1H, s), 8.02 (1H, dd, J=9.3, 1.8Hz), 8.18 (1H, dd, J=5.1, 0.9Hz), 8.34 (1H, dd, J=4.5, 1.8Hz).
Elemental analysis: for C₂₀H₁₉N₅
Calculated: C, 72.93; H, 5.81; N, 21.26.
Found: C, 73.08; H, 5.72; N, 21.33.

### 5 (Example 7)

### 3-[2-(cyclohexylamino)pyridin-4-yl]-N,N-dimethyl-2-(3-methylphenyl)imidazo[1,2-b]pyridazin-6-amine

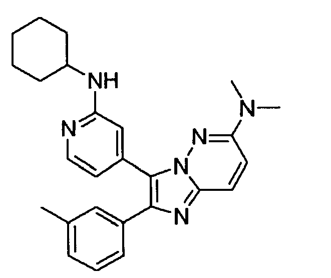

4-[6-Chloro-2-(3-methylphenyl)imidazo[1,2-b]pyridazin-3-yl]-N-cyclohexylpyridin-2-amine (100 mg, 0.239 mmol) obtained in Example 2 was stirred in 2.0 M dimethylamine-tetrahydrofuran solution (2.0 mL) under microwave irradiation at 150°C for 3.5 hr. The reaction mixture was allowed to be cooled to room temperature. To the reaction mixture was added saturated aqueous sodium hydrogen carbonate solution, and the mixture was extracted with ethyl acetate. The extract was washed with saturated brine, dried over anhydrous sodium sulfate, and the solvent was removed by evaporation under reduced pressure. The obtained residue was purified by silica gel column chromatography (ethyl acetate: hexane = 3:7 - 8:2) and recrystallized from ethyl acetate to give the title compound (yield 78.8 mg, 77%).

Melting point 191-192°C (ethyl acetate)
¹H-NMR (CDCl₃) δ: 1.09-1.72 (8H, m), 1.91-1.94 (2H, m), 2.35 (3H, s), 3.10 (6H, s), 3.27-3.32 (1H, m), 4.45 (1H, d, J=7.4Hz), 6.65 (1H, s), 6.80 (1H, d, J=9.8Hz), 6.93 (1H, dd, J=5.4Hz, 1.4Hz), 7.09-7.11 (1H, m), 7.17-7.22 (1H, m), 7.37 (1H, d, J=7.2Hz), 7.54 (1H, s), 7.74 (1H, d, J=9.8Hz), 8.07 (1H, dd, J=5.4, 0.8Hz).
Elemental analysis: for C₂₆H₃₀N₆
Calculated: C, 73.21; H, 7.09; N, 19.70.
Found: C, 73.05; H, 7.09; N, 19.69.

### (Example 8)

The compounds of Examples 8-1 - 8-3 below were synthesized in the same manner as in Example 7 respectively using 30% methylamine-ethanol solution, cyclohexylamine or benzylamine, instead of 2.0 M dimethylamine-tetrahydrofuran solution.

### Example compound 8-1: 3-[2-(cyclohexylamino)pyridin-4-yl]-N-methyl-2-(3-methylphenyl)imidazo[1,2-b]pyridazin-6-amine

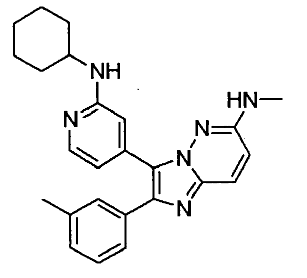

¹H-NMR (CDCl₃) δ: 1.16-1.30 (5H, m), 1.61-1.75 (3H, m), 1.94 (2H, d, J=11.3Hz), 2.35 (3H, s), 2.96 (3H, d, J=5.1Hz), 3.24-3.38 (1H, m), 4.43 (1H, d, J=5.1Hz), 4.47 (1H, d, J=8.4Hz), 6.48 (1H, d, J=9.6Hz), 6.68 (1H, s), 6.93 (1H, dd, J=5.3, 0.9Hz), 7.11 (1H, d, J=7.5Hz), 7.20 (1H, t, J=7.5Hz), 7.37 (1H, d, J=7.5Hz), 7.55 (1H, s), 7.68 (1H, d, J=9.6Hz), 8.08 (1H, d, J=5.3Hz).

### Example compound 8-2: N-cyclohexyl-3-[2-(cyclohexylamino)pyridin-4-yl]-2-(3-methylphenyl)imidazo[1,2-b]pyridazin-6-amine

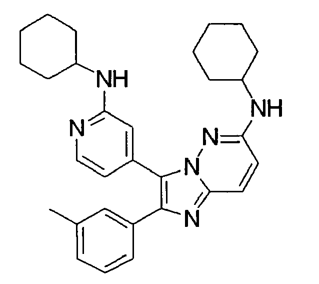

¹H-NMR (CDCl₃) δ: 1.12-1.80 (16H, m), 1.90-1.95 (2H, m), 2.08-2.12 (2H, m), 2.35 (3H, s), 3.24-3.36 (1H, m), 3.57-3.71 (1H, m), 4.24 (1H, d, J=7.4Hz), 4.43 (1H, d, J=7.4Hz), 6.44 (1H, d, J=9.5Hz), 6.60 (1H, s), 6.94 (1H, dd, J=5.5, 1.2Hz), 7.11 (1H, m), 7.20 (1H, t, J=7.4Hz), 7.38 (1H, d, J=7.4Hz), 7.55 (1H, s), 7.67 (1H, d, J=9.5Hz), 8.08 (1H, d, J=5.5Hz).

### Example compound 8-3: N-benzyl-3-[2-(cyclohexylamino)pyridin-4-yl]-2-(3-methylphenyl)imidazo[1,2-b]pyridazin-6-amine

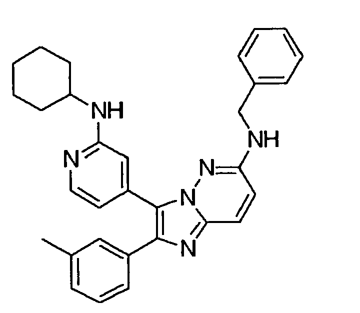

¹H-NMR (CDCl₃) δ: 1.13-1.29 (5H, m), 1.52-1.73 (3H, m), 1.87-1.97 (2H, m), 2.34 (3H, s), 3.25-3.37 (1H, m), 4.34 (1H, d, J=8.0Hz), 4.52 (2H, d, J=5.5Hz), 4.74-4.80 (1H, m), 6.50 (1H, d, J=9.6Hz), 6.54 (1H, s), 6.75 (1H, dd, J=5.2, 1.4Hz), 7.09 (1H, d, J=8.0Hz), 7.16-7.21 (1H, m), 7.24-7.36 (6H, m), 7.52 (1H, s), 7.69 (1H, d, J=9.6Hz), 8.02 (1H, dd, J=5.2, 0.7Hz).

### (Example 9)

### N-Cyclohexyl-4-[6-methoxy-2-(3-methylphenyl)imidazo[1,2-b]pyridazin-3-yl]pyridin-2-amine

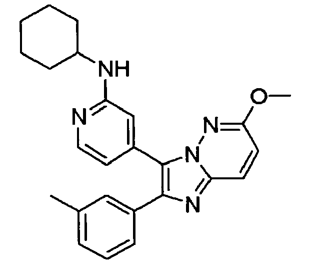

To a solution of 4-[6-chloro-2-(3-methylphenyl)imidazo[1,2-b]pyridazin-3-yl]-N-cyclohexylpyridin-2-amine (300 mg, 0.718 mmol) obtained in Example 2 in methanol (6 mL) was added 28% sodium methoxide-methanol solution (0.20 mL), and the mixture was heated under reflux for 15 hr. The solvent was removed by evaporation under reduced pressure. Water was added to the residue, and the mixture was extracted with ethyl acetate. The extract was dried over sodium sulfate, and the solvent was removed by evaporation under reduced pressure. The obtained residue was purified by silica gel chromatography (ethyl acetate: hexane = 3:7 - 8:2) and recrystallized from ethyl acetate to give the title compound (yield 184 mg, 62%).
Melting point 188-189°C (ethyl acetate)
¹H-NMR (CDCl₃) δ: 1.10-1.32 (5H, m), 1.57-1.75 (3H, m), 1.94 (2H, d, J=11.1Hz), 2.36 (3H, s), 3.24-3.36 (1H, m), 3.97 (3H, s), 4.50 (1H, d, J=8.1Hz), 6.61 (1H, s), 6.76 (1H, d, J=9.6Hz), 6.90 (1H, dd, J=5.3, 1.3Hz), 7.13-7.16 (1H, m), 7.22 (1H, t, J=7.5Hz), 7.38 (1H, d, J=7.5Hz), 7.56 (1H, s), 7.86 (1H, d, J=9.6Hz), 8.12 (1H, d, J=5.3Hz).
Elemental analysis: for C₂₅H₂₇N₅O
Calculated: C, 72.61; H, 6.58; N, 16.94.
Found: C, 72.46; H, 6.53; N, 16.90.

### (Example 10)

### N-Cyclohexyl-4-[2-(3-methylphenyl)-6-(methylthio)imidazo[1,2-b]pyridazin-3-yl]pyridin-2-amine

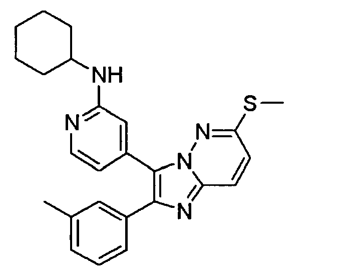

To a solution of 4-[6-chloro-2-(3-methylphenyl)imidazo[1,2-b]pyridazin-3-yl]-N-cyclohexylpyridin-2-amine (500 mg, 1.20 mmol) obtained in Example 2 in ethanol (7 mL) was added 15% sodium methanethiolate-ethanol solution (3.0 mL), and the mixture was heated under reflux for 2 hr. The solvent was removed by evaporation under reduced pressure. Water was added to the residue, and the mixture was extracted with ethyl acetate. The extract was dried over sodium sulfate, and the solvent was removed by evaporation under reduced pressure. The obtained residue was purified by silica gel chromatography (ethyl acetate: hexane = 3:7 - 8:2) and recrystallized from ethyl acetate to give the title compound (yield 411 mg, 86%).
Melting point 150-151°C (ethyl acetate)
¹H-NMR (CDCl₃) δ: 1.09-1.33 (5H, m), 1.63-1.72 (3H, m), 1.92 (2H, d, J=10.7Hz),2.36 (3H, s), 2.57 (3H, s), 3.25-3.37 (1H, m), 4.48 (1H, d, J=8.1Hz), 6.56 (1H,s), 6.89-6.97 (2H, m), 7.14 (1H, d, J=7.7Hz), 7.22 (1H, t, J=7.7Hz), 7.41 (1H, d, J=7.7Hz), 7.58 (1H, s), 7.78 (1H, d, J=9.2Hz), 8.13 (1H, d, J=5.3Hz).
Elemental analysis: for C₂₅H₂₇N₅S
Calculated: C, 69.90; H, 6.34; N, 16.30.
Found: C, 69.84; H, 6.31; N, 16.33.

### (Example 11)

### N-Cyclohexyl-4-[2-(3-methylphenyl)-6-(methylsulfonyl)imidazo[1,2-b]pyridazin-3-yl]pyridin-2-amine

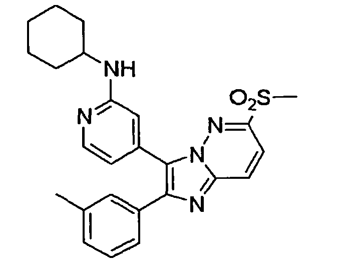

To a solution of N-cyclohexyl-4-[2-(3-methylphenyl)-6-(methylthio)imidazo[1,2-b]pyridazin-3-yl]pyridin-2-amine (300 mg, 698 mmol) obtained in Example 10 in methanol (10 mL) was added a solution of Oxone (registered trade mark, 1.00 g, 1.63 mmol) in water (10 mL), and the mixture was stirred at room temperature for 3 hr. The solvent was removed by evaporation under reduced pressure. Saturated aqueous sodium hydrogen carbonate solution was added to the residue, and the mixture was extracted with ethyl acetate. The extract was dried over sodium sulfate, and the solvent was removed by evaporation under reduced pressure. The obtained residue was purified by silica gel chromatography (ethyl acetate: hexane = 3:7 - 8:2) and recrystallized from ethyl acetate to give the title compound (yield 134 mg, 42%).
Melting point 196-197°C (ethyl acetate)
¹H-NMR (CDCl₃) δ: 1.12-1.36 (5H, m), 1.63-1.74 (3H, m), 1.94-1.98 (2H, m), 2.38 (3H, s), 3.30 (3H, s), 3.34-3.38 (1H, m), 4.59 (1H, d, J=8.1Hz), 6.57 (1H, s), 6.79 (1H, dd, J=5.2, 1.4Hz), 7.19-7.21 (1H, m), 7.26 (1H, t, J=7.7Hz), 7.45 (1H,d, J=7.7Hz), 7.62 (1H, s), 7.74 (1H, d, J=9.3Hz), 8.16 (1H, dd, J=5.2, 0.6Hz), 8.22 (1H, d, J=9.3Hz).
Elemental analysis: for C₂₅H₂₇N₅O₂S
Calculated: C, 65.05; H, 5.90; N, 15.17.
Found: C, 65.18; H, 5.96; N, 15.34.

### (Example 12)

### 4-[6-Chloro-2-(3-methylphenyl)imidazo[1,2-b]pyridazin-3-yl]pyridin-2-amine

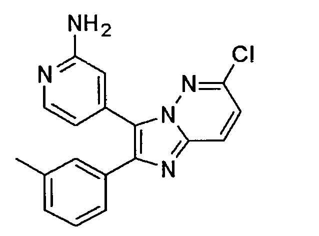

2-(2-Aminopyridin-4-yl)-2-bromo-1-(3-methylphenyl)ethanone hydrobromide (20.0 g, 51.8 mmol) obtained by the method described in WO01/74811 and 3-amino-6-chloropyridazine (6.71 g, 51.8 mmol) were heated under reflux for 14 hr in ethanol (200 mL). The mixture was allowed to be cooled to room temperature, and the solvent was removed by evaporation under reduced pressure. Saturated aqueous sodium hydrogen carbonate solution was added to the residue. The precipitated solid was collected by filtration, washed with water and dried. The obtained powder was dissolved in tetrahydrofuran and filtrated, and the solvent was removed by evaporation under reduced pressure. The obtained crude crystals were recrystallized from ethyl acetate to give the title compound (yield 10.5 g, 60%).
Melting point 247-248°C (ethyl acetate)
¹H-NMR (CDCl₃) δ: 2.36 (3H, s), 4.51 (2H, brs), 6.80 (1H, dd, J=1.4, 0.8Hz), 6.83 (1H, ddd, J=5.2, 1.4, 0.8Hz), 7.11 (1H, d, J=9.3Hz), 7.14-7.24 (2H, m), 7.36 (1H, d, J=7.4Hz), 7.58 (1H, s), 7.94 (1H, d, J=9.3Hz), 8.14 (1H, d, J=5.2Hz).
Elemental analysis: for C_{1B}H₁₄ClN₅·0.75H₂O
Calculated: C, 61.89; H, 4.47; N, 20.05.
Found: C, 61.75; H, 4.39; N, 20.34.

### (Example 13)

The compounds of Examples 13-1 - 13-4 below were synthesized in the same manner as in Example 12 respectively using 2-(2-aminopyridin-4-yl)-2-bromo-1-(3-chlorophenyl)ethanone hydrobromide, 2-(2-aminopyridin-4-yl)-2-bromo-1-(4-fluoro-3-methylphenyl)ethanone hydrobromide, 2-(2-aminopyridin-4-yl)-2-bromo-1-(4-chlorophenyl)ethanone hydrobromide or 2-(2-aminopyridin-4-yl)-2-bromo-1-(4-fluorophenyl)ethanone hydrobromide obtained by the method described in WO 01/74811, instead of 2-(2-aminopyridin-4-yl)-2-bromo-1-(3-methylphenyl)ethanone hydrobromide.

### Example compound 13-1: 4-[6-chloro-2-(3-chlorophenyl)imidazo[1,2-b]pyridazin-3-yl]pyridin-2-amine

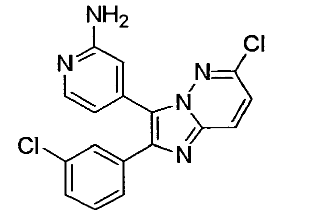

Melting point 268-269°C (tetrahydrofuran)
¹H-NMR (DMSO-d₆) δ: 6.19 (2H, brs), 6.57 (1H, dd, J=5.2, 1.4Hz), 6.60 (1H, dd, J=1.4, 0.8Hz), 7.40-7.44 (2H, m), 7.47 (1H, d, J=9.3Hz), 7.52-7.59 (1H, m), 7.68-7.72 (1H, m), 8.06 (1H, dd, J=5.2, 0.8Hz), 8.31 (1H, d, J=9.3Hz).
Elemental analysis: for C₁₇H₁₁Cl₂N₅
Calculated: C, 57.32; H, 3.11; N, 19.66.
Found: C, 57.13; H, 3.08; N, 19.46.

### Example compound 13-2: 4-[6-chloro-2-(4-fluoro-3-methylphenyl)imidazo[1,2-b]pyridazin-3-yl]pyridin-2-amine

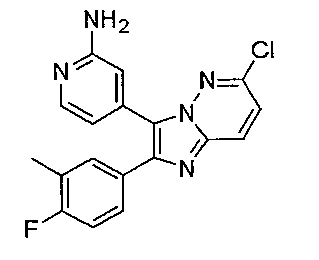

The obtained crude crystals were directly used for the reaction of Example 16 without purification.
¹H-NMR (DMSO-d₆) δ: 2.24 (3H, d, J=1.5Hz), 6.21 (2H, brs), 6.58 (1H, dd, J=5.3, 1.3Hz), 6.63 (1H, s), 7.12-7.20 (1H, m), 7.34-7.42 (1H, m), 7.46 (1H, d, J=9.5Hz), 7.67 (1H, dd, J=7.5, 1.5Hz), 8.04 (1H, dd, J=5.3, 0.4Hz), 8.29 (1H, d, J=9.5Hz).

### Example compound 13-3: 4-[6-chloro-2-(4-chlorophenyl)imidazo[1,2-b]pyridazin-3-yl]pyridin-2-amine

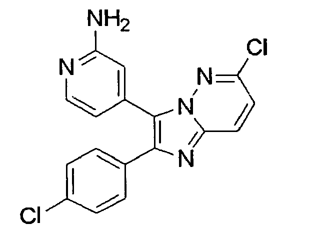

The obtained crude crystals were directly used for the reaction of Example 16 without purification.
¹H-NMR (DMSO-d₆) δ: 6.17 (2H, brs), 6.67 (1H, dd, J=5.4, 1.2Hz), 6.59 (1H, s), 7.46 (1H, d, J=9.5Hz), 7.47 (2H, d, J=8.7Hz), 7.65 (2H, d, J=8.7Hz), 8.04 (1H, d, J=5.4Hz), 8.29 (1H, d, J=9.5Hz). Example compound 13-4: 4-[6-chloro-2-(4-fluorophenyl)imidazo[1,2-b]pyridazin-3-yl]pyridin-2-amine

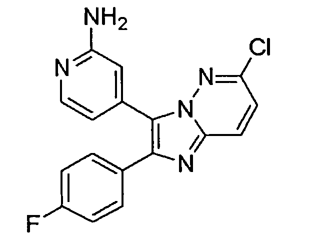

The obtained crude crystals were directly used for the reaction of Example 16 without purification.

### (Example 14)

### 4-[2-(3-Methylphenyl)imidazo[1,2-b]pyridazin-3-yl]pyridin-2-amine

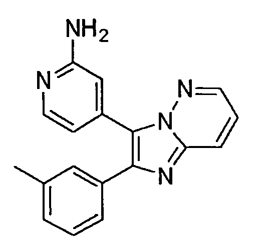

4-[6-Chloro-2-(3-methylphenyl)imidazo[1,2-b]pyridazin-3-yl]pyridin-2-amine (5.00 g, 14.9 mmol) obtained in Example 12 and 10% palladium carbon powder (50% water-containing product) (1.00 g) were stirred in acetic acid solvent (50 mL) under hydrogen atmosphere at 50°C for 3 hr at the hydrogen pressure of 0.5 MPa. The reaction mixture was allowed to be cooled to room temperature and filtrated, and the solvent was removed by evaporation under reduced pressure. Saturated aqueous sodium hydrogen carbonate solution was added to the residue, and the precipitated crude crystals were collected by filtration. The crude crystals were washed with water and dried. The obtained crude crystals were recrystallized from ethanol to give the title compound (yield 2.43 g, 54%).
Melting point 188-189°C (ethanol)
MS (ESI+): 302 (M+H).
¹H-NMR (CDCl₃) δ: 2.37 (3H, s), 4.50 (2H, brs), 6.83 (1H, dd, J=1.4, 0.8Hz), 6.87 (1H, dd, J=5.2, 1.4Hz), 7.10 (1H, dd, J=9.3, 4.4Hz), 7.13-7.19 (1H, m), 7.22 (1H, t, J=7.1Hz), 7.37-7.42 (1H, m), 7.60-7.63 (1H, m), 8.01 (1H, dd, J=9.3, 1.6Hz), 8.14 (1H, dd, J=5.2, 0.8Hz), 8.33 (1H, dd, J=4.4, 1.6Hz).
Elemental analysis: for C₁₈H₁₅N₅·0.5H₂O
Calculated: C, 69.66; H, 5.20; N, 22.57.
Found: C, 69.72; H, 5.18; N, 22.40.

### (Example 15)

### 4-[2-(3-Chlorophenyl)imidazo[1,2-b]pyridazin-3-yl]pyridin-2-amine

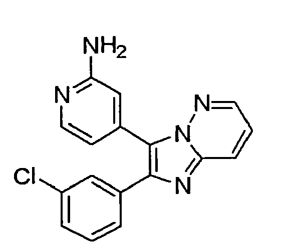

4-[6-Chloro-2-(3-chlorophenyl)imidazo[1,2-b]pyridazin-3-yl]pyridin-2-amine (5.62 g, 15.8 mmol) obtained in Example 13-1, 10% palladium carbon powder (50% water-containing product) (0.56 g) and triethylamine (4.4 mL, 31.6 mmol) were stirred in N,N-dimethylformamide (200 mL) under hydrogen atmosphere at room temperature for 1 hr. The mixture was filtrated, and the solvent was removed by evaporation under reduced pressure. Saturated aqueous sodium hydrogen carbonate solution was added to the residue, and the precipitated crude crystals were collected by filtration. The crude crystals were washed with water and dried to give the title compound (yield 4.83 g, 95%). The crude crystals were directly used for the reactions of Examples 21 and 28 without further purification.
MS (ESI+): 322 (M+H).
¹H-NMR (DMSO-d₆) δ: 6.13 (2H, brs), 6.59 (1H, dd, J=5.3, 1.3Hz), 6.65 (1H, s), 7.35 (1H, dd, J=9.2, 4.3Hz), 7.39-7.45 (2H, m), 7.56-7.62 (1H, m), 7.71-7.76 (1H, m), 8.04 (1H, dd, J=5.3, 0.6Hz), 8.23 (1H, dd, J=9.2, 1.7Hz), 8.55 (1H, dd, J=4.3, 1. 7Hz).

### (Example 16)

The compounds of Examples 16-1 - 16-3 below were synthesized in the same manner as in Example 15 respectively using 4-[6-chloro-2-(4-fluoro-3-methylphenyl)imidazo[1,2-b]pyridazin-3-yl]pyridin-2-amine (Example compound 13-2), 4-[6-chloro-2-(4-chlorophenyl)imidazo[1,2-b]pyridazin-3-yl]pyridin-2-amine (Example compound 13-3) or 4-[6-chloro-2-(4-fluorophenyl)imidazo[1,2-b]pyridazin-3-yl]pyridin-2-amine (Example compound 13-4), instead of 4-[6-chloro-2-(3-chlorophenyl)imidazo[1,2-b]pyridazin-3-yl]pyridin-2-amine. Example compound 16-1: 4-[2-(4-fluoro-3-methylphenyl)imidazo[1,2-b]pyridazin-3-yl]pyridin-2-amine

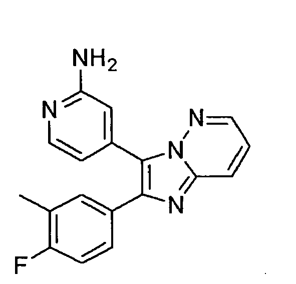

4-[6-Chloro-2-(4-fluoro-3-methylphenyl)imidazo[1,2-b]pyridazin-3-yl]pyridin-2-amine (8.0 g, 23 mmol) obtained in Example 13-2, 10% palladium carbon powder (50% water-containing product) (0.80 g) and triethylamine (6.3 mL, 45 mmol) were stirred in N,N-dimethylformamide (320 mL) at room temperature for 2 hr under hydrogen atmosphere. The mixture was filtrated, and the solvent was removed by evaporation under reduced pressure. Saturated aqueous sodium hydrogen carbonate solution was added to the residue, and the resulted mixture was extracted with ethyl acetate/tetrahydrofuran. The extract was dried over anhydrous sodium sulfate, and the solvent was removed by evaporation under reduced pressure to give the title compound (yield 7.2 g, 100%).
Melting point 179-180°C (ethanol)
¹H-NMR (CDCl₃) δ: 2.29 (3H, d, J=1.9Hz), 4.52 (2H, brs), 6.82 (1H, dd, J=1.6, 0.6Hz), 6.85 (1H, dd, J=5.4, 1.6Hz), 6.96 (1H, dd, J=9.3, 8.5Hz), 7.11 (1H, dd, J=9.2, 4.4Hz), 7.35-7.41 (1H, m), 7.63 (1H, dd, J=8.0, 2.1Hz), 8.00 (1H, dd, J=9.2, 1.7Hz), 8.15 (1H, dd, J=5.4, 0.6Hz), 8.34 (1H, dd, J=4.4, 1.7Hz).
Elemental analysis: for C₁₈H₁₄FN₅
Calculated: C, 67.70; H, 4.42; N, 21.93.
Found: C, 67.67; H, 4.43; N, 21.85.

### Example compound 16-2: 4-[2-(4-chlorophenyl)imidazo[1,2-b]pyridazin-3-yl]pyridin-2-amine

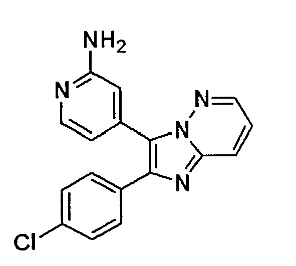

Melting point 252-253°C (ethyl acetate)
¹H-NMR (DMSO-d₆) δ: 6.10 (2H, brs), 6.57 (1H, dd, J=5.2, 1.7Hz), 6.63 (1H, s), 7.32 (1H, dd, J=9.1, 4.4Hz), 7.46 (2H, d, J=8.8Hz), 7.67 (2H, d, J=8.8Hz), 8.01 (1H, d, J=5.2Hz), 8.21 (1H, dd, J=9.1, 1.7Hz), 8.53 (1H, dd, J=4.4, 1.7Hz).
Elemental analysis: for C₁₇H₁₂ClN₅·0.5H₂O
Calculated: C, 61.73; H, 3.96; N, 21.17.
Found: C, 62.16; H, 4.20; N, 21.41.

### Example compound 16-3: 4-[2-(4-fluorophenyl)imidazo[1,2-b]pyridazin-3-yl]pyridin-2-amine

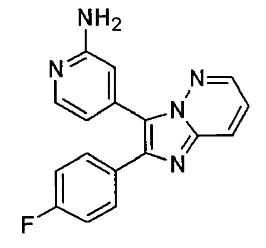

¹H-NMR (CDCl₃) δ: 6.10 (2H, brs), 6.58 (1H, dd, J=5.3, 1.5Hz), 6.65 (1H, s), 7.21-7.29 (2H, m), 7.33 (1H, dd, J=9.2, 4.4Hz), 7.66-7.76 (2H, m), 8.02 (1H, d, J=5.3Hz), 8.21 (1H, dd, J=9.2, 1.5Hz), 8.54 (1H, dd, J=4.4, 1.5Hz).

### (Example 17)

### N-{4-[6-Chloro-2-(3-methylphenyl)imidazo[1,2-b]pyridazin-3-yl]pyridin-2-yl}acetamide

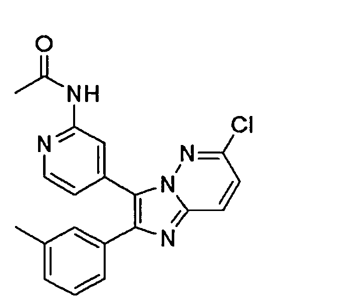

To a solution of 4-[6-chloro-2-(3-methylphenyl)imidazo[1,2-b]pyridazin-3-yl]pyridin-2-amine (150 mg, 0.447 mmol) obtained in Example 12 in dichloromethane-N,N-dimethylformamide (1:1) mixed solvent (10 mL) were added triethylamine (74.7 µL, 0.644 mmol) and acetyl chloride (38.1 µL, 0.536 mmol), and the mixture was stirred at room temperature for 20 min. Triethylamine (74.7 µL, 0.644 mmol) and acetyl chloride (38.1 µL, 0.536 mmol) were added to the mixture, and the mixture was further stirred at room temperature for 20 min. Saturated aqueous sodium hydrogen carbonate solution was added to the reaction mixture and the mixture was extracted with dichloromethane. The extract was washed with saturated brine and dried over anhydrous magnesium sulfate, and the solvent was removed by evaporation under reduced pressure. The obtained residue was dissolved in ammonia-ethanol solution (2.0 M, 5 mL). The mixture was stirred at room temperature for 5 min, and the solvent was removed by evaporation under reduced pressure. The obtained residue was purified by silica gel chromatography (ethyl acetate: hexane = 1:3 - 3:2) and recrystallized from ethyl acetate-hexane to give the title compound (yield 52.0 mg, 31%).
Melting point 228-229°C (ethyl acetate-hexane)
MS (ESI+): 378 (M+H).
¹H-NMR (CDCl₃) δ: 2.22 (3H, s), 2.35 (3H, s), 7.09-7.27 (4H, m), 7.34 (1H, d, J=7.4Hz), 7.57 (1H, s), 7.98 (1H, d, J=9.3Hz), 8.17 (1H, s), 8.31 (1H, d, J=5.2Hz), 8.58 (1H, s).
Elemental analysis: for C₂₀H₁₆ClN₅O
Calculated: C, 63.58; H, 4.27; N, 18.54.
Found: C, 63.48; H, 4.14; N, 18.48.

### (Example 18)

### N-{4-[6-Chloro-2-(3-methylphenyl)imidazo[1,2-b]pyridazin-3-yl]pyridin-2-yl}-4-methoxybenzamide

The compound was synthesized in the same manner as in Example 17 and using 4-methoxybenzoyl chloride instead of acetyl chloride. 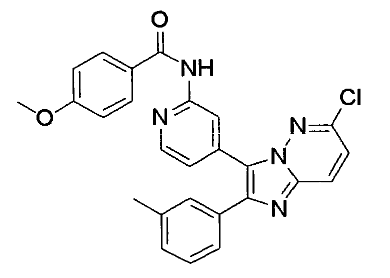 Melting point 204-205°C (ethyl acetate)
¹H-NMR (CDCl₃) δ: 2.36 (3H, s), 3.88 (3H, s), 6.96-7.02 (2H, m), 7.12-7.19 (3H, m), 7.22 (1H, t, J=7.4Hz), 7.37 (1H, d, J=7.4Hz), 7.60 (1H, s), 7.89-7.92 (2H, m), 7.98 (1H, d, J=9.4Hz), 8.35 (1H, dd, J=5.3, 0.8Hz), 8.58 (1H, s), 8.76 (1H, dd, J=1.4, 0.8Hz).
Elemental analysis: for C₂₆H₂₀ClN₅O₂
Calculated: C, 66.45; H, 4.29; N, 14.90.
Found: C, 66.36; H, 4.28; N, 14.86.

### (Example 19)

### N-{4-[2-(3-Methylphenyl)imidazo[1,2-b]pyridazin-3-yl]pyridin-2-yl}benzamide

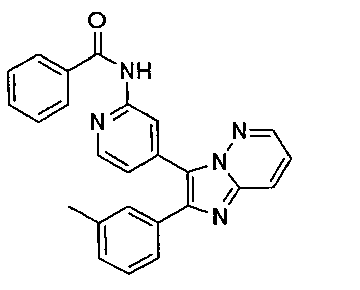

To a solution of 4-[2-(3-methylphenyl)imidazo[1,2-b]pyridazin-3-yl]pyridin-2-amine (150 mg, 0.498 mmol) obtained in Example 14 in dichloromethane (8 mL) were added triethylamine (208 µL, 1.49 mmol) and benzoyl chloride (173 µL, 1.49 mmol). The mixture was stirred at room temperature for 30 min, and saturated aqueous sodium hydrogen carbonate solution was added to the reaction mixture. Ammonia-ethanol solution (2.0 M, 10 mL) was added to the resulted mixture and the mixture was stirred at room temperature for 30 min. The solvent was removed by evaporation under reduced pressure, and the obtained residue was purified by silica gel chromatography (ethyl acetate: hexane = 3:7 - 8:2) and recrystallized from ethyl acetate to give the title compound (yield 145 mg, 72%).
Melting point 178-180°C (ethyl acetate)
¹H-NMR (CDCl₃) δ: 2.37 (3H, s), 7.12-7.19 (3H, m), 7.23 (1H, t, J=7.5Hz) , 7.40 (1H, d, J=7.5Hz ), 7.47-7.60 (3H, m), 7.63 (1H, s), 7.93-7.96 (2H, m), 8.05 (1H, dd, J=9.1, 1.6Hz), 8.26 (1H, dd, J=5.3, 0.8Hz), 8.41 (1H, dd, J=4.4, 1.6Hz), 8.88 (1H, s), 8.92 (1H, d, J=0.8Hz).
Elemental analysis: for C₂₅H₁₉N₅O
Calculated: C, 74.06; H, 4.72; N, 17.27.
Found: C, 73.81; H, 4.71; N, 17.24.

### (Example 20)

The compounds of Examples 20-1 - 20-18 below were synthesized in the same manner as in Example 19 respectively using 4-methylbenzoyl chloride, 4-methoxybenzoyl chloride, 4-chlorobenzoyl chloride, 3-fluorobenzoyl chloride, 2-fluorobenzoyl chloride, 2-acetoxypropionyl chloride (synthesized by the method described in Tetrahedron Asymmetry vol. 10, pp. 2997-3002, 1999), acetyl chloride, phenylacetyl chloride, propionyl chloride, cyclopropanecarbonyl chloride, 4-fluorobenzoyl chloride, 4-methoxyphenylacetyl chloride, 4-tert-butylbenzoyl chloride, 3,4-dichlorobenzoyl chloride, butyryl chloride, pivaloyl chloride, isobutyryl chloride or 3,3-dimethylbutyryl chloride, instead of benzoyl chloride.

### Example compound 20-1: 4-methyl-N-{4-[2-(3-methylphenyl)imidazo[1,2-b]pyridazin-3-yl]pyridin-2-yl}benzamide

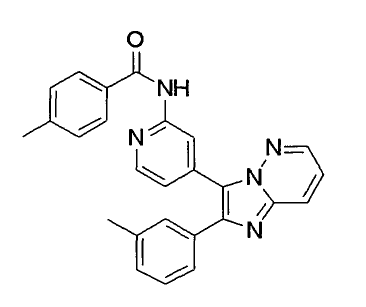

To a solution of 4-[2-(3-methylphenyl)imidazo[1,2-b]pyridazin-3-yl]pyridin-2-amine (1.0 g, 3.3 mmol) obtained in Example 14 in tetrahydrofuran (20 mL) were added triethylamine (500 mg, 4.9 mmol) and 4-methylbenzoyl chloride (770 mg, 5.0 mmol). The mixture was stirred at room temperature for 1 hr, and 5% aqueous sodium hydrogen carbonate solution was added to the reaction mixture. The resulted mixture was extracted with ethyl acetate. To the extract was added ammonia-ethanol solution (2.0 M, 20 mL), and the mixture was stirred at room temperature overnight. The mixture was dried over anhydrous sodium sulfate, and the solvent was removed by evaporation under reduced pressure. The obtained residue was purified by silica gel chromatography (ethyl acetate: hexane = 2:8 - 7:3) and recrystallized from ethyl acetate-tetrahydrofuran to give the title compound (yield 740 mg, 53%).
Melting point 194-196°C (ethyl acetate-tetrahydrofuran)
¹H-NMR (CDCl₃) δ: 2.37 (3H, s), 2.44 (3H, s), 7.12-7.27 (4H, m), 7.31 (2H, d, J=7.9Hz), 7.40 (1H, d, J=7.4Hz), 7.62 (1H, s), 7.84 (2H, m), 8.05 (1H, dd, J=9.2, 1.7Hz) , 8.32 (1H, dd, J=5.1, 0.8Hz), 8.41 (1H, dd, J=4.3, 1.7Hz), 8.65 (1H, s), 8.91 (1H, dd, J=1.5, 0.8Hz).
Elemental analysis: for C₂₆H₂₁N₅O
Calculated: C, 74.44; H, 5.05; N, 16.70.
Found: C, 74.39; H, 5.06; N, 16.75.

### Example compound 20-2: 4-methoxy-N-{4-[2-(3-methylphenyl)imidazo[1,2-b]pyridazin-3-yl]pyridin-2-yl}benzamide

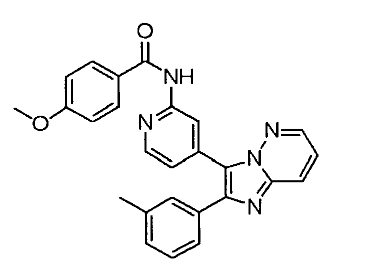

Melting point 177-178°C (ethyl acetate)
¹H-NMR (CDCl₃) δ: 2.37 (3H, s), 3.88 (3H, s), 6.99 (2H, d, J=8.9Hz), 7.11-7.27 (4H, m), 7.40 (1H, d, J=7.5Hz), 7.62 (1H, s), 7.92 (2H, d, J=8.9Hz), 8.04 (1H, dd, J=9.1, 1.6Hz), 8.30 (1H, d, J=5.1Hz), 8.40 (1H, dd, J=4.4, 1.6Hz), 8.68 (1H, s), 8.90 (1H, s).
Elemental analysis: for C₂₆H₂₁N₅O₂
Calculated: C, 71.71; H, 4.86; N, 16.08.
Found: C, 71.68; H, 4.85; N, 16.11.

### Example compound 20-3: 4-chloro-N-{4-[2-(3-methylphenyl)imidazo[1,2-b]pyridazin-3-yl]pyridin-2-yl}benzamide

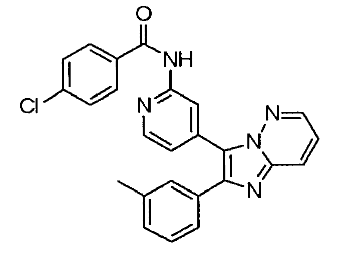

Melting point 211-212°C (ethyl acetate)
¹H-NMR (CDCl₃) δ: 2.37 (3H, s), 7.13-7.27 (4H, m), 7.39 (1H, d, J=7.4Hz), 7.46-7.52 (2H, m), 7.62 (1H, s), 7.85-7.91 (2H, m), 8.05 (1H, dd, J=9.2, 1.7Hz), 8.32 (1H, dd, J=5.3, 0.8Hz), 8.41 (1H, dd, J=4.3, 1.7Hz), 8.64 (1H, s), 8.88 (1H, dd, J=1.3, 0.8Hz).
Elemental analysis: for C₂₅H₁₈ClN₅O
Calculated: C, 68.26; H, 4.12; N, 15.92.
Found: C, 68.20; H, 4.14; N, 15.96.

### Example compound 20-4: 3-fluoro-N-{4-[2-(3-methylphenyl)imidazo[1,2-b]pyridazin-3-yl]pyridin-2-yl}benzamide

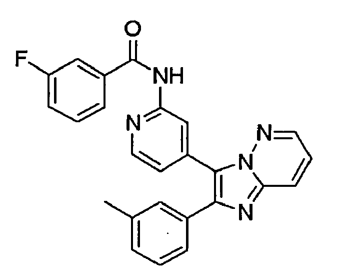

Melting point 204-205°C (ethyl acetate)
¹H-NMR (CDCl₃) δ: 2.37 (3H, s), 7.13-7.21 (3H, m), 7.24-7.31 (2H, m), 7.39 (1H, d, J=7.4Hz), 7.45-7.52 (1H, m), 7.61-7.72 (3H, m), 8.05 (1H, dd, J=9.1, 1.6Hz), 8.31 (1H, d, J=5.3Hz), 8.41 (1H, dd, J=4.4, 1.6Hz), 8.73 (1H, s), 8.89 (1H, s).
Elemental analysis: for C₂₅H₁₈FN₅O
Calculated: C, 70.91; H, 4.28; N, 16.54.
Found: C, 70.80; H, 4.45; N, 16.14.

### Example compound 20-5: 2-fluoro-N-{4-[2-(3-methylphenyl)imidazo[1,2-b]pyridazin-3-yl]pyridin-2-yl}benzamide

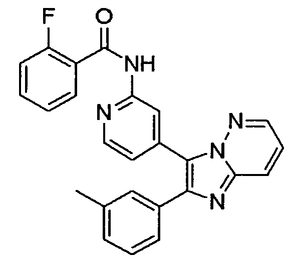

Melting point 180-181°C (ethyl acetate)
¹H-NMR (CDCl₃) δ: 2.37 (3H, s), 7.12-7.26 (5H, m), 7.29-7.35 (1H, m), 7.40 (1H, d, J=7.4Hz), 7.52-7.59 (1H, m), 7.63 (1H, s), 8.05 (1H, dd, J=9.2, 1.7Hz) , 8.15 (1H, m), 8.36 (1H, dd, J=5.3, 0.8Hz), 8.41 (1H, dd, J=4.4, 1.7Hz), 8.90 (1H, s), 9.17 (1H, d, J=14.0Hz).
Elemental analysis: for C₂₅H₁₈FN₅O
Calculated: C, 70.91; H, 4.28; N, 16.54.
Found: C, 70.87; H, 4.32; N, 16.45.

### Example compound 20-6: 1-methyl-2-({4-[2-(3-methylphenyl)imidazo[1,2-b]pyridazin-3-yl]pyridin-2-yl}amino)-2-oxoethyl acetate

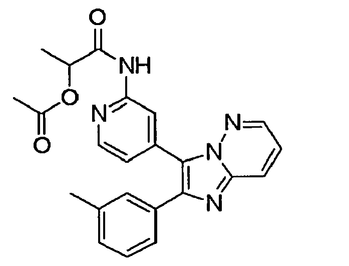

¹H-NMR (CDCl₃) δ: 1.58 (3H, d, J=6.9Hz), 2.23 (3H, s), 2.36 (3H, s), 5.37 (1H, q, J=6.9Hz), 7.12-7.27 (4H, m), 7.36 (1H, d, J=7.4Hz), 7.59 (1H, s), 8.04 (1H, dd, J=9.2, 1.7Hz), 8.31 (1H, d, J=5.3Hz), 8.39 (1H, dd, J=4.3, 1.7Hz), 8.56 (1H, s), 8.74 (1H, s).

### Example compound 20-7: N-{4-[2-(3-methylphenyl)imidazo[1,2-b]pyridazin-3-yl]pyridin-2-yl}acetamide

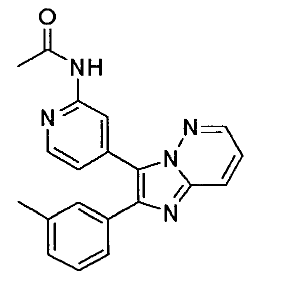

Melting point 227-228°C (ethyl acetate-hexane)
MS (ESI+): 344 (M+H).
¹H-NMR (CDCl₃) δ: 2.22 (3H, s), 2.36 (3H, s), 7.01-7.24 (4H, m), 7.37 (1H, d, J=7.0Hz), 7.60 (1H, s), 8.03 (1H, dd, J=9.2, 1.5Hz), 8.22-8.32 (2H, m), 8.37 (1H, dd, J=4.4, 1.5Hz), 8.67 (1H, s).
Elemental analysis: for C₂₀H₁₇N₅O
Calculated: C, 69.96; H, 4.99; N, 20.40.
Found: C, 69.72; H, 4.81; N, 20.22.

### Example compound 20-8: N-{4-[2-(3-methylphenyl)imidazo[1,2-b]pyridazin-3-yl]pyridin-2-yl}-2-phenylacetamide

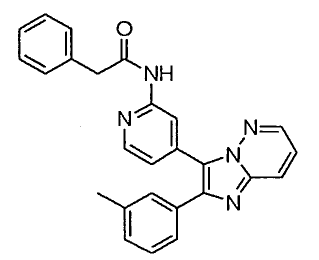

MS (ESI+): 420 (M+H).
¹H-NMR (CDCl₃) δ: 2.35 (3H, s), 3.78 (2H, s), 7.06-7.23 (4H, m), 7.30-7.46 (6H, m), 7.58 (1H, s), 7.91 (1H, s), 8.03 (1H, dd, J=9.1, 1.6Hz), 8.22 (1H, dd, J=5.3, 0.8Hz), 8.38 (1H, dd, J=4.5, 1.6Hz), 8.72 (1H, s).

### Example compound 20-9: N-{4-[2-(3-methylphenyl)imidazo[1,2-b]pyridazin-3-yl]pyridin-2-yl}propanamide

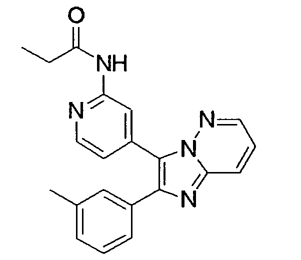

Melting point 138-139°C (ethyl acetate-hexane)
MS (ESI+): 358 (M+H).
¹H-NMR (CDCl₃) δ: 1.18-1.34 (3H, m), 2.36 (3H, s), 2.46 (2H, q, J=7.6Hz), 7.07-7.24 (4H, m), 7.37 (1H, d, J=7.5Hz), 7.60 (1H, s), 7.99 (1H, s), 8.03 (1H, dd, J=9.1, 1.6Hz), 8.27 (1H, dd, J=5.3, 0.8Hz), 8.39 (1H, dd, J=4.4, 1.6Hz), 8.72 (1H, s).
Elemental analysis: for C₂₁H₁₉N₅O
Calculated: C, 70.57; H, 5.36; N, 19.59.
Found: C, 70.27; H, 5.53; N, 19.69.

### Example compound 20-10: N-{4-[2-(3-methylphenyl)imidazo[1,2-b]pyridazin-3-yl]pyridin-2-yl}cyclopropanecarboxamide

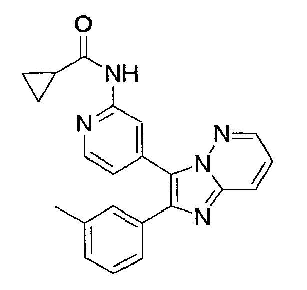

To a solution of 4-[2-(3-methylphenyl)imidazo[1,2-b]pyridazin-3-yl]pyridin-2-amine (55 g, 0.18 mol) obtained in Example 14 in tetrahydrofuran (1000 mL) was added cyclopropanecarbonyl chloride (30 g, 0.28 mol), and the mixture was stirred at room temperature for 30 min. Triethylamine (39 mL, 0.28 mol) was added to the mixture, and the mixture was stirred at room temperature for 2 hr. Saturated brine was added to the reaction mixture, and the resulted mixture was extracted with ethyl acetate. The extract was dried over anhydrous sodium sulfate, and the solvent was removed by evaporation under reduced pressure. Tetrahydrofuran (100 mL) and ammonia-ethanol solution (2.0 M, 100 mL) were added to the obtained residue, and the mixture was stirred at room temperature for 14 hr. The mixture was filtrated, and the resulted precipitation was collected and recrystallized from ethanol to give the title compound (yield 48 g, 58%).
Melting point 203-204°C (ethanol)
MS (ESI+): 370 (M+H).
¹H-NMR (CDCl₃) δ: 0.82-0.96 (2H, m), 1.05-1.16 (2H, m), 1.48-1.63 (1H, m), 2.36 (3H, s), 7.05-7.25 (4H, m), 7.36 (1H, d, J=7.3Hz), 7.59 (1H, s), 8.02 (1H, dd, J=9.2, 1.7Hz), 8.22-8.32 (1H, m), 8.32-8.44 (2H, m), 8.70 (1H, s).
Elemental analysis: for C₂₂H₁₉N₅O
Calculated: C, 71.53; H, 5.18; N, 18.96.
Found: C, 71.30; H, 5.29; N, 19.06.

### Example compound 20-11: 4-fluoro-N-{4-[2-(3-methylphenyl)imidazo[1,2-b]pyridazin-3-yl]pyridin-2-yl}benzamide

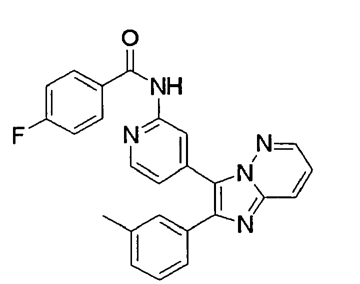

To a solution of 4-[2-(3-methylphenyl)imidazo[1,2-b]pyridazin-3-yl]pyridin-2-amine (300 mg, 1.0 mmol) obtained in Example 14 in tetrahydrofuran (100 mL) was added 4-fluorobenzoyl chloride (320 mg, 2.0 mol), and the mixture was stirred at room temperature for 5 min. Triethylamine (0.28 mL, 2.0 mmol) was added to the mixture, and the mixture was stirred at room temperature for 2 hr. Saturated aqueous sodium hydrogen carbonate solution was added to the mixture, and the resulted mixture was extracted with ethyl acetate. The extract was dried over anhydrous sodium sulfate, and the solvent was removed by evaporation under reduced pressure. Tetrahydrofuran (3 mL) and ammonia-ethanol solution (2.0 M, 3 mL) were added to the obtained residue, the mixture was stirred at room temperature for 14 hr, and the solvent was removed by evaporation under reduced pressure. The obtained residue was purified by silica gel chromatography (ethyl acetate: hexane = 5:5 - ethyl acetate) and recrystallized from ethanol to give the title compound (yield 220 mg, 52%).
MS (ESI+) : 424 (M+H).
¹H-NMR (CDCl₃) δ: 2.37 (3H, s), 7.10-7.25 (6H, m), 7.39 (1H, d, J=7.5Hz), 7.62 (1H, s), 7.89-8.00 (2H, m), 8.05 (1H, dd, J=9.2, 1.6Hz), 8.27-8.35 (1H, m), 8.41(1H, dd, J=4.4, 1.6Hz), 8.65 (1H, s), 8.88 (1H, s).

### Example compound 20-12: 2-(4-methoxyphenyl)-N-{4-[2-(3-methylphenyl)imidazo[1,2-b]pyridazin-3-yl]pyridin-2-yl}acetamide

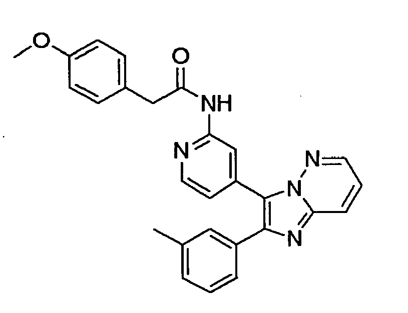

Melting point 213-214°C (ethyl acetate-hexane)
MS (ESI+): 450 (M+H).
¹H-NMR (CDCl₃) δ: 2.35 (3H, s), 3.72 (2H, s), 3.83 (3H, s), 6.86-6.99 (2H, m), 7.05-7.45 (7H, m), 7.58 (1H, s), 7.92 (1H, s), 8.03 (1H, dd, J=9.2, 1.7 Hz), 8.22 (1H, d, J=5.3 Hz), 8.38 (1H, dd, J=4.5, 1.7 Hz), 8.72 (1H, s).
Elemental analysis: for C₂₇H₂₃N₅O₂
Calculated: C, 72.14; H, 5.16; N, 15.58.
Found: C, 71.93; H, 5.26; N, 15.22.

### Example compound 20-13: 4-tert-butyl-N-{4-[2-(3-methylphenyl)imidazo[1,2-b]pyridazin-3-yl]pyridin-2-yl}benzamide formate

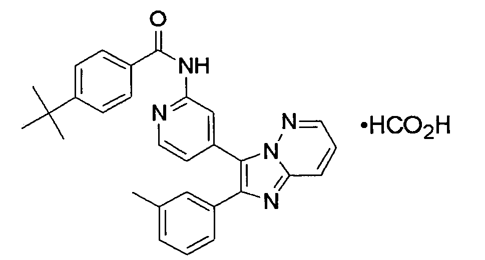

MS (ESI+) : 462 (M+H).
¹H-NMR (CD₃OD) δ: 1.36 (9H, s), 2.33 (3H, s), 7.19-7.29 (3H, m), 7.33-7.39(2H, m), 7.50 (1H, s), 7.56 (2H, d, J=8.5Hz), 7.89 (2H, d, J=8.5Hz), 8.07-8.12 (1H, m), 8.33-8.38 (1H, m), 8.50-8.53 (1H, m), 8.63 (1H, s), hidden (3H).

### Example compound 20-14: 3,4-dichloro-N-{4-[2-(3-methylphenyl)imidazo[1,2-b]pyridazin-3-yl]pyridin-2-yl}benzamide formate

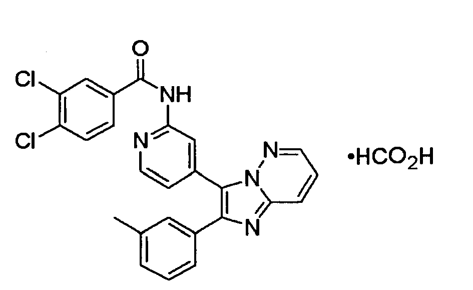

MS (ESI+): 474 (M+H).
¹H-NMR (DMSO-d₆) δ: 2.31 (3H, s), 7.16-7.21 (1H, m), 7.26 (1H, t, 7.6Hz), 7.31 (1H, dd, J=5.1, 1.5Hz), 7.36-7.40 (2H, m), 7.60 (1H, s), 7.79 (1H, d, J=8.5Hz), 7.96-7.99 (1H, m), 8.23-8.30 (2H, m), 8.49-8.53 (2H, m), 8.58 (1H, dd, J=4.5, 1.5Hz), 11.20 (1H, s), hidden (2H).

### Example compound 20-15: N-{4-[2-(3-methylphenyl)imidazo[1,2-b]pyridazin-3-yl]pyridin-2-yl}butanamide trifluoroacetate

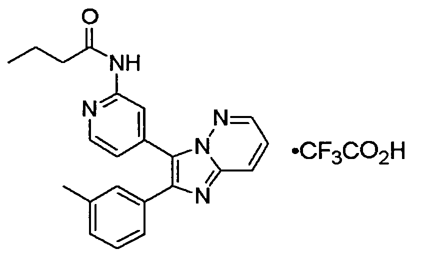

MS (ESI+): 372 (M+H).

### Example compound 20-16: 2,2-dimethyl-N-{4-[2-(3-methylphenyl)imidazo[1,2-b]pyridazin-3-yl]pyridin-2-yl}propanamide formate

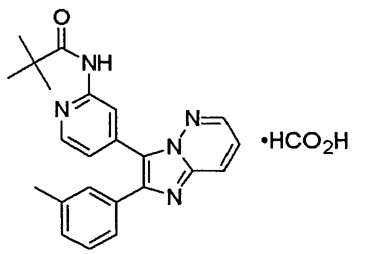

MS (ESI+): 386 (M+H).
¹H-NMR (DMSO-d₆) δ: 1.23 (9H, s), 2.30 (3H, s), 7.15-7.20 (2H, m), 7.25 (1H, t, J=7.5Hz), 7.32-7.39 (2H, m), 7.58 (1H, s), 8.24 (1H, dd, J=9.2, 1.5Hz), 8.38-8.40 (1H, m), 8.42 (1H, dd, J=5.2, 0.7Hz), 8.56 (1H, dd, J=4.4, 1.5Hz), 9.96 (1H, s), hidden (2H).

### Example compound 20-17: 2-methyl-N-{4-[2-(3-methylphenyl)imidazo[1,2-b]pyridazin-3-yl]pyridin-2-yl}propanamide trifluoroacetate

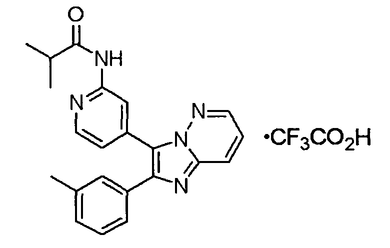

MS (ESI+): 372 (M+H).

### Example compound 20-18: 3,3-dimethyl-N-{4-[2-(3-methylphenyl)imidazo[1,2-b]pyridazin-3-yl]pyridin-2-yl}butanamide trifluoroacetate

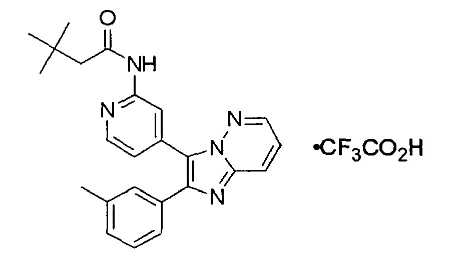

MS (ESI+): 400 (M+H).
¹H-NMR (CD₃OD) δ: 1.06 (9H, s), 2.29 (2H, s), 2.33 (3H, s), 7.17-7.41 (5H, m), 7.46 (1H, s), 8.09 (1H, dd, J=9.2, 1.5Hz), 8.32 (1H, d, J=5.3Hz), 8.40 (1H, s), 8.52 (1H, dd, J=4.4, 1.5Hz), hidden (2H).

### (Example 21)

The compounds of Examples 21-1 - 21-40 below were synthesized in the same manner as in Example 20 respectively using 4-[2-(3-chlorophenyl)imidazo[1,2-b]pyridazin-3-yl]pyridin-2-amine (Example compound 15), 4-[2-(4-fluoro-3-methylphenyl)imidazo[1,2-b]pyridazin-3-yl]pyridin-2-amine (Example compound 16-1), 4-[2-(4-chlorophenyl)imidazo[1,2-b]pyridazin-3-yl]pyridin-2-amine (Example compound 16-2) or 4-[2-(4-fluorophenyl)imidazo[1,2-b]pyridazin-3-yl]pyridin-2-amine (Example compound 16-3), instead of 4-[2-(3-methylphenyl)imidazo[1,2-b]pyridazin-3-yl]pyridin-2-amine.

### Example compound 21-1: N-{4-[2-(3-chlorophenyl)imidazo[1,2-b]pyridazin-3-yl]pyridin-2-yl}benzamide

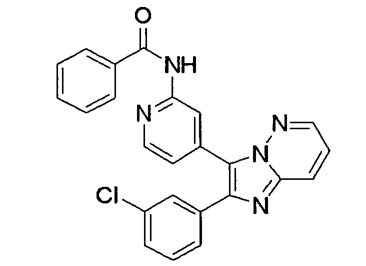

Melting point 213-214°C (ethanol)
MS (ESI+): 426 (M+H).
¹H-NMR (CDCl₃) δ: 7.13-7.20 (2H, m), 7.24-7.36 (2H, m), 7.47-7.62 (4H, m), 7.78 (1H, dd, J=1.9, 1.7Hz), 7.90-7.96 (2H, m), 8.04 (1H, dd, J=9.1, 1.7Hz), 8.35 (1H, dd, J=5.2, 0.6Hz), 8.41 (1H, dd, J=4.4, 1.7Hz), 8.71 (1H, brs), 8.85 (1H, dd, J=1.4, 0.8Hz).
Elemental analysis: for C₂₄H₁₆ClN₅O·0.2H₂O
Calculated: C, 67.12; H, 3.85; N, 16.31.
Found: C, 67.38; H, 3.80; N, 16.19.

### Example compound 21-2: N-{4-[2-(3-chlorophenyl)imidazo[1,2-b]pyridazin-3-yl]pyridin-2-yl}-4-methylbenzamide trifluoroacetate

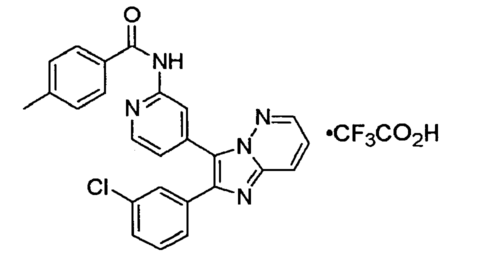

MS (ESI+): 440 (M+H).
¹H-NMR (DMSO-d₆) δ: 2.38 (3H, s), 7.29-7.34 (3H, m), 7.38-7.46 (3H, m), 7.54-7.60 (1H, m), 7.75 (1H, s), 7.93 (2H, d, J=8.1Hz), 8.29 (1H, dd, J=9.2, 1.5Hz), 8.50 (1H, s), 8.53 (1H, d, J=5.1Hz), 8.60 (1H, dd, J=4.3, 1.5Hz), 10.90 (1H, s), hidden (1H).

### Example compound 21-3: N-{4-[2-(3-chlorophenyl)imidazo[1,2-b]pyridazin-3-yl]pyridin-2-yl}-4-methoxybenzamide

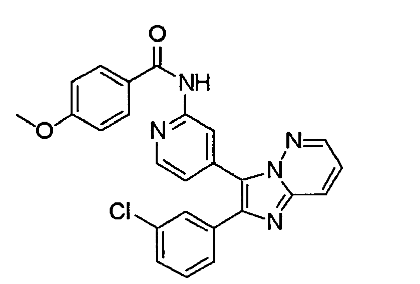

Melting point 223-224°C (tetrahydrofuran-water)
¹H-NMR (CDCl₃) δ: 3.88 (3H, s), 6.97-7.02 (2H, m), 7.12-7.22 (2H, m), 7.25-7.35 (2H, m), 7.50-7.54 (1H, m), 7.78-7.80 (1H, m), 7.88-7.94 (2H, m), 8.05 (1H, dd, J=9.2, 1.7Hz), 8.35 (1H, dd, J=5.2, 0.7Hz), 8.41 (1H, dd, J=4.3, 1.7Hz), 8.66 (1H, s), 8.84-8.85 (1H, m).
Elemental analysis: for C₂₅H₁₈ClN₅O₂
Calculated: C, 65.86; H, 3.98; N, 15.36.
Found: C, 65.82; H, 3.97; N, 15.33.

### Example compound 21-4: 4-chloro-N-{4-[2-(3-chlorophenyl)imidazo[1,2-b]pyridazin-3-yl]pyridin-2-yl}benzamide formate

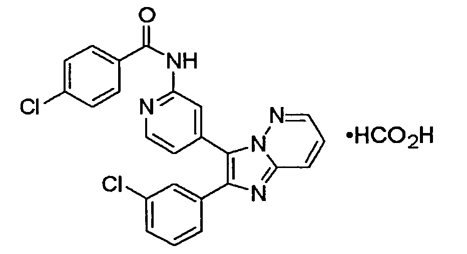

MS (ESI+): 460 (M+H).

### Example compound 21-5: N-{4-[2-(3-chlorophenyl)imidazo[1,2-b]pyridazin-3-yl]pyridin-2-yl}-4-fluorobenzamide formate

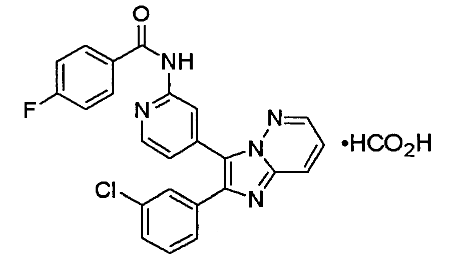

MS (ESI+): 444 (M+H).

### Example compound 21-6: N-{4-[2-(3-chlorophenyl)imidazo[1,2-b]pyridazin-3-yl]pyridin-2-yl}acetamide trifluoroacetate

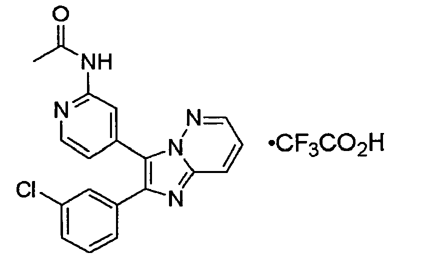

MS (ESI+): 364 (M+H).

### Example compound 21-7: N-{4-[2-(3-chlorophenyl)imidazo[1,2-b]pyridazin-3-yl]pyridin-2-yl}propanamide

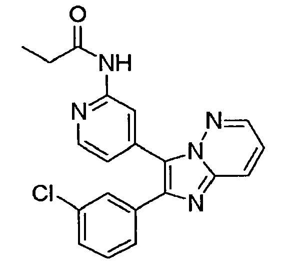

Melting point 192-193°C (ethanol)
MS (ESI+): 378 (M+H).
¹H-NMR (CDCl₃) δ: 1.26 (3H, t, J=7.7Hz), 2.46 (2H, q, J=7.7Hz), 7.10-7.18 (2H, m), 7.23-7.35 (2H, m), 7.46-7.51 (1H, m), 7.74 (1H, dd, J=1.6, 0.9Hz), 7.96 (1H, brs), 8.03 (1H, dd, J=9.1, 1.6Hz), 8.31 (1H, d, J=5.2Hz), 8.38 (1H, dd, J=4.4, 1.6Hz), 8.66 (1H, s).
Elemental analysis: for C₂₀H₁₆ClN₅O
Calculated: C, 63.58; H, 4.27; N, 18.54.
Found: C, 63.34; H, 4.11; N, 18.35.

### Example compound 21-8: N-{4-[2-(3-chlorophenyl)imidazo[1,2-b]pyridazin-3-yl]pyridin-2-yl}cyclopropanecarboxamide trifluoroacetate

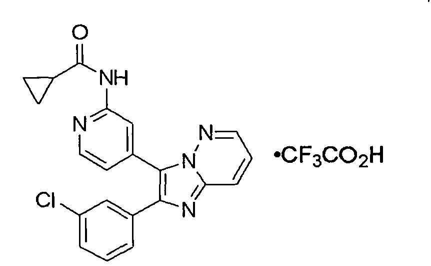

MS (ESI+): 390 (M+H).
¹H-NMR (DMSO-d₆) δ: 0.73-0.87 (4H, m), 1.98-2.05 (1H, m), 7.22 (1H, dd, J=5.1, 1.5Hz), 7.36-7.45 (3H, m), 7.51-7.53 (1H, m), 7.71 (1H, s), 8.27 (1H, dd, J=9.2, 1.5Hz), 8.36 (1H, s), 8.45 (1H, d, J= 5.1Hz), 8.57 (1H, dd, J=4.4, 1.5Hz), 10.99 (1H, s), hidden (1H).

### Example compound 21-9: 3,4-dichloro-N-{4-[2-(3-chlorophenyl)imidazo[1,2-b]pyridazin-3-yl]pyridin-2-yl}benzamide formate

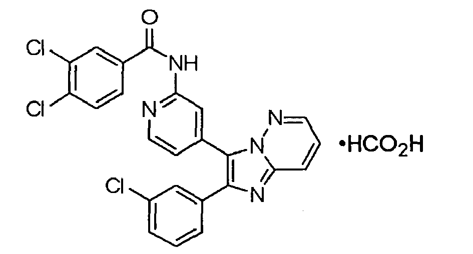

MS (ESI+): 494 (M+H).

### Example compound 21-10: N-{4-[2-(3-chlorophenyl)imidazo[1,2-b]pyridazin-3-yl]pyridin-2-yl}butanamide trifluoroacetate

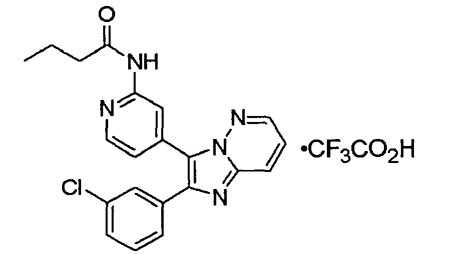

MS (ESI+): 392 (M+H).

### Example compound 21-11: N-{4-[2-(3-chlorophenyl)imidazo[1,2-b]pyridazin-3-yl]pyridin-2-yl}-2,2-dimethylpropanamide formate

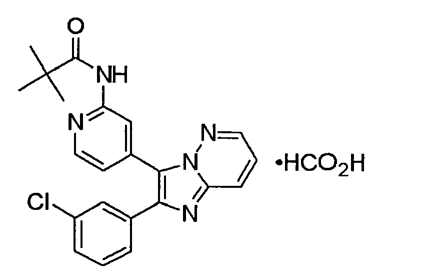

MS (ESI+): 406 (M+H).
¹H-NMR (DMSO-d₆) δ: 1.24 (9H, s), 7.23 (1H, dd, J=5.1, 1.3Hz), 7.36-7.45 (3H, m), 7.50 (1H, m), 7.72 (1H, s), 8.28 (1H, dd, J=9.2, 1.5Hz), 8.39 (1H, s), 8.46 (1H, d, J=5.1Hz) , 8.59 (1H, dd, J=4.5, 1.5Hz), 10.00 (1H, s), hidden (2H).

### Example compound 21-12: N-{4-[2-(3-chlorophenyl)imidazo[1,2-b]pyridazin-3-yl]pyridin-2-yl}-2-methylpropanamide trifluoroacetate

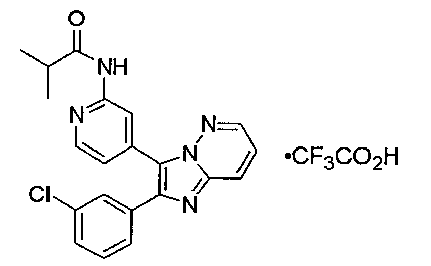

MS (ESI+): 392 (M+H).

### Example compound 21-13: N-{4-[2-(3-chlorophenyl)imidazo[1,2-b]pyridazin-3-yl]pyridin-2-yl}-3,3-dimethylbutanamide trifluoroacetate

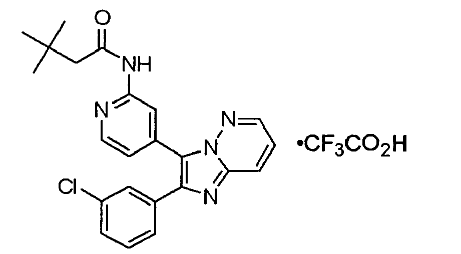

MS (ESI+) : 420 (M+H).
¹H-NMR (DMSO-d₆) δ: 0.98 (9H, s), 2.27 (2H, s), 7.30 (1H, dd, J=5.3, 1.5Hz), 7.36-7.44 (3H, m), 7.52-7.58 (1H, m), 7.70 (1H, s), 8.27 (1H, dd, J=9.2, 1.5Hz), 8.36 (1H, s), 8.45 (1H, d, J=5.3Hz), 8.59 (1H, dd, J=4.4, 1.5Hz), 10.52 (1H, s), hidden (1H) .

### Example compound 21-14: N-{4-[2-(4-fluoro-3-methylphenyl)imidazo[1,2-b]pyridazin-3-yl]pyridin-2-yl}benzamide

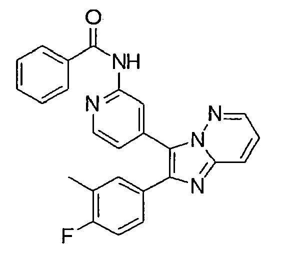

Melting point 189-190°C (ethanol)
MS (ESI+): 424 (M+H).
¹H-NMR (CDCl₃) δ: 2.29 (3H, d, J=1.9Hz), 6.96 (1H, dd, J=9.3, 8.8Hz), 7.14 (1H, dd, J=9.1, 4.4Hz), 7.17 (1H, dd, J=5.2, 1.4Hz), 7.35-7.41 (1H, m), 7.47-7.66 (4H, m), 7.90-7.95 (2H, m), 8.03 (1H, dd, J=9.1, 1.7Hz), 8.33 (1H, dd, J=5.2, 0.8Hz), 8.40 (1H, dd, J=4.4, 1.7Hz), 8.69 (1H, brs), 8.86 (1H, dd, J=1.4, 0.8Hz).
Elemental analysis: for C₂₅H₁₈FN₅O
Calculated: C, 70.91; H, 4.28; N, 16.54.
Found: C, 70.77; H, 4.33; N, 16.53.

### Example compound 21-15: N-{4-[2-(4-fluoro-3-methylphenyl)imidazo[1,2-b]pyridazin-3-yl]pyridin-2-yl}-4-methylbenzamide trifluoroacetate

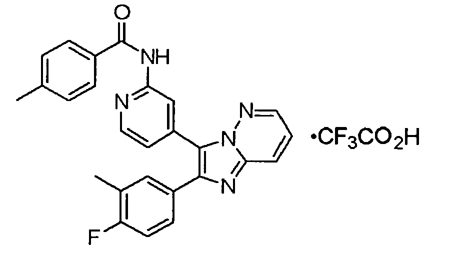

MS (ESI+): 438 (M+H).
¹H-NMR (DMSO-d₆) δ: 2.24 (3H, s), 2.38 (3H, s), 7.12-7.20 (1H, m), 7.28-7.35 (3H, m), 7.36-7.45 (2H, m), 7.67-7.72 (1H, m), 7.93 (2H, d, J=8.1Hz), 8.26 (1H, dd, J=9.2, 1.5Hz), 8.48-8.52 (2H, m), 8.59 (1H, dd, J=4.3, 1.5Hz), 10.89 (1H, s), hidden (1H).

### Example compound 21-16: N-{4-[2-(4-fluoro-3-methylphenyl)imidazo[1,2-b]pyridazin-3-yl]pyridin-2-yl}-4-methoxybenzamide

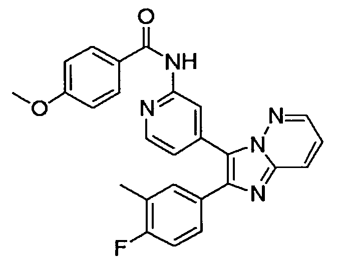

To a solution of 4-[2-(4-fluoro-3-methylphenyl)imidazo[1,2-b]pyridazin-3-yl]pyridin-2-amine (1.0 g, 3.3 mmol) obtained in Example 16-1 in pyridine (20 mL) was added 4-methoxybenzoyl chloride (1.3 g, 7.6 mmol), and the mixture was stirred at 50°C for 3 hr. Additional 4-methoxybenzoyl chloride (1.3 g, 7.6 mmol) was added to the mixture, and the mixture was stirred at 50°C overnight. Saturated aqueous sodium hydrogen carbonate solution was added to the reaction mixture. The resulted mixture was extracted with ethyl acetate. Ammonia-ethanol solution (2.0 M, 20 mL) was added to the extract, and the mixture was stirred at room temperature for 12 hr. The mixture was dried over anhydrous sodium sulfate, and the solvent was removed by evaporation under reduced pressure. The obtained residue was purified by silica gel chromatography (ethyl acetate: hexane = 2:8 - 7:3) and recrystallized from ethyl acetate-tetrahydrofuran to give the title compound (yield 740 mg, 53%).
Melting point 222-223°C (ethyl acetate-tetrahydrofuran)
¹H-NMR (CDCl₃) δ: 2.29 (3H, d, J=1.7Hz), 3.88 (3H, s), 6.93-7.02 (3H, m), 7.10-7.19 (2H, m), 7.35-7.43 (1H, m), 7.64 (1H, dd, J=7.4, 1.7Hz), 7.88-7.94 (2H, m), 8.03 (1H, dd, J=9.2, 1.7Hz), 8.31 (1H, dd, J=5.2, 0.7Hz), 8.40 (1H, dd, J=4.4, 1.7Hz), 8.71 (1H, s), 8.85-8.87 (1H, m).
Elemental analysis: for C₂₆H₂₀FN₅O₂
Calculated: C, 68.86; H, 4.45; N, 15.44.
Found: C, 68.75; H, 4.49; N, 15.36.

### Example compound 21-17: 4-chloro-N-{4-[2-(4-fluoro-3-methylphenyl)imidazo[1,2-b]pyridazin-3-yl]pyridin-2-yl}benzamide

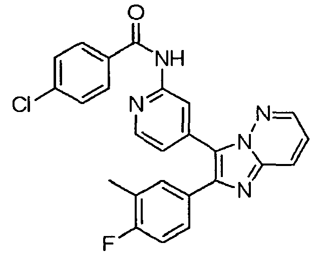

Melting point 207-209°C (ethanol-tetrahydrofuran)
¹H-NMR (CDCl₃) δ: 2.29 (3H, s), 6.98 (1H, t, J=9.0Hz), 7.11-7.23 (2H, m), 7.36-7.4.0 (1H, m), 7.49 (2H, d, J=8.5Hz), 7.64 (1H, d, J=7.4Hz) , 7.88 (2H, d, J=8.5Hz), 8.04 (1H, d, J=9.0Hz), 8.32 (1H, d, J=5.1Hz), 8.36-8.47 (1H, m), 8.72 (1H, s), 8.84 (1H, s). Elemental analysis: for C₂₅H₁₇ClFN₅0
Calculated: C, 65.58; H, 3.74; N, 15.29.
Found: C, 65.55; H, 3.73; N, 15.29.

### Example compound 21-18: N-{4-[2-(4-fluoro-3-methylphenyl)imidazo[1,2-b]pyridazin-3-yl]pyridin-2-yl}propanamide

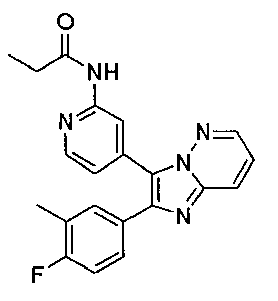

Melting point 166-167°C (ethanol)
MS (ESI+): 376 (M+H).
¹H-NMR (CDCl₃) δ: 1.26 (3H, t, J=7.7Hz), 2.28 (3H, d, J=1.6Hz), 2.46 (2H, q, J=7.7Hz), 6.95 (1H, t, J=8.9Hz), 7.10-7.16 (2H, m), 7.30-7.38 (1H, m), 7.60 (1H, dd, J=7.4, 1.9Hz), 8.01 (1H, dd, J=9.3, 1.7Hz), 8.03 (1H, s), 8.28 (1H, dd, J=5.2, 0.8Hz), 8.37 (1H, dd, J=4.4, 1.9Hz), 8.67 (1H, s).
Elemental analysis: for C₂₁H₁₈FN₅O
Calculated: C, 67.19; H, 4.83; N, 18.66.
Found: C, 67.31; H, 4.97; N, 18.77.

### Example compound 21-19: N-{4-[2-(4-fluoro-3-methylphenyl)imidazo[1,2-b]pyridazin-3-yl]pyridin-2-yl}cyclopropanecarboxamide trifluoroacetate

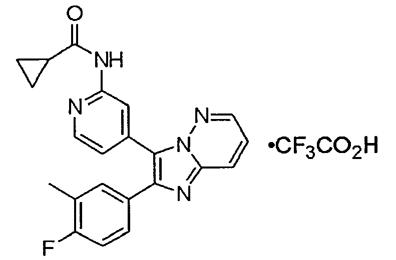

MS (ESI+): 388 (M+H).
¹H-NMR (DMSO-d₆) δ: 0.71-0.85 (4H, m), 1.97-2.09 (1H, m), 2.23 (3H, s), 7.10-7.18 (1H, m), 7.20 (1H, dd, J=5.3, 1.5Hz), 7.32-7.41 (2H, m), 7.66 (1H, dd, J=7.5, 1.5Hz), 8.23 (1H, dd, J=9.0, 1.5Hz), 8.38 (1H, s), 8.42 (1H, d, J=5.3Hz), 8.56 (1H, dd, J=4.5, 1.5Hz), 10.96 (1H, s), hidden (1H).

### Example compound 21-20: 4-fluoro-N-{4-[2-(4-fluoro-3-methylphenyl)imidazo[1,2-b]pyridazin-3-yl]pyridin-2-yl}benzamide formate

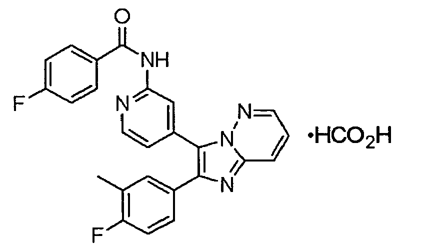

MS (ESI+) : 442 (M+H).
¹H-NMR (DMSO-d₆) δ: 2.25 (3H, d, J=1.5Hz), 7.12-7.20 (1H, m), 7.30-7.44 (5H, m), 7.70 (1H, dd, J=7.5, 1.5Hz), 8.06-8.13 (2H, m), 8.26 (1H, dd, J=9.2, 1.5Hz), 8.47-8.53 (2H, m), 8.58 (1H, dd, J=4.4, 1.5Hz), 11.02 (1H, s), hidden (2H).

### Example compound 21-21: 4-tert-butyl-N-{4-[2-(4-fluoro-3-methylphenyl)imidazo[1,2-b]pyridazin-3-yl]pyridin-2-yl}benzamide formate

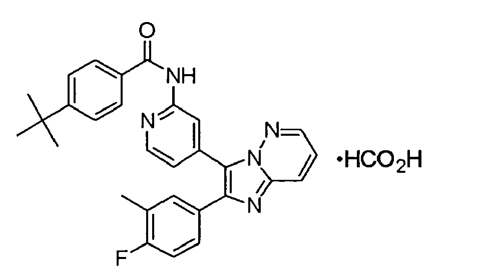

MS (ESI+): 480 (M+H).
¹H-NMR (DMSO-d₆) δ: 1.32 (9H, s), 2.25 (3H, d, J=1.5Hz), 7.12-7.19 (1H, m), 7.29 (1H, dd, J=5.1, 1.7Hz), 7.36-7.44 (2H, m), 7.50-7.56 (2H, m), 7.70 (1H, dd, J=7.6, 1.7Hz), 7.94-8.00 (2H, m), 8.26 (1H, dd, J=9.2, 1.5Hz), 8.49-8.53 (2H, m), 8.58 (1H, dd, J=4.5, 1.5Hz), 10.86 (1H, s), hidden (2H).

### Example compound 21-22: 3,4-dichloro-N-{4-[2-(4-fluoro-3-methylphenyl)imidazo[1,2-b]pyridazin-3-yl]pyridin-2-yl}benzamide formate

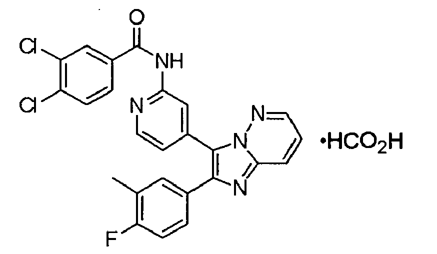

MS (ESI+): 492 (M+H).

### Example compound 21-23: N-{4-[2-(4-fluoro-3-methylphenyl)imidazo[1,2-b]pyridazin-3-yl]pyridin-2-yl}butanamide trifluoroacetate

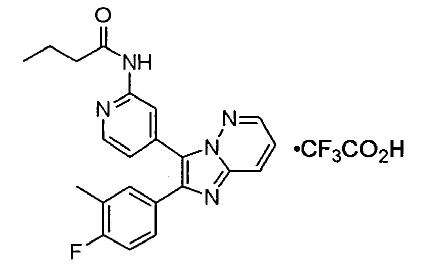

MS (ESI+): 390 (M+H).

### Example compound 21-24: N-{4-[2-(4-fluoro-3-methylphenyl)imidazo[1,2-b]pyridazin-3-yl]pyridin-2-yl}-2,2-dimethylpropanamide formate

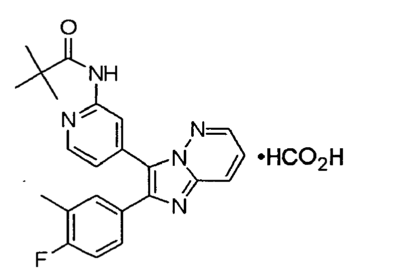

MS (ESI+): 404 (M+H).
¹H-NMR (CD₃OD) δ: 1.31 (9H, s), 2.25 (3H, s), 7.05 (1H, t, J=9.1Hz), 7.22-7.29 (1H, m), 7.34-7.40 (2H, m), 7.53 (1H, d, J=7.4Hz), 8.04-8.12 (1H, m), 8.34 (1H, d, J=5.1Hz), 8.46 (1H, s), 8.48-8.53 (1H, m), hidden (3H).

### Example compound 21-25: N-{4-[2-(4-fluoro-3-methylphenyl)imidazo[1,2-b]pyridazin-3-yl]pyridin-2-yl}-2-methylpropanamide trifluoroacetate

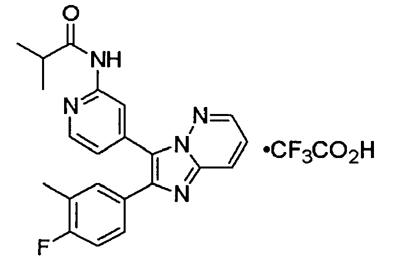

MS (ESI+): 390 (M+H).

### Example compound 21-26: N-{4-[2-(4-fluoro-3-methylphenyl)imidazo[1,2-b]pyridazin-3-yl]pyridin-2-yl}-3,3-dimethylbutanamide trifluoroacetate

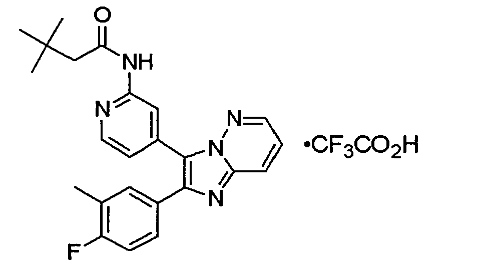

MS (ESI+): 418 (M+H).
¹H-NMR (DMSO-d₆) δ: 0.99 (9H, s), 2.23 (3H, d, J=0.8Hz), 2.27 (2H, s), 7.09-7.18 (1H, m), 7.29 (1H, dd, J=5.1, 1.3Hz), 7.34-7.42 (2H, m), 7.63-7.66 (1H, m), 8.24 (1H, dd, J=9.1, 1.5Hz), 8.36 (1H, s), 8.43 (1H, d, J=5.1Hz), 8.57 (1H, dd, J=4.3, 1.5Hz), 10.49 (1H, s), hidden (1H).

### Example compound 21-27: N-{4-[2-(4-chlorophenyl)imidazo[1,2-b]pyridazin-3-yl]pyridin-2-yl}benzamide

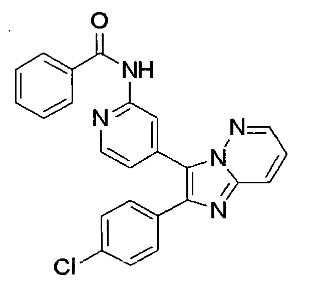

Melting point 218-219°C (ethanol)
¹H-NMR (CDCl₃) δ: 7.13-7.20 (2H, m), 7.36 (2H, d, 8.7Hz), 7.48-7.60 (3H, m), 7.65 (2H, d, J=8.7Hz), 7.91-7.98 (2H, m), 8.05 (1H, dd, J=9.2, 1.7Hz), 8.36 (1H, dd, J=5.1, 0.8Hz), 8.41 (1H, dd, J=4.4, 1.7Hz), 8.72 (1H, s), 8.85 (1H, dd, J=1.4, 0.8Hz).
Elemental analysis: for C₂₄H₁₆ClN₅O
Calculated: C, 67.69; H, 3.79; N, 16.44.
Found: C, 67.74; H, 3.81; N, 16.51.

### Example compound 21-28: N-{4-[2-(4-chlorophenyl)imidazo[1,2-b]pyridazin-3-yl]pyridin-2-yl}propanamide

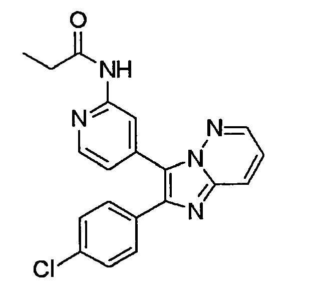

Melting point 204-205°C (ethanol)
¹H-NMR (CDCl₃) δ: 1.26 (3H, t, J=7.5Hz), 2.46 (2H, q, J=7.5Hz), 7.12 (1H, dd, J=5.3, 1.3Hz), 7.15 (1H, dd, J=9.1, 4.3Hz), 7.34 (2H, d, J=8.7Hz), 7.62 (2H, d, J=8.7Hz), 8.03 (1H, dd, J=9.1, 1.7Hz), 8.04 (1H, s), 8.31 (1H, dd, J=5.3, 0.7Hz), 8.39 (1H, dd, J=4.3, 1.7Hz), 8.66 (1H, s).
Elemental analysis: for C₂₀H₁₆ClN₅O
Calculated: C, 63.58; H, 4.27; N, 18.54.
Found: C, 63.38; H, 4.22; N, 18.47.

### Example compound 21-29: N-{4-[2-(4-fluorophenyl)imidazo[1,2-b]pyridazin-3-yl]pyridin-2-yl}-4-methylbenzamide trifluoroacetate

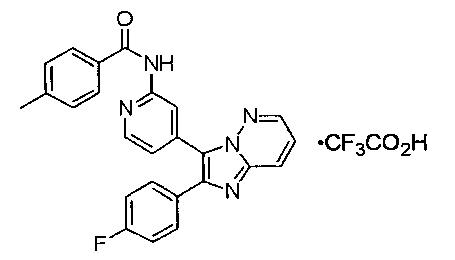

MS (ESI+): 424 (M+H).
¹H-NMR (DMSO-d₆) δ: 2.38 (3H, s), 7.23-7.34 (5H, m), 7.39 (1H, dd, J=9.2, 4.5Hz), 7.67-7.74 (2H, m), 7.93 (2H, d, J=8.1Hz), 8.27 (1H, dd, J=9.2, 1.6Hz), 8.45-8.54 (2H, m), 8.59 (1H, dd, J=4.5, 1.6Hz), 10.88 (1H, s), hidden (1H).

### Example compound 21-30: N-{4-[2-(4-fluorophenyl)imidazo[1,2-b]pyridazin-3-yl]pyridin-2-yl}-4-methoxybenzamide trifluoroacetate

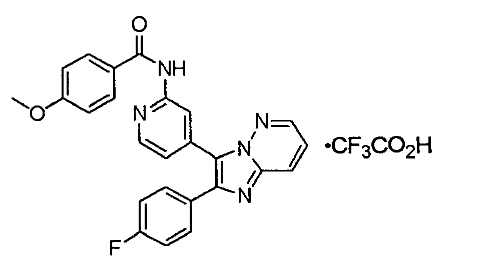

MS (ESI+): 440 (M+H).
¹H-NMR (DMSO-d₆) δ: 3.84 (3H, s), 7.04 (2H, d, J=8.9Hz), 7.22-7.30 (3H, m), 7.39 (1H, dd, J=9.1, 4.5Hz), 7.65-7.76 (2H, m), 8.03 (2H, d, J=8.9Hz), 8.27 (1H, dd, J=9.1, 1.5Hz), 8.48 (1H, s), 8.50 (1H, d, J=5.3Hz), 8.58 (1H, dd, J=4.5, 1.5Hz), 10.82 (1H, s), hidden (1H).

### Example compound 21-31: 4-chloro-N-{4-[2-(4-fluorophenyl)imidazo[1,2-b]pyridazin-3-yl]pyridin-2-yl}benzamide formate

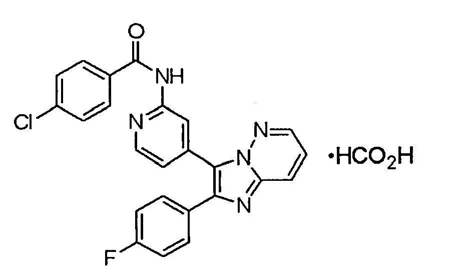

MS (ESI+): 444 (M+H).
¹H-NMR (DMSO-d₆) δ: 7.23-7.41 (4H, m), 7.59 (2H, d, J=8.5Hz), 7.67-7.74 (2H, m), 8.03 (2H, d, J=8.5Hz), 8.28 (1H, dd, J=9.1, 1.2Hz), 8.48 (1H, s), 8.52 (1H, d, J=5.1Hz), 8.56-8.60 (1H, m), 11.09 (1H, s), hidden (2H).

### Example compound 21-32: N-{4-[2-(4-fluorophenyl)imidazo[1,2-b]pyridazin-3-yl]pyridin-2-yl}acetamide trifluoroacetate

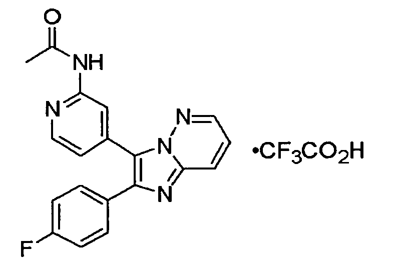

MS (ESI+): 348 (M+H).

### Example compound 21-33: N-{4-[2-(4-fluorophenyl)imidazo[1,2-b]pyridazin-3-yl]pyridin-2-yl}cyclopropanecarboxamide trifluoroacetate

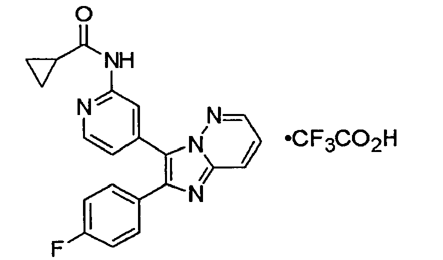

MS (ESI+): 374 (M+H).
¹H-NMR (DMSO-d₆) δ: 0.73-0.85 (4H, m), 1.98-2.07 (1H, m), 7.18-7.29 (3H, m), 7.36 (1H, dd, J=9.2, 4.5Hz), 7.63-7.70 (2H, m), 8.25 (1H, dd, J=9.2, 1.5Hz), 8.36 (1H, s), 8.43 (1H, d, J=5.1Hz), 8.56 (1H, dd, J=4.5, 1.5Hz), 10.97 (1H, s), hidden (1H).

### Example compound 21-34: 4-fluoro-N-{4-[2-(4-fluorophenyl)imidazo[1,2-b]pyridazin-3-yl]pyridin-2-yl}benzamide formate

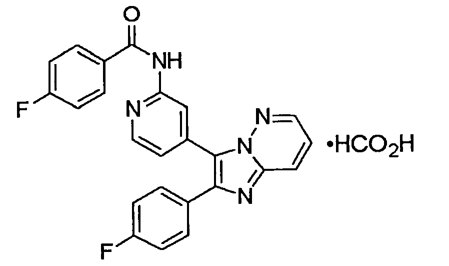

MS (ESI+): 428 (M+H).
¹H-NMR (DMSO-d₆) δ: 7.26-7.41 (6H, m), 7.71 (2H, dd, J=8.9, 5.5Hz), 8.10 (2H, dd, J=8.9, 5.5Hz), 8.27 (1H, dd, J=9.2, 1.5Hz), 8.48 (1H, s), 8.52 (1H, d, J=5.1Hz), 8.59 (1H, dd, J=4.3, 1.5Hz), 11.03 (1H, s), hidden (2H).

### Example compound 21-35: 4-tert-butyl-N-{4-[2-(4-fluorophenyl)imidazo[1,2-b]pyridazin-3-yl]pyridin-2-yl}benzamide formate

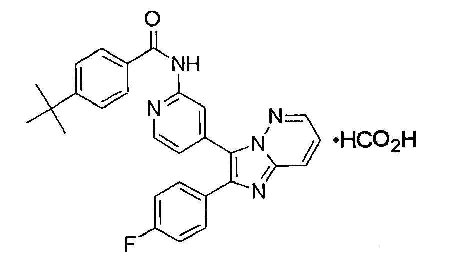

MS (ESI+): 466 (M+H).
¹H-NMR (DMSO-d₆) δ: 1.32 (9H, s), 7.22-7.31 (3H, m), 7.38 (1H, dd, J=9.1, 4.4Hz), 7.53 (2H, d, J=8.5Hz), 7.67-7.75 (2H, m), 7.97 (2H, d, J=8.5Hz), 8.27 (1H, dd, J=9.1, 1.4Hz), 8.49-8.53 (2H, m), 8.59 (1H, dd, J=4.4, 1.4Hz), 10.87 (1H, s), hidden (2H).

### Example compound 21-36: 3,4-dichloro-N-{4-[2-(4-fluorophenyl)imidazo[1,2-b]pyridazin-3-yl]pyridin-2-yl}benzamide formate

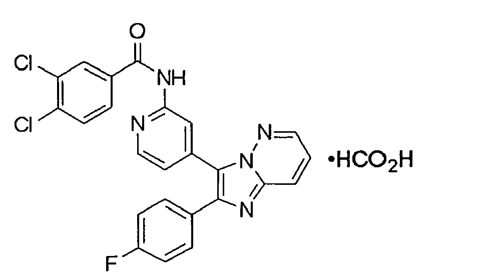

MS (ESI+): 478 (M+H).
¹H-NMR (DMSO-d₆) δ: 7.23-7.29 (2H, m), 7.33 (1H, dd, J=5.1, 1.5Hz), 7.39 (1H, dd, J=9.2, 4.3Hz), 7.66-7.75 (2H, m), 7.79 (1H, d, J=8.5Hz), 7.95-7.98 (1H, m), 8.23-8.33 (2H, m), 8.47 (1H, s), 8.53 (1H, d, J=5.1Hz), 8.59 (1H, dd, J=4.3, 1.5Hz), 11.21 (1H, s), hidden (2H).

### Example compound 21-37: N-{4-[2-(4-fluorophenyl)imidazo[1,2-b]pyridazin-3-yl]pyridin-2-yl}butanamide trifluoroacetate

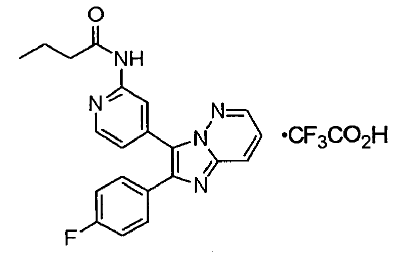

MS (ESI+): 376 (M+H).

### Example compound 21-38: N-{4-[2-(4-fluorophenyl)imidazo[1,2-b]pyridazin-3-yl]pyridin-2-yl}-2,2-dimethylpropanamide formate

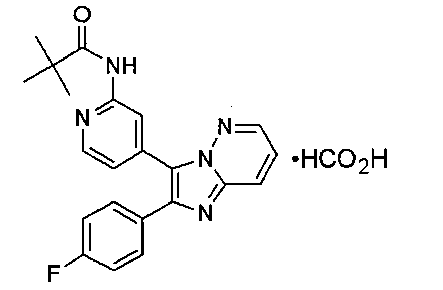

MS (ESI+): 390 (M+H).

### Example compound 21-39: N-{4-[2-(4-fluorophenyl)imidazo[1,2-b]pyridazin-3-yl]pyridin-2-yl}-2-methylpropanamide trifluoroacetate

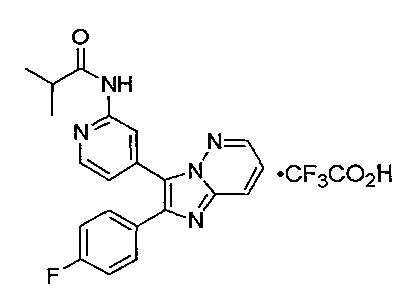

MS (ESI+): 376 (M+H).

### Example compound 21-40: N-{4-[2-(4-fluorophenyl)imidazo[1,2-b]pyridazin-3-yl]pyridin-2-yl}-3,3-dimethylbutanamide trifluoroacetate

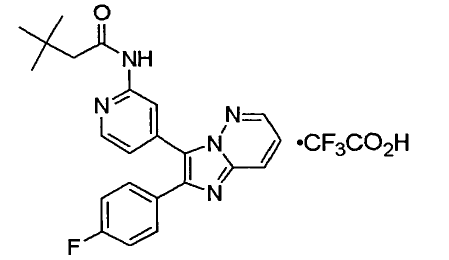

MS (ESI+): 404 (M+H).
¹H-NMR (DMSO-d₆) δ: 0.99 (9H, s), 2.28 (2H, s), 7.21-7.27 (2H, m), 7.30 (1H, dd, J=5.1, 1.5Hz), 7.39 (1H, dd, J=9.2, 4.3Hz), 7.63-7.71 (2H, m), 8.27 (1H, dd, J=9.2, 1.5Hz), 8.32 (1H, s), 8.43 (1H, d, J=5.1Hz), 8.60 (1H, dd, J=4.3, 1.5Hz), 10.61 (1H, s), hidden (1H) .

### (Example 22)

### N-{4-[2-(3-Methylphenyl)imidazo[1,2-b]pyridazin-3-yl]pyridin-2-yl}-N-propionylpropanamide

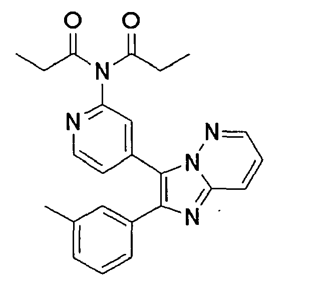

To a solution of 4-[2-(3-methylphenyl)imidazo[1,2-b]pyridazin-3-yl]pyridin-2-amine (150 mg, 0.498 mmol) obtained in Example 14 in dichloromethane - N,N-dimethylformamide (1:1) mixed solvent (6 mL) were added triethylamine (175 µL, 1.25 mmol) and propionyl chloride (109 µL, 1.25 mmol), and the mixture was stirred at room temperature for 30 min. Saturated aqueous sodium hydrogen carbonate solution was added to the reaction mixture, and the mixture was extracted with dichloromethane. The extract was washed with saturated brine and dried over anhydrous magnesium sulfate, and the solvent was removed by evaporation under reduced pressure. The obtained residue was purified by silica gel chromatography (ethyl acetate: hexane = 2:3 - 3:1) and recrystallized from ethyl acetate-hexane to give the title compound (yield 70.0 mg, 34%).
Melting point 210-211°C (ethyl acetate-hexane)
MS (ESI+): 414 (M+H).
¹H-NMR (CDCl₃) δ: 1.11 (6H, t, J=7.3Hz), 2.39 (3H, s), 2.59 (4H, q, J=7.3Hz), 7.11-7.32 (3H, m), 7.39 (1H, d, J=7.3Hz), 7.48 (1H, d, J=0.8Hz), 7.58 (1H, s), 7.84 (1H, dd, J=5.3, 1.5Hz), 8.06 (1H, dd, J=9.2, 1.7Hz), 8.40 (1H, dd, J=4.5, 1.7Hz), 8.68 (1H, d, J=5.3Hz).
Elemental analysis: for C₂₄H₂₃N₅O₂
Calculated: C, 69.72; H, 5.61; N, 16.94.
Found: C, 69.73; H, 5.68; N, 16.93.

### (Example 23)

The compounds of Examples 23-1 and 23-2 below were synthesized in the same manner as in Example 22 respectively using cyclopropanecarbonyl chloride or 4-fluorobenzoyl chloride, instead of propionyl chloride.

### Example compound 23-1: N-(cyclopropylcarbonyl)-N-{4-[2-(3-methylphenyl)imidazo[1,2-b]pyridazin-3-yl]pyridin-2-yl}cyclopropanecarboxamide

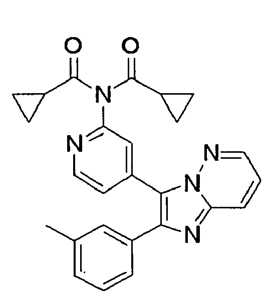

Melting point 173-175°C (ethyl acetate-hexane)
MS (ESI+): 438 (M+H).
¹H-NMR (CDCl₃) δ: 0.83-0.98 (4H, m), 1.08-1.21 (4H, m), 1.97-2.14 (2H, m), 2.38 (3H, s), 7.11-7.31 (3H, m), 7.39 (1H, d, J=7.3Hz), 7.58 (1H, s), 7.64 (1H, d, J=0.8 Hz), 7.76 (1H, dd, J=5.3, 1.5Hz), 8.06 (1H, dd, J=9.2, 1.7Hz), 8.38 (1H, dd, J=4.3, 1.7Hz), 8.68 (1H, d, J=5.3Hz).
Elemental analysis: for C₂₆H₂₃N₅O₂
Calculated: C, 71.38; H, 5.30; N, 16.01.
Found: C, 71.25; H, 5.40; N, 15.92.

### Example compound 23-2: 4-fluoro-N-(4-fluorobenzoyl)-N-{4-[2-(3-methylphenyl)imidazo[1,2-b]pyridazin-3-yl]pyridin-2-yl}benzamide

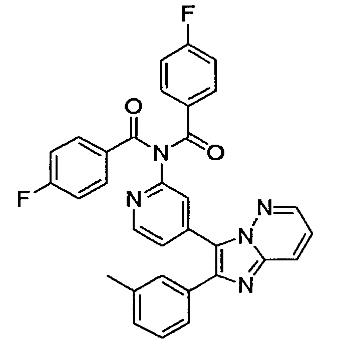

MS (ESI+): 546 (M+H).
¹H-NMR (CDCl₃) δ: 2.39 (3H, s), 6.98-7.10 (4H, m), 7.11-7.36 (4H, m), 7.47 (1H, dd, J=5.3, 1.6Hz) , 7.56 (1H, s), 7.66 (1H, s), 7.74-7.87 (4H, m), 8.03 (1H, dd, J=9.1, 1.6Hz), 8.31 (1H, dd, J=4.3, 1.6Hz), 8.43 (1H, dd, J=5.3, 0.8Hz).

### (Example 24)

### N-{4-[2-(3-Methylphenyl)imidazo[1,2-b]pyridazin-3-yl]pyridin-2-yl}-N-(phenylsulfonyl)benzenesulfonamide

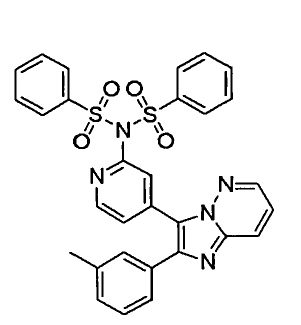

To a solution of 4-[2-(3-methylphenyl)imidazo[1,2-b]pyridazin-3-yl]pyridin-2-amine (100 mg, 0.332 mmol) obtained in Example 14 in dichloromethane (3 mL) were added triethylamine (140 µL, 1.00 mmol) and benzenesulfonyl chloride (128 µL, 1.00 mmol), and the mixture was stirred at room temperature for 2 hr. Saturated aqueous sodium hydrogen carbonate solution was added to the reaction mixture and the mixture was extracted with dichloromethane. The extract was dried over anhydrous sodium sulfate, and the solvent was removed by evaporation under reduced pressure. The obtained residue was purified by silica gel chromatography (ethyl acetate: hexane = 1:1 - ethyl acetate) and recrystallized from ethyl acetate-hexane to give the title compound (yield 132 mg, 68%).
MS (ESI+): 582 (M+H).
¹H-NMR (CDCl₃) δ: 2.43 (3H, s), 6.49 (1H, dd, J=7.8, 2.0Hz), 7.19-7.43 (6H, m), 7.45-7.69 (5H, m), 7.81-7.95 (4H, m), 8.06 (1H, dd, J=9.1, 1.6Hz), 8.21 (1H, d, J=7.8Hz), 8.47 (1H, dd, J=4.5, 1.6Hz), 8.51 (1H, d, J=1.6Hz).

### (Example 25)

### N-{4-[2-(3-Methylphenyl)imidazo[1,2-b]pyridazin-3-yl]pyridin-2-yl}benzenesulfonamide

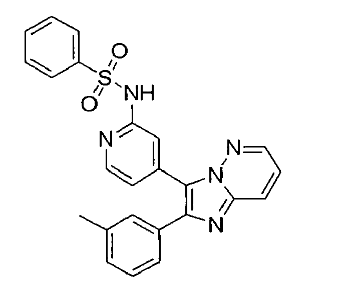

N-{4-[2-(3-Methylphenyl)imidazo[1,2-b]pyridazin-3-yl]pyridin-2-yl}-N-(phenylsulfonyl)benzenesulfonamide (85.0 mg, 0.146 mmol) obtained in Example 24 was dissolved in ammonia-ethanol solution (2.0 M, 5 mL). The mixture was stirred at room temperature for 2 hr, and the solvent was removed by evaporation under reduced pressure. Saturated aqueous sodium hydrogen carbonate solution was added to the obtained residue, and the mixture was extracted with ethyl acetate. The extract was washed with saturated brine and dried over anhydrous magnesium sulfate. The solvent was removed by evaporation under reduced pressure, and the resulted residue was recrystallized from ethyl acetate-hexane to give the title compound (yield 15.3 mg, 24%).
MS (ESI+): 442 (M+H).
¹H-NMR (CDCl₃) δ: 2.39 (3H, s), 6.93 (1H, dd, J=6.0, 1.6Hz), 7.11-7.63 (8H, m), 7.86-8.02 (3H, m), 8.06 (1H, dd, J=9.2, 1.7Hz), 8.20 (1H, d, J=6.0Hz), 8.33 (1H, dd, J=4.5, 1.7Hz), 12.04-13.12 (1H, m).

### (Example 26)

### N-{4-[2-(3-Methylphenyl)imidazo[1,2-b]pyridazin-3-yl]pyridin-2-yl}-N'-phenylurea

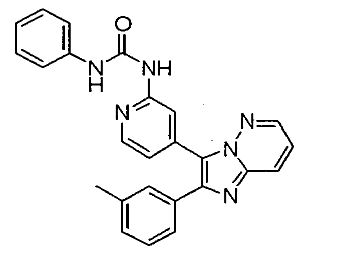

To a solution of 4-[2-(3-methylphenyl)imidazo[1,2-b]pyridazin-3-yl]pyridin-2-amine (100 mg, 0.332 mmol) obtained in Example 14 in dichloromethane (3 mL) was added phenyl isocyanate (90.0 mL, 0.828 mmol), and the mixture was stirred at room temperature for 3 hr. Saturated aqueous sodium hydrogen carbonate solution was added to the reaction mixture. The mixture was extracted with dichloromethane and dried over anhydrous sodium sulfate, and the solvent was removed by evaporation under reduced pressure. The obtained residue was purified by silica gel chromatography (ethyl acetate: hexane = 3:2 - ethyl acetate) and recrystallized from ethyl acetate-hexane to give the title compound (yield 58.4 mg, 42%).
Melting point 247-249°C
MS (ESI+): 421 (M+H).
¹H-NMR (CDCl₃) δ: 2.38 (3H, s), 7.07 (1H, t, J=7.4Hz), 7.12-7.41 (8H, m), 7.55-7.66 (3H, m), 8.06 (1H, dd, J=9.1, 1.6Hz), 8.11 (1H, s), 8.26 (1H, d, J=5.5Hz), 8.34 (1H, dd, J=4.3, 1.6Hz), 11.77 (1H, s).
Elemental analysis: for C₂₅H₂₀N₆O
Calculated: C, 71.41; H, 4.79; N, 19.99.
Found: C, 71.20; H, 4.63; N, 19.91.

### (Example 27)

The compounds of Examples 27-1 - 27-7 below were synthesized in the same manner as in Example 26 respectively using p-tolyl isocyanate, m-tolyl isocyanate, o-tolyl isocyanate, ethyl isocyanate, n-propyl isocyanate, isopropyl isocyanate or cyclohexyl isocyanate, instead of phenyl isocyanate.

### Example compound 27-1: N-(4-methylphenyl)-N'-{4-[2-(3-methylphenyl)imidazo[1,2-b]pyridazin-3-yl]pyridin-2-yl}urea

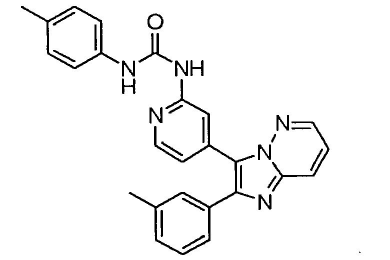

¹H-NMR (CDCl₃) δ: 2.32 (3H, s), 2.37 (3H, s), 7.07-7.28 (6H, m), 7.32-7.41 (2H, m), 7.47 (2H, d, J=8.3Hz), 7.60 (1H, s), 8.03-8.09 (1H, m), 8.23-8.32 (3H, m), 11.63 (1H, s).

### Example compound 27-2: N-(3-methylphenyl)-N'-{4-[2-(3-methylphenyl)imidazo[1,2-b]pyridazin-3-yl]pyridin-2-yl}urea

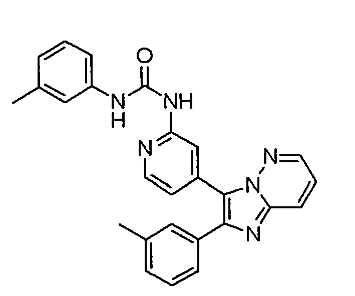

Melting point 202-203°C (ethyl acetate)
¹H-NMR (CDCl₃) δ: 2.29 (3H, s), 2.36 (3H, s), 6.88 (1H, d, J=7.5Hz), 7.07-7.27 (5H, m), 7.36-7.47 (4H, m), 7.60 (1H, s), 8.05 (1H, dd, J=9.2, 1.7Hz), 8.23 (1H, d, J=5.5Hz), 8.29 (1H, dd, J=4.3, 1.7Hz), 8.72 (1H, s), 11.61 (1H, s).
Elemental analysis: for C₂₆H₂₂N₆O
Calculated: C, 71.87; H, 5.10; N, 19.34.
Found: C, 71.80; H, 5.15; N, 19.30.

### Example compound 27-3: N-(2-methylphenyl)-N'-{4-[2-(3-methylphenyl)imidazo[1,2-b]pyridazin-3-yl]pyridin-2-yl}urea

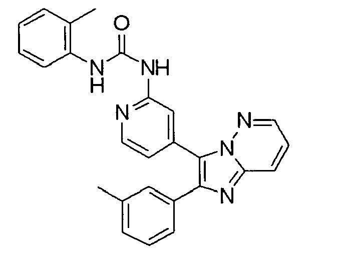

¹H-NMR (CDCl₃) δ: 2.38 (3H, s), 2.43 (3H, s), 7.00 (1H, t, J=7.4Hz), 7.11-7.27 (6H, m), 7.38 (2H, s), 7.61 (1H, s), 8.05-8.12 (2H, m), 8.23 (1H, d, J=5.5Hz), 8.31 (1H, d, J=3.0Hz), 8.38 (1H, s), 11.72 (1H, s).

### Example compound 27-4: N-ethyl-N'-(4-[2-(3-methylphenyl)imidazo[1,2-b]pyridazin-3-yl]pyridin-2-yl)urea

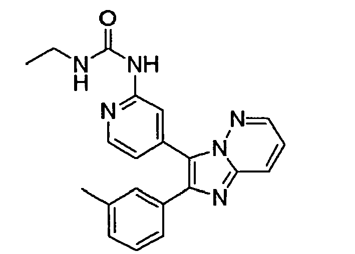

Melting point 212-213°C (ethyl acetate)
¹H-NMR (CDCl₃) δ: 1.24 (3H, t, J=7.3Hz), 2.36 (3H, s), 3.35-3.44 (2H, m), 7.06 (1H, dd, J=5.3, 1.4Hz), 7.11-7.25 (4H, m), 7.35 (1H, d, J=7.4Hz), 7.58 (1H, s), 8.04 (1H, dd, J=9.0, 1.7Hz), 8.16 (1H, d, J=5.3Hz), 8.24 (1H, s), 8.36 (1H, dd, J=4.4, 1.7Hz), 9.18 (1H, s).
Elemental analysis: for C₂₁H₂₀N₆O
Calculated: C, 67.73; H, 5.41; N, 22.57.
Found: C, 67.60; H, 5.42; N, 22.40.

### Example compound 27-5: N-{4-[2-(3-methylphenyl)imidazo[1,2-b]pyridazin-3-yl]pyridin-2-yl}-N'-propylurea

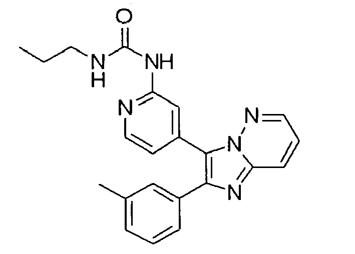

¹H-NMR (CDCl₃) δ: 0.99 (3H, t, J=7.4Hz), 1.58-1.70 (2H, m), 2.37 (3H, s), 3.30-3.38 (2H, m), 7.08-7.24 (5H, m), 7.31-7.38 (1H, m), 7.58 (1H, s), 7.66 (1H, s), 8.04 (1H, dd, J=9.1, 1.7Hz), 8.18 (1H, d, J=5.5Hz), 8.36 (1H, dd, J=4.5, 1.7Hz), 9.25-9.35 (1H, m). Example compound 27-6: N-isopropyl-N'-{4-[2-(3-methylphenyl)imidazo[1,2-b]pyridazin-3-yl]pyridin-2-yl}urea 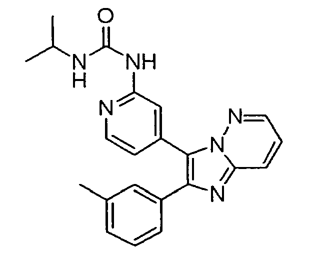 Melting point 193-195°C (ethyl acetate)
¹H-NMR (CDCl₃) δ: 1.27 (6H, d, J=6.6Hz), 2.37 (3H, s), 4.02-4.14 (1H, m), 7.08 (1H, dd, J=5.5, 1.4Hz), 7.12-7.23 (4H, m), 7.35 (1H, d, J=7.4Hz), 7.58 (2H, s), 8.04 (1H, dd, J=9.2, 1.7Hz), 8.18 (1H, d, J=5.5Hz), 8.36 (1H, dd, J=4.4, 1.7Hz), 9.13 (1H, d, J=7.4Hz).
Elemental analysis: for C₂₂H₂₂N₆O
Calculated: C, 68.38; H, 5.74; N, 21.75.
Found: C, 68.23; H, 5.83; N, 21.41.

### Example compound 27-7: N-cyclohexyl-N'-{4-[2-(3-methylphenyl)imidazo[1,2-b]pyridazin-3-yl]pyridin-2-yl}urea

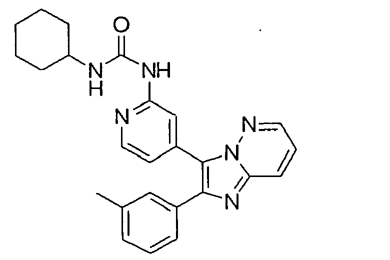

¹H-NMR (CDCl₃) δ: 1.21-1.50 (5H, m), 1.53-1.61 (1H, m), 1.67-1.79 (2H, m), 1.93-2.05 (2H, m), 2.37 (3H, s), 3.70-3.86 (1H, m), 7.07 (1H, dd, J=5.5, 1.3Hz), 7.12-7.25 (4H, m), 7.35 (1H, d, J=7.4Hz), 7.59 (2H, s), 8.04 (1H, dd, J=9.2, 1.7Hz), 8.18 (1H, d, J=5.5Hz), 8.36 (1H, dd, J=4.4, 1.7Hz), 9.24 (1H, d, J=7.2Hz).

### (Example 28)

The compounds of Examples 28-1 - 28-11 below were synthesized in the same manner as in Example 27 respectively using 4-[2-(4-fluoro-3-methylphenyl)imidazo[1,2-b]pyridazin-3-yl]pyridin-2-amine (Example compound 16-1), 4-[2-(3-chlorophenyl)imidazo[1,2-b]pyridazin-3-yl]pyridin-2-amine (Example compound 15), 4-[2-(4-chlorophenyl)imidazo[1,2-b]pyridazin-3-yl]pyridin-2-amine (Example compound 16-2) or 4-[2-(4-fluorophenyl)imidazo[1,2-b]pyridazin-3-yl]pyridin-2-amine (Example compound 16-3), instead of 4-[2-(3-methylphenyl) imidazo [1,2-b]pyridazin-3-yl]pyridin-2-amine. Example compound 28-1: N-{4-[2-(4-fluoro-3-methylphenyl)imidazo[1,2-b]pyridazin-3-yl]pyridin-2-yl}-N'-(3-methylphenyl)urea 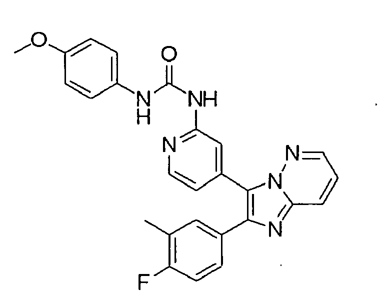 Melting point 213-215°C (ethyl acetate)
¹H-NMR (CDCl₃) δ: 2.30 (3H, d, J=1.7Hz), 3.80 (3H, s), 6.82-6.89 (2H, m), 7.00 (1H, t, J=8.9Hz), 7.10 (1H, dd, J=5.5, 1.3Hz), 7.16 (1H, dd, J=9.2, 4.4Hz), 7.30 (1H, s), 7.35-7.40 (1H, m), 7.46-7.52 (2H, m), 7.61 (1H, dd, J=7.4, 1.8Hz), 7.96 (1H, s), 8.05 (1H, dd, J=9.2, 1.7Hz), 8.27 (1H, d, J=5.5Hz), 8.33 (1H, dd, J=4.4, 1.7Hz), 11.52 (1H, s).
Elemental analysis: for C₂₆H₂₁FN₆O₂
Calculated: C, 66.66; H, 4.52; N, 17.94.
Found: C, 66.52; H, 4.47; N, 17.59.

### Example compound 28-2: N-(3,4-dichlorophenyl)-N'-{4-[2-(4-fluoro-3-methylphenyl)imidazo[1,2-b]pyridazin-3-yl]pyridin-2-yl}urea

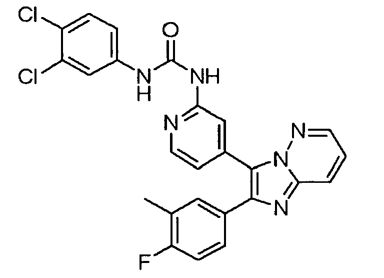

¹H-NMR (DMSO-d₆) δ: 2.26 (3H, d, J=1.3Hz), 7.11-7.24 (2H, m), 7.32-7.48 (3H, m), 7.56 (1H, d, J=8.9Hz), 7.67 (1H, dd, J=7.6, 1.6Hz), 7.78 (1H, s), 7.96 (1H, d, 2.5Hz), 8.25 (1H, dd, J=9.2, 1.7Hz), 8.41 (1H, d, J=5.3Hz), 8.59 (1H, dd, J=4.5, 1.7Hz), 9.76 (1H, s), 10.83 (1H, s).

### Example compound 28-3: N-ethyl-N'-{4-[2-(4-fluoro-3-methylphenyl)imidazo[1,2-b]pyridazin-3-yl]pyridin-2-yl}urea

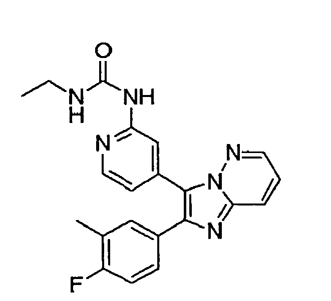

To a solution of 4-[2-(4-fluoro-3-methylphenyl)imidazo[1,2-b]pyridazin-3-yl]pyridin-2-amine (200 mg, 0.63 mmol) obtained in Example 16-1 in tetrahydrofuran (5 mL) was added ethyl isocyanate (100 µL, 1.3 mmol), and the mixture was stirred at 65°C for 14 hr. To the reaction mixture was added ethyl isocyanate (100 µL, 1.3 mmol), and the mixture was stirred at 50°C for 24 hr. Additional ethyl isocyanate (100 µL, 1.3 mmol) was added to the mixture, and the mixture was stirred at 50°C for 8 hr. The solvent was removed by evaporation under reduced pressure, and the obtained residue was purified by silica gel chromatography (ethyl acetate) and recrystallized from ethanol to give the title compound (yield 190 mg, 76%). Melting point 223-224°C (ethanol)
MS (ESI+): 391 (M+H).
¹H-NMR (CDCl₃) δ: 1.25 (3H, t, J=7.1Hz), 2.29 (3H, d, J=1.7Hz), 3.40 (2H, dq, J=5.5, 7.1Hz), 6.97 (1H, t, J=8.9Hz), 7.04 (1H, dd, J=5.4, 1.4Hz), 7.14 (1H, dd, J=9.2, 4.4Hz), 7.15 (1H, s), 7.30-7.37 (1H, m), 7.59 (1H, dd, J=7.2, 1.6Hz), 7.79 (1H, brs), 8.02 (1H, dd, J=9.2, 1.7Hz), 8.19 (1H, d, J=5.4Hz), 8.35 (1H, dd, J=4.4, 1.7Hz), 9.22 (1H, t, J=5.5Hz).
Elemental analysis: for C₂₁H₁₉FN₆O
Calculated: C, 64.60; H, 4.91; N, 21.53.
Found: C, 64.44; H, 4.96; N, 21.41.

### Example compound 28-4: N-{4-[2-(4-fluoro-3-methylphenyl)imidazo[1,2-b]pyridazin-3-yl]pyridin-2-yl}-N'-propylurea

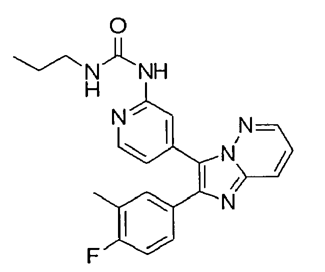

Melting point 231-232°C (ethyl acetate)
¹H-NMR (CDCl₃) δ: 0.99 (3H, t, J=7.4Hz), 1.58-1.70 (2H, m), 2.29 (3H, d, J=1.5Hz), 3.30-3.37 (2H, m), 6.98 (1H, t, J=9.0Hz), 7.05 (1H, m), 7.14-7.19 (2H, m), 7.32-7.38 (1H, m), 7.61 (1H, dd, J=7.4, 1.5Hz), 7.76-6.91 (1H, brs), 8.03 (1H, dd, J=9.1, 1.7Hz), 8.20 (1H, d, J=5.5Hz), 8.37 (1H, dd, J=4.3, 1.7Hz), 9.29 (1H, s).
Elemental analysis: for C₂₂H₂₁FN₆O
Calculated: C, 65.33; H, 5.23; N, 20.78.
Found: C, 65.10; H, 5.24; N, 20.63.

### Example compound 28-5: N-{4-[2-(4-fluoro-3-methylphenyl)imidazo[1,2-b]pyridazin-3-yl]pyridin-2-yl}-N'-isopropylurea

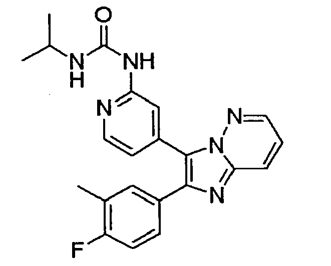

Melting point 232-233°C (ethyl acetate)
¹H-NMR (CDCl₃) δ: 1.27 (6H, d, J=6.6Hz), 2.29 (3H, d, J=1.7Hz), 4.01-4.14 (1H, m), 6.94-7.02 (1H, m), 7.04 (1H, dd, J=5.5, 1.3Hz), 7.11-7.19 (2H, m), 7.30-7.38 (1H, m), 7.61 (1H, dd, J=7.4, 1.6Hz), 7.75 (1H, s), 8.03 (1H, dd, J=9.2, 1.7Hz), 8.20 (1H, d, J=5.5Hz), 8.36 (1H, dd, J=4.3, 1.7Hz), 9.11 (1H, d, J=7.0Hz).
Elemental analysis: for C₂₂H₂₁FN₆O
Calculated: C, 65.33; H, 5.23; N, 20.78.
Found: C, 65.20; H, 5.32; N, 20.50.

### Example compound 28-6: N-cyclohexyl-N'-{4-[2-(4-fluoro-3-methylphenyl)imidazo[1,2-b]pyridazin-3-yl]pyridin-2-yl}urea

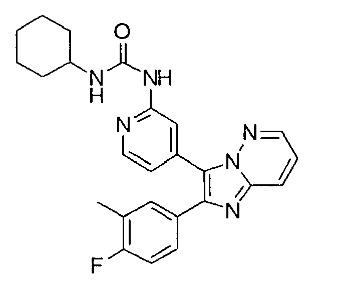

Melting point 229-230°C (ethyl acetate)
¹H-NMR (CDCl₃) δ: 1.24-1.76 (8H, m), 1.98-2.01 (2H, m), 2.30 (3H, s), 3.75-3.87 (1H, m), 6.99 (1H, t, J=8.9Hz), 7.05 (1H, m), 7.11-7.21 (2H, m), 7.32-7.37 (1H, m), 7.52 (1H, s), 7.61 (1H, dd, J=7.4, 1.8Hz), 8.03 (1H, dd, J=9.1, 1.7Hz), 8.20 (1H, d, J=5.3Hz), 8.36 (1H, dd, J=4.3, 1.7Hz), 9.25 (1H, d, J=6.8Hz).
Elemental analysis: for C₂₅H₂₅FN₆O
Calculated: C, 67.55; H, 5.67; N, 18.91.
Found: C, 67.49; H, 5.81; N, 18.65.

### Example compound 28-7: N-{4-[2-(3-chlorophenyl)imidazo[1,2-b]pyridazin-3-yl]pyridin-2-yl}-N'-ethylurea

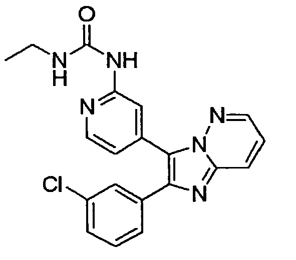

Melting point 230-231°C (ethanol)
MS (ESI+): 393 (M+H).
¹H-NMR (CDCl₃) δ: 1.25 (3H, t, J=7.1Hz), 3.41 (2H, dq, J=5.6, 7.1Hz), 7.05 (1H, dd, J=5.4, 1.4Hz), 7.10 (1H, s), 7.15 (1H, dd, J=9.3, 4.4Hz), 7.29 (1H, d, J=7.7Hz), 7.31-7.37 (1H, m), 7.43-7.48 (1H, m), 7.59 (1H, brs), 7.75 (1H, t, J=1.9Hz), 8.04 (1H, dd, J=9.3, 1.7Hz), 8.22 (1H, d, J=5.4Hz), 8.37 (1H, dd, J=4.4, 1.7Hz), 9.22 (1H, t, J=5.6Hz).
Elemental analysis: for C₂₀H₁₇ClN₆O
Calculated: C, 61.15; H, 4.36; N, 21.39.
Found: C, 61.06; H, 4.48; N, 21.11.

### Example compound 28-8: N-{4-[2-(4-chlorophenyl)imidazo[1,2-b]pyridazin-3-yl]pyridin-2-yl}-N'-ethylurea

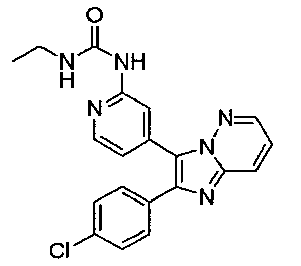

Melting point 223-224°C (ethanol)
¹H-NMR (CDCl₃) δ: 1.24 (3H, t, J=7.2Hz), 3.40 (2H, dq, J=5.4, 7.2Hz), 7.03 (1H, dd, J=5.4, 1.4Hz), 7.16 (1H, s), 7.17 (1H, dd, J=9.1, 4.5Hz), 7.36 (2H, d, J=8.7Hz), 7.62 (2H, d, J=8.7Hz), 7.95 (1H, s), 8.04 (1H, dd, J=9.1, 1.5Hz), 8.22 (1H, d, J=5.4Hz), 8.37 (1H, dd, J=4.5, 1.5Hz), 9.22 (1H, t, J=5.4Hz).
Elemental analysis: for C₂₀H₁₇ClN₆O
Calculated: C, 61.15; H, 4.36; N, 21.39.
Found: C, 61.05; H, 4.47; N, 21.24.

### Example compound 28-9: N-{4-[2-(4-fluorophenyl)imidazo[1,2-b]pyridazin-3-yl]pyridin-2-yl}-N'-propylurea

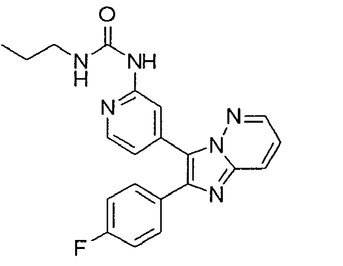

Melting point 219-220°C (ethyl acetate)
¹H-NMR (CDCl₃) δ: 0.98 (3H, t, J=7.4Hz), 1.57-1.70 (2H, m), 3.29-3.37 (2H, m), 7.03-7.18 (5H, m), 7.62-7.69 (2H, m), 7.93 (1H, s), 8.04 (1H, dd, J=9.2, 1.7Hz), 8.21 (1H, d, J=5.5Hz), 8.37 (1H, dd, J=4.5, 1.7Hz), 9.29 (1H, s).
Elemental analysis: for C₂₁H₁₉FN₆O
Calculated: C, 64.60; H, 4.91; N, 21.53.
Found: C, 64.42; H, 4.89; N, 21.34.

### Example compound 28-10: N-{4-[2-(4-fluorophenyl)imidazo[1,2-b]pyridazin-3-yl]pyridin-2-yl}-N'-isopropylurea

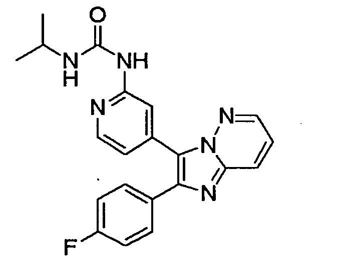

Melting point 222-223°C (ethyl acetate)
¹H-NMR (CDCl₃) δ: 1.26 (6H, d, J=6.6Hz), 4.00-4.14 (1H, m), 7.02-7.18 (5H, m), 7.62-7.69 (2H, m), 7.93 (1H, s), 8.04 (1H, dd, J=9.2, 1.7Hz), 8.21 (1H, d, J=5.3Hz), 8.36 (1H, dd, J=4.3, 1.7Hz), 9.11 (1H, d, J=7.4Hz).
Elemental analysis: for C₂₁H₁₉FN₆O Calculated: C, 64.60; H, 4.91; N, 21.53. Found: C, 64.51; H, 4.88; N, 21.40.

### Example compound 28-11: N-cyclohexyl-N'-{4-[2-(4-fluorophenyl)imidazo[1,2-b]pyridazin-3-yl]pyridin-2-yl}urea

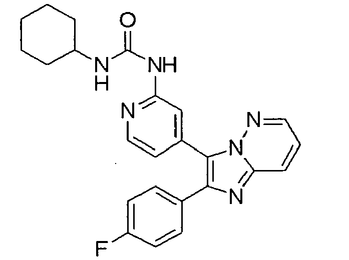

¹H-NMR (CDCl₃) δ: 1.24-1.61 (6H, m), 1.68-1.79 (2H, m), 1.91-2.06 (2H, m), 3.74-3.85 (1H, m), 7.00-7.19 (5H, m), 7.61-7.72 (3H, m), 8.04 (1H, dd, J=9.1, 1.6Hz), 8.21 (1H, d, J=5.3Hz), 8.36 (1H, dd, J=4.4, 1.6Hz), 9.24 (1H, d, J=7.4Hz).

### (Example 29)

### 2-Hydroxy-N-{4-[2-(3-methylphenyl)imidazo[1,2-b]pyridazin-3-yl]pyridin-2-yl}propanamide

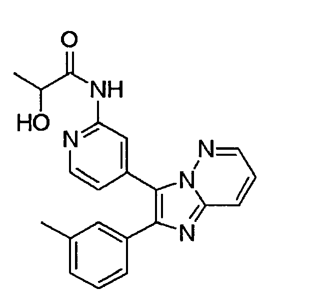

To 1-methyl-2-({4-[2-(3-methylphenyl)imidazo[1,2-b]pyridazin-3-yl]pyridin-2-yl}amino)-2-oxoethyl acetate (150 mg, 0.361 mmol) obtained in Example 20-6 in tetrahydrofuran-methanol(3:1) mixed solvent (2 mL) was added 1 M aqueous sodium hydroxide solution (0.397 mL, 0.397 mmol), and the mixture was stirred at room temperature for 1 hr. The solvent was removed by evaporation under reduced pressure. Water was added to the residue, and the mixture was extracted with ethyl acetate-tetrahydrofuran (1:1). The extract was dried over anhydrous sodium sulfate, and the solvent was removed by evaporation under reduced pressure. The obtained residue was purified by silica gel chromatography (ethyl acetate: hexane = 3:7 - 8:2) and recrystallized from ethyl acetate to give the title compound (yield 80.0 mg, 59%).
Melting point 223-224°C (ethyl acetate)
¹H-NMR (CDCl₃) δ: 1.31 (3H, d, J=6.8Hz), 2.30 (3H, s), 4.18-4.28 (1H, m), 5.91 (1H, s), 7.18 (1H, d, J=7.5Hz), 7.22-7.28 (2H, m), 7.37 (2H, m), 7.57 (1H, s), 8.25 (1H, dd, J=9.1, 1.6Hz), 8.41-8.45 (2H, m), 8.57 (1H, dd, J=4.4, 1.6Hz), 9.78 (1H, s).
Elemental analysis: for C₂₁H₁₉N₅O₂
Calculated: C, 67.55; H, 5.13; N, 18.76.
Found: C, 67.62; H, 5.13; N, 18.84.

### (Example 30)

### 2,2,2-Trichloroethyl {4-[2-(3-methylphenyl)imidazo[1,2-b]pyridazin-3-yl]pyridin-2-yl}carbamate

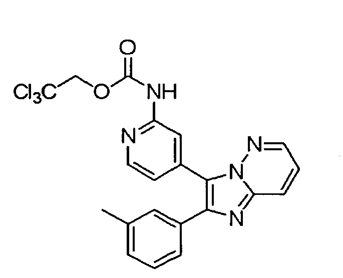

To a solution of 4-[2-(3-methylphenyl)imidazo[1,2-b]pyridazin-3-yl]pyridin-2-amine (2.5 g, 8.3 mmol) obtained in Example 14 in pyridine (50 mL) was added 2,2,2-trichloroethyl chloroformate (2.3 mL, 16.7 mmol), and the mixture was stirred at room temperature for 3 hr. 5% Aqueous sodium hydrogen carbonate solution was added to the reaction mixture, and the mixture was extracted with chloroform. A solution of ammonia in ethanol (2.0 M, 50 mL) was added to the extract. The mixture was stirred at room temperature overnight and dried over anhydrous sodium sulfate, and the solvent was removed by evaporation under reduced pressure. The obtained residue was purified by silica gel chromatography (ethyl acetate: hexane = 3:7 - 8:2) and the obtained crude crystals were washed with diisopropylether to give the title compound (yield 2.9 g, 73%).
¹H-NMR (CDCl₃) δ: 2.37 (3H, s), 4.85 (2H, s), 7.12-7.25 (4H, m), 7.37 (1H, d, J=7.4Hz), 7.61 (1H, s), 8.05 (1H, dd, J=9.1, 1.7Hz), 8.40 (1H, dd, J=4.4, 1.7Hz), 8.47 (1H, dd, J=5.4, 0.7Hz), 8.52 (1H, s), 9.72 (1H, s).

### (Example 31)

### N-{4-[2-(3-Methylphenyl)imidazo[1,2-b]pyridazin-3-yl]pyridin-2-yl}piperidine-1-carboxamide

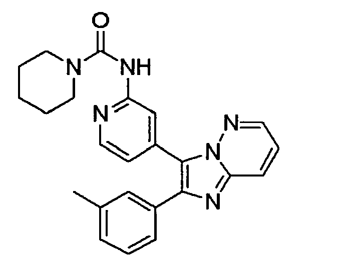

A solution of 2,2,2-trichloroethyl {4-[2-(3-methylphenyl)imidazo[1,2-b]pyridazin-3-yl]pyridin-2-yl}carbamate (150 mg, 0.315 mmol) obtained in Example 30, piperidine (37.4 µL, 0.378 mmol) and N-ethyldiisopropylamine (65.8 µL, 0.378 mmol) in dimethylsulfoxide (3 mL) was stirred at 70°C for 12 hr. 5% Aqueous sodium hydrogen carbonate solution was added to the reaction mixture, and the mixture was extracted with ethyl acetate. The extract was washed with water and dried over anhydrous sodium sulfate, and the solvent was removed by evaporation under reduced pressure. The obtained residue was purified by silica gel chromatography (ethyl acetate: hexane = 1:1 - 9:1) and the obtained crude crystals were recrystallized from ethanol-water to give the title compound (yield 73.6 mg, 57%).
Melting point 173-175°C (ethanol-water)
¹H-NMR (CDCl₃) δ: 1.64 (6H, s), 2.36 (3H, s), 3.47-3.52 (4H, m), 6.99 (1H, dd, J=5.1, 1.5Hz), 7.10 (1H, dd, J=9.2, 4.5Hz), 7.15 (1H, d, J=7.5Hz), 7.19-7.24 (1H, m), 7.30 (1H, s), 7.39 (1H, d, J=7.5Hz), 7.61 (1H, s), 8.01 (1H, dd, J=9.2, 1.7Hz), 8.21 (1H, dd, J=5.1, 0.7Hz), 8.38 (1H, dd, J=4.5, 1.7Hz), 8.53-8.54 (1H, m).
Elemental analysis: for C₂₄H₂₄N₆O
Calculated: C, 69.88; H, 5.86; N, 20.37.
Found: C, 69.81; H, 5.94; N, 20.18.

### (Example 32)

The compounds of Examples 32-1 and 32-2 below were synthesized in the same manner as in Example 31 and using diethylamine and 1,2,3,4-tetrahydroisoquinoline instead of piperidine.

### Example compound 32-1: N,N-diethyl-N'-{4-[2-(3-methylphenyl)imidazo[1,2-b]pyridazin-3-yl]pyridin-2-yl}urea

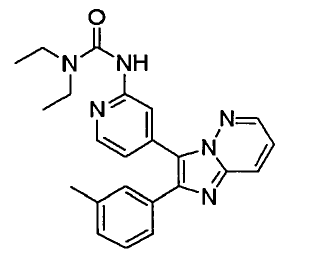

Melting point 166-167°C (ethanol-water)
¹H-NMR (CDCl₃) δ: 1.25 (6H, t, J=7.2Hz), 2.36 (3H, s), 3.41 (4H, q, J=7.2Hz), 6.98 (1H, dd, J=5.3, 1.5Hz), 7.10 (1H, dd, J=9.2, 4.5Hz), 7.14-7.24 (3H, m), 7.39 (1H, d, J=7.5Hz), 7.61 (1H, s), 8.01 (1H, dd, J=9.2, 1.7Hz), 8.21 (1H, dd, J=5.3, 0.7Hz), 8.38 (1H, dd, J=4.5, 1.7Hz), 8.62 (1H, m).
Elemental analysis: for C₂₃H₂₄N₆O
Calculated: C, 68.98; H, 6.04; N, 20.99.
Found: C, 68.87; H, 6.19; N, 20.79.

### Example compound 32-2: N-{4-[2-(3-methylphenyl)imidazo[1,2-b]pyridazin-3-yl]pyridin-2-yl}-3,4-dihydroisoquinoline-2(1H)-carboxamide

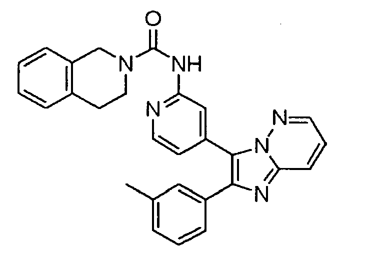

Melting point 122-124°C (ethanol-water)
¹H-NMR (CDCl₃) δ: 2.36 (3H, s), 2.96 (2H, t, J=5.8Hz), 3.77 (2H, t, J=5.8Hz), 4.72 (2H, s), 7.02 (1H, dd, J=5.3, 1.5Hz), 7.09-7.25 (7H, m), 7.35-7.41 (2H, m), 7.61 (1H, s), 8.02 (1H, dd, J=9.1, 1.7Hz), 8.23 (1H, dd, J=5.3, 0.8Hz), 8.38 (1H, dd, J=4.3, 1.7Hz), 8.59-8.60 (1H, m).

### (Example 33)

### 3-(2-Aminopyridin-4-yl)-2-(4-fluoro-3-methylphenyl)-N-methylimidazo[1,2-b]pyridazin-6-amine

The compound was synthesized in the same manner as in Example 7 using 4-[6-chloro-2-(4-fluoro-3-methylphenyl)imidazo[1,2-b]pyridazin-3-yl]pyridin-2-amine obtained in Example 13-2 instead of 4-[6-chloro-2-(3-methylphenyl)imidazo[1,2-b]pyridazin-3-yl]-N-cyclohexylpyridin-2-amine, and 30% methylamine-ethanol solution instead of 2.0 M dimethylamine-tetrahydrofuran solution. 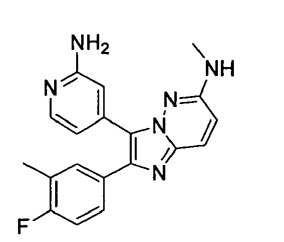 MS (ESI+): 349 (M+H).
¹H-NMR (DMSO-d₆) δ: 2.22 (3H, d, J=1.5Hz), 2.76 (3H, d, J=4.7Hz), 5.98 (2H, s), 6.60 (1H, dd, J=5.3, 1.5Hz), 6.69-6.76 (2H, m), 7.00-7.14 (2H, m), 7.27-7.39 (1H, m), 7.56 (1H, dd, J=7.6, 1.6Hz), 7.74 (1H, d,J=9.6Hz), 7.94 (1H, d, J=5.3Hz).

### (Example 34)

### 4-[6-Chloro-2-(3-methylphenyl)imidazo[1,2-b]pyridazin-3-yl]-2-pyrimidinamine

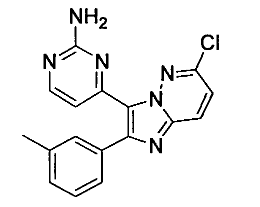

To a solution (10 mL) of tert-butyl 4-[2-hydroxy-2-(3-methylphenyl)ethenyl]-2-pyrimidinylcarbamate (1.0 g, 3.1 mmol) obtained in Reference Example 5 and sodium acetate (0.50 g, 6.11 mmol) in acetic acid was added dropwise bromine (0.16 mL, 3.1 mmol) at room temperature. The reaction mixture was directly stirred at room temperature for 2 hr. The acetic acid was evaporated under reduced pressure, and aqueous sodium hydrogen carbonate solution was added to the residue. The mixture was extracted with ethyl acetate, and the extract was dried and concentrated. The residue was dissolved in ethanol (10 mL), and 3-amino-6-chloropyridazine (0.40 g, 3.1 mmol) was added to the solution. The reaction mixture was heated under reflux for 14 hr, and allowed to be cooled to room temperature. The solvent was removed by evaporation under reduced pressure. Aqueous sodium hydrogen carbonate solution was added to the residue, and the mixture was extracted with ethyl acetate-THF. The extract was dried and concentrated, and the residue was purified by silica gel chromatography (ethyl acetate: hexane = 1:1 - 9:1) to give the title compound (yield 0.23 g, 22%).
Melting point 237-238°C (ethyl acetate-hexane)
MS (ESI+): 337 (M+H).
¹H-NMR (CDCl₃) δ: 2.38 (3H, s), 5.10 (2H, s), 7.08 (1H, d, J=5.1Hz), 7.14 (1H, d, J=9.5Hz), 7.16-7.20 (1H, m), 7.24 (1H, t, J=7.5Hz), 7.40-7.50 (1H, m), 7.61-7.65 (1H, m), 7.96 (1H, d, J=9.5Hz), 8.42 (1H, d, J=5.1Hz).

### (Example 35)

### 4-[6-Chloro-2-(4-fluoro-3-methylphenyl)imidazo[1,2-b]pyridazin-3-yl]-2-pyrimidinamine

The compound was synthesized in the same manner as in Example 34 and using tert-butyl 4-[2-hydroxy-2-(4-fluoro-3-methylphenyl)ethenyl]-2-pyrimidinylcarbamate obtained in Reference Example 6 instead of tert-butyl 4-[2-hydroxy-2-(3-methylphenyl)ethenyl]-2-pyrimidinylcarbamate. 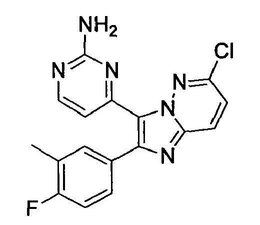 Melting point 196-197°C
¹H-NMR (CDCl₃) δ: 2.25 (3H, d, J=1.7Hz), 6.85 (2H, s), 6.72 (1H, d, J=4.9Hz), 7.16 (1H, t, J=9.2Hz), 7.50 (1H, d, J=9.6Hz), 7.52-7.59 (1H, m), 7.79 (1H, dd, J=8.0, 1.7Hz), 8.32 (1H, d, J=9.6Hz), 8.44 (1H, d, J=4.9Hz).

### (Example 36)

The compounds of Examples 36-1 - 36-4 below were synthesized in the same manner as in Example 12 using 2-(2-aminopyridin-4-yl)-2-bromo-1-(4-fluoro-3-methylphenyl)ethanone hydrobromide obtained by the method described in WO 01/74811 instead of 2-(2-aminopyridin-4-yl)-2-bromo-1-(3-methylphenyl)ethanone hydrobromide, and 6-chloro-4-methylpyridazin-3-amine obtained in Reference Example 2, 6-chloro-5-tert-butylpyridazin-3-amine obtained in Reference Example 3, 6-chloro-4-methoxypyridazin-3-amine obtained in Reference Example 4 or 6-chloro-4-(methylthio)pyridazin-3-amine obtained in Reference Example 4, instead of 3-amino-6-chloropyridazine.

### Example compound 36-1: 4-[6-chloro-2-(4-fluoro-3-methylphenyl)-8-methylimidazo[1,2-b]pyridazin-3-yl]-2-pyridylamine

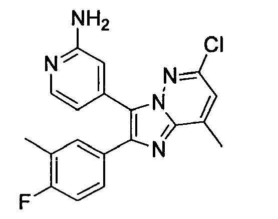

Melting point 218-219°C (ethanol)
¹H-NMR (CDCl₃) δ: 2.29 (3H, d, J=1.7Hz), 2.73 (3H, d, J=0.9Hz), 4.52 (2H, s), 6.79 (1H, s), 6.80 (1H, dd, J=5.2, 1.5Hz), 6.92-7.02 (2H, m), 7.31-7.40 (1H, m), 7.59 (1H, dd, J=7.5, 1.7Hz), 8.11-8.16 (1H, m).
Elemental analysis: for C₁₉H₁₅FClN₅
Calculated: C, 62.04; H, 4.11; N, 19.04.
Found: C, 61.94; H, 4.22; N, 18.68.

### Example compound 36-2: 4-[7-tert-butyl-6-chloro-2-(4-fluoro-3-methylphenyl)imidazo[1,2-b]pyridazin-3-yl]-2-pyridylamine

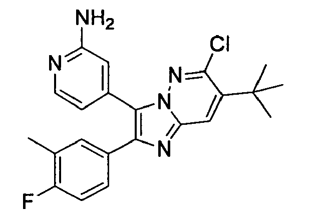

Melting point 232-233°C (ethanol)
MS (ESI+): 410 (M+H).
¹H-NMR (CDCl₃) δ: 1.55 (9H, s), 2.29 (3H, d, J=1.7Hz), 4.52 (2H, s), 6.82 (1H, s), 6.84 (1H, dd, J=5.3, 1.4Hz), 6.97 (1H, t, J=9.0Hz), 7.34-7.41 (1H, m), 7.60 (1H, dd, J=7.7, 1.9Hz), 8.01 (1H, s), 8.14 (1H, dd, J=5.3, 0.9Hz).
Elemental analysis: for C₂₂H₂₁FClN₅
Calculated: C, 64.47; H, 5.16; N, 17.09.
Found: C, 64.52; H, 5.17; N, 17.16.

### Example compound 36-3: 4-[6-chloro-2-(4-fluoro-3-methylphenyl)-8-methoxyimidazo[1,2-b]pyridazin-3-yl]-2-pyridylamine

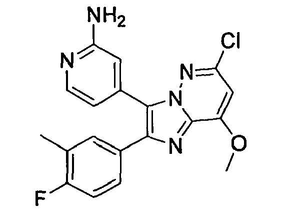

Melting point 200-201°C (ethanol)
¹H-NMR (CDCl₃) δ: 2.27 (3H, d, J=1.9Hz), 4.16 (3H, s), 4.52 (2H, s), 6.49 (1H, s), 6.76-6.79 (1H, m), 6.81 (1H, dd, J=5.3, 1.4Hz), 6.90-6.98 (1H, m), 7.31-7.39 (1H, m), 7.64 (1H, dd, J=7.4, 1.7Hz), 8.15 (1H, dd, J=5.3, 0.8Hz).

### Example compound 36-4: 4-[6-chloro-2-(4-fluoro-3-methylphenyl)-8-(methylthio)imidazo[1,2-b]pyridazin-3-yl]-2-pyridylamine

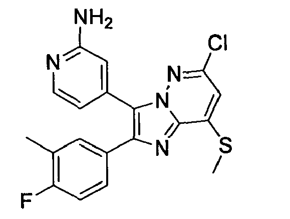

Melting point 237-238°C (ethanol)
¹H-NMR (CDCl₃) δ: 2.28 (3H, d, J=1.7Hz), 2.65 (3H, s), 4.95 (2H, brs), 6.77 (1H, s), 6.82 (1H, dd, J=5.2, 1.3Hz), 6.87 (1H, dd, J=1.3, 0.7Hz), 6.96 (1H, t, J=9.0Hz), 7.30-7.39 (1H, m), 7.59 (1H, dd, J=7.4, 1.6Hz), 8.06 (1H, d, J=5.2Hz).

### (Example 37)

The compounds of Examples 37-1 - 37-4 below were synthesized in the same manner as in Example 15 respectively using 4-[6-chloro-2-(3-methylphenyl)imidazo[1,2-b]pyridazin-3-yl]-2-pyrimidinamine (Example compound 34), 4-[6-chloro-2-(4-fluoro-3-methylphenyl)-8-methylimidazo[1,2-b]pyridazin-3-yl]-2-pyridylamine (Example compound 36-1), 4-[7-tert-butyl-6-chloro-2-(4-fluoro-3-methylphenyl)imidazo[1,2-b]pyridazin-3-yl]-2-pyridylamine (Example compound 36-2) or 4-[6-chloro-2-(4-fluoro-3-methylphenyl)-8-methoxyimidazo[1,2-b]pyridazin-3-yl]-2-pyridylamine (Example compound 36-3), instead of 4-[6-chloro-2-(3-chlorophenyl)imidazo[1,2-b]pyridazin-3-yl]pyridin-2-amine. Example compound 37-1: 4-[2-(3-methylphenyl)imidazo[1,2-b]pyridazin-3-yl]-2-pyrimidinamine 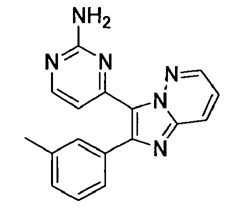 Melting point 250-251°C (ethanol)
¹H-NMR (DMSO-d₆) δ: 2.31 (3H, s), 6.09 (2H, brs), 6.56 (1H, dd, J=5.2, 1.7Hz), 6.66 (1H, s), 7.16 (1H, d, J=7.4Hz), 7.26 (1H, dd, J=7.7, 7.4Hz), 7.32 (1H, dd, J=9.4, 4.4Hz), 7.38 (1H, d, J=7.7Hz), 7.59 (1H, s), 8.01 (1H, d, J=5.2Hz), 8.21 (1H, dd, J=9.1, 1.7Hz), 8.53 (1H, dd, J=4.4, 1.7Hz).

### Example compound 37-2: 4-[2-(4-fluoro-3-methylphenyl)-8-methylimidazo[1,2-b]pyridazin-3-yl]-2-pyridylamine

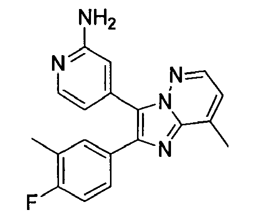

Melting point 212-213°C (ethanol)
¹H-NMR (CDCl₃) δ: 2.29 (3H, d, J=1.7Hz), 2.74 (3H, d, J=1.0Hz), 4.51 (2H, s), 6.82 (1H, s), 6.84 (1H, dd, J=5.3, 1.5Hz), 6.92 (1H, dd, J=4.5, 1.0Hz), 6.97 (1H, t, J=9.0Hz), 7.34-7.42 (1H, m), 7.62 (1H, dd, J=7.4, 1.8Hz), 8.14 (1H, dd, J=5.3, 0.6Hz), 8.22 (1H, d, J=4.5Hz).
Elemental analysis: for C₁₉H₁₆FN₅
Calculated: C, 68.46; H, 4.84; N, 21.01.
Found: C, 68.19; H, 5.11; N, 20.68.

### Example compound 37-3: 4-[7-tert-butyl-2-(4-fluoro-3-methylphenyl)imidazo[1,2-b]pyridazin-3-yl]-2-pyridylamine

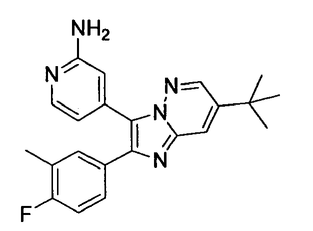

Melting point 212-213°C (ethanol)
¹H-NMR (CDCl₃) δ: 1.42 (9H, s), 2.29 (3H, d, J=1.7Hz), 4.51 (2H, s), 6.83 (1H, s), 6.86 (1H, dd, J=5.3, 1.4Hz), 7.00 (1H, t, J=9.0Hz), 7.35-7.43 (1H, m), 7.63 (1H, dd, J=7.5, 1.9Hz), 7.88 (1H, d, J=2.3Hz), 8.14 (1H, d, J=5.3Hz), 8.43 (1H, d, J=2.3Hz). Elemental analysis: for C₂₂H₂₂FN₅
Calculated: C, 70.38; H, 5.91; N, 18.65.
Found: C, 70.39; H, 5.97; N, 18.58. °

### Example compound 37-4: 4-[2-(4-fluoro-3-methylphenyl)-8-methoxyimidazo[1,2-b]pyridazin-3-yl]-2-pyridylamine

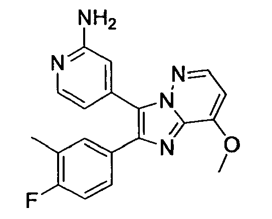

Melting point 219-220°C (ethanol)
¹H-NMR (CDCl₃) δ: 2.27 (3H, d, J=1.9Hz), 4.14 (3H, s), 4.50 (2H, s), 6.43 (1H, d, J=5.3Hz), 6.76-6.82 (1H, m), 6.85 (1H, dd, J=5.6, 1.1Hz), 6.89-7.00 (1H, m), 7.32-7.43 (1H, m), 7.68 (1H, dd, J=7.6, 1.5Hz), 8.16 (1H, d, J=5.3Hz), 8.20 (1H, d, J=5.6Hz).

### (Example 38)

### N-(Cyclohexylmethyl)-4-[2-(3-methylphenyl)imidazo[1,2-b]pyridazin-3-yl]pyridin-2-amine

The compound was synthesized in the same manner as in Example 5 and using cyclohexylmethylamine instead of 1-cyclopropylmethylamine. 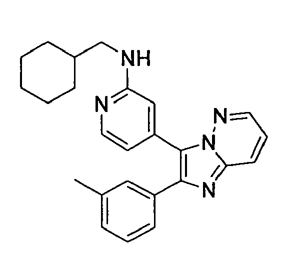 Melting point 128-130°C (ethyl acetate)
¹H-NMR (CDCl₃) δ: 0.87-1.01 (2H, m), 1.16-1.29 (3H, m), 1.42-1.58 (1H, m), 1.65-1.80 (5H, m), 2.37 (3H, s), 3.05 (2H, t, J=6.3Hz), 4.65-4.69 (1H, m), 6.64 (1H, s), 6.81 (1H, dd, J=5.3, 1.5Hz), 7.10 (1H, dd, J=9.0, 4.3Hz), 7.14-7.19 (1H, m), 7.21-7.25 (1H, m), 7.42 (1H, d, J=7.5Hz), 7.65 (1H, s), 8.02 (1H, dd, J=9.0, 1.7Hz), 8.14-8.20 (1H, m), 8.35 (1H, dd, J=4.3, 1.7Hz). Elemental analysis: for C₂₅H₂₇N₅
Calculated: C, 75.54; H, 6.85; N, 17.62.
Found: C, 75.41; H, 6.79; N, 17.34.

### (Example 39)

The compounds of Examples 39-1 - 39-3 below were synthesized in the same manner as in Example 19 using 4-methoxybenzoyl chloride instead of benzoyl chloride, and 4-[6-chloro-2-(4-fluoro-3-methylphenyl)imidazo[1,2-b]pyridazin-3-yl]pyridin-2-amine (Example compound 13-2), 4-[2-(3-chlorophenyl)imidazo[1,2-b]pyridazin-3-yl]pyridin-2-amine (Example compound 15) or 4-[2-(4-fluoro-3-methylphenyl)imidazo[1,2-b]pyridazin-3-yl]pyridin-2-amine (Example compound 16-1), instead of 4-[2-(3-methylphenyl)imidazo[1,2-b]pyridazin-3-yl]pyridin-2-amine. Example compound 39-1: N-{4-[6-chloro-2-(4-fluoro-3-methylphenyl)imidazo[1,2-b]pyridazin-3-yl]pyridin-2-yl}-4-methoxybenzamide 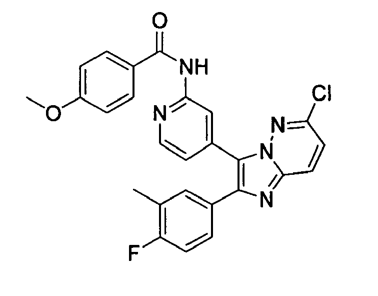 MS (ESI+): 488 (M+H).
¹H-NMR (CDCl₃) δ: 2.28 (3H, d, J=1.3Hz), 3.89 (3H, s), 6.93-7.02 (3H, m), 7.15 (1H, d, J=9.4Hz), 7.19 (1H, dd, J=5.3, 1.5Hz), 7.36 (1H, m), 7.59-7.64 (1H, m), 7.90 (2H, d, J=8.9Hz), 7.97 (1H, d, J=9.4Hz), 8.37 (1H, d, J=5.3Hz), 8.59 (1H, s), 8.72 (1H, s). Example compound 39-2: N-{4-[2-(3-chlorophenyl)imidazo[1,2-b]pyridazin-3-yl]pyridin-2-yl}-4-methoxybenzamide 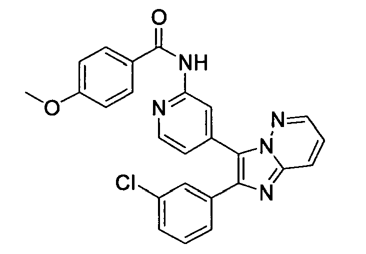 Melting point 223-224°C (tetrahydrofuran-water)
¹H-NMR (CDCl₃) δ: 3.38 (3H, s), 6.97-7.02 (2H, m), 7.12-7.22 (2H, m), 7.25-7.35 (2H, m), 7.50-7.54 (1H, m), 7.78-7.80 (1H, m), 7.88-7.94 (2H, m), 8.05 (1H, dd, J=9.2, 1.7Hz), 8.35 (1H, dd, J=5.2, 0.7Hz), 8.41 (1H, dd, J=4.3, 1.7Hz), 8.66 (1H, s), 8.84-8.85 (1H, m).
Elemental analysis: for C₂₅H₁₈ClN₅O₂
Calculated: C, 65.86; H, 3.98; N, 15.36.
Found: C, 65.82; H, 3.97; N, 15.33.

### Example compound 39-3: N-{4-[2-(4-fluoro-3-methylphenyl)imidazo[1,2-b]pyridazin-3-yl]pyridin-2-yl}-4-methoxybenzamide

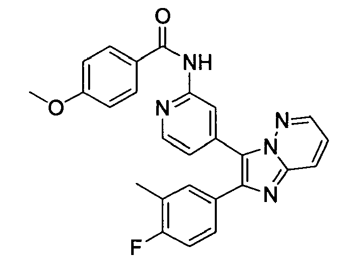

Melting point 222-223°C (ethanol-tetrahydrofuran)
¹H-NMR (CDCl₃) δ: 2.29 (3H, d, J=1.7Hz), 3.88 (3H, s), 6.93-7.02 (3H, m), 7.10-7.19 (2H, m), 7.35-7.43 (1H, m), 7.64 (1H, dd, J=7.4, 1.6Hz), 7.88-7.94 (2H, m), 8.03 (1H, dd, J=9.2, 1.7Hz), 8.31 (1H, dd, J=5.2, 0.8Hz), 8.40 (1H, dd, J=4.4, 1.6Hz), 8.71 (1H, s), 8.86 (1H, dd, J=1.3, 0.8Hz).
Elemental analysis: for C₂₆H₂₀FN₅O₂
Calculated: C, 68.86; H, 4.45; N, 15.44.
Found: C, 68.75; H, 4.49; N, 15.36.

### (Example 40)

The compounds of Examples 40-1 and 40-2 below were synthesized in the same manner as in Example 19 and using 4-[2-(4-fluorophenyl)imidazo[1,2-b]pyridazin-3-yl]pyridin-2-amine (Example compound 16-3) instead of 4-[2-(3-methylphenyl)imidazo[1,2-b]pyridazin-3-yl]pyridin-2-amine, and isonicotinoyl chloride hydrochloride or 6-chloronicotinoyl chloride instead of benzoyl chloride.

### Example compound 40-1: N-{4-[2-(4-fluorophenyl)imidazo[1,2-b]pyridazin-3-yl]pyridin-2-yl}isonicotinamide

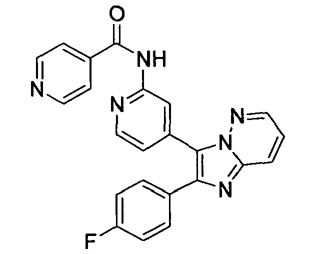

Melting point 226-227°C (ethanol-tetrahydrofuran)
MS (ESI+): 411 (M+H).
¹H-NMR (CDCl₃) δ: 7.04-7.13 (2H, m), 7.18 (1H, dd, J=9.2, 4.5Hz), 7.24-7.26 (1H, m), 7.64-7.70 (2H, m), 7.74-7.78 (2H, m), 8.06 (1H, dd, J=9.2, 1.7Hz), 8.32-8.37 (1H, m), 8.42 (1H, dd, J=4.5, 1.7Hz), 8.80-8.86 (4H, m).
Elemental analysis: for C₂₃H₁₅FN₆O
Calculated: C, 67.31; H, 3.68; N, 20.48.
Found: C, 67.01; H, 3.76; N, 20.36.

### Example compound 40-2: 6-chloro-N-{4-[2-(4-fluorophenyl)imidazo[1,2-b]pyridazin-3-yl]pyridin-2-yl}nicotinamide

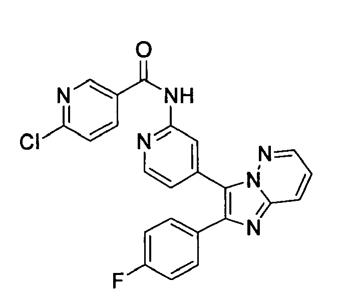

Melting point 247-248°C (ethanol-tetrahydrofuran)
MS (ESI+) : 445 (M+H).
¹H-NMR (CDCl₃) δ: 7.04-7.12 (2H, m), 7.18 (1H, dd, J=9.2, 4.4Hz), 7.24 (1H, dd, J=5.2, 1.4Hz), 7.48 (1H, dd, J=8.3, 0.6Hz), 7.64-7.70 (2H, m), 8.05 (1H, dd, J=9.2, 1.7Hz), 8.20 (1H, dd, J=8.3, 2.5Hz), 8.32 (1H, dd, J=5.2, 0.6Hz), 8.41 (1H, dd, J=4.4, 1.7Hz), 8.78-8.80 (1H, m), 8.88 (1H, s), 8.94 (1H, dd, J=2.5, 0.6Hz).
Elemental analysis: for C₂₃H₁₄ClFN₆O
Calculated: C, 62.10; H, 3.17; N, 18.89.
Found: C, 61.94; H, 3.23; N, 18.67.

### (Example 41)

The compounds of Examples 41-1 - 41-5 below were synthesized in the same manner as in Example 19 using 4-fluorobenzoyl chloride instead of benzoyl chloride, and 4-[2-(4-fluoro-3-methylphenyl)imidazo[1,2-b]pyridazin-3-yl]pyridin-2-amine (Example compound 16-1), 4-[6-chloro-2-(4-fluoro-3-methylphenyl)-8-methylimidazo[1,2-b]pyridazin-3-yl]-2-pyridylamine (Example compound 36-1), 4-[7-tert-butyl-6-chloro-2-(4-fluoro-3-methylphenyl)imidazo[1,2-b]pyridazin-3-yl]-2-pyridylamine (Example compound 36-2), 4-[2-(4-fluoro-3-methylphenyl)-8-methylimidazo[1,2-b]pyridazin-3-yl]-2-pyridylamine (Example compound 37-2) or 4-[7-tert-butyl-2-(4-fluoro-3-methylphenyl)imidazo[1,2-b]pyridazin-3-yl]-2-pyridylamine (Example compound 37-3), instead of 4-[2-(3-methylphenyl)imidazo[1,2-b]pyridazin-3-yl]pyridin-2-amine. Example compound 41-1: 4-fluoro-N-{4-[2-(4-fluoro-3-methylphenyl)imidazo[1,2-b]pyridazin-3-yl]pyridin-2-yl}benzamide

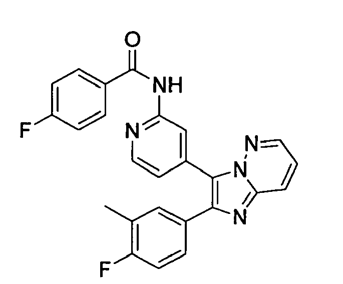

To a solution of 4-[2-(4-fluoro-3-methylphenyl)imidazo[1,2-b]pyridazin-3-yl]pyridin-2-amine (400 mg, 1.3 mmol) obtained in Example 16-1 in tetrahydrofuran (20 mL) were added triethylamine (320 mg, 3.2 mmol) and 4-fluorobenzoyl chloride (500 mg, 3.2 mmol), and the mixture was stirred at room temperature for 3 hr. 5% Aqueous sodium hydrogen carbonate solution was added to the reaction mixture. The obtained mixture was extracted with ethyl acetate/tetrahydrofuran. Ammonia-ethanol solution (2.0 M, 20 mL) was added to the extract, and the mixture was stirred at room temperature overnight. The mixture was dried over anhydrous sodium sulfate, and the solvent was removed by evaporation under reduced pressure. The obtained residue was purified by silica gel chromatography (ethyl acetate: hexane = 3:7 - 8:2) and recrystallized from ethanol to give the title compound (yield 450 mg, 82%).
Melting point 212-214°C (ethanol)
MS (ESI+): 442 (M+H).
¹H-NMR (CDCl₃) δ: 2.29 (3H, d, J=1.9Hz), 6.94-7.01 (1H, m), 7.13-7.23 (4H, m), 7.35-7.41 (1H, m), 7.64 (1H, dd, J=7.6, 1.8Hz), 7.92-7.98 (2H, m), 8.04 (1H, dd, J=9.1, 1.7Hz), 8.31 (1H, d, J=5.2Hz), 8.41 (1H, dd, J=4.4, 1.7Hz), 8.67 (1H, s), 8.84 (1H, s).
Elemental analysis: for C₂₇H₁₇F₂N₅O
Calculated: C, 68.02; H, 3.88; N, 15.87.
Found: C, 67.80; H, 3.88; N, 15.69.

### Example compound 41-2: N-{4-[6-chloro-2-(4-fluoro-3-methylphenyl)-8-methylimidazo[1,2-b]pyridazin-3-yl]-2-pyridyl}-4-fluorobenzamide

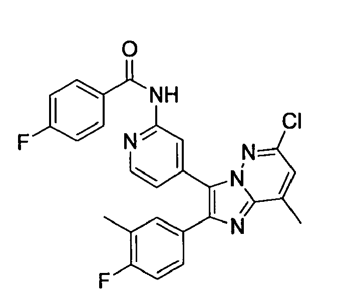

Melting point 235-236°C (ethanol)
¹H-NMR (CDCl₃) δ: 2.28 (3H, d, J=1.7Hz), 2.75 (3H, d, J=0.9Hz), 6.95 (1H, d, J=8.9Hz), 7.00 (1H, d, J=0.9Hz), 7.14-7.23 (3H, m), 7.32-7.39 (1H, m), 7.60 (1H, dd, J=7.4, 1.6Hz), 7.90-7.99 (2H, m), 8.34 (1H, dd, J=5.3, 0.6Hz), 8.64 (1H, s), 8.71 (1H, d, J=0.6Hz).
Elemental analysis: for C₂₆H₁₈F₂ClN₅O
Calculated: C, 63.74; H, 3.70; N, 14.30.
Found: C, 63.59; H, 3.75; N, 14.22.

### Example compound 41-3: N-{4-[7-tert-butyl-6-chloro-2-(4-fluoro-3-methylphenyl)imidazo[1,2-b]pyridazin-3-yl]-2-pyridyl}-4-fluorobenzamide

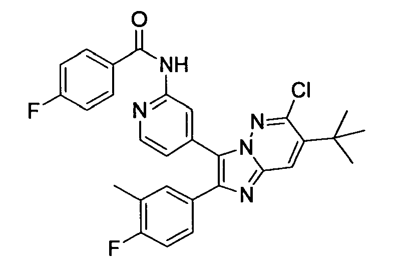

Melting point 181-182°C (ethanol)
MS (ESI+) : 532 (M+H).
¹H-NMR (CDCl₃) δ: 1.56 (9H, s), 2.29 (3H, d, J=1.9Hz), 6.98 (1H, t, J=9.1Hz), 7.15-7.24 (3H, m), 7.34-7.41 (1H, m), 7.61 (1H, dd, J=7.4, 2.2Hz), 7.93-8.00 (2H, m), 8.03 (1H, s), 8.34 (1H, d, J=5.2Hz), 8.67 (1H, s), 8.77 (1H, s).
Elemental analysis: for C₂₉H₂₄F₂ClN₅O·0.75H₂O
Calculated: C, 63.85; H, 4.71; N, 12.84.
Found: C, 63.99; H, 4.78; N, 12.87.

### Example compound 41-4: N-{4-[2-(4-fluoro-3-methylphenyl)-8-methylimidazo[1,2-b]pyridazin-3-yl]-2-pyridyl}-4-fluorobenzamide

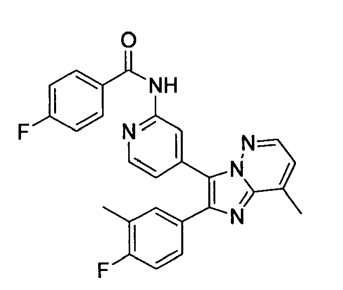

Melting point 243-244°C (ethanol)
¹H-NMR (CDCl₃) δ: 2.29 (3H, d, J=1.7Hz), 2.76 (3H, s), 6.96 (1H, dd, J=5.1, 1.1Hz), 6.98 (1H, d, J=9.2Hz), 7.14-7.23 (3H, m), 7.34-7.41 (1H, m), 7.62 (1H, dd, J=7.4, 2.0Hz), 7.91-8.00 (2H, m), 8.28 (1H, d, J=4.5Hz), 8.29 (1H, d, J=5.1Hz), 8.71 (1H, s), 8.84 (1H, s).
Elemental analysis: for C₂₆H₁₉F₂N₅O
Calculated: C, 68.56; H, 4.20; N, 15.38.
Found: C, 68.53; H, 4.15; N, 15.36.

### Example compound 41-5: N-{4-[7-tert-butyl-2-(4-fluoro-3-methylphenyl)imidazo[1,2-b]pyridazin-3-yl]-2-pyridyl}-4-fluorobenzamide

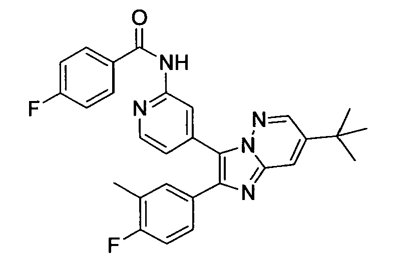

Melting point 201-202°C (ethanol)
¹H-NMR (CDCl₃) δ: 1.42 (9H, s), 2.29 (3H, d, J=1.9Hz), 6.95 (1H, t, J=9.1Hz), 7.17 (1H, dd, J=5.2, 1.4Hz), 7.18-7.24 (2H, m), 7.36-7.42 (1H, m), 7.62 (1H, dd, J=7.0, 1.8Hz), 7.90 (1H, d, J=2.3Hz), 7.95-8.00 (2H, m), 8.29 (1H, dd, J=5.2, 0.8Hz), 8.51 (1H, d, J=2.3Hz), 8.69 (1H, s), 8.89 (1H, dd, J=1.4, 0.8Hz).
Elemental analysis: for C₂₉H₂₅F₂N₅O
Calculated: C, 70.01; H, 5.06; N, 14.08.
Found: C, 70.00; H, 4.94; N, 14.15.

### (Example 42)

The compounds of Examples 42-1 - 42-37 below were synthesized in the same manner as in Example 19 using 4-[2-(4-fluoro-3-methylphenyl)imidazo[1,2-b]pyridazin-3-yl]pyridin-2-amine (Example compound 16-1) instead of 4-[2-(3-methylphenyl)imidazo[1,2-b]pyridazin-3-yl]pyridin-2-amine, and 3,3,3-trifluoropropionyl chloride, 2-methylpropionyl chloride, cyclopropanecarbonyl chloride, butyryl chloride, 3-(methylthio)propionyl chloride, 2-(methylthio)acetyl chloride, 4-chlorobenzoyl chloride, 4-fluoro-2-methoxybenzoyl chloride, 4-(methylthio)benzoyl chloride, 4-(dimethylamino)benzoyl chloride hydrochloride, 4-fluoro-3-methoxybenzoyl chloride, 3-fluoro-4-methoxybenzoyl chloride, 4-(trifluoromethyl)benzoyl chloride, 4-(trifluoromethoxy)benzoyl chloride, 4-chloro-2-methylbenzoyl chloride, 2-methylbenzoyl chloride, 3-methylbenzoyl chloride, 4-methoxy-2-methylbenzoyl chloride, 2-naphthoyl chloride, 1-naphthoyl chloride, 2-pyridinecarbonyl chloride hydrochloride, 2-chloronicotinoyl chloride, isonicotinoyl chloride hydrochloride, 6-chloronicotinoyl chloride, 2-chloroisonicotinoyl chloride, 6-trifluoromethylnicotinoyl chloride hydrochloride, 6-methylnicotinoyl chloride hydrochloride, 5-chloronicotinoyl chloride hydrochloride, 2-methylnicotinoyl chloride hydrochloride, 5-methylnicotinoyl chloride hydrochloride, 2-chloro-6-methylisonicotinoyl chloride, 3-thenoyl chloride, 3-furoyl chloride, 2-pyrazinecarbonyl chloride hydrochloride, 1,3-benzothiazole-6-carbonyl chloride, 6-quinolinecarbonyl chloride hydrochloride or 8-quinolinecarbonyl chloride hydrochloride, instead of benzoyl chloride.

### Example compound 42-1: 3,3,3-trifluoro-N-{4-[2-(4-fluoro-3-methylphenyl)imidazo[1,2-b]pyridazin-3-yl]pyridin-2-yl}propanamide

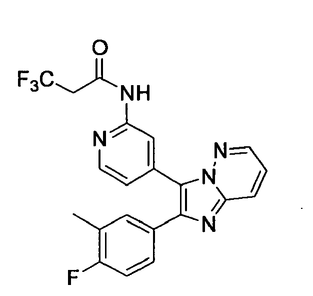

Melting point 220-221°C (ethanol)
MS (ESI+): 430 (M+H).
¹H-NMR (CDCl₃) δ: 2.28 (3H, d, J=1.7Hz), 3.32-3.35 (2H, m), 6.93-7.01 (1H, m), 7.16 (1H, dd, J=9.0, 1.7Hz), 7.24 (1H, dd, J=5.3, 1.5Hz), 7.32-7.38 (1H, m), 7.60 (1H, dd, J=7.4, 1.6Hz), 8.04 (1H, dd, J=9.0, 1.7Hz), 8.32 (1H, dd, J=5.3, 0.8Hz), 8.39 (1H, dd, J=4.3, 1.7Hz), 8.50 (1H, s), 8.66 (1H, s).
Elemental analysis: for C₂₁H₁₅F₄N₅O
Calculated: C, 58.74; H, 3.52; N, 16.31.
Found: C, 58.62; H, 3.44; N, 16.16.

### Example compound 42-2: N-{4-[2-(4-fluoro-3-methylphenyl)imidazo[1,2-b]pyridazin-3-yl]pyridin-2-yl}-2-methylpropanamide

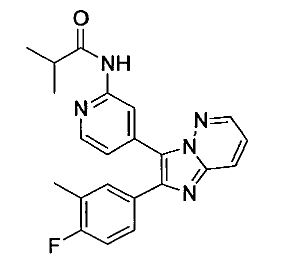

Melting point 214-215°C (ethanol)
MS (ESI+): 390 (M+H).
¹H-NMR (CDCl₃) δ: 1.28 (6H, d, J=6.9Hz), 2.28 (3H, d, J=1.9Hz), 2.52-2.65 (1H, m), 6.91 (1H, m), 7.10 (2H, m), 7.32-7.39 (1H, m), 7.58-7.63 (1H, m), 7.98-8.05 (2H, m), 8.29 (1H, dd, J=5.2, 0.8Hz), 8.39 (1H, dd, J=4.5, 1.8Hz), 8.70-8.74 (1H, m).
Elemental analysis: for C₂₂H₂₀FN₅O
Calculated: C, 67.85; H, 5.18; N, 17.98.
Found: C, 68.09; H, 5.31; N, 18.18.

### Example compound 42-3: N-{4-[2-(4-fluoro-3-methylphenyl)imidazo[1,2-b]pyridazin-3-yl]pyridin-2-yl}cyclopropanecarboxamide

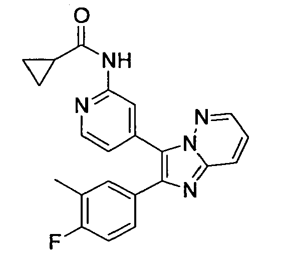

To a solution of 4-[2-(4-fluoro-3-methylphenyl)imidazo[1,2-b]pyridazin-3-yl]pyridin-2-amine (500 mg, 1.6 mmol) obtained in Example 16-1 and cyclopropanecarbonyl chloride (330 mg, 3.2 mmol) in tetrahydrofuran (10 mL) was added triethylamine (320 mg, 3.2 mmol), and the mixture was stirred at room temperature for 30 min. Saturated aqueous sodium hydrogen carbonate solution was added to the reaction mixture, and the obtained mixture was extracted with ethyl acetate. Ammonia-ethanol solution (2.0 M, 20 mL) was added to the extract, and the mixture was stirred at room temperature for 1 hr. The mixture was dried over anhydrous sodium sulfate, and the solvent was removed by evaporation under reduced pressure. The obtained residue was purified by silica gel chromatography (ethyl acetate: hexane = 1:9 - 6:4) and recrystallized from ethanol to give the title compound (yield 380 mg, 62%).
Melting point 217-218°C (ethanol)
MS (ESI+): 388 (M+H).
¹H-NMR (CDCl₃) δ: 0.86-0.94 (2H, m), 1.07-1.15 (2H, m), 1.53-1.60 (1H, m), 2.28 (3H, d, J=1.9Hz), 6.92-6.99 (1H, m), 7.08-7.16 (2H, m), 7.31-7.39 (1H, m), 7.58-7.64 (1H, m), 8.01 (1H, dd, J=9.1, 1.7Hz), 8.30 (1H, dd, J=5.2, 0.8Hz), 8.35 (1H, s), 8.38 (1H, dd, J=4.7, 1.7Hz), 8.66 (1H, s).
Elemental analysis: for C₂₂H₁₈FN₅O
Calculated: C, 68.21; H, 4.68; N, 18.08.
Found: C, 68.33; H, 4.86; N, 18.25.

### Example compound 42-4: N-{4-[2-(4-fluoro-3-methylphenyl)imidazo[1,2-b]pyridazin-3-yl]pyridin-2-yl}butanamide

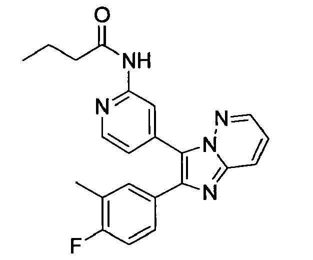

Melting point 181-183°C (ethanol)
MS (ESI+): 390 (M+H).
¹H-NMR (CDCl₃) δ: 1.01 (3H, t, J=7.4Hz), 1.71-1.83 (2H, m), 2.28 (3H, d, J=1.9Hz), 2.40 (2H, t, J=7.4Hz), 6.93-6.99 (1H, m), 7.11-7.17 (2H, m), 7.33-7.38 (1H, m), 7.59-7.63 (1H, m), 8.02 (1H, dd, J=9.3, 1.7Hz), 8.09 (1H, s), 8.30 (1H, dd, J=5.2, 0.8Hz), 8.39 (1H, dd, J=4.4, 1.7Hz), 8.69 (1H, s).
Elemental analysis: for C₂₂H₂₀FN₅O
Calculated: C, 67.85; H, 5.18; N, 17.98.
Found: C, 68.08; H, 5.30; N, 18.19.

### Example compound 42-5: N-{4-[2-(4-fluoro-3-methylphenyl)imidazo[1,2-b]pyridazin-3-yl]pyridin-2-yl}-3-(methylthio)propanamide

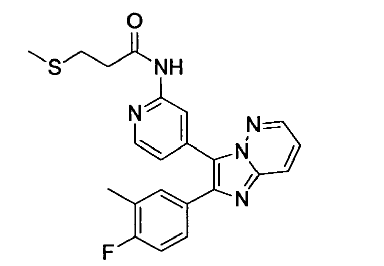

Melting point 158-159°C (ethanol)
¹H-NMR (CDCl₃) δ: 2.17 (3H, s), 2.28 (3H, d, J=1.6Hz), 2.72 (2H, t, J=6.9Hz), 2.89 (2H, t, J=6.9Hz), 6.93 (1H, t, J=8.9Hz), 7.14 (1H, dd, J=9.1, 4.4Hz), 7.18 (1H, dd, J=5.2, 1.6Hz), 7.32-7.39 (1H, m), 7.59-7.64 (1H, m), 8.02 (1H, dd, J=9.1, 1.7Hz), 8.25 (1H, s), 8.32 (1H, dd, J=5.2, 0.8Hz), 8.38 (1H, dd, J=4.4, 1.7Hz), 8.66 (1H, s).
Elemental analysis: for C₂₂H₂₀FN₅OS
Calculated: C, 62.69; H, 4.78; N, 16.62.
Found: C, 62.64; H, 4.62; N, 16.66.

### Example compound 42-6: N-{4-[2-(4-fluoro-3-methylphenyl)imidazo[1,2-b]pyridazin-3-yl]pyridin-2-yl}-2-(methylthio) acetamide

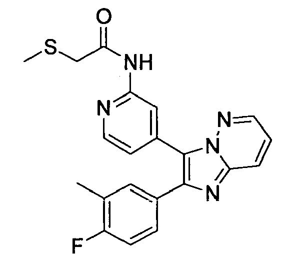

To a solution of methylthioacetic acid (220 mg, 2.0 mmol) in tetrahydrofuran (5 mL) were added oxalyl chloride (260 mg, 2.0 mmol) and a catalytic amount of dimethylformamide, and the mixture was stirred at room temperature for 2 hr. The solvent was removed by evaporation under reduced pressure. To a solution of the obtained residue in tetrahydrofuran (20 mL) were added 4-[2-(4-fluoro-3-methylphenyl)imidazo[1,2-b]pyridazin-3-yl]pyridin-2-amine (500 mg, 1.6 mmol) obtained in Example 16-1 and triethylamine (200 mg, 2.0 mmol), and the mixture was stirred at room temperature for 14 hr. A solution of methylthioacetyl chloride (2.0 mmol) in tetrahydrofuran (5 mL), which was additionally prepared, was added to the reaction mixture, and the mixture was stirred at room temperature for 14 hr. Saturated aqueous sodium hydrogen carbonate solution was added to the reaction mixture. The obtained mixture was extracted with ethyl acetate. The extract was dried over anhydrous sodium sulfate, and the solvent was removed by evaporation under reduced pressure. The obtained residue was purified by silica gel chromatography (ethyl acetate: hexane = 8:2 - ethyl acetate) and recrystallized from ethanol to give the title compound (yield 210 mg, 33%).
Melting point 155-156°C (ethanol)
¹H-NMR (CDCl₃) δ: 2.22 (3H, s), 2.28 (3H, d, J=1.9Hz), 3.37 (2H, s), 6.96 (1H, t, J=9.1Hz), 7.15 (1H, dd, J=9.1, 4.4Hz), 7.21 (1H, dd, J=5.2, 1.7Hz), 7.33-7.39 (1H, m), 7.60-7.65 (1H, m), 8.03 (1H, dd, J=9.1, 1.7Hz), 8.36 (1H, dd, J=5.2, 0.8Hz), 8.38 (1H, dd, J=4.4, 1.7Hz), 8.67 (1H, s), 9.26 (1H, s).
Elemental analysis: for C₂₁H₁₈FN₅OS
Calculated: C, 61.90; H, 4.45; N, 17.19.
Found: C, 61.70; H, 4.42; N, 17.07.

### Example compound 42-7: 4-chloro-N-{4-[2-(4-fluoro-3-methylphenyl)imidazo[1,2-b]pyridazin-3-yl]pyridin-2-yl}benzamide

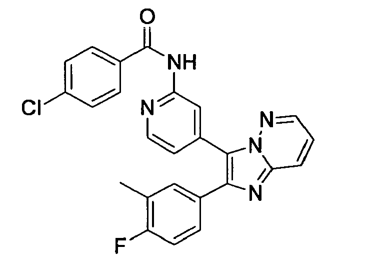

Melting point 208-209°C (ethanol-tetrahydrofuran) ¹H-NMR (CDCl₃) δ: 2.29 (3H, d, J=2.1Hz), 6.95-7.01 (1H, m), 7.13-7.21 (2H, m), 7.35-7.40 (1H, m), 7.46-7.51 (2H, m), 7.63 (1H, dd, J=7.5, 2.1Hz), 7.85-7.90 (2H, m), 8.04 (1H, dd, J=9.0, 1.4Hz), 8.32 (1H, dd, J=5.3, 0.8Hz), 8.40 (1H, dd, J=4.1, 1.4Hz), 8.71 (1H, s), 8.84 (1H, dd, J=1.4, 0.8Hz).
Elemental analysis: for C₂₅H₁₇ClFN₅O
Calculated: C, 65.58; H, 3.74; N, 15.29.
Found: C, 65.55; H, 3.73; N, 15.29.

### Example compound 42-8: 4-fluoro-N-{4-[2-(4-fluoro-3-methylphenyl)imidazo[1,2-b]pyridazin-3-yl]pyridin-2-yl}-2-methoxybenzamide

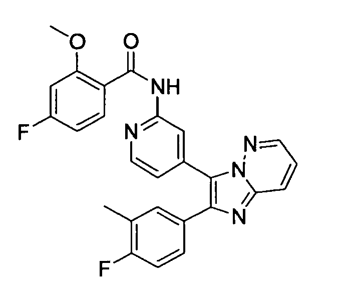

Melting point 251-252°C (ethanol-tetrahydrofuran-water)
MS (ESI+): 472 (M+H).
¹H-NMR (CDCl₃) δ: 2.28 (3H, d, J=1.9Hz), 4.11 (3H, s), 6.75-6.89 (2H, m), 6.93-7.01 (1H, m), 7.12-7.22 (2H, m), 7.36-7.43 (1H, m), 7.62-7.66 (1H, m), 8.03 (1H, dd, J=9.3, 1.7Hz), 8.27 (1H, dd, J=8.8, 7.1Hz) 8.35-8.47 (2H, m), 8.86-8.90 (1H, m), 10.28 (1H, s).
Elemental analysis: for C₂₆H₁₉F₂N₅O₂
Calculated: C, 66.24; H, 4.06; N, 14.85.
Found: C, 66.20; H, 4.04; N, 14.87.

### Example compound 42-9: N-{4-[2-(4-fluoro-3-methylphenyl)imidazo[1,2-b]pyridazin-3-yl]pyridin-2-yl}-4-(methylthio)benzamide

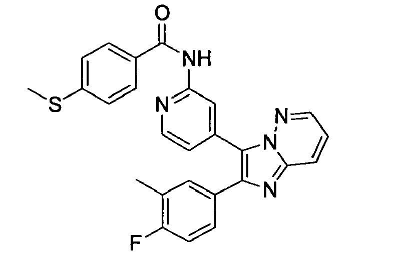

Melting point 191-192°C (ethanol-tetrahydrofuran)
MS (ESI+) : 470 (M+H).
¹H-NMR (CDCl₃) δ: 2.29 (3H, d, J=1.7Hz), 2.54 (3H, s), 6.94-7.01 (1H, m), 7.13-7.18 (2H, m), 7.30-7.35 (2H, m), 7.35-7.41 (1H, m), 7.62-7.66 (1H, m), 7.83-7.87 (2H, m), 8.03 (1H, dd, J=9.4, 1.7Hz), 8.34 (1H, dd, J=5.2, 1.7Hz), 8.40 (1H, dd, J=4.4, 1.7Hz), 8.65 (1H, s), 8.85-8.87 (1H, m).
Elemental analysis: for C₂₆H₂₀FN₅OS·0.3H₂O
Calculated: C, 65.75; H, 4.37; N, 14.75.
Found: C, 65.87; H, 4.40; N, 14.71.

### Example compound 42-10: 4-(dimethylamino)-N-{4-[2-(4-fluoro-3-methylphenyl)imidazo[1,2-b]pyridazin-3-yl]pyridin-2-yl}benzamide

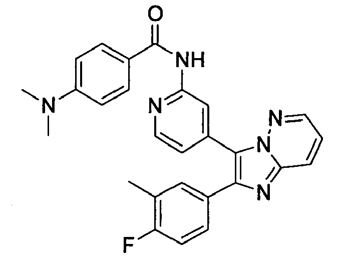

Melting point 245-246°C (ethyl acetate-hexane)
MS (ESI+): 467 (M+H).
¹H-NMR (CDCl₃) δ: 2.28 (3H, d, J=1.7Hz), 3.06 (6H, s), 6.69-6.75 (2H, m), 6.94-7.00 (1H, m), 7.09-7.16 (2H, m), 7.36-7.42 (1H, m), 7.64 (1H, dd, J=7.4, 1.6Hz), 7.81-7.87 (2H, m), 8.02 (1H, dd, J=9.2, 1.7Hz), 8.32 (1H, dd, J=5.2, 0.6Hz), 8.40 (1H, dd, J=4.3, 1.7Hz), 8.58 (1H, s), 8.85-8.88 (1H, m).
Elemental analysis: for C₂₇H₂₃FN₆O
Calculated: C, 69.51; H, 4.97; N, 18.01.
Found: C, 69.34; H, 4.91; N, 17.92.

### Example compound 42-11: 4-fluoro-N-{4-[2-(4-fluoro-3-methylphenyl)imidazo[1,2-b]pyridazin-3-yl]pyridin-2-yl}-3-methoxybenzamide

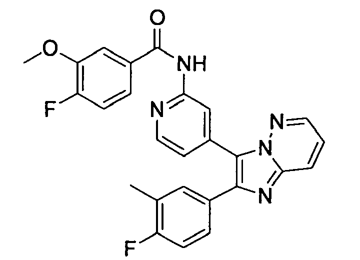

Melting point 217-218°C (ethanol-tetrahydrofuran)
MS (ESI+): 472 (M+H).
¹H-NMR (CDCl₃) δ: 2.29 (3H, d, J=1.7Hz), 3.96 (3H, s), 6.93-7.02 (1H, m), 7.12-7.23 (3H, m), 7.35-7.42 (1H, m), 7.43-7.49 (1H, m), 7.59-7.67 (2H, m), 8.04 (1H, dd, 9.1, 1.6Hz), 8.31 (1H, d, 5.3Hz), 8.41 (1H, dd, J=4.4, 1.6Hz), 8.83 (1H, s), 8.86 (1H, s).
Elemental analysis: for C₂₆H₁₉F₂N₅O₂·0.1H₂O
Calculated: C, 65.98; H, 4.09; N, 14.80.
Found: C, 65.84; H, 4.07; N, 14.71.

### Example compound 42-12: 3-fluoro-N-{4-[2-(4-fluoro-3-methylphenyl)imidazo[1,2-b]pyridazin-3-yl]pyridin-2-yl}-4-methoxybenzamide

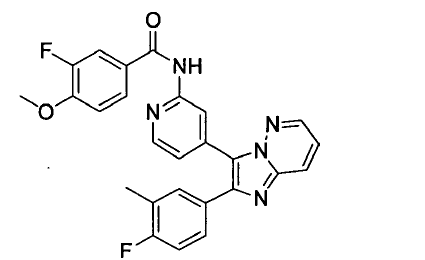

Melting point 202-203°C (ethanol-tetrahydrofuran-water)
MS (ESI+): 472 (M+H).
¹H-NMR (CDCl₃) δ: 2.29 (3H, d, J=1.1Hz), 3.97 (3H, s), 6.92-7.08 (2H, m), 7.11-7.21 (2H, m), 7.34-7.43 (1H, m), 7.61-7.76 (3H, m), 8.04 (1H, dd, J=9.1, 1.6Hz), 8.33 (1H, d, J=5.1Hz), 8.40 (1H, dd, J=4.4, 1.6Hz), 8.64 (1H, s), 8.83 (1H, s).
Elemental analysis: for C₂₆H₁₉F₂N₅O₂·0.3H₂O
Calculated: C, 65.49; H, 4.14; N, 14.69.
Found: C, 65.57; H, 4.11; N, 14.54.

### Example compound 42-13: N-{4-[2-(4-fluoro-3-methylphenyl)imidazo[1,2-b]pyridazin-3-yl]pyridin-2-yl}-4-(trifluoromethyl)benzamide

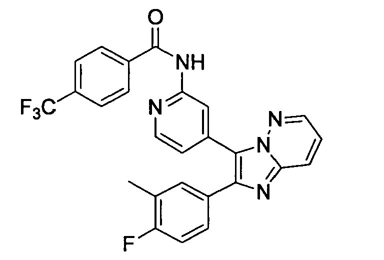

Melting point 224-225°C (ethanol-tetrahydrofuran)
MS (ESI+): 492 (M+H).
¹H-NMR (CDCl₃) δ: 2.29 (3H, d, J=1.7Hz), 6.95-7.02 (1H, m), 7.17 (1H, dd, J=9.1, 4.3Hz), 7.23 (1H, dd, J=5.3, 1.5Hz), 7.35-7.41 (1H, m), 7.64 (1H, dd, J=7.4, 1.7Hz), 7.79 (2H, d, J=8.3Hz), 8.02-8.08 (3H, m), 8.35 (1H, d, J=5.3Hz), 8.42 (1H, dd, J=4.3, 1.7Hz), 8.70 (1H, s), 8.86 (1H, s).
Elemental analysis: for C₂₆H₁₇F₄N₅O
Calculated: C, 63.54; H, 3.49; N, 14.25.
Found: C, 63.52; H, 3.42; N, 14.23.

### Example compound 42-14: N-{4-[2-(4-fluoro-3-methylphenyl)imidazo[1,2-b]pyridazin-3-yl]pyridin-2-yl}-4-(trifluoromethoxy)benzamide

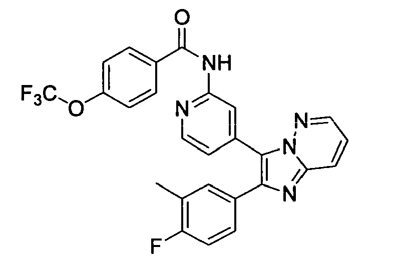

Melting point 225-226°C (ethanol-tetrahydrofuran)
MS (ESI+): 508 (M+H).
¹H-NMR (CDCl₃) δ: 2.29 (3H, d, J=1.5Hz), 6.92-7.04 (1H, m), 7.16 (1H, dd, J=9.2, 4.5Hz), 7.20 (1H, dd, J=5.2, 1.5Hz), 7.31-7.42 (3H, m), 7.64 (1H, dd, J=7.4, 1.6Hz), 7.95-8.02 (2H, m), 8.04 (1H, dd, J=9.2, 1.7Hz), 8.30 (1H, dd, J=5.2, 0.5Hz), 8.41 (1H, dd, J=4.5, 1.7Hz), 8.79 (1H, s), 8.84-8.86 (1H, m).
Elemental analysis: for C₂₆H₁₇F₄N₅O₂
Calculated: C, 61.54; H, 3.38; N, 13.80.
Found: C, 61.49; H, 3.29; N, 13.77.

### Example compound 42-15: 4-chloro-N-{4-[2-(4-fluoro-3-methylphenyl)imidazo[1,2-b]pyridazin-3-yl]pyridin-2-yl}-2-methylbenzamide

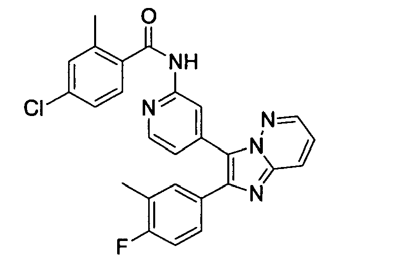

Melting point 196-197°C (ethanol)
MS (ESI+): 472 (M+H).
¹H-NMR (CDCl₃) δ: 2.30 (3H, d, J=1.7Hz), 2.51 (3H, s), 6.95-7.02 (1H, m), 7.16 (1H, dd, J=9.2, 4.5Hz), 7.21 (1H, dd, J=5.3, 1.4Hz), 7.24-7.30 (2H, m), 7.35-7.41 (1H, m), 7.51 (1H, d, J=8.1Hz), 7.64 (1H, dd, J=7.5, 1.7Hz), 8.04 (1H, dd, J=9.2, 1.7Hz), 8.24 (1H, dd, J=5.3, 0.6Hz), 8.41 (1H, dd, J=4.5, 1.7Hz), 8.51 (1H, s), 8.80 (1H, s).
Elemental analysis: for C₂₆H₁₉ClFN₅O·H₂O
Calculated: C, 63.74; H, 4.32; N, 14.29.
Found: C, 64.06; H, 4.56; N, 14.43.

### Example compound 42-16: N-{4-[2-(4-fluoro-3-methylphenyl) imidazo[1,2-b]pyridazin-3-yl]pyridin-2-yl}-2-methylbenzamide

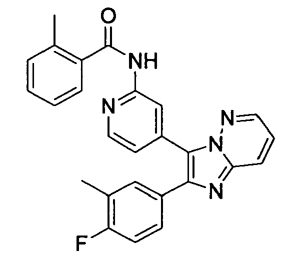

Melting point 265-266°C (ethanol-tetrahydrofuran)
MS (ESI+): 438 (M+H).
¹H-NMR (CDCl₃) δ: 2.30 (3H, d, J=1.9Hz), 2.53 (3H, s), 6.95-7.02 (1H, m), 7.15 (1H, dd, J=9.1, 4.4Hz), 7.19 (1H, dd, J=5.2, 1.7Hz), 7.25-7.30 (2H, m), 7.36-7.42 (2H, m), 7.55-7.59 (1H, m), 7.63-7.66 (1H, m), 8.04 (1H, dd, J=9 .1, 1. 7Hz), 8.27 (1H, dd, J=5.2, 0.8Hz), 8.41 (1H, dd, J=4.4, 1.7Hz), 8.43 (1H, s), 8.82-8.86 (1H, m).
Elemental analysis: for C₂₆H₂₀FN₅O Calculated: C, 71.38; H, 4.61; N, 16.01. Found: C, 71.23; H, 4.67; N, 15.89.

### Example compound 42-17: N-{4-[2-(4-fluoro-3-methylphenyl)imidazo[1,2-b]pyridazin-3-yl]-2-pyridyl}-3-methylbenzamide

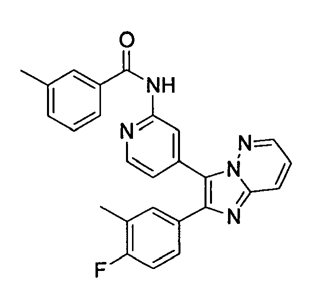

Melting point 203-204°C (ethanol)
¹H-NMR (CDCl₃) δ: 2.29 (3H, d, J=1.7Hz), 2.44 (3H, s), 6.92-7.02 (1H, m), 7.15 (1H, dd, J=9.3, 4.4Hz), 7.18 (1H, dd, J=5.3, 1.5Hz), 7.35-7.43 (3H, m), 7.64 (1H, dd, J=7.4, 1.7Hz), 7.68-7.74 (1H, m), 7.75 (1H, s), 8.03 (1H, dd, J=9.3, 1.6Hz), 8.33 (1H, dd, J=5.3, 0.6Hz), 8.40 (1H, dd, J=4.4, 1.6Hz), 8.70 (1H, s), 8.89 (1H, dd, J=1.5, 0.6Hz).
Elemental analysis: for C₂₆H₂₀FN₅O Calculated: C, 71.38; H, 4.61; N, 16.01. Found: C, 71.24; H, 4.55; N, 16.02.

### Example compound 42-18: N-{4-[2-(4-fluoro-3-methylphenyl)imidazo[1,2-b]pyridazin-3-yl]pyridin-2-yl}-4-methoxy-2-methylbenzamide

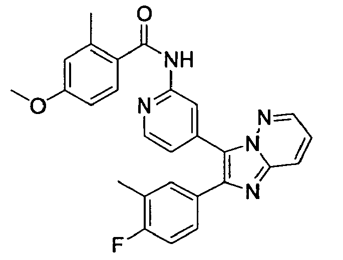

Melting point 200-202°C (ethanol)
MS (ESI+) : 468 (M+H).
¹H-NMR (CDCl₃) δ: 2.29 (3H, d, J=1.7Hz), 2.54 (3H, s), 3.84 (3H, s), 6.76-6.81 (2H, m), 6.94-7.01 (1H, m), 7.12-7.18 (2H, m), 7.36-7.42 (1H, m), 7.54-7.59 (1H, m), 7.65 (1H, dd, J=7.4, 1.7Hz), 8.03 (1H, dd, J=9.1, 1.6Hz), 8.26 (1H, dd, J=5.2, 0.7Hz), 8.40 (1H, dd, J=4.4, 1.6Hz), 8.45 (1H, s), 8.82 (1H, s).
Elemental analysis: for C₂₇H₂₂FN₅O₂·1.2H₂O
Calculated: C, 66.30; H, 5.03; N, 14.32.
Found: C, 66.29; H, 5.14; N, 14.34.

### Example compound 42-19: N-{4-[2-(4-fluoro-3-methylphenyl)imidazo[1,2-b]pyridazin-3-yl]pyridin-2-yl}-2-naphthamide

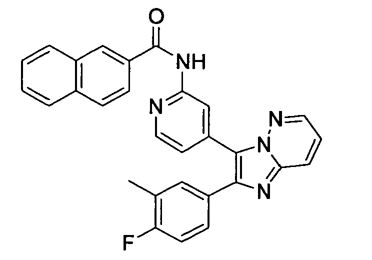

Melting point 187-188°C (ethanol)
¹H-NMR (CDCl₃) δ: 2.30 (3H, d, J=1.7Hz), 6.99 (1H, t, J=8.9Hz), 7.16 (1H, dd, J=9.1, 4.4Hz), 7.21 (1H, dd, J=5.2, 1.4Hz), 7.37-7.44 (1H, m), 7.55-7.68 (3H, m), 7.90-8.01 (4H, m), 8.05 (1H, dd, J=9.1, 1.7Hz), 8.38 (1H, dd, J=5.2, 0.6Hz), 8.42 (1H, dd, J=4.4, 1.7Hz), 8.47 (1H, s), 8.82 (1H, s), 8.93 (1H, dd, J=1.4, 0.6Hz).
Elemental analysis: for C₂₉H₂₀FN₅O
Calculated: C, 73.56; H, 4.26; N, 14.79.
Found: C, 73.52; H, 4.18; N, 14.70.

### Example compound 42-20: N-{4-[2-(4-fluoro-3-methylphenyl)imidazo[1,2-b]pyridazin-3-yl]pyridin-2-yl}-1-naphthamide

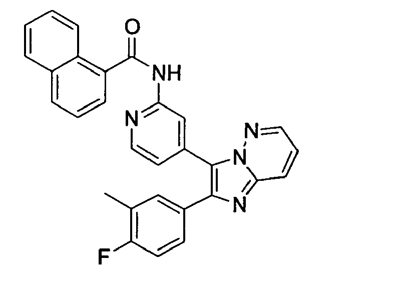

Melting point 252-253°C (ethanol-tetrahydrofuran)
¹H-NMR (CDCl₃) δ: 2.32 (3H, d, J=1.6Hz), 7.01 (1H, t, J=9.1Hz), 7.17 (1H, dd, J=9.1, 4.4Hz), 7.22 (1H, dd, J=5.2, 1.7Hz), 7.38-7.46 (1H, m), 7.51-7.70 (4H, m), 7.84 (1H, dd, J=7.1, 1.1Hz), 7.90-7.95 (1H, m), 8.01 (1H, d, J=8.2Hz), 8.05 (1H, dd, J=9.1, 1.7Hz), 8.29 (1H, dd, J=5.2, 0.8Hz), 8.41-8.47 (2H, m), 8.68 (1H, s), 8.97 (1H, s).
Elemental analysis: for C₂₉H₂₀FN₅O
Calculated: C, 73.56; H, 4.26; N, 14.79.
Found: C, 73.34; H, 4.41; N, 14.62.

### Example compound 42-21: N-{4-[2-(4-fluoro-3-methylphenyl)imidazo[1,2-b]pyridazin-3-yl]pyridin-2-yl}pyridine-2-carboxamide

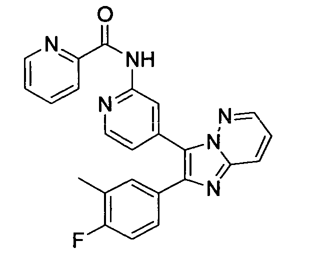

Melting point 234-235°C (ethanol-tetrahydrofuran-water)
¹H-NMR (CDCl₃) δ: 2.28 (3H, d, J=1.5Hz), 6.94-6.70 (1H, m), 7.15 (1H, dd, J=9.2, 4.3Hz), 7.21 (1H, dd, J=5.2, 1.4Hz), 7.36-7.43 (1H, m), 7.49-7.53 (1H, m), 7.65 (1H, dd, J=7.4, 1.6Hz), 7.89-7.93 (1H, m), 8.04 (1H, dd, J=9.2, 1.7Hz), 8.28 (1H, d, J=7.9Hz), 8.38-8.45 (2H, m), 8.66 (1H, d, J=4.1Hz), 8.88 (1H, s), 10.67 (1H, s).
Elemental analysis: for C₂₄H₁₇FN₆O
Calculated: C, 67.92; H, 4.04; N, 19.80.
Found: C, 67.74; H, 3.98; N, 19.77.

### Example compound 42-22: 2-chloro-N-{4-[2-(4-fluoro-3-methylphenyl)imidazo[1,2-b]pyridazin-3-yl]pyridin-2-yl}nicotinamide

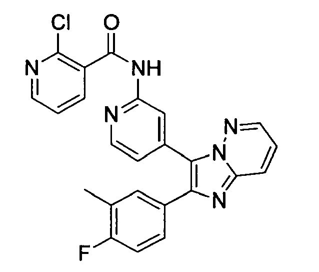

To a solution of 4-[2-(4-fluoro-3-methylphenyl)imidazo[1,2-b]pyridazin-3-yl]pyridin-2-amine (400 mg, 1.3 mmol) obtained in Example 16-1 in tetrahydrofuran (12 mL) were added triethylamine (150 mg, 1.5 mmol) and 2-chloronicotinoyl chloride (260 mg, 1.5 mmol), and the mixture was stirred at room temperature for 5 hr. 5% Aqueous sodium hydrogen carbonate solution was added to the reaction mixture, and the obtained mixture was extracted with ethyl acetate. Ammonia-ethanol solution (2.0 M, 12 mL) was added to the extract, and the mixture was stirred at room temperature overnight. The mixture was dried over anhydrous sodium sulfate, and the solvent was removed by evaporation under reduced pressure. The obtained residue was purified by basic silica gel chromatography (ethyl acetate: hexane = 3:7 - 8:2) and recrystallized from ethyl acetate-hexane to give the title compound (yield 420 mg, 73%).
MS (ESI+): 459 (M+H).
¹H-NMR (CDCl₃) δ: 2.30 (3H, d, J=1.1Hz), 6.96-7.02 (1H, m), 7.17 (1H, dd, J=9.2, 4.5Hz), 7.23-7.31 (1H, m), 7.36-7.43 (2H, m), 7.64 (1H, dd, J=7.4, 1.6Hz), 8.05 (1H, dd, J=9.2, 1.5Hz), 8.17 (1H, dd, J=7.6, 2.0Hz), 8.30 (1H, d, J=5.3Hz), 8.42 (1H, dd, J=4.5, 1.5Hz), 8.54 (1H, dd, J=4.8, 2.0Hz), 8.81 (1H, s), 9.09 (1H, s).

### Example compound 42-23: N-{4-[2-(4-fluoro-3-methylphenyl)imidazo[1,2-b]pyridazin-3-yl]pyridin-2-yl}isonicotinamide

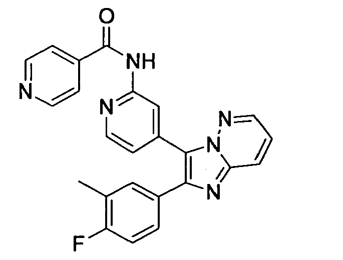

To a solution of 4-[2-(4-fluoro-3-methylphenyl)imidazo[1,2-b]pyridazin-3-yl]pyridin-2-amine (1.0 g, 3.1 mmol) obtained in Example 16-1 in tetrahydrofuran (20 mL) were added triethylamine (1.9 g, 19 mmol) and isonicotinoyl chloride hydrochloride (1.7 g, 9.5 mmol), and the mixture was stirred at room temperature for 3 hr. 5% Aqueous sodium hydrogen carbonate solution was added to the reaction mixture, and the obtained mixture was extracted with ethyl acetate/tetrahydrofuran. To the extract was added ammonia-ethanol solution (2.0 M, 20 mL), and the mixture was stirred at room temperature overnight. The mixture was dried over anhydrous sodium sulfate, and the solvent was removed by evaporation under reduced pressure. The obtained residue was purified by basic silica gel chromatography (ethyl acetate: hexane = 3:7 - 8:2) and recrystallized from ethanol-tetrahydrofuran to give the title compound (yield 720 mg, 54%).
Melting point 219-220°C (ethanol-tetrahydrofuran)
MS (ESI+): 425 (M+H).
¹H-NMR (CDCl₃) δ: 2.29 (3H, d, J=1.7Hz), 6.95-7.01 (1H, m), 7.17 (1H, dd, J=9.1, 4.4Hz), 7.23-7.27 (1H, m), 7.34-7.41 (1H, m), 7.63 (1H, dd, J=7.5, 1.9Hz), 7.75-7.79 (2H, m), 8.05 (1H, dd, J=9.1, 1.6Hz), 8.34 (1H, d, J=5.3Hz), 8.41 (1H, dd, J=4.4, 1.6Hz), 8.80-8.86 (4H, m).
Elemental analysis: for C₂₄H₁₇FN₆O
Calculated: C, 67.92; H, 4.04; N, 19.80.
Found: C, 67.56; H, 4.06; N, 19.76.

### Example compound 42-24: 6-chloro-N-{4-[2-(4-fluoro-3-methylphenyl)imidazo[1,2-b]pyridazin-3-yl]pyridin-2-yl}nicotinamide

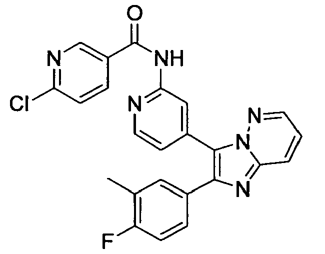

Melting point 225-226°C (ethanol-tetrahydrofuran-water)
¹H-NMR (CDCl₃) δ: 2.29 (3H, d, J=1.5Hz), 6.95-7.01 (1H, m), 7.17 (1H, dd, J=9.2, 4.3Hz), 7.23-7.27 (1H, m), 7.34-7.40 (1H, m), 7.49 (1H, d, J=8.3Hz), 7.63 (1H, dd, J=7.4, 1.9Hz), 8.05 (1H, dd, J=9.2, 1.7Hz), 8.20 (1H, dd, J=8.3, 2.5Hz), 8.34 (1H, d, J=5.3Hz), 8.41 (1H, dd, J=4.3, 1.7Hz), 8.73 (1H, s), 8.81 (1H, s), 8.95 (1H, d, J=2.5Hz).
Elemental analysis: for C₂₄H₁₆ClFN₆O
Calculated: C, 62.82; H, 3.51; N, 18.31.
Found: C, 62.79; H, 3.53; N, 18.35.

### Example compound 42-25: 2-chloro-N-{4-[2-(4-fluoro-3-methylphenyl)imidazo[1,2-b]pyridazin-3-yl]pyridin-2-yl}isonicotinamide

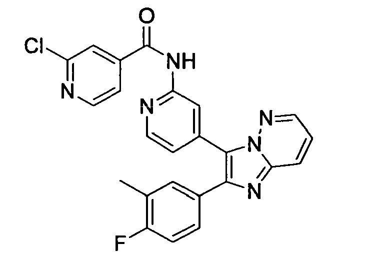

Melting point 224-225°C (ethanol-tetrahydrofuran-water)
MS (ESI+): 459 (M+H).
¹H-NMR (CDCl₃) δ: 2.30 (3H, s), 6.93-7.06 (1H, m), 7.18 (1H, dd, J=9.1, 4.4Hz), 7.24-7.44 (2H, m), 7.59-7.71 (2H, m), 7.82 (1H, s), 8.01-8.09 (1H, m), 8.32 (1H, d, J=5.1Hz), 8.39-8.45 (1H, m), 8.59 (1H, d, J=5.1Hz), 8.81 (1H, s), 8.87 (1H, s).
Elemental analysis: for C₂₄H₁₆ClFN₆O·0.3H₂O
Calculated: C, 62.09; H, 3.60; N, 18.10.
Found: C, 62.20; H, 3.59; N, 17.88.

### Example compound 42-26: N-{4-[2-(4-fluoro-3-methylphenyl)imidazo[1,2-b]pyridazin-3-yl]pyridin-2-yl}-6-(trifluoromethyl)nicotinamide

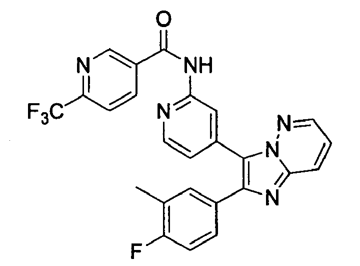

Melting point 225-226°C (ethanol-tetrahydrofuran)
MS (ESI+): 493 (M+H).
¹H-NMR (CDCl₃) δ: 2.30 (3H, d, J=1.5Hz), 6.95-7.03 (1H, m), 7.18 (1H, dd, J=9.1, 4.4Hz), 7.25-7.34 (1H, m), 7.35-7.41 (1H, m), 7.64 (1H, dd, J=7.4, 1.6Hz), 7.86 (1H, d, J=8.1Hz), 8.06 (1H, dd, J=9.1, 1.6Hz), 8.36 (1H, d, J=5.1Hz), 8.40-8.48 (2H, m), 8.74 (1H, s), 8.83 (1H, s), 9.26 (1H, d, J=1.5Hz).
Elemental analysis: for C₂₅H₁₆F₄N₆O
Calculated: C, 60.98; H, 3.28; N, 17.07.
Found: C, 60.89; H, 3.13; N, 16.97.

### Example compound 42-27: N-{4-[2-(4-fluoro-3-methylphenyl)imidazo[1,2-b]pyridazin-3-yl]pyridin-2-yl}-6-methylnicotinamide

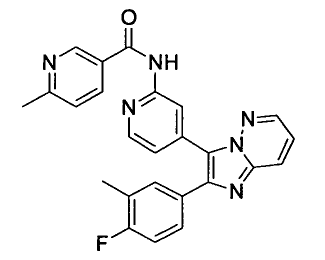

To a suspension of 6-methylnicotinic acid (230 mg, 1.7 mmol) in tetrahydrofuran (5 mL) were added oxalyl chloride (210 mg, 1.7 mmol) and a catalytic amount of dimethylformamide, and the mixture was stirred at room temperature for 30 min. The solvent was removed by evaporation under reduced pressure. A suspension of the obtained residue in tetrahydrofuran (1 mL) was added to a solution of 4-[2-(4-fluoro-3-methylphenyl) imidazo [1,2-b]pyridazin-3-yl]pyridin-2-amine (150 mg, 0.47 mmol) obtained in Example 16-1 and triethylamine (170 mg, 1.7 mmol) in tetrahydrofuran (3 mL), and the mixture was stirred at room temperature for 3 hr. 5% Aqueous sodium hydrogen carbonate solution was added to the reaction mixture, and the mixture was extracted with ethyl acetate/tetrahydrofuran.

Ammonia-ethanol solution (2.0 M, 3 mL) was added to the extract, and the mixture was stirred at room temperature overnight. The mixture was dried over anhydrous sodium sulfate, and the solvent was removed by evaporation under reduced pressure. The obtained residue was purified by basic silica gel chromatography (ethyl acetate: hexane = 3:7 - 8:2) and recrystallized from ethanol-tetrahydrofuran to give the title compound (yield 25 mg, 12%). Melting point 214-215°C (ethanol-tetrahydrofuran)
MS (ESI+): 439 (M+H).
¹H-NMR (CDCl₃) δ: 2.29 (3H, d, J=1.3Hz), 2.66 (3H, s), 6.94-7.02 (1H, m), 7.16 (1H, dd, J=9.1, 4.4Hz), 7.22 (1H, dd, J=5.1, 1.4Hz), 7.31 (1H, d, J=8.1Hz), 7.35-7.41 (1H, m), 7.61-7.66 (1H, m), 8.04 (1H, dd, J=9.1, 1.6Hz), 8.13 (1H, dd, J=8.1, 2.1Hz), 8.36 (1H, d, J=5.1Hz), 8.41 (1H, dd, J=4.4, 1.6Hz), 8.64 (1H, s), 8.85 (1H, s), 9.06 (1H, d, J=2.1Hz).
Elemental analysis: for C₂₅H₁₉FN₆O·0.3H₂O
Calculated: C, 67.65; H, 4.45; N, 18.93.
Found: C, 67.50; H, 4.36; N, 18.88.

### Example compound 42-28: 5-chloro-N-{4-[2-(4-fluoro-3-methylphenyl)imidazo[1,2-b]pyridazin-3-yl]pyridin-2-yl}nicotinamide

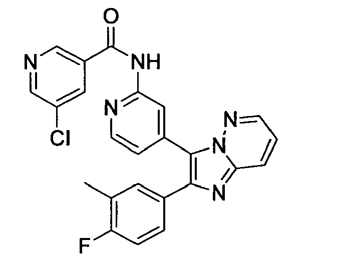

Melting point 252-254°C (ethanol-tetrahydrofuran)
MS (ESI+): 459 (M+H).
¹H-NMR (DMSO-d₆) δ: 2.25 (3H, d, J=1.1Hz), 7.11-7.22 (1H, m), 7.33 (1H, dd, J=5.3, 1.3Hz), 7.35-7.46 (2H, m), 7.69 (1H, dd, J=7.6, 1.9Hz), 8.26 (1H, dd, J=9.2, 1.5Hz), 8.45-8.49 (1H, m), 8.50-8.51 (1H, m), 8.53 (1H, dd, J=5.3Hz), 8.59 (1H, dd, J=4.5, 1.5Hz), 8.83 (1H, d, J=2.3Hz), 9.05 (1H, d, J=1.9Hz), 11.35 (1H, s).
Elemental analysis: for C₂₄H₁₆ClFN₆O
Calculated: C, 62.82; H, 3.51; N, 18.31.
Found: C, 62.81; H, 3.52; N, 18.11.

### Example compound 42-29: N-{4-[2-(4-fluoro-3-methylphenyl)imidazo[1,2-b]pyridazin-3-yl]pyridin-2-yl}-2-methylnicotinamide

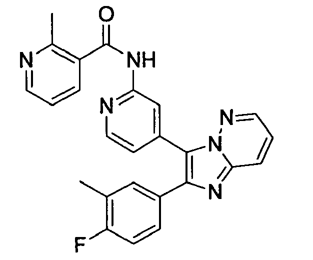

Melting point 258-259°C (ethanol)
MS (ESI+): 439 (M+H).
¹H-NMR (CDCl₃) δ: 2.30 (3H, d, J=1.7Hz), 2.77 (3H, s), 6.95-7.03 (1H, m), 7.17 (1H, dd, J=9.1, 4.4Hz), 7.21-7.26 (2H, m), 7.35-7.41 (1H, m), 7.63-7.65 (1H, m), 7.85 (1H, dd, J=7.7, 1.7Hz), 8.05 (1H, dd, J=9.1, 1.6Hz), 8.17 (1H, dd, J=5.3, 0.8Hz), 8.42 (1H, dd, J=4.4, 1.6Hz), 8.63 (1H, dd, J=4.9, 1.7Hz), 8.72 (1H, s), 8.83 (1H, s).
Elemental analysis: for C₂₅H₁₉FN₆O
Calculated: C, 68.48; H, 4.37; N, 19.17.
Found: C, 68.12; H, 4.59; N, 18.96.

### Example compound 42-30: N-{4-[2-(4-fluoro-3-methylphenyl)imidazo[1,2-b]pyridazin-3-yl]pyridin-2-yl}-5-methylnicotinamide

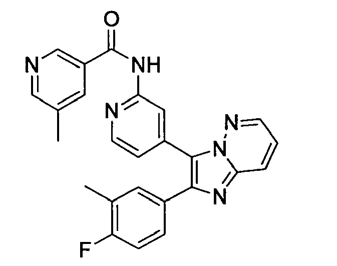

Melting point 238-240°C (ethanol-tetrahydrofuran)
MS (ESI+): 439 (M+H).
¹H-NMR (CDCl₃) δ: 2.29 (3H, d, J=1.7Hz), 2.45 (3H, s), 6.95-7.02 (1H, m), 7.16 (1H, dd, J=9.1, 4.4Hz), 7.23 (1H, dd, J=5.3, 1.5Hz), 7.35-7.41 (1H, m), 7.64 (1H, dd, J=7.4, 1.7Hz), 8.02-8.07 (2H, m), 8.36 (1H, d, J=5.3Hz), 8.41 (1H, dd, J=4.4, 1.6Hz), 8.62-8.65 (2H, m), 8.84 (1H, s), 8.96-8.98 (1H, m).

### Example compound 42-31: 2-chloro-N-{4-[2-(4-fluoro-3-methylphenyl)imidazo[1,2-b]pyridazin-3-yl]pyridin-2-yl}-6-methylisonicotinamide

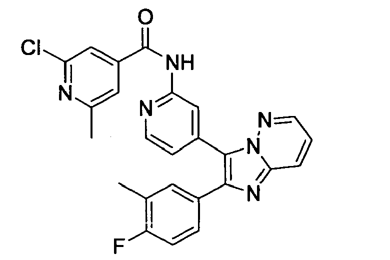

A mixture of 2-chloro-6-methylisonicotinic acid (670 mg, 3.9 mmol) and phosphorus oxychloride (15 mL) was stirred at 50°C for 1 hr, and phosphorus oxychloride was removed by evaporation under reduced pressure. A suspension of the obtained residue in tetrahydrofuran (3 mL) was added to a solution of 4-[2-(4-fluoro-3-methylphenyl)imidazo[1,2-b]pyridazin-3-yl]pyridin-2-amine (460 mg, 1.4 mmol) obtained in Example 16-1 and triethylamine (790 mg, 7.8 mmol) in tetrahydrofuran (10 mL), and the mixture was stirred at room temperature for 3 hr. 5% Aqueous sodium hydrogen carbonate solution was added to the reaction mixture. The obtained mixture was extracted with ethyl acetate/tetrahydrofuran. Ammonia-ethanol solution (2.0 M, 10 mL) was added to the extract, and the mixture was stirred at room temperature overnight. The mixture was dried over anhydrous sodium sulfate, and the solvent was removed by evaporation under reduced pressure. The obtained residue was purified by basic silica gel chromatography (ethyl acetate: hexane = 3:7 - 8:2) and recrystallized from ethanol-tetrahydrofuran to give the title compound (yield 630 mg, 92%).
Melting point 298-300°C (ethanol-tetrahydrofuran)
MS (ESI+): 473 (M+H).
¹H-NMR (CDCl₃) δ: 2.30 (3H, d, J=1.9Hz), 2.64 (3H, s), 6.95-7.02 (1H, m), 7.17 (1H, dd, J=9.2, 4.5Hz), 7.25-7.29 (1H, m), 7.34-7.40 (1H, m), 7.53 (1H, d, J=0.8Hz), 7.60-7.65 (2H, m), 8.05 (1H, dd, J=9.2, 1.7Hz), 8.35 (1H, dd, J=5.3, 0.8Hz), 8.41 (1H, dd, J=4.5, 1.7Hz), 8.71 (1H, s), 8.80 (1H, s).

### Example compound 42-32: N-{4-[2-(4-fluoro-3-methylphenyl)imidazo[1,2-b]pyridazin-3-yl]pyridin-2-yl}thiophene-3-carboxamide

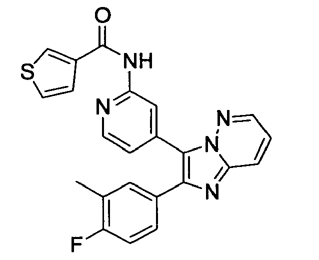

Melting point 217-218°C (ethanol-tetrahydrofuran-water)
MS (ESI+): 430 (M+H).
¹H-NMR (CDCl₃) δ: 2.29 (3H, s), 6.93-7.01 (1H, m), 7.10-7.22 (2H, m), 7.32-7.46 (2H, m), 7.55 (1H, dd, J=5.1, 1.3Hz), 7.61-7.66 (1H, m), 7.98-8.11 (2H, m), 8.32 (1H, dd, J=5.1, 0.8Hz), 8.40 (1H, dd, J=4.7, 1.7Hz), 8.59 (1H, s), 8.83 (1H, dd, J=1.3, 0.8Hz).
Elemental analysis: for C₂₃H₁₆FN₅OS
Calculated: C, 64.32; H, 3.76; N, 16.31.
Found: C, 64.23; H, 3.73; N, 16.22.

### Example compound 42-33: N-{4-[2-(4-fluoro-3-methylphenyl)imidazo[1,2-b]pyridazin-3-yl]pyridin-2-yl}-3-furamide

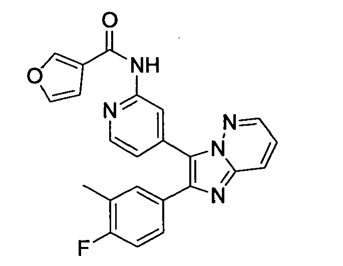

Melting point 199-200°C (ethanol-tetrahydrofuran-water)
MS (ESI+): 414 (M+H).
¹H-NMR (CDCl₃) δ: 2.29 (3H, d, J=1.5Hz), 6.78 (1H, dd, J=1.9, 0.8Hz), 6.93-7.02 (1H, m), 7.11-7.21 (2H, m), 7.34-7.41 (1H, m), 7.48-7.53 (1H, m), 7.63 (1H, dd, J=7.4, 1.6Hz), 8.03 (1H, dd, J=9.2, 1.7Hz), 8.07-8.11 (1H, m), 8.30-8.42 (3H, m), 8.78-8.82 (1H, m).
Elemental analysis: for C₂₃H₁₆FN₅O₂
Calculated: C, 66.82; H, 3.90; N, 16.94.
Found: C, 66.55; H, 3.88; N, 16.77.

### Example compound 42-34: N-{4-[2-(4-fluoro-3-methylphenyl)imidazo[1,2-b]pyridazin-3-yl]pyridin-2-yl}pyrazine-2-carboxamide

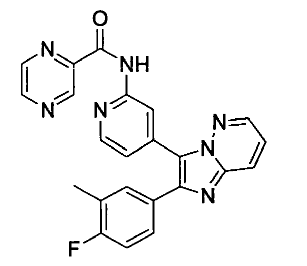

Melting point 229-230°C (ethanol)
MS (ESI+): 426 (M+H).
¹H-NMR (CDCl₃) δ: 2.29 (3H, d, J=1.7Hz), 6.94-7.02 (1H, m), 7.17 (1H, dd, J=9.1, 4.4Hz), 7.23-7.28 (1H, m), 7.35-7.42 (1H, m), 7.64 (1H, dd, J=7.4, 1.7Hz), 8.05 (1H, dd, J=9.1, 1.6Hz), 8.40-8.45 (2H, m), 8.65 (1H, dd, J=2.5, 1.5Hz), 8.84 (1H, d, J=2.5Hz), 8.86-8.89 (1H, m), 9.5 (1H, d, J=1.5Hz), 10.31 (1H, s).
Elemental analysis: for C₂₃H₁₆FN₇O
Calculated: C, 64.94; H, 3.79; N, 23.05.
Found: C, 64.77; H, 3.93; N, 22.84.

### Example compound 42-35: N-{4-[2-(4-fluoro-3-methylphenyl)imidazo[1,2-b]pyridazin-3-yl]pyridin-2-yl}-1,3-benzothiazole-6-carboxamide

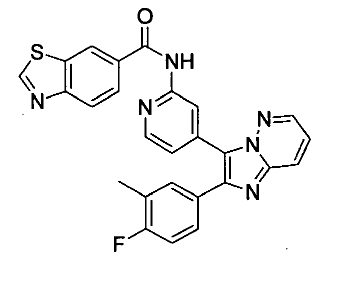

A mixture of 1,3-benzothiazole-6-carboxylic acid (340 mg, 1.9 mmol) and phosphorus oxychloride (1 mL) was stirred at 80°C for 3 hr, and phosphorus oxychloride was removed by evaporation under reduced pressure. To a solution of the obtained residue in tetrahydrofuran (10 mL) was added a solution of 4-[2-(4-fluoro-3-methylphenyl)imidazo[1,2-b]pyridazin-3-yl]pyridin-2-amine (300 mg, 0.94 mmol) obtained in Example 16-1 and triethylamine (190 mg, 1.9 mmol), and the mixture was stirred at room temperature for 14 hr. Saturated aqueous sodium hydrogen carbonate solution was added to the reaction mixture, and the obtained mixture was extracted with ethyl acetate. The extract was dried over anhydrous sodium sulfate, and the solvent was removed by evaporation under reduced pressure. A solution of tetrahydrofuran (3 mL) and ammonia-ethanol (2.0 M, 5 mL) was added to the obtained residue. The mixture was stirred at room temperature for 14 hr, and the solvent was removed by evaporation under reduced pressure. The obtained residue was purified by silica gel chromatography (ethyl acetate) and recrystallized from tetrahydrofuran-ethanol to give the title compound (yield 124 mg, 27%).
Melting point 252-253°C (ethanol-tetrahydrofuran)
¹H-NMR (CDCl₃) δ: 2.30 (3H, d, J=1.9Hz), 6.99 (1H, t, J=9.1Hz), 7.18 (1H, dd, J=9.1, 4.5Hz), 7.22 (1H, dd, J=5.2, 1.4Hz), 7.38-7.46 (1H, m), 7.65 (1H, dd, J=4.4, 1.7Hz), 8.02-8.09 (2H, m), 8.26 (1H, d, J=8.5Hz), 8.36 (1H, dd, J=5.2, 0.8Hz), 8.42 (1H, dd, J=4.5, 1.7Hz), 8.63 (1H, d, J=1.7Hz), 8.78 (1H, s), 8.88 (1H, dd, J=1.4, 0.8Hz), 9.18 (1H, s).
Elemental analysis: for C₂₆H₁₇FN₆OS
Calculated: C, 64.99; H, 3.57; N, 17.49.
Found: C, 64.82; H, 3.66; N, 17.18.

### Example compound 42-36: N-{4-[2-(4-fluoro-3-methylphenyl)imidazo[1,2-b]pyridazin-3-yl]pyridin-2-yl}-6-quinolinecarboxamide

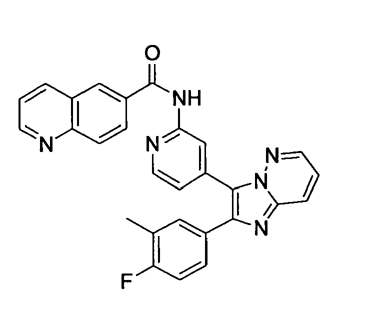

A mixture of quinoline-6-carboxylic acid (330 mg, 1.9 mmol) and phosphorus oxychloride (1 mL) was stirred at 80°C for 3 hr, and phosphorus oxychloride was removed by evaporation under reduced pressure. To a solution of the obtained residue in tetrahydrofuran (10 mL) were added 4-[2-(4-fluoro-3-methylphenyl)imidazo[1,2-b]pyridazin-3-yl]pyridin-2-amine (300 mg, 0.94 mmol) obtained in Example 16-1 and triethylamine (190 mg, 1.9 mmol), and the mixture was stirred at room temperature for 14 hr. Saturated aqueous sodium hydrogen carbonate solution was added to the reaction mixture, and the obtained mixture was extracted with ethyl acetate. The extract was dried over anhydrous sodium sulfate, and the solvent was removed by evaporation under reduced pressure. To the obtained residue was added a solution of tetrahydrofuran (3 mL) and ammonia-ethanol (2.0 M, 5 mL), and the mixture was stirred at room temperature for 14 hr. The solvent was removed by evaporation under reduced pressure, and the obtained residue was purified by silica gel chromatography (ethyl acetate) and recrystallized from ethanol-tetrahydrofuran to give the title compound (yield 220 mg, 49%).
Melting point 210-211°C (ethanol-tetrahydrofuran)
¹H-NMR (CDCl₃) δ: 2.30 (3H, d, J=1.6Hz), 6.99 (1H, t, J=8.9Hz), 7.17 (1H, dd, J=9.1, 4.4Hz), 7.23 (1H, dd, J=5.2, 1.7Hz), 7.37-7.44 (1H, m), 7.53 (1H, dd, J=8.2, 4.1Hz), 7.66 (1H, dd, J=7.7, 1.9Hz), 8.06 (1H, dd, J=9.1, 1.7Hz), 8.21-8.27 (2H, m), 8.30
(1H, dd, J=8.2, 1.6Hz), 8.38 (1H, d, J=5.2Hz), 8.43 (1H, dd, J=4.4, 1.7Hz), 8.47 (1H, s), 8.85 (1H, s), 8.92 (1H, d, J=0.8Hz), 9.05 (1H, dd, J=4.1, 1.7Hz).
Elemental analysis: for C₂₈H₁₉FN₆O·0.25H₂O
Calculated: C, 70.21; H, 4.10; N, 17.55.
Found: C, 70.34; H, 4.04; N, 17.61.

### Example compound 42-37: N-{4-[2-(4-fluoro-3-methylphenyl) imidazo[1,2-b]pyridazin-3-yl]pyridin-2-yl}-8-quinolinecarboxamide

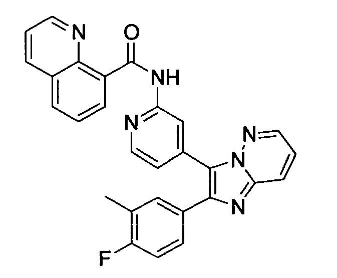

Melting point 229-230°C (ethanol-tetrahydrofuran)
MS (ESI+): 408 (M+H).
¹H-NMR (CDCl₃) δ: 2.28 (3H, d, J=1.7Hz), 6.96 (1H, t, J=9.1Hz), 7.14 (1H, dd, J=9.1, 4.4Hz), 7.18 (1H, dd, J=5.2, 1.7Hz), 7.24-7.29 (1H, m), 7.38-7.46 (1H, m), 7.58 (1H, dd, J=8.3, 4.4Hz), 7.66 (1H, d, J=8.5Hz), 7.73 (1H, t, J=7.8Hz), 8.01-8.08 (2H, m), 8.35 (1H, dd, J=8.5, 1.7Hz), 8.41 (1H, dd, J=4.4, 1.7Hz), 8.49 (1H, dd, J=5.2, 0.8Hz), 8.93 (1H, dd, J=7.8, 1.7Hz), 8.99 (1H, dd, J=1.7, 0.8Hz), 9.17 (1H, dd, J=4.4, 1.7Hz).

### (Example 43)

### N-{4-[2-(3-Methylphenyl)imidazo[1,2-b]pyridazin-3-yl]pyrimidin-2-yl}benzamide

The compound was synthesized in the same manner as in Example 19 using 4-[2-(3-methylphenyl)imidazo[1,2-b]pyridazin-3-yl]-2-pyrimidinamine (Example compound 37-1) instead of 4-[2-(3-methylphenyl)imidazo[1,2-b]pyridazin-3-yl]pyridin-2-amine. 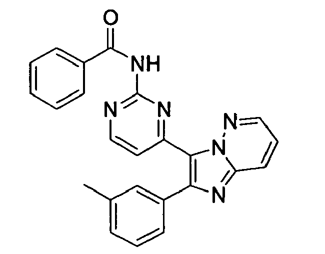 Melting point 200-201°C (ethanol)
MS (ESI+): 407 (M+H).
¹H-NMR (CDCl₃) δ: 2.39 (3H, s), 7.19-7.31 (3H, m), 7.44-7.53 (3H, m), 7.54-7.61 (1H, m), 7.64 (1H, d, J=5.2Hz), 7.66 (1H, s), 7.87-7.93 (2H, m), 8.08 (1H, dd, J=9.3, 1.6Hz), 8.46 (1H, dd, J=4.4, 1.7Hz), 8.61 (1H, brs), 8.81 (1H, d, J=5.2Hz).

### (Example 44)

The compounds of Examples 44-1 - 44-3 below were synthesized in the same manner as in Example 43 respectively using 4-methylbenzoyl chloride, 4-methoxybenzoyl chloride or isonicotinoyl chloride hydrochloride, instead of benzoyl chloride.

### Example compound 44-1: 4-methyl-N-{4-[2-(3-methylphenyl)imidazo[1,2-b]pyridazin-3-yl]pyrimidin-2-yl}benzamide

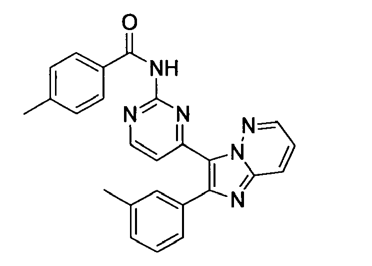

Melting point 211-212°C (ethanol)
MS (ESI+): 421 (M+H).
¹H-NMR (CDCl₃) δ: 2.39 (3H, s), 2.43 (3H, s), 7.19-7.26 (2H, m), 7.27-7.32 (3H, m), 7.47 (1H, d, J=7.6Hz), 7.63 (1H, d, J=5.2Hz), 7.66 (1H, s), 7.80 (2H, d, J=8.1Hz), 8.08 (1H, dd, J=9.1, 1.6Hz), 8.46 (1H, dd, J=4.3, 1.7Hz), 8.53 (1H, s), 8.81 (1H, d, J=5.2Hz).

### Example compound 44-2: 4-methoxy-N-{4-[2-(3-methylphenyl)imidazo[1,2-b]pyridazin-3-yl]pyrimidin-2-yl}benzamide

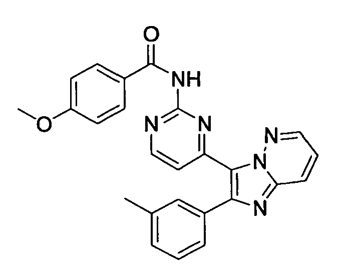

Melting point 193-194°C (ethanol)
MS (ESI+): 437 (M+H).
¹H-NMR (CDCl₃) δ: 2.39 (3H, s), 3.88 (3H, s), 7.19-7.32 (3H, m), 7.19-7.32 (4H, m), 7.47 (1H, d, J=7.4Hz), 7.61 (1H, d, J=5.2Hz), 7.66 (1H, s), 7.88 (2H, d, J=8.9Hz), 8.08 (1H, dd, J=9.0, 1.5Hz), 8.46 (1H, dd, J=4.4, 1.6Hz), 8.49 (1H, s), 8.81 (1H, d, J=5.2Hz).

### Example compound 44-3: N-{4-[2-(3-methylphenyl)imidazo[1,2-b]pyridazin-3-yl]pyrimidin-2-yl}isonicotinamide

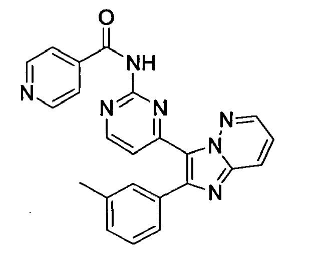

Melting point 181-182°C (ethanol)
MS (ESI+): 408 (M+H).
¹H-NMR (CDCl₃) δ: 2.39 (3H, s), 7.19-7.31 (3H, m), 7.45 (1H, d, J=7.3Hz), 7.64 (1H, s), 7.67-7.73 (3H, m), 8.09 (1H, dd, J=9.2, 1.7Hz), 8.46 (1H, dd, J=4.3, 1.7Hz), 8.73 (1H, s), 8.77-8.83 (3H, m).

### (Example 45)

The compounds of Examples 45-1 and 45-2 below were synthesized in the same manner as in Example 7 respectively using N-{4-[6-chloro-2-(3-methylphenyl)imidazo[1,2-b]pyridazin-3-yl]pyridin-2-yl}-4-methoxybenzamide (Example compound 18) or N-{4-[6-chloro-2-(4-fluoro-3-methylphenyl)imidazo[1,2-b]pyridazin-3-yl]pyridin-2-yl}-4-methoxybenzamide (Example compound 39-1), instead of 4-[6-chloro-2-(3-methylphenyl)imidazo[1,2-b]pyridazin-3-yl]-N-cyclohexylpyridin-2-amine.

### Example compound 45-1: N-{4-[6-(dimethylamino)-2-(3-methylphenyl)imidazo[1,2-b]pyridazin-3-yl]pyridin-2-yl}-4-methoxybenzamide

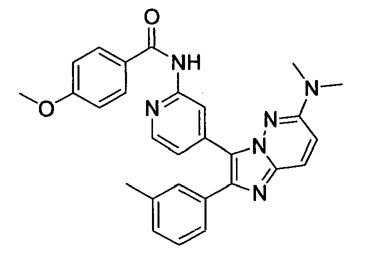

Melting point 193-194°C (ethanol-water)
¹H-NMR (CDCl₃) δ: 2.36 (3H, s), 3.13 (6H, s), 3.88 (3H, s), 6.84 (1H, d, J=10.0Hz), 6.96-7.02 (2H, m), 7.10-7.15 (2H, m), 7.18-7.24 (1H, m), 7.36 (1H, d, J=7.7Hz), 7.57 (1H, s), 7.76 (1H, d, J=10.0Hz), 7.84-7.94 (2H, m), 8.18 (1H, dd, J=5.3, 0.6Hz), 8.61 (1H, s), 9.10-9.14 (1H, m).

### Example compound 45-2: N-{4-[6-(dimethylamino)-2-(4-fluoro-3-methylphenyl)imidazo[1,2-b]pyridazin-3-yl]pyridin-2-yl}-4-methoxybenzamide

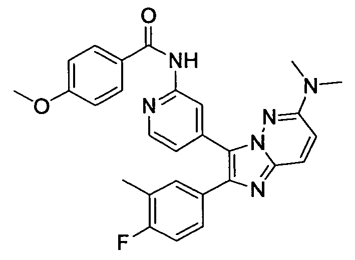

Melting point 224-225°C (ethanol-tetrahydrofuran-water)
MS (ESI+): 497 (M+H).
¹H-NMR (CDCl₃) δ: 2.28 (3H, d, J=1.3Hz), 3.13 (6H, s), 3.88 (3H, s), 6.85 (1H, d, J=10.0Hz), 6.92-7.02 (3H, m), 7.12 (1H, dd, J=5.3, 1.5Hz), 7.31-7.38 (1H, m), 7.57 (1H, dd, J=7.5, 1.5Hz), 7.75 (1H, d, J=10.0Hz), 7.87-7.94 (2H, m), 8.21 (1H, d, J=5.3Hz), 8.61 (1H, s), 9.06-9.09 (1H, m).
Elemental analysis: for C₂₈H₂₅FN₆O₂·O.5H₂O
Calculated: C, 66.52; H, 5.18; N, 16.62.
Found: C, 66.56; H, 5.16; N, 16.51.

### (Example 46)

### N-{4-[2-(4-Fluoro-3-methylphenyl)-6-(methylamino)imidazo[1,2-b]pyridazin-3-yl]pyridin-2-yl}-4-methoxybenzamide

The compound was synthesized in the same manner as in Example 45 using 30% methylamine-ethanol solution instead of 2.0 M dimethylamine-tetrahydrofuran solution. 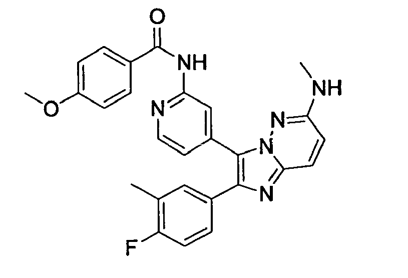 Melting point 178-180°C (ethanol-tetrahydrofuran-water)
MS (ESI+): 483 (M+H).
¹H-NMR (CDCl₃) δ: 2.28 (3H, d, J=1.7Hz), 3.00 (3H, d, J=5.0Hz), 3.88 (3H, s), 4.45-4.51 (1H, m), 6.52 (1H, d, J=9.4Hz), 6.92-7.01 (3H, m), 7.13 (1H, dd, J=5.2, 1.7Hz), 7.30-7.36 (1H, m), 7.54-7.57 (1H, m), 7.67 (1H, d, J=9.4Hz), 7.88-7.93 (2H, m), 8.22 (1H, dd, J=5.2, 0.8Hz), 8.62 (1H, s), 9.07 (1H, dd, J=1.7, 0.8Hz).
Elemental analysis: for C₂₇H₂₃FN₆O₂·1.5H₂O
Calculated: C, 63.64; H, 5.14; N, 16.49.
Found: C, 63.80; H, 4.96; N, 16.43.

### (Example 47)

The compounds of Examples 47-1 - 47-6 below were synthesized in the same manner as in Example 26 using 4-[2-(4-fluoro-3-methylphenyl)imidazo[1,2-b]pyridazin-3-yl]pyridin-2-amine (Example compound 16-1) instead of 4-[2-(3-methylphenyl)imidazo[1,2-b]pyridazin-3-yl]pyridin-2-amine, and methyl isocyanate, tert-butyl isocyanate, sec-butyl isocyanate, 3-chloropropyl isocyanate, 4-cyanophenyl isocyanate or 3-thienyl isocyanate instead of phenyl isocyanate.

### Example compound 47-1: N-{4-[2-(4-fluoro-3-methylphenyl)imidazo[1,2-b]pyridazin-3-yl]pyridin-2-yl}-N'-methylurea

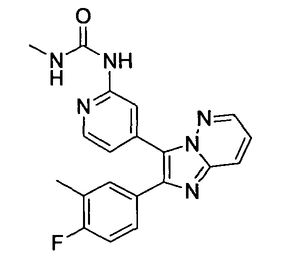

Melting point 225-227°C (ethanol-water)
MS (ESI+): 377 (M+H).
¹H-NMR (CDCl₃) δ: 2.29 (3H, d, J=1.7Hz), 2.95 (3H, d, J=4.7Hz), 6.95-7.01 (1H, m), 7.06 (1H, dd, J=5.5, 1.3Hz), 7.12-7.20 (2H, m), 7.31-7.38 (1H, m), 7.60 (1H, dd, J=7.4, 1.6Hz), 7.94 (1H, s), 8.03 (1H, dd, J=9.0, 1.7Hz), 8.20 (1H, d, J=5.5Hz), 8.37 (1H, dd, J=4.5, 1.7Hz), 9.13-9.24 (1H, m).
Elemental analysis: for C₂₀H₁₇FN₆O Calculated: C, 63.82; H, 4.55; N, 22.33. Found: C, 63.63; H, 4.64; N, 22.03.

### Example compound 47-2: N-(tert-butyl)-N'-{4-[2-(4-fluoro-3-methylphenyl)imidazo[1,2-b]pyridazin-3-yl]pyridin-2-yl}urea

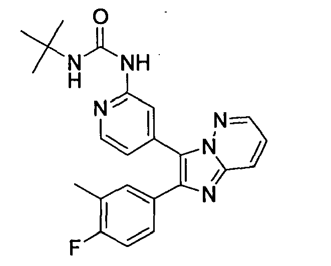

Melting point 234-235°C (ethanol-tetrahydrofuran-water)
MS (ESI+): 419 (M+H).
¹H-NMR (CDCl₃) δ: 1.43 (9H, s), 2.29 (3H, d, J=1.9Hz), 6.94-7.01 (2H, m), 7.14 (1H, dd, J=9.1, 4.3Hz), 7.20 (1H, s), 7.32-7.38 (1H, m), 7.59-7.64 (1H, m), 7.99-8.06 (2H, m), 8.17 (1H, dd, J=5.5, 0.4Hz), 8.35 (1H, dd, J=4.3, 1.7Hz), 9.25 (1H, s).
Elemental analysis: for C₂₃H₂₃FN₆O
Calculated: C, 66.01; H, 5.54; N, 20.08.
Found: C, 65.96; H, 5.55; N, 20.06.

### Example compound 47-3: N-(sec-butyl)-N'-{4-[2-(4-fluoro-3-methylphenyl)imidazo[1,2-b]pyridazin-3-yl]pyridin-2-yl}urea

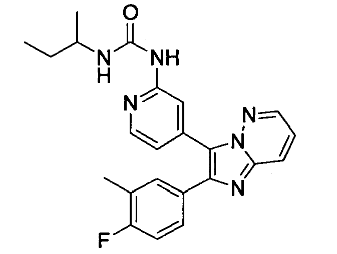

Melting point 196-197°C (ethanol-tetrahydrofuran-water)
¹H-NMR (CDCl₃) δ: 0.97 (3H, t, J=7.4Hz), 1.23 (3H, d, J=6.6Hz), 1.54-1.65 (2H, m), 2.29 (3H, d, J=1.7Hz), 3.83-3.97 (1H, m), 6.93-7.05 (2H, m), 7.15 (1H, dd, J=9.1, 4.4Hz), 7.20 (1H, s), 7.32-7.39 (1H, m), 7.61 (1H, dd, J=7.5, 1.7Hz), 7.99 (1H, s), 8.03 (1H, dd, J=9.1, 1.6Hz), 8.19 (1H, d, J=5.5Hz), 8.36 (1H, dd, J=4.4, 1.6Hz), 9.11 (1H, d, J=7.5Hz).
Elemental analysis: for C₂₃H₂₃FN₆O
Calculated: C, 66.01; H, 5.54; N, 20.08.
Found: C, 65.96; H, 5.54; N, 19.99.

### Example compound 47-4: N-(3-chloropropyl)-N'-{4-[2-(4-fluoro-3-methylphenyl)imidazo[1,2-b]pyridazin-3-yl]pyridin-2-yl}urea

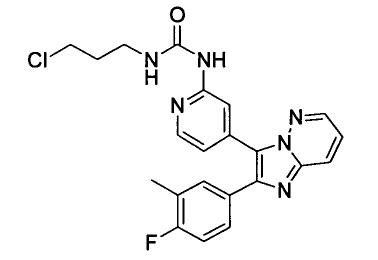

Melting point 170-171°C (ethanol-water)
MS (ESI+): 439 (M+H).
¹H-NMR (CDCl₃) δ: 2.03-2.12 (2H, m), 2.29 (3H, d, J=1.7Hz), 3.48-3.56 (2H, m), 3.64 (2H, t, J=6.5Hz), 6.95-7.02 (1H, m), 7.05 (1H, dd, J=5.5, 1.4Hz), 7.16 (1H, dd, J=9.2, 4.4Hz), 7.27 (1H, s), 7,33-7.38 (1H, m), 7.59-7.63 (1H, m), 8.04 (1H, dd, J=9.2, 1.7Hz), 8.19 (1H, d, J=5.5Hz), 8.37 (1H, dd, J=4.4, 1.7Hz), 8.47 (1H, s), 9.40-9.47 (1H, m).
Elemental analysis: for C₂₂H₂₀ClFN₆O
Calculated: C, 60.21; H, 4.59; N, 19.15.
Found: C, 60.19; H, 4.72; N, 19.16.

### Example compound 47-5: N-(4-cyanophenyl)-N'-{4-[2-(4-fluoro-3-methylphenyl)imidazo[1,2-b]pyridazin-3-yl]pyridin-2-yl}urea

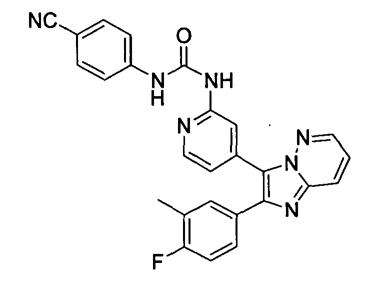

Melting point 204-206°C (ethanol)
¹H-NMR (CDCl₃) δ: 2.30 (3H, d, J=1.9Hz), 6.96-7.03 (1H, m), 7.18-7.23 (2H, m), 7.33-7.39 (2H, m), 7.54-7.59 (2H, m), 7.60-7.64 (1H, m), 7.71-7.76 (2H, m), 8.08 (1H, dd, J=9.3, 1.7Hz), 8.26-8.31 (2H, m), 8.34 (1H, dd, J=4.4, 1.7Hz), 12.34 (1H, s).
Elemental analysis: for C₂₆H₁₈FN₇O·0.8H₂O
Calculated: C, 65.35; H, 4.13; N, 20.52.
Found: C, 65.42; H, 4.37; N, 20.27.

### Example compound 47-6: N-{4-[2-(4-fluoro-3-methylphenyl)imidazo[1,2-b]pyridazin-3-yl]pyridin-2-yl}-N'-3-thienylurea

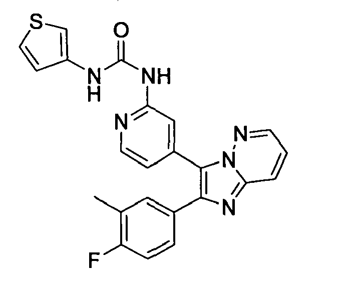

Melting point 234-235°C (ethanol-tetrahydrofuran-water)
MS (ESI+): 445 (M+H).
¹H-NMR (DMSO-d₆) δ: 2.25(3H, d, J=1.7Hz), 7.11-7.16 (3H, m), 7.35-7.41 (3H, m), 7.47 (1H, dd, J=5.2, 3.3Hz), 7.67 (1H, dd, J=7.4, 1.7Hz), 7.77 (1H, s), 8.24 (1H, dd, J=9.2, 1.5Hz), 8.38 (1H, dd, J=5.2, 0.6Hz),8.58 (1H, dd, J=4.5, 1.5Hz), 9.62 (1H, s), 10.71 (1H, s).
Elemental analysis: for C₂₃H₁₇FN₆OS
Calculated: C, 62.15; H, 3.86; N, 18.91.
Found: C, 62.02; H, 4.02; N, 18.69.

### (Example 48)

The compounds of Examples 48-1 - 48-5 below were synthesized in the same manner as in Example 26 using ethyl isocyanate instead of phenyl isocyanate, and 4-[6-chloro-2-(4-fluoro-3-methylphenyl)-8-methylimidazo[1,2-b]pyridazin-3-yl]-2-pyridylamine (Example compound 36-1), 4-[7-tert-butyl-6-chloro-2-(4-fluoro-3-methylphenyl)imidazo[1,2-b]pyridazin-3-yl]-2-pyridylamine (Example compound 36-2), 4-[2-(4-fluoro-3-methylphenyl)-8-methylimidazo[1,2-b]pyridazin-3-yl]-2-pyridylamine (Example compound 37-2), 4-[7-tert-butyl-2-(4-fluoro-3-methylphenyl)imidazo[1,2-b]pyridazin-3-yl]-2-pyridylamine (Example compound 37-3) or 3-(2-aminopyridin-4-yl)-2-(4-fluoro-3-methylphenyl)-N-methylimidazo[1,2-b]pyridazin-6-amine (Example compound 33), instead of 4-[2-(3-methylphenyl)imidazo[1,2-b]pyridazin-3-yl]pyridin-2-amine. Example compound 48-1: N-{4-[6-chloro-2-(4-fluoro-3-methylphenyl)-8-methylimidazo[1,2-b]pyridazin-3-yl]-2-pyridyl}-N'-ethylurea 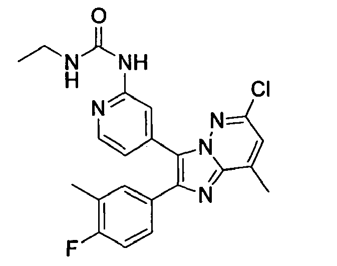 Melting point 229-230°C (ethanol)
MS (ESI+): 439 (M+H).
¹H-NMR (CDCl₃) δ: 1.24 (3H, t, J=7.2Hz), 2.29 (3H, s), 2.73 (3H, s), 3.41 (2H, dq, J=7.2, 5.4Hz), 6.93-7.07 (3H, m), 7.10 (1H, s), 7.28-7.37 (1H, m), 7.56 (1H, dd, J=7.4, 1.9Hz), 7.81 (1H, s), 8.19 (1H, d, J=5.4Hz), 9.23 (1H, t, J=5.4Hz).
Elemental analysis: for C₂₂H₂₀ClFN₆O
Calculated: C, 60.21; H, 4.59; N, 19.15.
Found: C, 60.22; H, 4.54; N, 19.12.

### Example compound 48-2: N-{4-[7-tert-butyl-6-chloro-2-(4-fluoro-3-methylphenyl)imidazo[1,2-b]pyridazin-3-yl]-2-pyridyl}-N'-ethylurea

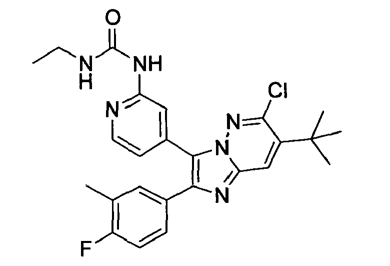

Melting point 246-247°C (ethanol)
MS (ESI+): 481 (M+H).
¹H-NMR (CDCl₃) δ: 1.25 (3H, t, J=7.3Hz), 1.55 (9H, s), 2.29 (3H, d, J=1.7Hz), 3.42 (2H, dq, J=7.3, 5.5Hz), 6.99 (1H, t, J=9.1Hz), 7.04 (1H, dd, J=5.5, 1.1Hz), 7.12 (1H, s), 7.30-7.37 (1H, m), 7.57 (1H, dd, J=7.4, 2.2Hz), 7.64 (1H, s), 8.03 (1H, s), 8.19 (1H, d, J=5.5Hz), 9.26 (1H, t, J=5.5Hz).
Elemental analysis: for C₂₅H₂₆ClFN₆O
Calculated: C, 62.43; H, 5.45; N, 17.47.
Found: C, 62.36; H, 5.49; N, 17.40.

### Example compound 48-3: N-{4-[2-(4-fluoro-3-methylphenyl)-8-methylimidazo[1,2-b]pyridazin-3-yl]-2-pyridyl}-N'-ethylurea

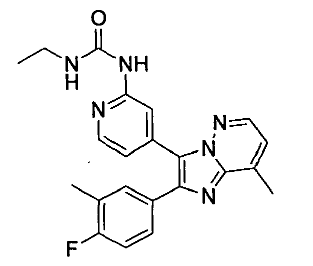

Melting point 240-241°C (ethanol-hexane)
MS (ESI+): 405 (M+H).
¹H-NMR (CDCl₃) δ: 1.25 (3H, t, J=7.2Hz), 2.30 (3H, d, J=1.7Hz), 2.75 (3H, s), 3.42 (2H, dq, J=7.2, 5.0Hz), 6.93-7.03 (2H, m), 7.04-7.10 (2H, m), 7.21 (1H, s), 7.30-7.38 (1H, m), 7.58 (1H, dd, J=7.7, 1.9Hz), 8.19 (1H, d, J=5.8Hz), 8.24 (1H, d, J=4.5Hz), 9.27 (1H, t, J=5.0Hz).
Elemental analysis: for C₂₂H₂₁FN₆O.0.5H₂O
Calculated: C, 63.91; H, 5.36; N, 20.33.
Found: C, 64.12; H, 5.08; N, 20.16.

### Example compound 48-4: N-{4-[7-tert-butyl-2-(4-fluoro-3-methylphenyl)imidazo[1,2-b]pyridazin-3-yl]-2-pyridyl}-N'-ethylurea

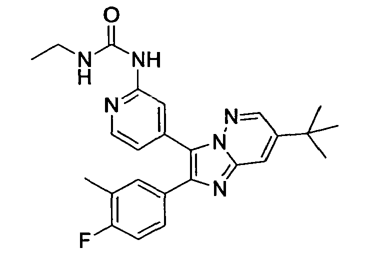

Melting point 212-213°C (ethanol)
MS (ESI+): 447 (M+H).
¹H-NMR (CDCl₃) δ: 1.25 (3H, t, J=7.1Hz), 1.42 (9H, s), 2.29 (3H, d, J=1.6Hz), 3.41 (2H, dq, J=7.1, 5.6Hz), 6.98 (1H, t, J=8.9Hz), 7.04 (1H, dd, J=5.5, 1.4Hz), 7.18 (1H, s), 7.32-7.38 (1H, m), 7.59 (1H, dd, J=7.4, 1.7Hz), 7.77 (1H, s), 7.89 (1H, d, J=2.3Hz), 8.18 (1H, d, J=5.5Hz), 8.45 (1H, d, J=2.3Hz), 9.25 (1H, t, J=5.6Hz).
Elemental analysis: for C₂₅H₂₇FN₆O
Calculated: C, 67.25; H, 6.09; N, 18.82.
Found: C, 66.97; H, 6.17; N, 18.72.

### Example compound 48-5: N-ethyl-N'-{4-[2-(4-fluoro-3-methylphenyl)-6-(methylamino)imidazo[1,2-b]pyridazin-3-yl]pyridin-2-yl}urea

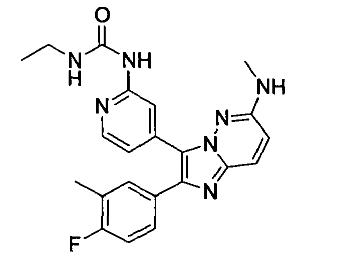

Melting point 263-264°C (ethanol)
MS (ESI+): 420 (M+H).
¹H-NMR (DMSO-d₆) δ: 1.09 (3H, t, J=7.1Hz), 2.23 (3H, d, J=1.4Hz), 2.77 (3H, d, J=5.0Hz), 3.15-3.24 (2H, m), 6.75 (1H, d, J=9.6Hz), 6.96 (1H, dd, J=5.2, 1.4Hz), 7.07-7.14 (2H, m), 7.24 (1H, m), 7.51-7.55 (1H, m), 7.72 (1H, s), 7.77 (1H, d, J=9.6Hz), 8.17 (1H, d, J=5.2Hz), 8.28-8.38 (1H, m), 9.26-9.39 (1H, m).
Elemental analysis: for C₂₂H₂₂FN₇O·0.2H₂O
Calculated: C, 62.46; H, 5.34; N, 23.18.
Found: C, 62.67; H, 5.51; N, 23.01.

### (Example 49)

### N-{4-[6-chloro-2-(4-fluoro-3-methylphenyl)imidazo[1,2-b]pyridazin-3-yl]pyridin-2-yl}-N'-isopropylurea

The compound was synthesized in the same manner as in Example 26 using isopropyl isocyanate instead of phenyl isocyanate, and 4-[6-chloro-2-(4-fluoro-3-methylphenyl)imidazo[1,2-b]pyridazin-3-yl]pyridin-2-amine (Example compound 13-2) instead of 4-[2-(3-methylphenyl)imidazo[1,2-b]pyridazin-3-yl]pyridin-2-amine. 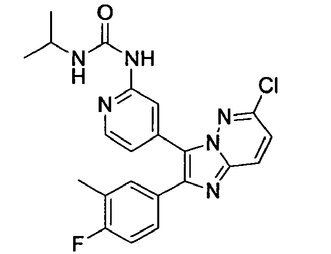 MS (ESI+): 439 (M+H).
¹H-NMR (CDCl₃) δ: 1.26 (6H, d, J=6.6Hz), 2.28 (3H, d, J=1.7Hz), 3.99-4.12 (1H, m), 6.94-7.02 (2H, m), 7.12-7.18 (2H, m), 7.29-7.36 (1H, m), 7.58 (1H, dd, J=7.4, 1.7Hz), 7.97 (1H, d, J=9.4Hz), 8.13 (1H, s), 8.20 (1H, d, J=5.5Hz), 9.10 (1H, d, J=7.4Hz).

### (Example 50)

### 2,2,2-Trichloroethyl {4-[2-(4-fluoro-3-methylphenyl)imidazo[1,2-b]pyridazin-3-yl]pyridin-2-yl}carbamate

The compound was synthesized in the same manner as in Example 30 using 4-[2-(4-fluoro-3-methylphenyl)imidazo[1,2-b]pyridazin-3-yl]pyridin-2-amine (Example compound 16-1) instead of 4-[2-(3-methylphenyl)imidazo[1,2-b]pyridazin-3-yl]pyridin-2-amine.

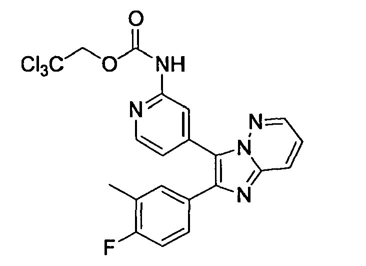

To a solution of 4-[2-(4-fluoro-3-methylphenyl)imidazo[1,2-b]pyridazin-3-yl]pyridin-2-amine (5.0 g, 16 mmol) obtained in Example 16-1 in pyridine (100 mL) was added 2,2,2-trichloroethyl chloroformate (4.3 mL, 31 mmol), and the mixture was stirred at 50°C overnight. Additional 2,2,2-trichloroethyl chloroformate (4.3 mL, 31 mmol) was added to the reaction mixture, and the mixture was stirred at 50°C overnight. 5% Aqueous sodium hydrogen carbonate solution was added to the mixture, and the mixture was extracted with chloroform. A solution of ammonia in ethanol (2.0 M, 100 mL) was added to the extract. The mixture was stirred at room temperature overnight and dried over anhydrous sodium sulfate. The solvent was removed by evaporation under reduced pressure, and the obtained residue was purified by silica gel chromatography (ethyl acetate: hexane = 3:7 - 8:2) to give the title compound (yield 2.6 g, 34%). ¹H-NMR (DMSO-d₆) δ: 2.23 (3H, d, J=1.4Hz), 4.94 (2H, s), 7.15 (1H, t, J=9.1Hz), 7.28 (1H, dd, J=5.2, 1.4Hz), 7.34-7.42 (2H, m), 7.65 (1H, dd, J=7.7, 2.2Hz), 8.08 (1H, s), 8.25 (1H, dd, J=9.3, 1.7Hz), 8.43 (1H, d, J=5.2Hz), 8.57 (1H, dd, J=4. 4, 1.7Hz), 10.85 (1H, s).

### (Example 51)

The compounds of Examples 51-1 - 51-17 below were synthesized in the same manner as in Example 31 using 2,2,2-trichloroethyl {4-[2-(4-fluoro-3-methylphenyl)imidazo[1,2-b]pyridazin-3-yl]pyridin-2-yl}carbamate (Example compound 50) instead of 2,2,2-trichloroethyl {4-[2-(3-methylphenyl)imidazo[1,2-b]pyridazin-3-yl]pyridin-2-yl}carbamate, and cyclopropylamine, 2,2,2-trifluoroethylamine, isobutylamine, 1-ethylpropylamine, 2-(methylsulfonyl)ethylamine, 4-(methylthio)aniline, pyridin-3-amine, 3-methyl-1H-pyrazol-5-amine, ammonia-dioxane solution, 4-aminocyclohexanol, 2-aminoethanol, 4-aminobutanol, 2-amino-1-methylethanol, 3-aminopropionitrile, tetrahydro-2H-pyran-4-amine, morpholine or 4-hydroxypiperidine instead of piperidine.

### Example compound 51-1: N-cyclopropyl-N'-{4-[2-(4-fluoro-3-methylphenyl)imidazo[1,2-b]pyridazin-3-yl]pyridin-2-yl}urea

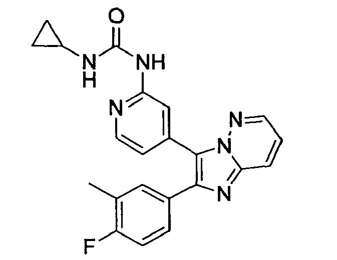

Melting point 222-223°C (ethanol-tetrahydrofuran-water)
MS (ESI+): 403 (M+H).
¹H-NMR (CDCl₃) δ: 0.57-0.87 (4H, m), 2.29 (3H, d, J=1.7Hz), 2.68-2.83 (1H, m), 6.93-7.01 (1H, m), 7.05 (1H, dd, J=5.5, 1.3Hz), 7.14 (1H, dd, J=9.1, 4.4Hz), 7.23-7.39 (2H, m), 7.59 (1H, dd, J=7.4, 1.7Hz), 7.86 (1H, s), 8.02 (1H, dd, J=9.1, 1.6Hz), 8.18 (1H, d, J=5.5Hz), 8.36 (1H, dd, J=4.4, 1.6Hz), 9.19 (1H, brs).
Elemental analysis: for C₂₂H₁₉FN₆O
Calculated: C, 65.66; H, 4.76; N, 20.88.
Found: C, 65.52; H, 4.69; N, 20.71.

### Example compound 51-2: N-{4-[2-(4-fluoro-3-methylphenyl)imidazo[1,2-b]pyridazin-3-yl]pyridin-2-yl}-N'-(2,2,2-trifluoroethyl)urea

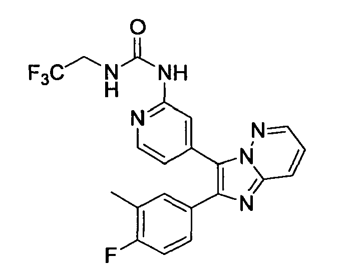

Melting point 229-230°C (ethanol-tetrahydrofuran-water)
MS (ESI+): 445 (M+H).
¹H-NMR (CDCl₃) δ: 2.30 (3H, d, J=1.7Hz), 3.96-4.07 (2H, m), 6.96-7.02 (1H, m), 7.11 (1H, dd, J=5.5, 1.3Hz), 7.17 (1H, dd, J=9.2, 4.5Hz), 7.27 (1H, s), 7.32-7.39 (1H, m), 7.61 (1H, dd, J=7.4, 1.6Hz), 8.04 (1H, dd, J=9.2, 1.7Hz), 8.21 (1H, d, J=5.5Hz), 8.37 (1H, dd, J=4.5, 1.7Hz), 8.54 (1H, s), 9.99 (1H, s).
Elemental analysis: for C₂₁H₁₆F₄N₆O
Calculated: C, 56.76; H, 3.63; N, 18.91.
Found: C, 56.71; H, 3.63; N, 18.85.

### Example compound 51-3: N-{4-[2-(4-fluoro-3-methylphenyl)imidazo[1,2-b]pyridazin-3-yl]pyridin-2-yl}-N'-isobutylurea

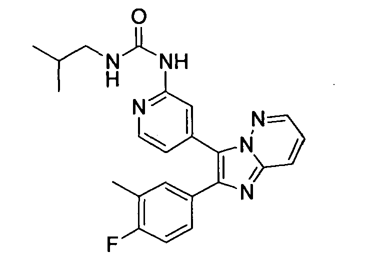

Melting point 213-214°C (ethanol-water)
MS (ESI+): 419 (M+H).
¹H-NMR (CDCl₃) δ: 0.98 (6H, d, J=6.8Hz), 1.86-1.96 (1H, m), 2.30 (3H, d, J=1.7Hz), 3.21 (2H, dd, J=6.6, 5.8Hz), 6.95-7.02 (1H, m), 7.05 (1H, dd, J=5.5, 1.5Hz), 7.12-7.19 (2H, m), 7.32-7.39 (1H, m), 7.61 (1H, dd, J=7.4, 1.6Hz), 7.81 (1H, s), 8.03 (1H, dd, J=9.1, 1.7Hz), 8.20 (1H, d, J=5.5Hz), 8.37 (1H, dd, J=4.3, 1.7Hz), 9.34-9.39 (1H, m).
Elemental analysis: for C₂₃H₂₃FN₆O
Calculated: C, 66.01; H, 5.54; N, 20.08.
Found: C, 66.01; H, 5.52; N, 20.10.

### Example compound 51-4: N-(1-ethylpropyl)-N'-{4-[2-(4-fluoro-3-methylphenyl)imidazo[1,2-b]pyridazin-3-yl]pyridin-2-yl}urea

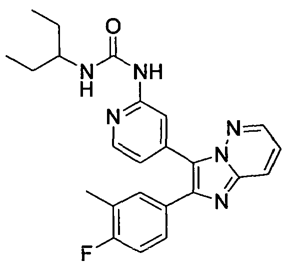

Melting point 187-188°C (ethanol-water)
¹H-NMR (CDCl₃) δ: 0.95 (6H, t, J=7.4Hz), 1.50-1.65 (4H, m), 2.29 (3H, d, J=1.1Hz), 3.70-3.82 (1H, m), 6.94-7.01 (2H, m), 7.14 (1H, dd, J=9.2, 4.5Hz), 7.30 (1H, s), 7.32-7.39 (1H, m), 7.62 (1H, dd, J=7.4, 1.5Hz), 8.03 (1H, dd, J=9.2, 1.5Hz), 8.16 (1H, d, J=5.5Hz), 8.35 (1H, dd, J=4.5, 1.5Hz), 8.60 (1H, s), 8.95-9.09 (1H, m).
Elemental analysis: for C₂₄H₂₅FN₆O
Calculated: C, 66.65; H, 5.83; N, 19.43.
Found: C, 66.46; H, 5.78; N, 19.35.

### Example compound 51-5: N-{4-[2-(4-fluoro-3-methylphenyl)imidazo[1,2-b]pyridazin-3-yl]pyridin-2-yl}-N'-[2-(methylsulfonyl)ethyl]urea

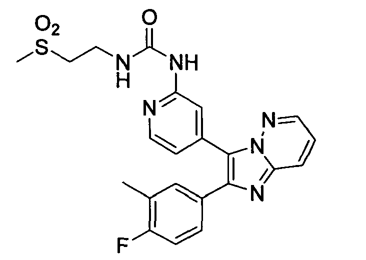

Melting point 204-205°C (ethanol)
MS (ESI+): 469 (M+H).
¹H-NMR (CDCl₃) δ: 2.30 (3H, d, J=1.9Hz), 2.98 (3H, s), 3.39 (2H, t, J=6.3Hz), 3.84-3.95 (2H, m), 6.95-7.05 (1H, m), 7.12 (1H, dd, J=5.5, 1.4Hz), 7.15-7.21 (2H, m), 7.30-7.37 (1H, m), 7.58-7.63 (1H, m), 7.69 (1H, s), 8.04 (1H, dd, J=9.1, 1.7Hz), 8.23 (1H, dd, J=5.5, 0.6Hz), 8.39 (1H, dd, J=4.4, 1.7Hz), 9.82 (1H, brs).
Elemental analysis: for C₂₂H₂₁FN₆O₃S
Calculated: C, 56.40; H, 4.52; N, 17.94.
Found: C, 56.27; H, 4.47; N, 18.07.

### Example compound 51-6: N-{4-[2-(4-fluoro-3-methylphenyl)imidazo[1,2-b]pyridazin-3-yl]pyridin-2-yl}-N'-[4-(methylthio)phenyl]urea

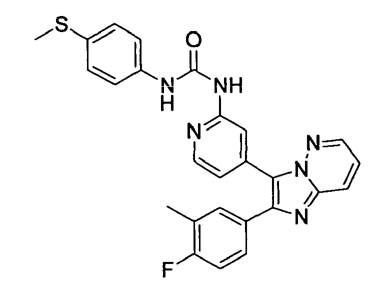

Melting point 219-220°C (ethanol)
MS (ESI+): 485 (M+H).
¹H-NMR (CDCl₃) δ: 2.29 (3H, d, J=1.7Hz), 2.47 (3H, s), 6.96-7.03 (1H, m), 7.09 (1H, dd, J=5.5, 1.5Hz), 7.14-7.22 (3H, m), 7.35-7.40 (1H, m), 7.41 (1H, m), 7.51-7.56 (2H, m), 7.61 (1H, dd, J=7.6, 2.0Hz), 8.05 (1H, dd, J=9.2, 1.7Hz), 8.24-8.29 (2H, m), 8.67 (1H, s), 11.80 (1H, s).
Elemental analysis: for C₂₆H₂₁FN₆OS
Calculated: C, 64.45; H, 4.37; N, 17.34.
Found: C, 64.33; H, 4.43; N, 17.31.

### Example compound 51-7: N-{4-[2-(4-fluoro-3-methylphenyl)imidazo[1,2-b]pyridazin-3-yl]pyridin-2-yl}-N'-(pyridin-3-yl)urea

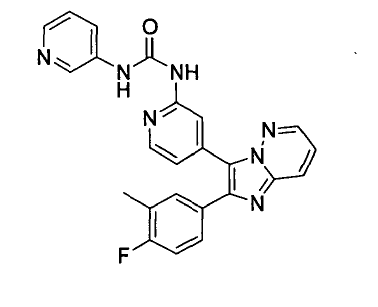

Melting point 233-234°C (ethanol-tetrahydrofuran)
MS (ESI+): 440 (M+H).
¹H-NMR (CDCl₃) δ: 2.29 (3H, d, J=1.7Hz), 6.95-7.03 (1H, m), 7.13 (1H, dd, J=5.5, 1.7Hz), 7.15-7.21 (2H, m), 7.34-7.41 (1H, m), 7.45 (1H, s), 7.59-7.64 (1H, m), 8.32 (1H, dd, J=9.3, 1.7Hz), 8.10-8.16 (1H, m), 8.27-8.30 (1H, m), 8.32 (1H, dd, J=4.7, 1.4Hz), 8.36 (1H, dd, J=4.4, 1.7Hz), 8.72-8.75 (1H, m), 8.99 (1H, s), 12.08 (1H, s).

### Example compound 51-8: N-{4-[2-(4-fluoro-3-methylphenyl)imidazo[1,2-b]pyridazin-3-yl]pyridin-2-yl}-N'-(3-methyl-1H-pyrazol-5-yl)urea

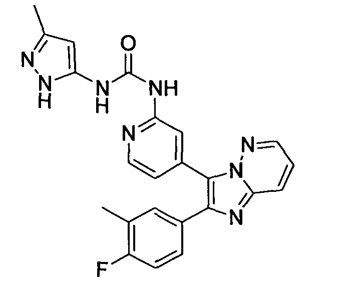

Melting point 239-240°C (ethanol-isopropyl ether)
MS (ESI+): 443 (M+H).
¹H-NMR (DMSO-d₆) δ: 2.19 (3H, s), 2.25 (3H, s), 6.08 (1H, brs), 7.09-7.21 (2H, m), 7.32-7.43 (2H, m), 7.68 (1H, dd, J=7.6, 1.8Hz), 7.84 (1H, brs), 8.25 (1H, dd, J=9.2, 1.4Hz), 8.37 (1H, d, J=5.3Hz), 8.58 (1H, dd, J=4.3, 1.4Hz), 9.22-9.95 (1H, m), 10.04-10.70 (1H, m), 12.00 (1H, m).
Elemental analysis: for C₂₃H₁₉FN₈O
Calculated: C, 62.44; H, 4.33; N, 25.33.
Found: C, 62.31; H, 4.33; N, 25.22.

### Example compound 51-9: N-{4-[2-(4-fluoro-3-methylphenyl)imidazo[1,2-b]pyridazin-3-yl]pyridin-2-yl}urea

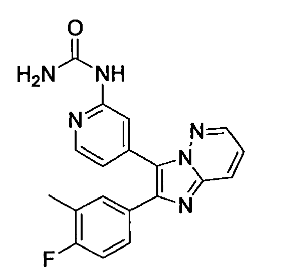

Melting point 254-255°C (ethanol-tetrahydrofuran-water)
MS (ESI+): 363 (M+H).
¹H-NMR (CDCl₃) δ: 2.25 (3H, d, J=1.1Hz), 6.84-7.24 (4H, m), 7.32-7.40 (2H, m), 7.62-7.74 (2H, m), 8.23 (1H, dd, J=9.2, 1.5Hz), 8.30 (1H, d, J=5.3Hz), 8.56 (1H, dd, J=4.5, 1.5Hz), 9.26 (1H, s).
Elemental analysis: for C₁₉H₁₅FN₆O
Calculated: C, 62.98; H, 4.17; N, 23.19.
Found: C, 62.82; H, 4.10; N, 23.03.

### Example compound 51-10: N-{4-[2-(4-fluoro-3-methylphenyl)imidazo[1,2-b]pyridazin-3-yl]pyridin-2-yl}-N'-(4-hydroxycyclohexyl)urea

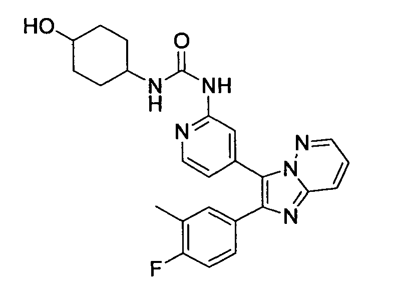

Melting point 218-220°C (ethanol)
MS (ESI+): 461 (M+H).
¹H-NMR (DMSO-d₆) δ: 1.16-1.31 (4H, m), 1.77-1.92 (4H, m), 2.25 (3H, d, J=1.3Hz), 3.38-3.52 (2H, m), 4.55 (1H, d, J=4.3Hz), 7.04 (1H, dd, J=5.3, 1.3Hz), 7.11-7.19 (1H, m), 7.32-7.40 (2H, m), 7.63-7.69 (2H, m), 8.01-8.05 (1H, m), 8.23 (1H, dd, J=9.2, 1.7Hz), 8.27 (1H, d, J=5.3Hz), 8.56 (1H, dd, J=4.5, 1.7Hz), 9.23 (1H, s).
Elemental analysis: for C₂₅H₂₅FN₆O₂
Calculated: C, 65.20; H, 5.47; N, 18.25.
Found: C, 65.02; H, 5.47; N, 18.23.

### Example compound 51-11: N-{4-[2-(4-fluoro-3-methylphenyl)imidazo[1,2-b]pyridazin-3-yl]pyridin-2-yl}-N'-(2-hydroxyethyl)urea

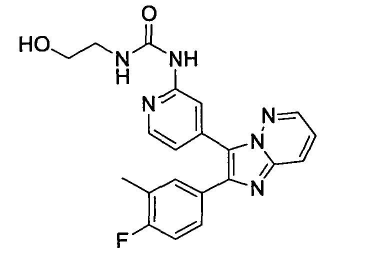

A solution (10 mL) of 2,2,2-trichloroethyl {4-[2-(4-fluoro-3-methylphenyl)imidazo[1,2-b]pyridazin-3-yl]pyridin-2-yl}carbamate (300 mg, 0.61 mmol) obtained in Example 50, 2-aminoethanol (56 mg, 0.92 mmol) and N-ethyldiisopropylamine (120 mg, 0.93 mmol) in dimethylsulfoxide was stirred at room temperature overnight. 5% Aqueous sodium hydrogen carbonate solution was added to the reaction mixture, and the mixture was extracted with ethyl acetate-tetrahydrofuran. The extract was washed with water, dried over anhydrous sodium sulfate, and the solvent was removed by evaporation under reduced pressure. The obtained crude crystals were recrystallized from ethanol to give the title compound (yield 170 mg, 67%).
Melting point 189-190°C (tetrahydrofuran-ethyl acetate)
MS (ESI+): 407 (M+H).
¹H-NMR (CDCl₃) δ: 2.30 (3H, d, J=1.5Hz), 3.17 (1H, t, J=5.3Hz), 3.52-3.59 (2H, m), 3.78-3.85 (2H, m), 6.95-7.02 (1H, m), 7.08 (1H, dd, J=5.5, 1.2Hz), 7.16 (1H, dd, J=9.1, 4.4Hz), 7.21 (1H, s), 7.31-7.37 (1H, m), 7.60 (1H, dd, J=7.4, 1.8Hz), 7.83 (1H, s), 8.04 (1H, dd, J=9.1, 1.6Hz), 8.20 (1H, d, J=5.5Hz), 8.39 (1H, dd, J=4.4, 1.6Hz), 9.60-9.68 (1H, m).
Elemental analysis: for C₂₁H₁₉FN₆O₂·0.2H₂O
Calculated: C, 61.52; H, 4.77; N, 20.50.
Found: C, 61.42; H, 4.81; N, 20.64.

### Example compound 51-12: N-{4-[2-(4-fluoro-3-methylphenyl)imidazo[1,2-b]pyridazin-3-yl]pyridin-2-yl}-N'-(4-hydroxybutyl)urea hydrochloride

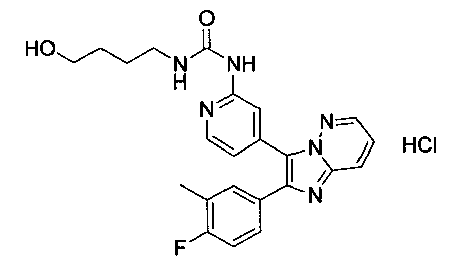

Melting point 188-189°C (ethanol-isopropyl ether)
¹H-NMR (DMSO-d₆) δ: 1.40-1.60 (4H, m), 2.27 (3H, d, J=1.7Hz), 3.13-3.25 (2H, m), 3.37-3.46 (2H, m), 7.15-7.25 (2H, m), 7.39-7.53 (2H, m), 7.66-7.84 (3H, m), 8.27 (1H, d, J=6.1Hz), 8.31 (1H, dd, J=9.1, 1.7Hz), 8.66 (1H, dd, J=4.4, 1.7Hz), 10.60-10.89 (1H, m), 2H (hidden).
Elemental analysis: for C₂₃H₂₄ClFN₆O₂·0.6H₂O
Calculated: C, 57.34; H, 5.27; N, 17.45.
Found: C, 57.19; H, 5.07; N, 17.32.

### Example compound 51-13: N-{4-[2-(4-fluoro-3-methylphenyl)imidazo[1,2-b]pyridazin-3-yl]pyridin-2-yl}-N'-(2-hydroxypropyl)urea

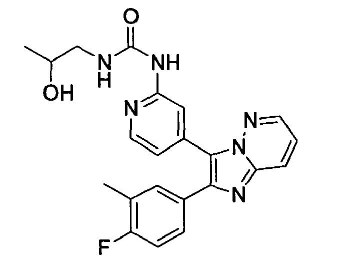

Melting point 139-140°C (ethanol)
MS (ESI+): 421 (M+H).
¹H-NMR (CDCl₃) δ: 1.23 (3H, d, J=6.3Hz), 2.30 (3H, d, J=1.9Hz), 3.21-3.37 (2H, m), 3.45-3.56 (1H, m), 3.97-4.11 (1H, m), 6.94-7.03 (1H, m), 7.05-7.11 (1H, m), 7.16 (1H, dd, J=9.3, 4.4Hz), 7.22 (1H, s), 7.30-7.38 (1H, m), 7.60 (1H, dd, J=7.7, 1.7Hz), 7.85 (1H, s), 8.04 (1H, dd, J=9.3, 1.7Hz), 8.16-8.22 (1H, s), 8.39 (1H, dd, J=4.4, 1.7Hz), 9.62 (1H, brs).
Elemental analysis: for C₂₂H₂₁FN₆O₂·0.5H₂O
Calculated: C, 61.53; H, 5.16; N, 19.57.
Found: C, 61.69; H, 5.08; N, 19.70.

### Example compound 51-14: N-(2-cyanoethyl)-N'-{4-[2-(4-fluoro-3-methylphenyl)imidazo[1,2-b]pyridazin-3-yl]pyridin-2-yl}urea

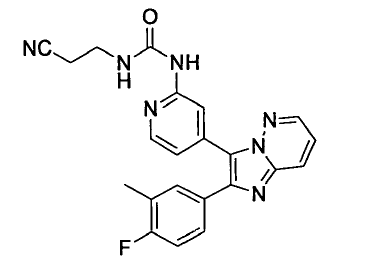

A solution (10 mL) of 2,2,2-trichloroethyl {4-[2-(4-fluoro-3-methylphenyl)imidazo[1,2-b]pyridazin-3-yl]pyridin-2-yl}carbamate (300 mg, 0.61 mmol) obtained in Example 50, 3-aminopropionitrile (64 mg, 0.91 mmol) and N-ethyldiisopropylamine (120 mg, 0.93 mmol) in dimethylsulfoxide was stirred at 80°C overnight. 5% Aqueous sodium hydrogen carbonate solution was added to the reaction mixture, and the mixture was extracted with ethyl acetate-tetrahydrofuran. The extract was washed with water, dried over anhydrous sodium sulfate, and the solvent was removed by evaporation under reduced pressure. The obtained residue was purified by silica gel chromatography (ethyl acetate: hexane = 5:1 - 9:1) and the obtained crude crystals were recrystallized from ethanol-isopropylether to give the title compound (yield 190 mg, 74%).
Melting point 213-214°C (ethanol-isopropyl ether)
¹H-NMR (CDCl₃) δ: 2.31 (3H, d, J=1.9Hz), 2.72 (2H, t, J=6.5Hz), 3.62-3.70 (2H, m), 6.97-7.04 (1H, m), 7.12 (1H, dd, J=5.5, 1.4Hz), 7.15-7.20 (2H, m), 7.32-7.38 (1H, m), 7.61 (1H, dd, J=7.4, 1.5Hz), 7.78 (1H, s), 8.04 (1H, dd, J=9.2, 1.7Hz), 8.23 (1H, d, J=5.5Hz), 8.38 (1H, dd, J=4.3, 1.7Hz), 9.83-9.91 (1H, m) .
Elemental analysis: for C₂₂H₁₈FN₇O
Calculated: C, 63.61; H, 4.37; N, 23.60.
Found: C, 63.39; H, 4.39; N, 23.44.

### Example compound 51-15: N-{4-[2-(4-fluoro-3-methylphenyl)imidazo[1,2-b]pyridazin-3-yl]pyridin-2-yl}-N'-(tetrahydro-2H-pyran-4-yl)urea

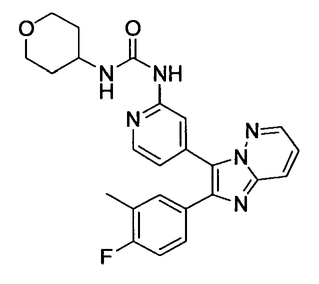

Melting point 240-241°C (ethanol)
MS (ESI+): 447 (M+H).
¹H-NMR (CDCl₃) δ: 1.58-1.73 (2H, m), 1.97-2.07 (2H, m), 2.30 (3H, d, J=1.9Hz), 3.49-3.59 (2H, m), 3.94-4.10 (3H, m), 6.99 (1H, t, J=8.9Hz), 7.09 (1H, dd, J=5.5, 1.4Hz), 7.12 (1H, s), 7.16 (1H, dd, J=9.3, 4.4Hz), 7.31-7.38 (1H, m), 7.45 (1H, s), 7.61 (1H, dd, J=7.1, 1.7Hz), 8.04 (1H, dd, J=9.2, 1.7Hz), 8.22 (1H, d, J=5.5Hz), 8.37 (1H, dd, J=4.4, 1.7Hz), 9.44 (1H, d, J=7.4Hz).
Elemental analysis: for C₂₄H₂₃FN₆O₂·0.5H₂O
Calculated: C, 63.29; H, 5.31; N, 18.45.
Found: C, 63.43; H, 5.36; N, 18.12.

### Example compound 51-16: N-{4-[2-(4-fluoro-3-methylphenyl)imidazo[1,2-b]pyridazin-3-yl]pyridin-2-yl}morpholine-4-carboxamide hydrochloride

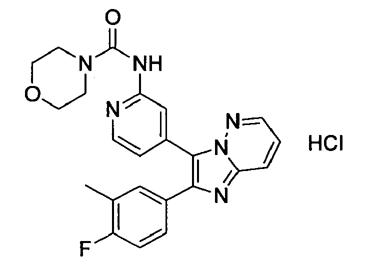

Melting point 164-166°C (ethanol-isopropyl ether) ¹H-NMR (DMSO-d₆) δ: 2.27 (3H, d, J=1.3Hz), 3.50-3.57 (4H, m), 3.59-3.67 (4H, m), 7.15-7.23 (1H, m), 7.27-7.31 (1H, m), 7.39-7.49 (2H, m), 7.68 (1H, dd, J=7.5, 1.7Hz), 8.17 (1H, d, J=1.1Hz), 8.30 (1H, dd, J=9.2, 1.5Hz), 8.34 (1H, d,J=6.0 Hz), 8.64-8.66 (1H, m), 10.35 (1H, brs), hidden (1H).
Elemental analysis: for C₂₃H₂₂ClFN₆O₂
Calculated: C, 58.91; H, 4.73; N, 17.92.
Found: C, 58.52; H, 4.60; N, 17.94.

### Example compound 51-17: N-{4-[2-(4-fluoro-3-methylphenyl)imidazo[1,2-b]pyridazin-3-yl]pyridin-2-yl}-4-hydroxypiperidine-1-carboxamide hydrochloride

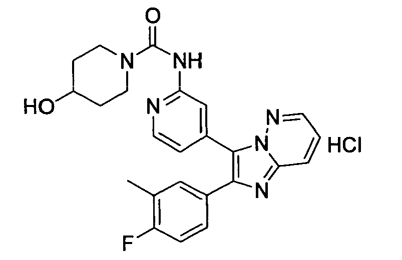

Melting point 156-157°C (ethanol-isopropyl ether)
¹H-NMR (DMSO-d₆) δ: 1.32-1.48 (2H, m), 1.73-1.85 (2H, m), 2.28 (3H, d, J=1.1Hz), 3.23-3.35 (2H, m), 3.71-3.80 (1H, m), 3.85-3.99 (2H, m), 7.16-7.27 (1H, m), 7.37 (1H, dd, J=6.6, 1.7Hz), 7.44-7.57 (2H, m), 7.68 (1H, dd, J=7.5, 1.5Hz), 8.25-8.39 (3H, m), 8.71 (1H, dd, J=4.5, 1.5Hz), 11.00 (1H, s), 2H (hidden).

### (Example 52)

### N-{4-[2-(3-Methylphenyl)imidazo[1,2-b]pyridazin-3-yl]pyridin-2-yl}-N'-(tetrahydro-2H-pyran-4-yl)urea

The compound was synthesized in the same manner as in Example 31 and using tetrahydro-2H-pyran-4-amine instead of piperidine. 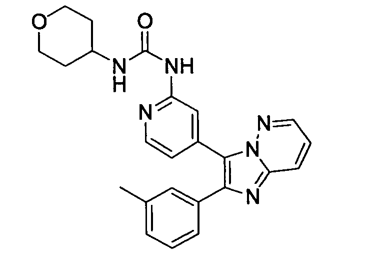 Melting point 222-222°C (ethanol)
¹H-NMR (CDCl₃) δ: 1.58-1.73 (2H, m), 1.97-2.07 (2H, m), 2.39 (3H, s), 3.49-3.61 (2H, m), 3.93-4.11 (3H, m), 6.94 (1H, s), 7.04 (1H, s), 7.12-7.20 (2H, m), 7.20-7.26 (2H, m), 7.35 (1H, d, J=7.2Hz), 7.58 (1H, s), 8.05 (1H, dd, J=9.2, 1.7Hz), 8.22 (1H, d, J=5.5Hz), 8.37 (1H, dd, J=4.4, 1.6Hz), 9.44 (1H, d, J=7.9Hz).
Elemental analysis: for C₂₄H₂₄N₆O₂
Calculated: C, 67.27; H, 5.65; N, 19.61.
Found: C, 67.35; H, 5.70; N, 19.74.

### (Example 53)

### N-{4-[2-(4-Fluoro-3-methylphenyl)-6-methoxyimidazo[1,2-b]pyridazin-3-yl]pyridin-2-yl}-N'-isopropylurea

The compound was synthesized in the same manner as in Example 9 using N-{4-[6-chloro-2-(4-fluoro-3-methylphenyl)imidazo[1,2-b]pyridazin-3-yl]pyridin-2-yl}-N'-isopropylurea (Example compound 49) instead of 4-[6-chloro-2-(3-methylphenyl)imidazo[1,2-b]pyridazin-3-yl]-N-cyclohexylpyridin-2-amine. 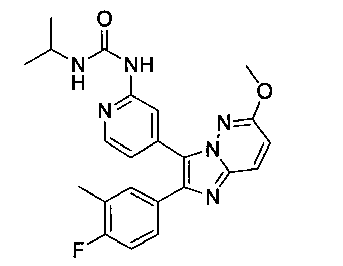 Melting point 233-234°C (ethanol)
MS (ESI+): 435 (M+H).
¹H-NMR (CDCl₃) δ: 1.27 (6H, d, J=6.6Hz), 2.29 (3H, d, J=1.7Hz), 3.99 (3H, s), 3.99-4.13 (1H, m), 6.81 (1H, d, J=9.6Hz), 6.93-7.00 (1H, m), 7.02 (1H, dd, J=5.5, 1.5Hz), 7.22 (1H, s), 7.27-7.34 (1H, m), 7.53-7.56 (1H, m), 7.86 (1H, d, J=9.6Hz), 7.90 (1H, s), 8.15 (1H, d, J=5.1Hz), 9.24 (1H, d, J=7.4Hz).
Elemental analysis: for C₂₃H₂₃FN₆O₂
Calculated: C, 63.58; H, 5.34; N, 19.34.
Found: C, 63.57; H, 5.42; N, 19.32.

### (Example 54)

### N-{4-[2-(4-Fluoro-3-methylphenyl)-6-(methylthio)imidazo[1,2-b]pyridazin-3-yl]pyridin-2-yl}-N'-isopropylurea

The compound was synthesized in the same manner as in Example 10 using N-{4-[6-chloro-2-(4-fluoro-3-methylphenyl)imidazo[1,2-b]pyridazin-3-yl]pyridin-2-yl}-N'-isopropylurea (Example compound 49) instead of 4-[6-chloro-2-(3-methylphenyl)imidazo[1,2-b]pyridazin-3-yl]-N-cyclohexylpyridin-2-amine. 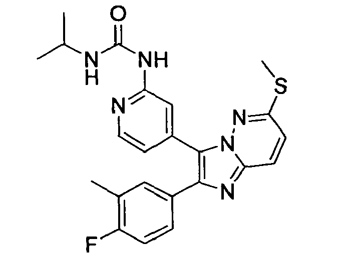 Melting point 241-242°C (ethanol)
MS (ESI+): 451 (M+H).
¹H-NMR (CDCl₃) δ: 1.27 (6H, d, J=6.6Hz), 2.29 (3H, d, J=1.7Hz), 2.58 (3H, s), 4.01-4.02 (1H, m), 6.94-7.00 (2H, m), 7.02 (1H, dd, J=5.5, 1.3Hz), 7.22 (1H, s), 7.28-7.35 (1H, m), 7.57 (1H, dd, J=7.4, 1.5Hz), 7.78 (1H, d, J=9.4Hz), 8.07 (1H, s), 8.15 (1H, d, J=5.5Hz), 9.24 (1H, d, J=7.4Hz).
Elemental analysis: for C₂₃H₂₃FN₆OS
Calculated: C, 61.32; H, 5.15; N, 18.65.
Found: C, 61.38; H, 5.19; N, 18.72.

### (Example 55)

### 1-{4-[2-(4-Fluoro-3-methylphenyl)imidazo[1,2-b]pyridazin-3-yl]pyridin-2-yl}tetrahydropyrimidine-2(1H)-one

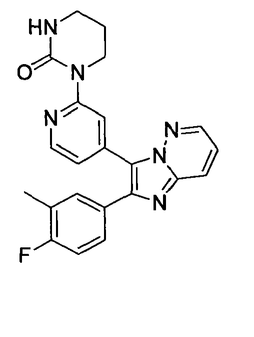

To a solution of N-(3-chloropropyl)-N'-{4-[2-(4-fluoro-3-methylphenyl)imidazo[1,2-b]pyridazin-3-yl]pyridin-2-yl}urea (250 mg, 0.570 mmol) obtained in Example 47-4 in tetrahydrofuran (5ml) was added potassium t-butoxide (90 mg, 0.68 mmol) at 0°C, and the mixture was stirred at 0°C for 1 hr. The solvent was removed by evaporation under reduced pressure. The obtained residue was washed with water and recrystallized from ethanol to give the title compound (yield 126 mg, 55%).
Melting point 213-215°C (ethanol)
MS (ESI+): 403 (M+H).
¹H-NMR (CDCl₃) δ: 2.06-2.16 (2H, m), 2.28 (3H, d, J=1.7Hz), 3.39-3.46 (2H, m), 4.05-4.12 (2H, m), 4.99 (1H, s), 6.92-7.00 (1H, m), 7.07-7.14 (2H, m), 7.37-7.44 (1H, m), 7.66 (1H, dd, J=7.4, 1.6Hz), 8.01 (1H, dd, J=9.0, 1.7Hz), 8.36 (1H, dd, J=4.5, 1.7Hz), 8.37-8.43 (2H, m).
Elemental analysis: for C₂₂H₁₉FN₆O·0.5H₂O
Calculated: C, 64.22; H, 4.90; N, 20.43.
Found: C, 64.45; H, 4.99; N, 20.51.

### (Example 56)

### N-{4-[2-(4-Fluoro-3-methylphenyl)imidazo[1,2-b]pyridazin-3-yl]pyridin-2-yl}-3-(methylsulfonyl)propanamide

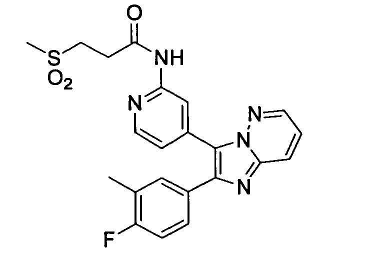

To a solution of N-{4-[2-(4-fluoro-3-methylphenyl)imidazo[1,2-b]pyridazin-3-yl]pyridin-2-yl}-3-(methylthio)propanamide (0.35 g, 0.83 mmol) obtained in Example 42-5 in DMF (5 mL) was added 70% m-chloroperbenzoic acid (0.38 g, 1.7 mmol), and the mixture was stirred at room temperature for 4 hr. Aqueous sodium hydrogen carbonate solution was added to the mixture, and the mixture was extracted with ethyl acetate-THF. The extract was dried over anhydrous sodium sulfate and concentrated. The obtained residue was purified by silica gel chromatography (ethyl acetate) and the obtained crude crystals were recrystallized from ethanol to give the title compound (yield 0.24 g, 63%).
Melting point 227-228°C (ethanol)
¹H-NMR (CDCl₃) δ: 2.29 (3H, d, J=1.6Hz), 2.98 (3H, s), 3.03 (2H, t, J=7.7Hz), 3.48 (2H, t, J=7.7Hz), 6.97 (1H, t, J=8.9Hz), 7.16 (1H, dd, J=9.1, 4.4Hz), 7.26 (1H, dd, J=5.2, 1.4Hz), 7.32-7.38 (1H, m), 7.59-7.64 (1H, m), 8.04 (1H, dd, J=9.1, 1.7Hz), 8.35 (1H, d, J=5.2Hz), 8.39 (1H, dd, J=4.4, 1.7Hz), 8.52 (1H, s), 8.56 (1H, s).
Elemental analysis: for C₂₂H₂₀FN₅O₃S
Calculated: C, 58.27; H, 4.45; N, 15.44.
Found: C, 58.21; H, 4.46; N, 15.35.

### (Example 57)

The compounds of Examples 57-1 and 57-2 below were synthesized in the same manner as in Example 56 respectively using N-{4-[2-(4-fluoro-3-methylphenyl)imidazo[1,2-b]pyridazin-3-yl]pyridin-2-yl}-2-(methylthio)acetamide (Example compound 42-6) or 4-[6-chloro-2-(4-fluoro-3-methylphenyl)-8-(methylthio)imidazo[1,2-b]pyridazin-3-yl]-2-pyridylamine (Example compound 36-4) instead of N-{4-[2-(4-fluoro-3-methylphenyl)imidazo[1,2-b]pyridazin-3-yl]pyridin-2-yl}-3-(methylthio)propanamide.

### Example compound 57-1: N-{4-[2-(4-fluoro-3-methylphenyl)imidazo[1,2-b]pyridazin-3-yl]pyridin-2-yl}-2-(methylsulfonyl)acetamide

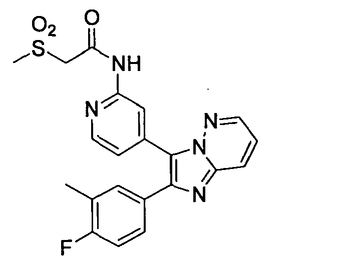

Melting point 240-241°C (ethanol)
¹H-NMR (CDCl₃) δ: 1.57 (3H, s), 2.29 (3H, d, J=1.9Hz), 4.06 (2H, s), 6.98 (1H, t, J=9.1Hz), 7.17 (1H, dd, J=9.1, 4.4Hz), 7.32 (1H, dd, J=5.2, 1.4Hz), 7.33-7.39 (1H, m), 7.58-7.64 (1H, m), 8.04 (1H, dd, J=9.1, 1.6Hz), 8.37-8.42 (2H, m), 8.55 (1H, s), 8.90 (1H, s).
Elemental analysis: for C₂₁H₁₈FN₅O₃S·0.25H₂O
Calculated: C, 56.81; H, 4.20; N, 15.77.
Found: C, 56.85; H, 4.14; N, 15.78.

### Example compound 57-2: 4-[6-chloro-2-(4-fluoro-3-methylphenyl)-8-(methylsulfinyl)imidazo[1,2-b]pyridazin-3-yl]-2-pyridylamine

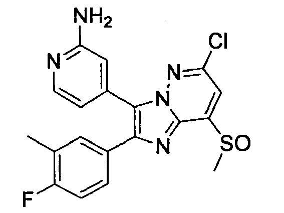

Melting point 216-217°C (ethanol)
¹H-NMR (CDCl₃) δ: 2.29 (3H, d, J=1.9Hz), 3.27 (3H, s), 4.58 (2H, brs), 6.76-6.81 (2H, m), 6.98 (1H, t, J=8.9Hz), 7.34-7.41 (1H, m), 7.54-7.59 (1H, m), 7.64 (1H, s), 8.18 (1H, dd, J=5.2, 0.9Hz).

### (Example 58)

### N-{4-[2-(4-Fluoro-3-methylphenyl)imidazo[1,2-b]pyridazin-3-yl]pyridin-2-yl}-2-methylisonicotinamide

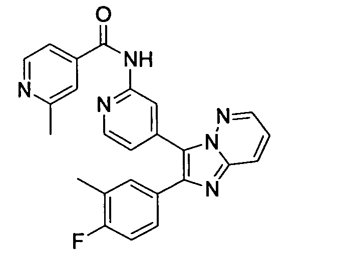

2-Chloro-N-{4-[2-(4-fluoro-3-methylphenyl)imidazo[1,2-b]pyridazin-3-yl]pyridin-2-yl}-6-methylisonicotinamide (9.0 g, 19 mmol) obtained in Example 42-31, triethylamine (3.9 g, 38 mmol) and 10% palladium carbon powder (50% water-containing product, 1.8 g) were stirred in dimethylformamide solvent (45 mL) under hydrogen atmosphere of 0.5 MPa at 50°C for 8 hr. The reaction mixture was allowed to be cooled to room temperature and filtrated, and the solvent was removed by evaporation under reduced pressure. Saturated aqueous sodium hydrogen carbonate solution was added to the residue, and the mixture was extracted with mixed solvent (tetrahydrofuran : ethyl acetate = 1:1). The extract was washed with saturated brine, dried over anhydrous sodium sulfate, and the solvent was removed by evaporation under reduced pressure. The obtained residue was purified by basic silica gel chromatography (ethyl acetate : hexane = 3:7 - 8:2) and the obtained crystals were recrystallized from ethanol to give the title compound (yield 4.4 g, 52%).
Melting point 200-202°C (ethanol)
MS (ESI+): 439 (M+H).
¹H-NMR (CDCl₃) δ: 2.30 (3H, d, J=1.5Hz), 2.68 (3H, s), 6.95-7.02 (1H, m), 7.17 (1H, dd, J=9.1, 4.4Hz), 7.23-7.28 (1H, m), 7.35-7.41 (1H, m), 7.53-7.56 (1H, m), 7.61-7.66 (2H, m), 8.05 (1H, dd, J=9.1, 1.6Hz), 8.36 (1H, d, J=5.3Hz), 8.41 (1H, dd, J=4.4, 1.6Hz), 8.66-8.73 (2H, m), 8.84 (1H, s).
Elemental analysis: for C₂₅H₁₉FN₆O
Calculated: C, 68.48; H, 4.37; N, 19.17.
Found: C, 68.10; H, 4.25; N, 19.03.

### (Example 59)

### N-{4-[2-(4-Fluoro-3-methylphenyl)imidazo[1,2-b]pyridazin-3-yl]pyridin-2-yl}nicotinamide

The compound was synthesized in the same manner as in Example 58 using 2-chloro-N-{4-[2-(4-fluoro-3-methylphenyl)imidazo[1,2-b]pyridazin-3-yl]pyridin-2-yl}nicotinamide (Example compound 42-22) instead of 2-chloro-N-{4-[2-(4-fluoro-3-methylphenyl)imidazo[1,2-b]pyridazin-3-yl]pyridin-2-yl}-6-methylisonicotinamide. 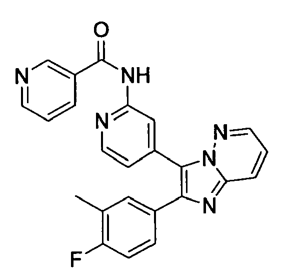 Melting point 221-222°C (ethanol-tetrahydrofuran-water)
MS (ESI+): 425 (M+H).
¹H-NMR (CDCl₃) δ: 2.29 (3H, d, J=1.7Hz), 6.95-7.01 (1H, m), 7.17 (1H, dd, J=9.1, 4.4Hz), 7.22 (1H, dd, J=5.2, 1.7Hz), 7.35-7.41 (1H, m), 7.44-7.49 (1H, m), 7.64 (1H, dd, J=7.4, 1.9Hz), 8.05 (1H, dd, J=9.1, 1.7Hz), 8.22-8.27 (1H, m), 8.30-8.34 (1H, m), 8.41 (1H, dd, J=4.4, 1.7Hz), 8.81 (1H, dd, J=4.7, 1.7Hz), 8.84-8.87 (1H, m), 8.89 (1H, s), 9.18 (1H, d, J=1.9Hz).
Elemental analysis: for C₂₄H₁₇FN₆O
Calculated: C, 67.92; H, 4.04; N, 19.80.
Found: C, 67.59; H, 3.99; N, 19.59.

### (Example 60)

### 4-Chloro-N-{4-[2-(4-fluoro-3-methylphenyl)imidazo[1,2-b]pyridazin-3-yl]pyridin-2-yl}benzamide hydrochloride

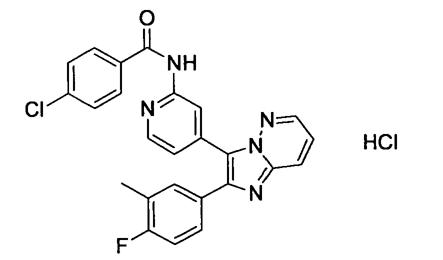

To a solution of 4-chloro-N-{4-[2-(4-fluoro-3-methylphenyl)imidazo[1,2-b]pyridazin-3-yl]pyridin-2-yl}benzamide (100 mg, 0.218 mmol) obtained in Example 21-17 in ethyl acetate (2 mL) was added a 4N solution of hydrogen chloride-ethyl acetate (170 µL, 0.680 mmol), and the solvent was removed by evaporation under reduced pressure. The obtained residue was recrystallized from ethanol-tetrahydrofuran to give the title compound (yield 77 mg, 72%).
Melting point 264-266°C (ethanol-tetrahydrofuran)
MS (ESI+): 458 (M+H).
¹H-NMR (CDCl₃) δ: 2.33 (3H, d, J=1.7Hz), 7.06-7.13 (1H, m), 7.32-7.40 (2H, m), 7.51-7.57 (5H, m), 8.07 (1H, d, J=6.6Hz), 8.14 (1H, dd, J=9.2, 1.2Hz), 8.28 (2H, d, J=8.5Hz), 8.53-8.65 (1H, m), 9.62 (1H, s), 12.19 (1H, s).
Elemental analysis: for C₂₅H₁₈Cl₂FN₅O
Calculated: C, 60.74; H, 3.67; N, 14.17.
Found: C, 60.63; H, 3.69; N, 14.10.

### (Example 61)

The compounds of Examples 61-1 - 61-6 below were synthesized in the same manner as in Example 60 respectively using N-{4-[2-(3-chlorophenyl)imidazo[1,2-b]pyridazin-3-yl]pyridin-2-yl}-4-methoxybenzamide (Example compound 21-3), 4-methyl-N-{4-[2-(3-methylphenyl)imidazo[1,2-b]pyridazin-3-yl]pyridin-2-yl}benzamide (Example compound 20-1), N-{4-[2-(4-fluoro-3-methylphenyl)imidazo[1,2-b]pyridazin-3-yl]pyridin-2-yl}-4-methoxybenzamide (Example compound 21-16), N-{4-[2-(4-fluoro-3-methylphenyl)imidazo[1,2-b]pyridazin-3-yl]pyridin-2-yl}-2-methylisonicotinamide (Example compound 58), 6-chloro-N-{4-[2-(4-fluoro-3-methylphenyl)imidazo[1,2-b]pyridazin-3-yl]pyridin-2-yl}nicotinamide (Example compound 42-24) or N-{4-[2-(4-fluoro-3-methylphenyl)imidazo[1,2-b]pyridazin-3-yl]pyridin-2-yl}nicotinamide (Example compound 59), instead of 4-chloro-N-{4-[2-(4-fluoro-3-methylphenyl)imidazo[1,2-b]pyridazin-3-yl]pyridin-2-yl}benzamide.

### Example compound 61-1: N-{4-[2-(3-chlorophenyl)imidazo[1,2-b]pyridazin-3-yl]pyridin-2-yl}-4-methoxybenzamide hydrochloride

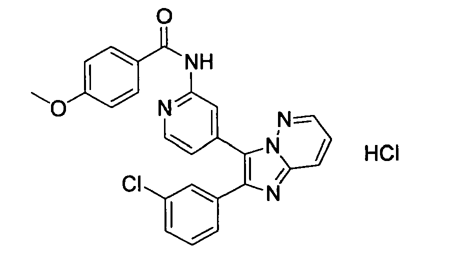

Melting point 236-238°C (ethanol-tetrahydrofuran)
MS (ESI+): 456 (M+H).
¹H-NMR (DMSO-d₆) δ: 3.85 (3H, s), 7.07 (2H, d, J=8.9Hz), 7.37-7.49 (4H, m), 7.54-7.60 (1H, m), 7.74-7.78 (1H, m), 8.06 (2H, d, J=8.9Hz), 8.32 (1H, dd, J=9.2, 1.5Hz), 8.50 (1H, s), 8.53 (1H, d, J=5.5Hz), 8.64 (1H, dd, J=4.5, 1.5Hz), 11.16 (1H, s), hidden (1H).
Elemental analysis: for C₂₅H₁₉Cl₂N₅O₂
Calculated: C, 60.99; H, 3.89; N, 14.22.
Found: C, 60.77; H, 4.12; N, 14.11.

### Example compound 61-2: 4-methyl-N-{4-[2-(3-methylphenyl)imidazo[1,2-b]pyridazin-3-yl]pyridin-2-yl}benzamide hydrochloride

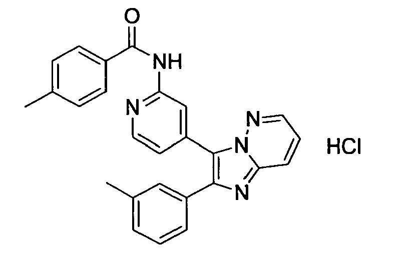

Melting point 263-264°C (ethanol)
MS (ESI+): 420 (M+H).
¹H-NMR (DMSO-d₆) δ: 2.33 (3H, s), 2.40 (3H, s), 7.21-7.26 (1H, m), 7.28-7.33 (1H, m), 7.34-7.42 (4H, m), 7.49 (1H, dd, J=9.2, 4.5Hz), 7.60 (1H, s), 7.98 (2H, d, J=8.1Hz), 8.33 (1H, dd, J=9.2, 1.5Hz), 8.51 (1H, d, J=5.5Hz), 8.55 (1H, d, J=0.9Hz), 8.68 (1H, dd, J=9.2, 1.5Hz), 11.35 (1H, s), hidden (1H).
Elemental analysis: for C₂₆H₂₂ClN₅O
Calculated: C, 68.49; H, 4.86; N, 15.36.
Found: C, 68.34; H, 4.85; N, 15.28.

### Example compound 61-3: N-{4-[2-(4-fluoro-3-methylphenyl) imidazo[1,2-b]pyridazin-3-yl]pyridin-2-yl}-4-methoxybenzamide hydrochloride

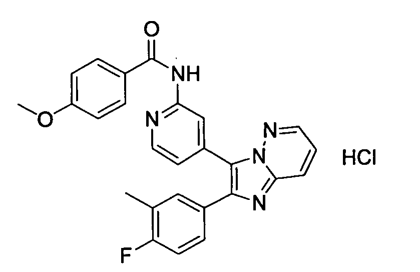

Melting point 233-234°C (ethanol)
MS (ESI+): 454 (M+H).
¹H-NMR (DMSO-d₆) δ: 2.33 (3H, d, J=1.5Hz), 3.89 (3H, s), 7.01-7.12 (3H, m), 7.32 (1H, dd, J=9.2, 4.5Hz), 7.35-7.41 (1H, m), 7.44-7.46 (1H, m), 7.56 (1H, dd, J=7.2, 1.5Hz), 8.06 (1H, d, J=6,4Hz), 8.12 (1H, dd, J=9.2, 1.3Hz), 8.29 (2H, d, J=8.9Hz), 8.57 (1H, dd, J=4.5, 1.3Hz), 9.60 (1H, s), 11.82 (1H, s), hidden (1H).
Elemental analysis: for C₂₆H₂₁ClFN₅O₂·0.4H₂O
Calculated: C, 62.82; H, 4.42; N, 14.09.
Found: C, 62.81; H, 4.66; N, 13.90.

### Example compound 61-4: N-{4-[2-(4-fluoro-3-methylphenyl)imidazo[1,2-b]pyridazin-3-yl]pyridin-2-yl}-2-methylisonicotinamide dihydrochloride

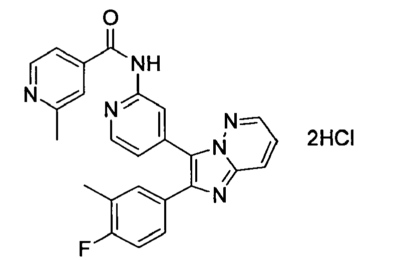

MS (ESI+): 439 (M+H).
¹H-NMR (DMSO-d₆) δ : 2.25 (3H, d, J=1.3Hz), 2.77 (3H, s), 7.13-7.22 (1H, m), 7.38-7.47 (3H, m), 7.69 (1H, dd, J=7.4, 1.8Hz), 8.15 (1H, dd, J=5.8, 1.1Hz), 8.26 (1H, s), 8.29 (1H, dd, J=9.2, 1.5Hz), 8.49-8.51 (1H, m), 8.54-8.58 (1H, m), 8.62 (1H, dd, J=4.5, 1.5Hz), 8.88 (1H, d, J=5.8Hz), 11.62 (1H, s), hidden (2H).
Elemental analysis: for C₂₅H₂₁Cl₂FN₆O·1.5H₂O
Calculated: C, 55.77; H, 4.49; N, 15.61; C1, 13.17; F, 3.53.
Found: C, 55.96; H, 4.47; N, 15.66; C1, 12.95; F, 3.65.

### Example compound 61-5: 6-chloro-N-{4-[2-(4-fluoro-3-methylphenyl)imidazo[1,2-b]pyridazin-3-yl]pyridin-2-yl}nicotinamide hydrochloride

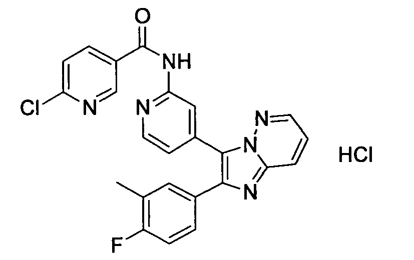

Melting point 250-251°C (ethanol)
¹H-NMR (DMSO-d₆) δ: 2.25 (3H, d, J=1.7Hz), 7.11-7.22 (1H, m), 7.34 (1H, dd, J=5.2, 1.7Hz), 7.36-7.47 (2H, m), 7.64-7.73 (2H, m), 8.28 (1H, dd, J=9.1, 1.7Hz), 8.37 (1H, dd, J=8.4, 2.6Hz), 8.48-8.55 (2H, m), 8.61 (1H, dd, J=4.4, 1.7 Hz), 8.94-9.00 (1H, m), 11.41 (1H, s), hidden (1H).
Elemental analysis: for C₂₄H₁₇Cl₂FN₆O
Calculated: C, 58.19; H, 3.46; N, 16.97; Cl, 14.31.
Found: C, 57.96; H, 3.60; N, 16.90; Cl, 14.24.

### Example compound 61-6: N-{4-[2-(4-fluoro-3-methylphenyl)imidazo[1,2-b]pyridazin-3-yl]pyridin-2-yl}nicotinamide dihydrochloride

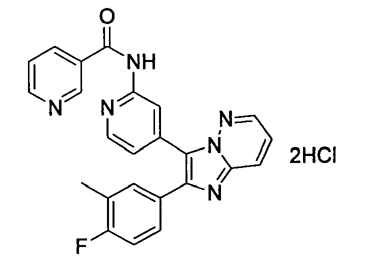

¹H-NMR (DMSO-d₆) δ: 2.26 (3H, d, J=1.4Hz), 7.16-7.25 (1H, m), 7.37-7.54 (3H, m), 7.70 (1H, dd, J=7.4, 1.7Hz), 7.95 (1H, dd, J=8.1, 5.4Hz), 8.34 (1H, dd, J=9.3, 1.7Hz), 8.53-8.59 (2H, m), 8.68 (1H, dd, J=4.4, 1.7Hz), 8.74-8.80 (1H, m), 8.98 (1H, dd, J=5.4, 1.5Hz), 9.34 (1H, d, J=1.9Hz), 11.72 (1H, s), hidden (2H).
Elemental analysis: for C₂₄H₁₉Cl₂FN₆O·2H₂O
Calculated: C, 54.04; H, 4.35; N, 15.76; C1, 13.29.
Found: C, 53.84; H, 4.55; N, 15.64; C1, 13.14.

### (Example 62)

### 4-Chloro-N-{4-[2-(4-fluoro-3-methylphenyl)imidazo[1,2-b]pyridazin-3-yl]pyridin-2-yl}benzamide methanesulfonate

The compound was synthesized in the same manner as in Example 60 using methanesulfonic acid instead of 4N hydrogen chloride-ethyl acetate solution. 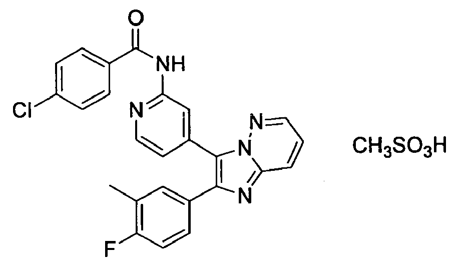 Melting point 239-241°C (ethanol)
MS (ESI+): 458 (M+H).
¹H-NMR (DMSO-d₆) δ: 2.26 (3H, s), 2.36 (3H, s), 7.16-7.22 (1H, m), 7.37-7.49 (3H, m), 7.62 (2H, d, J=8.7Hz), 7.69 (1H, dd, J=7.5, 1.5Hz), 8.04 (2H, d, J=8.7Hz), 8.31 (1H, dd, J=9.0, 1.5Hz), 8.47 (1H, s), 8.52 (1H, d, J=5.5Hz), 8.65 (1H, dd, J=4.5, 1.5Hz), 11.32 (1H, s), hidden (1H).
Elemental analysis: for C₂₆H₂₁ClFN₅O₄S·0.5H₂O
Calculated: C, 55.47; H, 3.94; N, 12.44.
Found: C, 55.62; H, 3.97; N, 12.40.

### (Example 63)

### N-{4-[2-(4-Fluoro-3-methylphenyl)imidazo[1,2-b]pyridazin-3-yl]pyridin-2-yl}-N'-(2-oxopropyl)urea

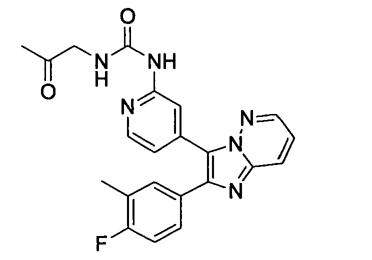

To a solution of dimethyl sulfoxide (140 µl, 1.9 mmol) in dichloromethane (1.0 ml) was added oxalyl chloride (78 µl, 0.89 mmol) at -50°C, and the mixture was stirred at -50°C for 2 min. To the reaction mixture was added a solution of N-{4-[2-(4-fluoro-3-methylphenyl)imidazo[1,2-b]pyridazin-3-yl]pyridin-2-yl}-N'-(2-hydroxypropyl)urea (170 mg, 0.40 mmol) obtained in Example 51-13 in dichloromethane (2.0 ml) at -50°C, and the mixture was further stirred at 0°C for 15 min. Triethylamine (560 µl, 4.0 mmol) was added to the reaction mixture at 0°C, and the mixture was further stirred at room temperature for 1 hr. Water was added to the reaction mixture, and the mixture was extracted with ethyl acetate-tetrahydrofuran (1:1) mixed solvent. The extract was dried over anhydrous sodium sulfate, and the solvent was removed by evaporation under reduced pressure. The obtained residue was purified by silica gel chromatography (ethyl acetate:methanol=10:0 - 8.5:1.5) and recrystallized from ethanol to give the title compound (yield 86 mg, 51%).
Melting point 208-209°C (ethanol)
MS (ESI+): 419 (M+H).
¹H-NMR (CDCl₃) δ: 2.23 (3H, s), 2.30 (3H, d, J=1.7Hz), 4.29 (2H, d, J=5.1Hz), 6.95-7.03 (1H, m, J=9.0Hz), 7.11 (1H, dd, J=5.2, 1.4Hz), 7.13-7.19 (2H, m), 7.31-7.39 (1H, m), 7.60 (1H, dd, J=7.4, 1.6Hz), 7.65 (1H, s), 8.03 (1H, dd, J=9.0, 1.7Hz), 8.27 (1H, d, J=5.2Hz), 8.37 (1H, dd, J=4.4, 1.7Hz), 9.81 (1H, s).
Elemental analysis: for C₂₂H₁₉FN₆O₂
Calculated: C, 63.15; H, 4.58; N, 20.08.
Found: C, 62.92; H, 4.63; N, 20.01.

### (Example 64)

### 6-Chloro-3-(2-ethoxypyridin-4-yl)-2-(3-methylphenyl)imidazo[1,2-b]pyridazine

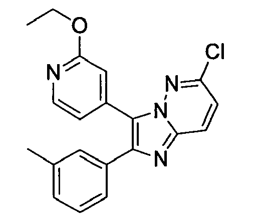

2-Bromo-2-(2-fluoropyridin-4-yl)-1-(3-methylphenyl)ethanone (1.1 g, 2.8 mmol) obtained in Reference Example 1 and 3-amino-6-chloropyridazine (0.30 g, 2.3 mmol) were heated under reflux for 24 hr in ethanol (20 mL). The reaction mixture was allowed to be cooled to room temperature, the solvent was removed by evaporation under reduced pressure. Saturated aqueous sodium hydrogen carbonate solution was added to the residue, and the mixture was extracted with ethyl acetate. The extract was washed with water and dried over anhydrous sodium sulfate, and the solvent was removed by evaporation under reduced pressure. The obtained crude crystals were purified by silica gel column chromatography (ethyl acetate: hexane = 1:9 - 3:7) to give the title compound (yield 30 mg, 3%) and 6-chloro-3-(2-fluoropyridin-4-yl)-2-(3-methylphenyl)imidazo[1,2-b]pyridazine (Example compound 1, yield 200 mg, 21%).
Melting point 144-146°C (ethyl acetate)
¹H-NMR (DMSO-d₆) δ: 1.34 (3H, t, J=7.0Hz), 2.31 (3H, s), 4.36 (2H, q, J=7.0Hz), 7.03 (1H, s), 7.03-7.05 (1H, m), 7.19-7.21 (1H, m), 7.27 (1H, t, J=7.7Hz), 7.29-7.31 (1H, m), 7.49 (1H, d, J=9.4Hz), 7.52 (1H, s), 8.27 (1H, d, J=5.3Hz), 8.32 (1H, d, J=9.4Hz).

| (Formulation Example 1) | | |
|---|---|---|
| (1) Example compound 28-3 | | 50 mg |
| (2) lactose | | 34 mg |
| (3) cornstarch | | 10.6 mg |
| (4) cornstarch (paste) | | 5 mg |
| (5) magnesium stearate | | 0.4 mg |
| (6) calcium carboxymethylcellulose | | 20 mg |
| | total | 120 mg |

According to a conventional method, the above (1) to (6) are mixed, and the mixture is compressed with a tabletting machine to give tablets.

| (Formulation Example 2) | |
|---|---|
| (1) Example compound 28-3 | 10.0 mg |
| (2) lactose | 60.0 mg |
| (3) cornstarch | 35.0 mg |
| (4) gelatin | 3.0 mg |
| (5) magnesium stearate | 2.0 mg |

A mixture of Example compound 28-3 (10.0 mg), lactose (60.0 mg) and cornstarch (35.0 mg) is granulated by passing through a 1 mm mesh sieve and using 0.03 ml of a 10% aqueous gelatin solution (3.0 mg as gelatin) and, thereafter, dried at 40°C and passed through the sieve again. The granules thus obtained are mixed with 2.0 mg of magnesium stearate and the mixture is compressed. The resulting core tablet is coated with a sugar coating of a suspension of sucrose, titanium dioxide, talc and arabic gum in water. The tablet coated with the coating is polished with beeswax to give a coated tablet.

| (Formulation Example 3) | |
|---|---|
| (1) Example compound 28-3 | 10.0 mg |
| (2) lactose | 70.0 mg |
| (3) cornstarch | 50.0 mg |
| (4) soluble starch | 7.0 mg |
| (5) magnesium stearate | 3.0 mg |

After Example compound 28-3 (10.0 mg) and magnesium stearate (3.0 mg) are granulated with 0.07 ml of an aqueous solution of soluble starch (7.0 mg as soluble starch), the granules are dried and mixed with lactose (70.0 mg) and cornstarch (50.0 mg). The mixture is compressed to give tablets.

### (Experimental Example 1)

The genetic manipulations described below were according to the methods described in the book (Maniatis et al., Molecular Cloning, Cold Spring Harbor Laboratory, 1989) or a method described in the protocol attached to the reagent.
(1) Cloning of human p38 MAP kinase gene and preparation of recombinant Baculovirus
   Cloning of human p38 MAP kinase gene was performed by a PCR method using a primer set P38-U:
   5'-ACCACTCGAGATGGACTACAAGGACGACGATGACAAGTCTCAGGAGAGGCCCACGTTCTACC-3' [SEQ ID NO:1]
      and PAG-L:
   5'-ACCCGGTACCACCAGGTGCTCAGGACTCCATCTCT-3' [SEQ ID NO:2] made by reference to the base sequence of p38 MAP kinase gene reported by Han et al. (Science 265 (5173), 808-811 (1994)) and employing kidney cDNA (Toyobo, QUICK-Clone cDNA) as a template.

   The PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution and 10 µL sterile distilled water were mixed. As the upper mixed solution, 1 µL human cardiac cDNA (1 ng/mL) as a template, 3 µL 10×LA PCR Buffer, 1 µL 2.5 mM dNTP solution, 0.5 µL TaKaRa LA Taq DNA polymerase (Takara Shuzo), and 24.5 µL sterile distilled water were mixed. One AmpliWax PCR Gem 100 (Takara Shuzo) was added to the prepared lower mixed solution to treat at 70°C for 5 minutes and for 5 minutes in an ice and, thereafter, the upper mixed solution was added thereto to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.1 kb DNA fragment containing p38 MAP kinase gene was recovered from the gel and, thereafter, which was inserted into pT7Blue-T vector (Takara Shuzo) to make the plasmid pHP38.
   The 4.8 kb XhoI-KpnI fragment of the plasmid pFASTBAC1 (CIBCOBRL) and the 1.1 kb XhoI-Kpn fragment of the above plasmid pHP38 were ligated to make the plasmid pFBHP38.
   The plasmid pFBHP38 and BAC-TO-BAC Baculovirus Expression System (GIBCOBRL) were used to prepare the recombinant Baculovirus virusstock BAC-HP38.
(2) Cloning of human MKK3 gene and preparation of recombinant Baculovirus
   Cloning of human MKK3 gene was performed by a PCR method using a primer set MKK-U:
   5'-ACAAGAATTCATAACATATGGCTCATCATCATCATCATCATTCCAAGCCACCCGCACCCAA-3' [SEQ ID NO:3]
      and MKK-L:
   5'-TCCCGTCTAGACTATGAGTCTTCTCCCAGGAT-3' [SEQ ID NO:4] made by reference to the base sequence of MKK3 gene reported by Derijard, B. et al., Science 267 (5198), 682-685 (1995) and employing kidney cDNA (Toyobo, QUICK-Clone cDNA) as a template.

   The PCR reaction was performed by a Hot Start method using AmpliWax PCR Gem 100 (Takara Shuzo). As the lower mixed solution, 2 µL 10×LA PCR Buffer, 3 µL 2.5 mM dNTP solution, each 2.5 µL of 12.5 µM primer solution, and 10 µL sterile distilled water were mixed. As the upper mixed solution, 1 µL human kidney cDNA (1 ng/mL), 3 µL 10×LA PCR Buffer, 1 µL 2.5 mM dNTP solution, 0.5 µL TaKaRa LA Taq DNA polymerase (Takara Shuzo) and 24.5 µL sterile distilled water were mixed. One AmpliWax PCR Gem 100 (Takara Shuzo) was added to the prepared lower mixed solution to treat at 70°C for 5 minutes and for 5 minutes in ice and, thereafter, the upper mixed solution was added thereto to prepare a reaction solution for PCR. A tube containing the reaction solution was set at a thermal cycler (Perkin Elmer), which was treated at 95°C for 2 minutes. Further, after repeating 35 times a cycle of 15 seconds at 95°C and 2 minutes at 68°C, treatment was performed at 72°C for 8 minutes. The resulting PCR product was subjected to agarose gel (1%) electrophoresis, 1.0 kb DNA fragment containing MKK3 gene was recovered from the gel and, thereafter, which was inserted into pT7Blue-T vector (Takara Shuzo) to make the plasmid pHMKK3.
   In order to mutate MKK3 into a constitutive active form (from Ser to Glu at 189 position, from Thr to Glu at position 193), a primer set SER-U:
   5'-GGCTACTTGGTGGACGAGGTGGCCAAGGAGATGGATGCCGGCTGC-3' [SEQ ID NO:5]
      and SER-L:
   5'-GCAGCCGGCATCCATCTCCTTGGCCACCTCGTCCACCAAGTAGCC-3' [SEQ ID NO:6]
   was used to introduce mutations by QuickChange Site-Directed Mutagenesis Kit (Stratagene), to obtain pcaMKK3.
   4.8 kb EcoRI-XbaI fragment of the plasmid pFASTBAC1 (CIBCOBRL) and the 1.0 kb EcoRI-XbaI fragment of the above plasmid pcaMKK3 were ligated to make the plasmid pFBcaMKK3.
   The plasmid pFBcaMKK3 and BAC-TO-BAC Baculovirus Expression System (GIBCOBRL) were used to prepare the recombinant Baculovirus virusstock BAC-caMKK3.
(3) Preparation of active form p38 MAP kinase
   The Sf-21 cells were seeded on 100 ml Sf-900II SFM medium (GIBCOBRL) to 1×10⁶ cells/mL and cultured at 27°C for 24 hours. After each 0.2 mL of the virusstock BAC-HP38 of recombinant Baculovirus and BAC-caMKK3 were added, the culturing was further performed for 48 hours. After the cells were separated from the culturing solution by centrifugation (3000 rpm, 10 min), the cells were washed twice with PBS. After the cells were suspended in 10 ml Lysis buffer (25 mM HEPES (pH 7.5), 1% Triton X, 130 mM NaCl, 1 mM EDTA, 1 mM DTT, 25 mM β-glycerophosphate, 20 mM leupeptin, 1 mM APMSF, 1 mM Sodium orthovanadate), the cells were lysed by treating twice with a homogenizer (POLYTRON) at 20000 rpm for 2 minutes. By using Anti-FLAG M2 Affinity Gel (Eastman Chemical), active form p38 MAP kinase was purified from the supernatant obtained by centrifugation (40000 rpm, 45 minutes).
(4) Measurement of the p38 MAP kinase inhibitory activity
   2.5 µL of a test compound dissolved in DMSO was added to 37.5 µL reaction solution (25 mM HEPES (pH 7.5), 10 mM Magnesium Acetate) containing 260 ng active form p38 MAP kinase and 1 µg Myelin Basic Protein, which was maintained at 30°C for 5 minutes. The reaction was initiated by adding 10 µL ATP solution (2.5 µM ATP, 0.1 µCi [g-³²P]ATP). After the reaction was performed at 30°C for 60 minutes, the reaction was stopped by adding 50 µL 20% TCA solution. After the reaction solution was allowed to stand at 0°C for 20 minutes, an acid insoluble fraction was transferred to GF/C filter (Packard Japan) using Cell Harvester (Packard Japan) and washed with 250 mM H₃PO₄. After drying at 45 °C for 60 minutes, 40 µM Microscint 0 (Packard Japan) was added and the radioactivity was measured with a TopCount (Packard Japan). The concentration (IC₅₀ value) necessary for inhibiting uptake of ³²P into an acid insoluble fraction by 50% was calculated with PRISM 2.01 (Graphpad Software).

The results are shown in Table 1.

**Table 1**

| Example compound | IC₅₀ (µM) |
|---|---|
| 1 | 0.11 |
| 2 | 0.19 |
| 4 | 0.16 |
| 5 | 0.0025 |
| 6-10 | 0.0026 |
| 7 | 0.0087 |
| 17 | 0.023 |
| 19 | 0.0084 |
| 20-1 | 0.0084 |
| 20-10 | 0.0097 |
| 20-11 | 0.014 |
| 21-16 | 0.0012 |
| 26 | 0.022 |
| 28-3 | 0.0079 |
| 28-5 | 0.014 |
| 41-1 | 0.0081 |
| 42-3 | 0.0087 |
| 42-6 | 0.0077 |
| 42-23 | 0.0067 |
| 42-27 | 0.0035 |
| 42-35 | 0.0039 |
| 42-36 | 0.0034 |
| 51-11 | 0.0070 |
| 51-14 | 0.0049 |
| 58 | 0.0039 |
| 59 | 0.0045 |

From the results, it is clear that Compound (I) has a superior p38 MAP kinase inhibitory activity.

### (Experimental Example 2)

### Measurement of TNF-_{α} production inhibitory activity

After THP-1 cells which had been cultured in PRMI 1640 medium (manufactured by Life Technologies, Inc.) containing 1% non-activated bovine fetal serum (manufactured by Life Technologies, Inc., U.S.A.) and 10 mM HEPES (pH 7.5) was plated on a 96-well plate to 1×10⁵ cells/well, 1 µL test compound dissolved in DMSO was added to there. After incubation at 37°C for 1 hour in a CO₂ incubator, LPS (Wako Pure Chemicals) was added to the final concentration 5 µg/mL. After cultured at 37°C for 4 hours in a CO₂ incubator, the supernatant was obtained by centrifugation. The concentration of TNF-α in the supernatant was measured with ELISA (R&D System, Quantikine Kit). The concentration (IC₅₀ value) necessary for inhibiting TNF-α production by 50% was calculated by PRIMS 2.01 (Graphpad Software).

The results are shown in Table 2.

**Table 2**

| Example compound | IC₅₀ (µM) |
|---|---|
| 1 | 0.95 |
| 2 | 0.028 |
| 4 | 0.32 |
| 5 | 0.0039 |
| 6-10 | 0.0098 |
| 7 | 0.00010 |
| 17 | 0.12 |
| 19 | 0.018 |
| 20-1 | 0.018 |
| 20-10 | 0.0054 |
| 20-11 | 0.0038 |
| 21-16 | 0.014 |
| 26 | 0.33 |
| 28-3 | 0.045 |
| 28-5 | 0.064 |
| 41-1 | 0.015 |
| 42-3 | 0.0095 |
| 42-6 | 0.0061 |
| 42-23 | 0.010 |
| 42-27 | 0.0015 |
| 42-35 | 0.0025 |
| 42-36 | 0.0071 |
| 51-11 | 0.010 |
| 51-14 | 0.0010 |
| 58 | 0.0022 |
| 59 | 0.0029 |

From the results, it is clear that Compound (I) has a superior TNF-α production inhibitory activity.

### (Experimental Example 3)

### Measurement of MMP-13 production inhibitory activity

Human normal articular chondrocytes (Camblex) were grown in a chondrocyte growth medium (CGM: Camblex), and the cells were suspended in a chondrocyte differentiation medium (CDM: Camblex) and plated on a 96 well plate at 2×10⁴ cells/well (150 µL/well). After culturing for 3 days under 37°C, 5% CO₂ conditions, the culture medium was exchanged to 0.1% BSA added α-modification of Eagle's (α-MEM: Gibco), and the cells were cultured for 4 hr. The compound was added thereto and, after culture for 1 hr, recombinant human IL-1β (rhIL-1β: R&D systems) was added at 0.1 ng/mL. After culture for 24 hr, the culture supernatant was recovered, and the concentration of MMP-13 in the culture supernatant was measured by the ELISA method (Amersham). The results are shown in Table 3.

**Table 3**

| Example compound | IC₅₀ (µM) |
|---|---|
| 1 | 0.60 |
| 2 | 0.011 |
| 4 | 1.52 |
| 5 | 0.011 |
| 6-10 | 0.082 |
| 7 | 0.0051 |
| 17 | 0.22 |
| 19 | 0.073 |
| 20-1 | 0.073 |
| 20-10 | 0.025 |
| 20-11 | 0.040 |
| 21-16 | 0.041 |
| 26 | 0.077 |
| 28-3 | 0.57 |
| 28-5 | 0.064 |
| 41-1 | 0.035 |
| 42-3 | 0.035 |
| 42-6 | 0.011 |
| 42-23 | 0.014 |
| 42-27 | 0.016 |
| 42-35 | 0.0062 |
| 42-36 | 0.011 |
| 51-11 | 0.064 |
| 51-14 | 0.032 |
| 58 | 0.041 |
| 59 | 0.020 |

From the results, it is clear that Compound (I) has a superior MMP-13 production inhibitory activity.

### Industrial Applicability

Since the compounds (I) and (I') of the present invention show superior p38 MAP kinase inhibitory activity, TNF-α inhibitory activity (TNF-α production inhibitory activity, TNF-α action inhibitory activity), MMP-13 production inhibitory activity (e.g., IL-1-induced MMP-13 production inhibitory activity, etc.), and the like, particularly, superior p38 MAP kinase inhibitory activity and MMP-13 production inhibitory activity, they are useful as starting materials for safe pharmaceutical agents based on these actions. In addition, the pharmaceutical agent of the present invention containing compound (I) or (I') shows low toxicity and can be safely administered orally or parenterally.

This application is based on a patent application No. 2004-381947 filed in Japan, the contents of which are incorporated in full herein by this reference. In addition, the references cited herein, including patent reference and non-patent reference, are hereby incorporated in full by reference, to the extent that they have been disclosed herein.

## Claims

1. A compound represented by formula [I] 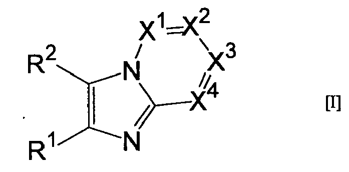 wherein
X¹, X² and X³ are each an optionally substituted CH or a nitrogen atom, and any one of X¹, X² and X³ is a nitrogen atom,
X⁴ is an optionally substituted CH,
R¹ is an optionally substituted phenyl group or an optionally substituted heterocyclic group, and
R² is an optionally substituted pyridin-4-yl group, an optionally substituted pyridine-N-oxide-4-yl group or an optionally substituted pyrimidin-4-yl group,
or a salt thereof.

2. The compound of claim 1, wherein
X¹, X² and X³ are each an optionally substituted CH or a nitrogen atom, and any one of X¹, X² and X³ is a nitrogen atom,
X⁴ is CH,
R¹ is an optionally substituted phenyl group or an optionally substituted heterocyclic group, and
R² is an optionally substituted pyridin-4-yl group or an optionally substituted pyrimidin-4-yl group.

3. The compound of claim 1, wherein R¹ is an optionally substituted phenyl group.

4. The compound of claim 1, wherein
X¹ is a nitrogen atom;
x² is CH optionally substituted by (a) halogen atom, (b) C₁₋₆ alkylthio group, (c) C₁₋₆ alkylsulfonyl group, (d) C₁₋₆ alkoxy group, or (e) a group represented by the formula 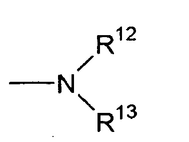 wherein
R¹² and R¹³ are each (i) a hydrogen atom, (ii) a C₁₋₆ alkyl group, (iii) a C₇₋₁₆ aralkyl group, or (iv) a C₃₋₁₀ cycloalkyl group;
X³ is CH optionally substituted by C₁₋₆ alkyl group;
X⁴ is CH optionally substituted by C₁₋₆ alkyl group, C₁₋₆ alkoxy group, C₁₋₆ alkylthio group or C₁₋₆ alkylsulfinyl group;
R¹ is a phenyl group optionally substituted by 1 or 2 substituents selected from halogen atom and C₁₋₆ alkyl group; and
R² is
(I) a pyridin-4-yl group substituted by substituent(s) selected from halogen atom, C₁₋₆ alkoxy group and C₁₋₆ alkyl group,
(II) a pyridin-4-yl group substituted by substituent(s) represented by the formula 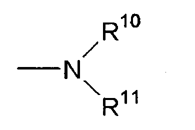 wherein
R¹⁰ and R¹¹ are each
(a) a hydrogen atom,
(b) a C₁₋₆ alkyl group optionally substituted by group(s) selected from
(i) C₁₋₆ alkoxy group,
(ii) amino group optionally having 1 or 2 C₁₋₆ alkyl groups,
(iii) hydroxy group,
(iv) C₃₋₁₀ cycloalkyl group,
(v) 5- to 10-membered heterocyclic group, and
(vi) 5- to 7-membered saturated cyclic amino group,
(c) a C₇₋₁₆ aralkyl group,
(d) a C₃₋₁₀ cycloalkyl group optionally condensed with C₆₋₁₄ aryl ring,
(e) a C₆₋₁₄ aryl group optionally substituted by substituent(s) selected from halogen atom, C₁₋₆ alkyl group and C₁₋₆ alkoxy group, or
(f) the formula -(C=O)-R⁵, -(C=O)-NR⁶R^{6'} or -SO₂-R⁷
wherein
R⁵ is
(i) a C₁₋₆ alkyl group optionally substituted by substituent(s) selected from C₁₋₆ alkyl-carbonyloxy group, hydroxy group, halogen atom, C₁₋₆ alkylthio group and C₁₋₆ alkylsulfonyl group;
(ii) a C₃₋₁₀ cycloalkyl group;
(iii) a C₆₋₁₄ aryl group optionally substituted by substituent(s) selected from C₁₋₆ alkoxy group optionally substituted by halogen atom, C₁₋₆ alkyl group optionally substituted by halogen atom, halogen atom, C₁₋₆ alkylthio group, C₁₋₆ alkylsulfonyl group and di-C₁₋₆ alkylamino group;
(iv) a C₇₋₁₆ aralkyl group optionally substituted by C₁₋₆ alkoxy group;
(v) a C₁₋₆ alkoxy group optionally substituted by halogen atom; or
(vi) a 5- to 10-membered heterocyclic group optionally substituted by substituent(s) selected from halogen atom and C₁₋₆ alkyl group optionally substituted by halogen atom,
R⁶ is
(i) a hydrogen atom;
(ii) a C₆₋₁₄ aryl group optionally substituted by substituent(s) selected from halogen atom, C₁₋₆ alkyl group, C₁₋₆ alkoxy group, C₁₋₆ alkyl-carbonyl group, C₁₋₆ alkylthio group and cyano group;
(iii) a C₁₋₆ alkyl group optionally substituted by substituent(s) selected from hydroxy group, C₁₋₆ alkylsulfonyl group, oxo group, cyano group and halogen atom;
(iv) a C₃₋₁₀ cycloalkyl group optionally substituted by hydroxy group; or
(v) a 5- to 10-membered heterocyclic group optionally substituted by C₁₋₆ alkyl group;
R^{6'} is a hydrogen atom or a C₁₋₆ alkyl group; or
R⁶ and R^{6'} form, together with the nitrogen atom they are bonded to, a 5- to 7-membered saturated cyclic amino group optionally condensed with C₆₋₁₄ aryl ring optionally substituted by hydroxy group; and
R⁷ is a C₆₋₁₄ aryl group; or
R¹⁰ and R¹¹ form, together with the nitrogen atom they are bonded to, a 5- to 7-membered saturated cyclic amino group optionally substituted by oxo group,
(III) a pyridine-N-oxide-4-yl group substituted by C₆₋₁₄ aryl-carbonylamino group optionally substituted by C₁₋₆ alkylsulfonyl group, or
(IV) a pyrimidin-4-yl group substituted by substituent(s) selected from (1) amino group, (2) C₆₋₁₄ aryl-carbonylamino group
wherein the aryl moiety is optionally substituted by C₁₋₆ alkyl group or C₁₋₆ alkoxy group, and (3) 5- to 10-membered heterocyclic group-carbonylamino group.

5. 4-Methyl-N-{4-[2-(3-methylphenyl)imidazo[1,2-b]pyridazin-3-yl]pyridin-2-yl}benzamide,
N-{4-[2-(3-methylphenyl)imidazo[1,2-b]pyridazin-3-yl]pyridin-2-yl}cyclopropanecarboxamide,
4-fluoro-N-{4-[2-(3-methylphenyl)imidazo[1,2-b]pyridazin-3-yl]pyridin-2-yl}benzamide,
N-{4-[2-(4-fluoro-3-methylphenyl)imidazo[1,2-b]pyridazin-3-yl]pyridin-2-yl}-4-methoxybenzamide,
N-ethyl-N'-{4-[2-(4-fluoro-3-methylphenyl)imidazo[1,2-b]pyridazin-3-yl]pyridin-2-yl}urea,
4-fluoro-N-{4-[2-(4-fluoro-3-methylphenyl)imidazo[1,2-b]pyridazin-3-yl]pyridin-2-yl}benzamide, N-{4-[2-(4-fluoro-3-methylphenyl)imidazo[1,2-b]pyridazin-3-yl]pyridin-2-yl}cyclopropanecarboxamide,
N-{4-[2-(4-fluoro-3-methylphenyl)imidazo[1,2-b]pyridazin-3-yl]pyridin-2-yl}-2-(methylthio)acetamide,
N-{4-[2-(4-fluoro-3-methylphenyl)imidazo[1,2-b]pyridazin-3-yl]pyridin-2-yl}isonicotinamide,
N-{4-[2-(4-fluoro-3-methylphenyl)imidazo[1,2-b]pyridazin-3-yl]pyridin-2-yl}-6-methylnicotinamide,
N-{4-[2-(4-fluoro-3-methylphenyl)imidazo[1,2-b]pyridazin-3-yl]pyridin-2-yl}-1,3-benzothiazole-6-carboxamide,
N-{4-[2-(4-fluoro-3-methylphenyl)imidazo[1,2-b]pyridazin-3-yl]pyridin-2-yl}-6-quinolinecarboxamide,
N-{4-[2-(4-fluoro-3-methylphenyl)imidazo[1,2-b]pyridazin-3-yl]pyridin-2-yl}-N'-(2-hydroxyethyl)urea, N-(2-cyanoethyl)-N'-{4-[2-(4-fluoro-3-methylphenyl)imidazo[1,2-b]pyridazin-3-yl]pyridin-2-yl}urea,
N-{4-[2-(4-fluoro-3-methylphenyl)imidazo[1,2-b]pyridazin-3-yl]pyridin-2-yl}-2-methylisonicotinamide,
N-{4-[2-(4-fluoro-3-methylphenyl)imidazo[1,2-b]pyridazin-3-yl]pyridin-2-yl}nicotinamide
or a salt thereof.

6. A prodrug of the compound of claim 1.

7. A pharmaceutical agent comprising a compound represented by formula [I'] 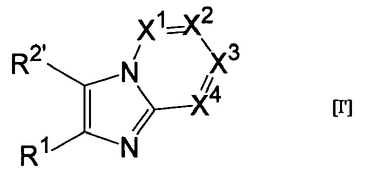 wherein
R^{2'} is an optionally substituted phenyl group or an optionally substituted heterocyclic group, and the other symbols are each as defined claim 1,
or a salt thereof or a prodrug thereof.

8. The pharmaceutical agent of claim 7, which is a p38 MAPK inhibitor and/or MMP-13 production inhibitor.

9. The pharmaceutical agent of claim 7, which is an agent for the prophylaxis or treatment of an inflammatory disease, an autoimmune disease, a debilitating disease, an osteoarticular degenerative disease, a neurodegenerative disease, a vascular disease, a neoplastic disease or an infectious disease.

10. A method for the prophylaxis or treatment of an inflammatory disease, an autoimmune disease, a debilitating disease, an osteoarticular degenerative disease, a neurodegenerative disease, a vascular disease, a neoplastic disease or an infectious disease, which comprises administering an effective amount of a compound represented by formula [I'] 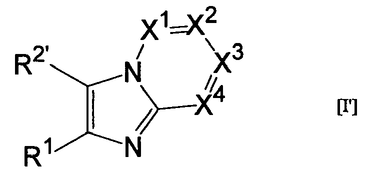 wherein
R^{2'} is an optionally substituted phenyl group or an optionally substituted heterocyclic group, and the other symbols are each as defined claim 1,
or a salt thereof or a prodrug thereof to a mammal.

11. Use of a compound represented by formula [I'] 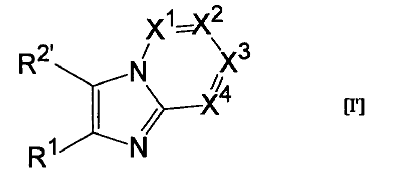 wherein
R^{2'} is an optionally substituted phenyl group or an optionally substituted heterocyclic group, and the other symbols are each as defined claim 1,
or a salt thereof or a prodrug thereof, for the production of an agent for the prophylaxis or treatment of an inflammatory disease, an autoimmune disease, a debilitating disease, an osteoarticular degenerative disease, a neurodegenerative disease, a vascular disease, a neoplastic disease or an infectious disease.

12. A production method of the compound of claim 1 or a salt thereof, which comprises condensing a compound represented by the formula [III] 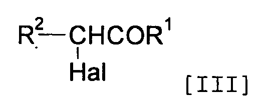 wherein
Hal is a halogen atom,
R¹ is an optionally substituted phenyl group or an optionally substituted heterocyclic group,
R² is an optionally substituted pyridin-4-yl group, an optionally substituted pyridine-N-oxide-4-yl group, or an optionally substituted pyrimidin-4-yl group,
or a salt thereof with a compound represented by formula [IV] 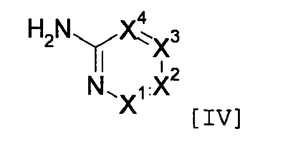 wherein
X¹, X² and X³ are each an optionally substituted CH or a nitrogen atom, and any one of X¹, X² and X³ is a nitrogen atom, and
X⁴ is an optionally substituted CH,
or a salt thereof, and, where desired, performing deprotection, acylation reaction, alkylation reaction, hydrogenation reaction, oxidation reaction, reduction reaction, carbon chain extension reaction and/or substituent exchange reaction.


SEQUENCE LISTING

<110>  TAKEDA PHARMACEUTICAL COMPANY LIMITED

<120>  Condensed imidazole compound and use thereof

<130>  09856

<150>JP 2004-381947
<151>2004-12-28

<160>  6

<170>  PatentIn version 3.3

<210>  1
<211>  62
<212>  DNA
<213>  Artificial

<220>
<223>  primer

<400>  1
accactcgag atggactaca aggacgacga tgacaagtct caggagaggc ccacgttcta      60

cc                                                                      62


<210>  2
<211>  35
<212>  DNA
<213>  Artificial

<220>
<223>  primer

<400>  2
acccggtacc accaggtgct caggactcca tctct                                  35


<210>  3
<211>  61
<212>  DNA
<213>  Artificial

<220>
<223>  primer

<400>  3
acaagaattc ataacatatg gctcatcatc atcatcatca ttccaagcca cccgcaccca      60

a                                                                       61


<210>  4
<211>  32
<212>  DNA
<213>  Artificial

<220>
<223>  primer

<400>  4
tcccgtctag actatgagtc ttctcccagg at                                     32


<210>  5

<211>  45
<212>  DNA
<213>  Artificial

<220>
<223>  primer

<400>  5
ggctacttgg tggacgaggt ggccaaggag atggatgccg gctgc                    45


<210>  6
<211>  45
<212>  DNA
<213>  Artificial

<220>
<223>  primer

<400>  6
gcagccggca tccatctcct tggccacctc gtccaccaag tagcc                    45